# EUROPEAN PATENT APPLICATION

(11) **EP 3 409 662 A1**
(43) Date of publication of application: **05.12.2018**
(21) Application number: 17744401.5
(22) Date of filing: 27.01.2017
(51) Int. Cl.: C07D 239/95, A61K 31/517, A61K 31/519, A61K 31/551, A61P 1/04, A61P 11/00, A61P 11/02, A61P 11/06, A61P 17/00, A61P 17/04, A61P 17/06, A61P 19/02, A61P 25/00, A61P 27/02, A61P 29/00, A61P 37/08, A61P 43/00, C07D 401/12, C07D 403/04, C07D 403/12

(54) **NOVEL COMPOUND AND PHARMACEUTICALLY ACCEPTABLE SALT THEREOF**

(30) Priority: 29.01.2016 JP 2016014957
(71) Applicant: Meiji Seika Pharma Co., Ltd., Tokyo 104-8002 (JP)
(72) Inventor: ARAI Mami, Yokohama-shi Kanagawa 222-8567 (JP); YAMAKAWA Takeru, Yokohama-shi Kanagawa 222-8567 (JP); NAKAJIMA Rie, Yokohama-shi Kanagawa 222-8567 (JP); MURASAKI Kota, Yokohama-shi Kanagawa 222-8567 (JP); WAKIYAMA Yoshinari, Yokohama-shi Kanagawa 222-8567 (JP); FUJIWARA Yuta, Yokohama-shi Kanagawa 222-8567 (JP); ISHIKAWA Makoto, Tokyo 104-8002 (JP); NINOMIYA Tomohisa, Tokyo 104-8002 (JP)
(74) Representative: J A Kemp
(86) International application number: PCT/JP2017/002975
(87) International publication number: WO 2017/131171

(57) **Abstract**

A compound represented by the following general formula (I) or a pharmacologically acceptable salt thereof: 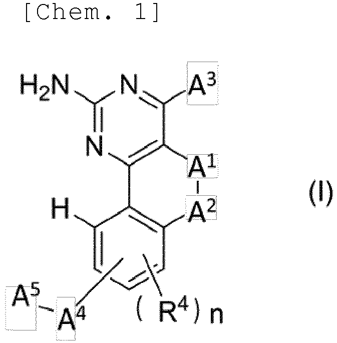 [in the formula (I), A¹ represents a methylene group or the like; A² represents a methylene group or an oxygen atom; A³ represents an azetidinyl group, a pyrrolidinyl group, a piperidinyl group, a piperazinyl group, or a homopiperazinyl group which is optionally substituted with -N(R¹)R², -N(R¹)R³, and/or -R³; A⁴ represents -O-, -S-, -S(O)-, -S(O)₂-, or -N(R¹)-; A⁵ represents an optionally substituted C₃₋₁₀ cycloalkylalkyl group or the like; R⁴ represents a halogen atom or the like; and n represents an integer of 0 or 1].

## Description

### [Technical Field]

The present invention relates to a novel compound and a pharmacologically acceptable salt thereof, and more particularly to a novel compound and a pharmacologically acceptable salt thereof which have a dual modulatory action on histamine H1 receptor and histamine H4 receptor and act as a dual modulator on the histamine H1 receptor and the histamine H4 receptor, as well as pharmaceutical compositions containing the same.

### [Background Art]

Histamine receptors are the receptors for histamine, which is one of bioactive amines, and there are four known subtypes H1 - H4. Among them, the histamine H1 receptor is a G protein-coupled receptor (GPCR) that couples to the Gα_{q/11} protein, and is distributed in the peripheries such as blood vessels and smooth muscles and the central nerves. Histamine, in the peripheries, causes vascular hyperpermeability and smooth muscle contraction, and in the central nerves, exhibits actions related to sedation, memory, and the like, through the histamine H1 receptor (NPL 1). Histamine H1 receptor antagonists, also called antihistamines, have already been used as therapeutic drugs for various allergic diseases, for example, allergic rhinitis, allergic conjunctivitis, and hives, or for insomnia.

The histamine H4 receptor, which is the fourth and latest histamine receptor identified in 2000, is a GPCR that couples to the Gα_{i/o} protein, and is distributed in the peripheral leukocytes, the bone marrow, the spleen, and the like. Moreover, the histamine H4 receptor is present in the mast cells, eosinophils, T-cells, and the like, the migrations of which are caused by the histamine stimulation via the H4 receptor. Based on the above and the like, the histamine H4 receptor is considered to be involved in immunomodulation of inflammations, allergies, and the like (NPL 1). In addition, histamine H4 receptor antagonists have been reported to have efficacy for asthma, pulmonary fibrosis, atopic dermatitis, pruritus, and the like in non-clinical animal models (NPL 2). Accordingly, a histamine H4 receptor antagonist has been expected to be a potential therapeutic drug for various immunoinflammatory diseases, for example, rheumatism, asthma, atopic dermatitis, allergic rhinitis, and the like.

The histamine H4 receptor antagonist JNJ-39758979 has been reported to have efficacy for histamine-induced pruritus (NPL 3) and atopic dermatitis pruritus (NPL 4), in clinical trials.

As described above, since allergic actions or inflammatory actions involving the histamine H1 receptor and the histamine H4 receptor overlap, the combination of antagonists for these two receptors is expected to provide greater therapeutic effects for allergic diseases than the use of either one of them (NPL 1). In fact, in non-clinical animal models, the effect of the combination of a histamine H1 receptor antagonist and a histamine H4 receptor antagonist has been reported, for pruritus (NPL 5), as well as the effect for atopic dermatitis (NPL 6) and for allergic conjunctivitis (NPL 7).

In clinical trials as well, it has been reported that the efficacy of cetirizine, a histamine H1 receptor antagonist, for histamine-induced pruritus, was higher in the group in which the histamine H4 receptor antagonist JNJ-39758979 had been administered in advance (NPL 3). Accordingly, there has been an implication that the effect of the combination of a histamine H1 receptor antagonist and a histamine H4 receptor antagonist for pruritus and inflammations can be greater than the use of an either one of them.

Under such a background, it is conceivable that a single compound having a dual modulatory function for these two receptors, or specifically a dual modulator for the histamine H1 receptor and the histamine H4 receptor would be a new preventive and/or therapeutic drug for allergic diseases and inflammatory diseases. However, regarding such modulators, there have only been reports on quinazoline derivative modulators (NPL 8) and mepyramine derivatives modulators (NPL 9).

In addition, compounds having structures similar to that of a fused tricyclic pyrimidine derivative, which is a compound of the present invention, are described in PTL 1, PTL 2, PTL 3, and the like. However, PTL 1 relates to a dopamine D4 receptor ligand, and this document does not describe the histamine H1 receptor or the histamine H4 receptor. Furthermore, the benzene ring in the skeleton of the fused pyrimidine derivative described in Example is not substituted. In addition, although both PTL 2 and PTL 3 are publications relating to histamine H4 receptor ligands, these documents do not describe a dual modulatory function for the histamine H1 receptor and the histamine H4 receptor.

### [Citation List]

### [Patent Literature]

[PTL 1] International Publication No.WO95/07893
[PTL 2] International Publication No.WO2008/060767
[PTL 3] International Publication No.WO2008/074445

### [Non Patent Literature]

[NPL 1] Robin L. Thurmond et al., Nat.Rev.Drug Discovery, Vol.7, p.41-53, 2008
[NPL 2] Ekaterini Tiligada, Perspectives in H4R Research and Therapeutic Exploitation, a Novel Drug Target For Immunoregulation and Inflammation, DE GRUYTER, p.333-352, 2013
[NPL 3] Robin L. Thurmond et al., J. Pharmacol. Exp. Ther., 350, p.181-187, 2014
[NPL 4] M. Furue et al., J. Dermatol., 41, p.1-11, 2014
[NPL 5] Robin L. Thurmond et al., J. ALLERGY CLIN. IMMUNOL. , Vol.119, No.1, p.176-183, 2007
[NPL 6] Y. Ohsawa and N. Hirasawa, Allergy, 67, p.1014-1022, 2012
[NPL 7] C. Kamei et al., Eur. J. Pharmacol., 608, p.71-75, 2009
[NPL 8] Rob Leurs et al., J. Med. Chem., 51, p.7855-7865, 2008
[NPL 9] Andrea Strasser et al., Bioorg. Med. Chem. Lett., 21, p.6274-6280, 2011

### [Summary of Invention]

### [Technical Problem]

The object of the present invention is to provide a compound that has a dual modulatory function for the histamine H1 receptor and the histamine H4 receptor and is useful in treating diseases attributable to the histamine H1 receptor and/or the histamine H4 receptor, and a pharmacologically acceptable salt thereof, as well as pharmaceutical compositions containing the same.

### [Solution to Problem]

The present inventors conducted diligent studies in order to solve the above-described problems, and consequently found that a fused tricyclic pyrimidine derivative having a specific structure and a pharmacologically acceptable salt thereof have an excellent dual modulatory function for the histamine H1 receptor and the histamine H4 receptor. As a result, the present inventors completed the present invention.

Specifically, the present invention encompasses the following inventions.
[1] A compound represented by the following general formula (I) or a pharmacologically acceptable salt thereof: 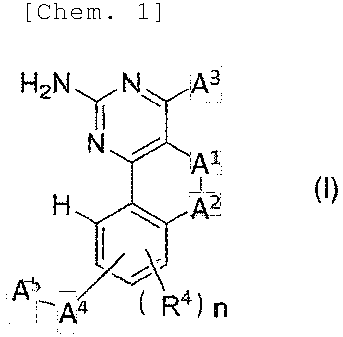 [in the formula (I),
   A¹ represents a single bond, a methylene group, an ethylene group, or a sulfur atom,
   A² represents a methylene group or an oxygen atom,
   A³ represents an azetidinyl group, a pyrrolidinyl group, a piperidinyl group, a piperazinyl group, or a homopiperazinyl group which is optionally substituted with at least one substituent selected from the group consisting of -N(R¹)R², -N(R¹)R³, and -R³,
   R¹ and R² may be the same or different and each represents a hydrogen atom, a C₁₋₆ alkyl group, or an optionally substituted C₆₋₁₀ monocyclic or polycyclic arylalkyl group,
   R³ represents a group represented by the following formula: -D¹-D²-D³ or a group represented by the following formula: -D¹-ON=D⁴ (except where -N(R¹)R³ is -N(R¹)R²),
      D¹ represents an optionally substituted C₁₋₆ alkylene group,
      D² represents a single bond, -O-, -OC(O)-, -S-, -S(O)₂-, -N(R¹)S(O)₂-,-C(O)-, or -C(O)N(R¹)-,
      D³ represents a hydrogen atom, an optionally substituted C₁₋₆ alkyl group, an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₁₋₆ alkyloxyalkyl group, a 3- to 10-membered monocyclic or bicyclic heterocyclyloxyalkyl group, an optionally substituted C₁₋₆ alkylthioalkyl group, or an optionally substituted C₁₋₆ alkylsulfonylalkyl group,
      D⁴ represents an optionally substituted C₃₋₁₀ cycloalkylene group or an optionally substituted 3-to 10-membered monocyclic or bicyclic divalent heterocyclyl group,
   A⁴ represents -O-, -S-, -S(O)-, -S(O)₂-, or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, an optionally substituted C₆₋₁₀ monocyclic or polycyclic arylalkylsulfonyl group, an optionally substituted C₆₋₁₀ monocyclic or polycyclic aryloxyalkylsulfonyl group, or an optionally substituted C₆₋₁₀ monocyclic or polycyclic arylaminocarbonyl group,
   R⁴ represents a halogen atom, a hydroxyl group, a C₁₋₆ alkyloxy group, or a cyano group, and
   n is an integer of 0 or 1,
   except where
   A¹ is a methylene group, an ethylene group, or a sulfur atom,
   A² is a methylene group or an oxygen atom,
   A³ is an azetidinyl group, a pyrrolidinyl group, or a piperidinyl group which is substituted with at least one substituent selected from the group consisting of -N(R¹)R², -N(R¹)R³, and -R³, a piperazinyl group in which one carbon atom on the ring is substituted with a hydroxyalkyl group or a C₁₋₆ alkyloxyalkyl group, or a homopiperazinyl group in which a nitrogen atom on the ring is optionally substituted with a C₁₋₆ alkyl group, a hydroxyalkyl group, a C₁₋₆ alkyloxyalkyl group, or a C₃₋₁₀ cycloalkyloxyalkyl group,
   A⁴ is -N(R¹)-, and
   A⁵ is a C₃₋₁₀ cycloalkylsulfonyl group, a C₆₋₁₀ monocyclic or polycyclic arylsulfonyl group, or a 3- to 10-membered monocyclic or bicyclic heterocyclylsulfonyl group].
[2] The compound or a pharmacologically acceptable salt thereof according to [1], wherein
   in the formula (I),
   A³ represents an azetidinyl group, a pyrrolidinyl group, a piperidinyl group, a piperazinyl group, or a homopiperazinyl group which is optionally substituted with at least one substituent selected from the group consisting of -N(R¹)R³ and -R³,
   R³ represents a group represented by the following formula: -D¹-D²-D³ or a group represented by the following formula: -D¹-ON=D⁴ (except where -N(R¹)R³ is -N(R¹)R²),
      D¹ represents a C₁₋₆ alkylene group,
      D² represents a single bond, -O-, -OC(O)-, -S(O)₂-, -N(R)S(O)₂-, -C(O)-, or -C(O)N(R¹)-,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
   n represents 0.
[3] The compound or a pharmacologically acceptable salt thereof according to [1], wherein
   in the formula (I),
   both A¹ and A² represent methylene groups,
   A³ represents an azetidinyl group, a pyrrolidinyl group, a piperidinyl group, a piperazinyl group, or a homopiperazinyl group which is optionally substituted with at least one substituent selected from the group consisting of -N(R¹)R³ and -R³,
      R³ represents a group represented by the following formula: -D¹-D²-D³ or a group represented by the following formula: -D¹-ON=D⁴ (except where -N(R¹)R³ is -N(R¹)R²),
      D¹ represents a C₁₋₆ alkylene group,
      D² represents a single bond, -O-, -OC(O)-, -S(O)₂-, -N(R)S(O)₂-, -C(O)-, or -C(O)N(R¹)-,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, an optionally substituted C₆₋₁₀ monocyclic or polycyclic arylaminocarbonyl group, and
   n represents 0.
[4] The compound or a pharmacologically acceptable salt thereof according to [1], wherein
   in the formula (I),
   both A¹ and A² represent methylene groups,
   A³ represents an azetidinyl group, a pyrrolidinyl group, a piperidinyl group, a piperazinyl group, or a homopiperazinyl group which is optionally substituted at least one -N(R¹)R²,
      R¹ and R² may be the same or different and each represents a hydrogen atom or a C₁₋₆ alkyl group,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
   n represents 0.
[5] The compound or a pharmacologically acceptable salt thereof according to [1], wherein
   in the formula (I),
   both A¹ and A² represent methylene groups,
   A³ represents a piperazinyl group optionally substituted with at least one -R³,
      R³ represents a group represented by the following formula: -D¹-D²-D³ or a group represented by the following formula: -D¹-ON=D⁴,
      D¹ represents a C₁₋₆ alkylene group,
      D² represents a single bond, -O-, -OC(O)-, -S(O)₂-, -N(R)S(O)₂-, -C(O)-, or -C(O)N(R¹)-,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
   n represents 0.
[6] The compound or a pharmacologically acceptable salt thereof according to [1], wherein
   in the formula (I),
   A¹ represents a single bond, a methylene group, or an ethylene group,
   A³ represents a pyrrolidinyl group, a piperidinyl group, piperazinyl group, or a homopiperazinyl group which is optionally substituted with at least one substituent selected from the group consisting of -N(R¹)R² and -R³,
      R¹ and R² may be the same or different and each represents a hydrogen atom or a C₁₋₆ alkyl group,
      R³ represents a group represented by the following formula: -D¹-D²-D³,
      D¹ represents a C₁₋₆ alkylene group,
      D² represents a single bond or -O-,
      D³ represents a hydrogen atom, an optionally substituted C₁₋₆ alkyl group, an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₁₋₆ alkyloxyalkyl group, a 3- to 10-membered monocyclic or bicyclic heterocyclyloxyalkyl group, or an optionally substituted C₁₋₆ alkylthioalkyl group,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
   n represents 0.
[7] The compound or a pharmacologically acceptable salt thereof according to [1], wherein
   the compound represented by the formula (I) is N-(2-amino-4-(4-methylpiperazin-1-yl)-5,6-dihydrobenzo [h]quinazolin-8-yl)benzenesulfonamide, 9-(benzyloxy)-4-(4-methylpiperazin-1-yl)-6,7-dihydro-5 H-benzo[6,7]cyclohepta[1,2-d]pyrimidin-2-amine, 8-(benzyloxy)-4-(4-methylpiperazin-1-yl)-6H-isothiochr omeno[4,3-d]pyrimidin-2-amine, 2-(4-(2-amino-8-(benzyloxy)-5,6-dihydrobenzo[h]quinazo lin-4-yl)piperazin-1-yl)ethan-1-ol, 2-(4-(2-amino-8-((4-chlorobenzyl)oxy)-5,6-dihydrobenzo [h]quinazolin-4-yl)piperazin-1-yl)ethyl 3-(2-methoxy-2-oxoethyl)azetidine-1-carboxylate, 1-(2-(4-(2-amino-8-((4-chlorobenzyl)oxy)-5,6-dihydrobe nzo[h]quinazolin-4-yl)piperazin-1-yl)ethyl) 3-methyl azetidine-1,3-dicarboxylate,
   methyl 1-(4-(4-(2-amino-8-((4-chlorobenzyl)oxy)-5,6-dihydrobe nzo[h]quinazolin-4-yl)piperazin-1-yl)butanoyl)azetidin e-3-carboxylate, N-(2-amino-4-(4-methylpiperazin-1-yl)-5,6-dihydrobenzo [h]quinazolin-8-yl)-4-chlorobenzenesulfonamide, N-(2-amino-4-(4-methylpiperazin-1-yl)-5,6-dihydrobenzo [h]quinazolin-8-yl)-3-chlorobenzenesulfonamide, N-(2-amino-4-(4-methylpiperazin-1-yl)-5,6-dihydrobenzo [h]quinazolin-8-yl)-5-chlorothiophene-2-sulfonamide, N-(2-amino-4-(4-methylpiperazin-1-yl)-5,6-dihydrobenzo [h]quinazolin-8-yl)-4-chloro-3-fluorobenzenesulfonamid e, N-(2-amino-4-(4-methylpiperazin-1-yl)-5,6-dihydrobenzo [h]quinazolin-8-yl)-4-chloro-2-fluorobenzenesulfonamid e, N-(2-amino-4-(4-methylpiperazin-1-yl)-5,6-dihydrobenzo [h]quinazolin-8-yl)-4-cyanobenzenesulfonamide, N-(2-amino-4-(4-methylpiperazin-1-yl)-5,6-dihydrobenzo [h]quinazolin-8-yl)-2-phenoxyethane-1-sulfonamide, N-(2-amino-4-(4-methylpiperazin-1-yl)-5,6-dihydrobenzo [h]quinazolin-8-yl)-1-phenylmethanesulfonamide, N-(2-amino-4-(4-methylpiperazin-1-yl)-5,6-dihydrobenzo [h]quinazolin-8-yl)-6-chloropyridine-3-sulfonamide, N-(2-amino-4-(4-methylpiperazin-1-yl)-5,6-dihydrobenzo [h]quinazolin-8-yl)-4-fluorobenzenesulfonamide, N-(2-amino-4-(4-methylpiperazin-1-yl)-5,6-dihydrobenzo [h]quinazolin-8-yl)-4-(trifluoromethyl)benzenesulfonam ide, N-(2-amino-4-(4-methylpiperazin-1-yl)-5,6-dihydrobenzo [h]quinazolin-8-yl)-3-phenoxypropane-1-sulfonamide, N-(2-amino-4-(4-methylpiperazin-1-yl)-5,6-dihydrobenzo [h]quinazolin-8-yl)-2-phenylethane-1-sulfonamide, N-(2-amino-4-(4-methylpiperazin-1-yl)-5,6-dihydrobenzo [h]quinazolin-8-yl)-3-phenylpropane-1-sulfonamide, N-(2-amino-4-(4-methylpiperazin-1-yl)-5,6-dihydrobenzo [h]quinazolin-8-yl)-4-methoxybenzenesulfonamide, N-(2-amino-4-(4-methylpiperazin-1-yl)-5,6-dihydrobenzo [h]quinazolin-8-yl)-4-(trifluoromethoxy)benzenesulfona mide,
   methyl 4-(N-(2-amino-4-(4-methylpiperazin-1-yl)-5,6-dihydrobe nzo[h]quinazolin-8-yl)sulfamoyl)benzoate, N-(2-amino-4-(4-methylpiperazin-1-yl)-5,6-dihydrobenzo [h]quinazolin-8-yl)cyclohexanesulfonamide, 1-(2-amino-4-(4-methylpiperazin-1-yl)-5,6-dihydrobenzo [h]quinazolin-8-yl)-3-(4-chlorophenyl)urea, N-(2-amino-4-(4-methylpiperazin-1-yl)-5,6-dihydrobenzo [h]quinazolin-8-yl)-N-(4-chlorobenzyl)-4-nitrobenzenes ulfonamide, N⁸-(4-chlorobenzyl)-4-(4-methylpiperazin-1-yl)-5,6-dih ydrobenzo[h]quinazolin-2,8-diamine, 4-(((2-amino-4-(4-methylpiperazin-1-yl)-5,6-dihydroben zo[h]quinazolin-8-yl)oxy)methyl)-N-methylbenzenesulfon amide, 2-(4-(2-(4-(2-amino-8-((4-chlorobenzyl)oxy)-5,6-dihydr obenzo[h]quinazolin-4-yl)piperazin-1-yl)ethoxy)phenyl) -2-methylpropanoic acid, 3-(3-((2-(4-(2-amino-8-((4-chlorobenzyl)oxy)-5,6-dihyd robenzo[h]quinazolin-4-yl)piperazin-1-yl)ethoxy)methyl )oxetan-3-yl)propanoic acid, 3-((2-(2-(4-(2-amino-8-((4-chlorobenzyl)oxy)-5,6-dihyd robenzo[h]quinazolin-4-yl)piperazin-1-yl)ethoxy)ethoxy )methyl)oxetane-3-carboxylic acid, 3-(2-(2-(4-(2-amino-8-((4-chlorobenzyl)oxy)-5,6-dihydr obenzo[h]quinazolin-4-yl)piperazin-1-yl)ethoxy)ethoxy) oxetane-3-carboxylic acid, 5-((2-(4-(2-amino-8-((4-chlorobenzyl)oxy)-5,6-dihydrob enzo[h]quinazolin-4-yl)piperazin-1-yl)ethoxy)methyl)te trahydrofuran-2-carboxylic acid, 2-(3-(2-(4-(2-amino-8-((4-chlorobenzyl)oxy)-5,6-dihydr obenzo[h]quinazolin-4-yl)piperazin-1-yl)ethoxy)cyclobu tyl)acetic acid, 2-(3-(2-(4-(2-amino-8-((4-chlorobenzyl)oxy)-5,6-dihydr obenzo[h]quinazolin-4-yl)piperazin-1-yl)ethoxy)cyclobu tyl)-2-methylpropanoic acid, 3-(2-(4-(2-amino-8-((4-(trifluoromethoxy)benzyl)oxy)-5 ,6-dihydrobenzo[h]quinazolin-4-yl)piperazin-1-yl)ethox y)bicyclo[3.1.0]hexane-6-carboxylic acid, 2-(3-(2-(4-(2-amino-8-((4-(trifluoromethoxy)benzyl)oxy )-5,6-dihydrobenzo[h]quinazolin-4-yl)piperazin-1-yl)et hoxy)cyclobutyl)-2-methylpropanoic acid, 2-(3-(2-(4-(2-amino-8-((4-(trifluoromethoxy)benzyl)oxy )-5,6-dihydrobenzo[h]quinazolin-4-yl)piperazin-1-yl)et hoxy)cyclobutyl)acetic acid, 4-(2-(4-(2-amino-8-((4-(trifluoromethoxy)benzyl)oxy)-5 ,6-dihydrobenzo[h]quinazolin-4-yl)piperazin-1-yl)ethox y)tetrahydrofuran-2-carboxylic acid, 4-((2-(4-(2-amino-8-((4-(trifluoromethoxy)benzyl)oxy)-5,6-dihydrobenzo[h]quinazolin-4-yl)piperazin-1-yl)etho xy)methyl)oxetane-2-carboxylic acid, N-(2-amino-4-(piperazin-1-yl)-5,6-dihydrobenzo[h]quina zolin-8-yl)-4-(trifluoromethoxy)benzenesulfonamide, ethyl 2-((2-(4-(2-amino-8-((4-chlorobenzyl)oxy)-5,6-dihydrob enzo[h]quinazolin-4-yl)piperazin-1-yl)ethoxy)methyl)th iazole-4-carboxylate,
   methyl 4-((2-(4-(2-amino-8-((4-chlorobenzyl)oxy)-5,6-dihydrob enzo[h]quinazolin-4-yl)piperazin-1-yl)ethoxy)methyl)-3 -fluorobenzoate, 4-((2-(4-(2-amino-8-((4-chlorobenzyl)oxy)-5,6-dihydrob enzo[h]quinazolin-4-yl)piperazin-1-yl)ethoxy)methyl)-3 -fluorobenzene carboxylic acid, 2-((2-(4-(2-amino-8-((4-chlorobenzyl)oxy)-5,6-dihydrob enzo[h]quinazolin-4-yl)piperazin-1-yl)ethoxy)methyl)th iazole-4-carboxylic acid, 4-((2-(4-(2-amino-8-((4-chlorobenzyl)oxy)-5,6-dihydrob enzo[h]quinazolin-4-yl)piperazin-1-yl)ethoxy)methyl)pe ntacyclo[4.2.0.0^{2,5}.0^{3,8}.0^{4,7}]octane-1-carboxylic acid, 6-((2-(4-(2-amino-8-((4-chlorobenzyl)oxy)-5,6-dihydrob enzo[h]quinazolin-4-yl)piperazin-1-yl)ethoxy)methyl)sp iro[3.3]heptane-2-carboxylic acid, 4-((2-(4-(2-amino-8-((4-chlorobenzyl)oxy)-5,6-dihydrob enzo[h]quinazolin-4-yl)piperazin-1-yl)ethoxy)methyl)-1 -methyl-1H-pyrrole-2-carboxylic acid, 4-(2-(4-(2-amino-8-((4-chlorobenzyl)oxy)-5,6-dihydrobe nzo[h]quinazolin-4-yl)piperazin-1-yl)ethoxy)bicyclo[2. 2.2]octane-1-carboxylic acid, 3-(2-(4-(2-amino-8-((4-chlorobenzyl)oxy)-5,6-dihydrobe nzo[h]quinazolin-4-yl)piperazin-1-yl)ethoxy)-4,7,7-tri methylbicyclo[2.2.1]heptane-1-carboxylic acid, 4-(4-(2-((2-(tetrazol-5-yl)propan-2-yl)oxy)ethyl)piper azin-1-yl)-8-((4-chlorobenzyl)oxy)-5,6-dihydrobenzo[h] quinazolin-2-amine, 3-((2-(4-(2-amino-8-((4-chlorobenzyl)oxy)-5,6-dihydrob enzo[h]quinazolin-4-yl)piperazin-1-yl)ethoxy)imino)cyc lobutane-1-carboxylic acid, 3-((2-(4-(2-amino-8-((4-(trifluoromethoxy)benzyl)oxy)-5,6-dihydrobenzo[hlquinazolin-4-yl)piperazin-1-yl)etho xy)methyl)cyclohexane-1-carboxylic acid, 3-((2-(4-(2-amino-8-((4-(trifluoromethoxy)benzyl)oxy)-5,6-dihydrobenzo[hlquinazolin-4-yl)piperazin-1-yl)etho xy)methyl)-N-cyanoazetidine-1-carboximideamide, 4-(4-(2-(azetidin-3-ylmethoxy)ethyl)piperazin-1-yl)-8-((4-(trifluoromethoxy)benzyl)oxy)-5,6-dihydrobenzo[h]q uinazolin-2-amine, 4-(piperazin-1-yl)-8-((4-(trifluoromethoxy)benzyl)oxy) -5,6-dihydrobenzo[h]quinazolin-2-amine, 3-((2-(4-(2-amino-8-((4-(trifluoromethoxy)benzyl)oxy)-5,6-dihydrobenzo[h]quinazolin-4-yl)piperazin-1-yl)etho xy)methyl)azetidine-1-carboxamide, 3-(3-((2-(4-(2-amino-8-((4-(trifluoromethoxy)benzyl)ox y)-5,6-dihydrobenzo[h]quinazolin-4-yl)piperazin-1-yl)e thoxy)methyl)azetidin-1-yl)-4-hydroxy-1,2,5-thiadiazol 1,1-dioxide, 8-(benzyloxy)-4-(piperazin-1-yl)-5,6-dihydrobenzo[h]qu inazolin-2-amine, 2-(4-(2-amino-8-(benzyloxy)-5,6-dihydrobenzo[h]quinazo lin-4-yl)piperazin-1-yl)ethan-1-ol, 2-(2-(2-(4-(2-amino-8-(benzyloxy)-5,6-dihydrobenzo[h]q uinazolin-4-yl)piperazin-1-yl)ethoxy)ethoxy)acetic acid, 4-(4-methylpiperazin-1-yl)-8-(thiophen-3-ylmethoxy)-5, 6-dihydrobenzo[h]quinazolin-2-amine, 8-((4-chlorobenzyl)oxy)-4-(4-methylpiperazin-1-yl)-5,6 -dihydrobenzo[h]quinazolin-2-amine, 4-(4-methylpiperazin-1-yl)-8-((tetrahydro-2H-pyran-4-y 1)methoxy)-5,6-dihydrobenzo[h]quinazolin-2-amine, 8-((4-chlorobenzyl)oxy)-4-(piperazin-1-yl)-5,6-dihydro benzo[h]quinazolin-2-amine, 8-(benzyloxy)-4-(3-(dimethylamino)pyrrolidin-1-yl)-5,6 -dihydrobenzo[h]quinazolin-2-amine, 8-((4-fluorobenzyl)oxy)-4-(4-methylpiperazin-1-yl)-5,6 -dihydrobenzo[h]quinazolin-2-amine, 8-(benzyloxy)-4-(4-(dimethylamino)piperidin-1-yl)-5,6-dihydrobenzo[h]quinazolin-2-amine, 8-(benzyloxy)-4-(4-methylpiperazin-1-yl)benzofuro[3,2-d]pyrimidin-2-amine, 2-(2-(2-(4-(2-amino-8-((4-chlorobenzyl)oxy)-5,6-dihydr obenzo[h]quinazolin-4-yl)piperazin-1-yl)ethoxy)ethoxy) acetic acid, N-(4-(4-(2-amino-8-(benzyloxy)-5,6-dihydrobenzo[h]quin azolin-4-yl)piperazin-1-yl)butyl)ethanesulfonamide, (4-(2-amino-8-(benzyloxy)-5,6-dihydrobenzo[h]quinazoli n-4-yl)-1-methylpiperazin-2-yl)methanol, 8-(benzyloxy)-4-(4-(3-(ethylsulfonyl)propyl)piperazin-1-yl)-5,6-dihydrobenzo[h]quinazolin-2-amine, 2-(2-(2-(4-(2-amino-8-(benzyloxy)-5,6-dihydrobenzo[h]q uinazolin-4-yl)piperazin-1-yl)ethoxy)ethoxy)-N-methyla cetamide, 2-((5-(4-(2-amino-8-(benzyloxy)-5,6-dihydrobenzo[h]qui nazolin-4-yl)piperazin-1-yl)pentyl)oxy)acetic acid, 2-(2-(2-(4-(2-amino-8-(benzyloxy)-5,6-dihydrobenzo[h]q uinazolin-4-yl)piperazin-1-yl)ethoxy)ethoxy)acetamide, 2-(2-(2-(4-(2-amino-8-((4-chlorobenzyl)oxy)-5,6-dihydr obenzo[h]quinazolin-4-yl)piperazin-1-yl)ethoxy)ethoxy) propanoic acid, N-(2-(2-(2-(4-(2-amino-8-(benzyloxy)-5,6-dihydrobenzo[ h]quinazolin-4-yl)piperazin-1-yl)ethoxy)ethoxy)ethyl)m ethanesulfonamide, 8-(benzyloxy)-4-(4-methylpiperazin-1-yl)-5H-chromeno[4 ,3-d]pyrimidin-2-amine, 2-(2-(2-(4-(2-amino-8-((4-chlorobenzyl)oxy)-5,6-dihydr obenzo[h]quinazolin-4-yl)piperazin-1-yl)ethoxy)ethoxy) -2-methylpropanoic acid, 2-(4-(4-(2-amino-8-((4-chlorobenzyl)oxy)-5,6-dihydrobe nzo[h]quinazolin-4-yl)piperazin-1-yl)butoxy)propanoic acid, 2-(2-(2-(4-(2-amino-8-((4-fluorobenzyl)oxy)-5,6-dihydr obenzo[h]quinazolin-4-yl)piperazin-1-yl)ethoxy)ethoxy) acetic acid, 4-(2-(4-(2-amino-8-((4-chlorobenzyl)oxy)-5,6-dihydrobe nzo[h]quinazolin-4-yl)piperazin-1-yl)acetamide)butanoi c acid, 2-(2-(2-(4-(2-amino-8-((4-chlorobenzyl)oxy)-5,6-dihydr obenzo[h]quinazolin-4-yl)piperazin-1-yl)ethoxy)ethoxy) butanoic acid, 2-(2-(2-(4-(2-amino-8-((4-chlorobenzyl)oxy)-5,6-dihydr obenzo[h]quinazolin-4-yl)piperazin-1-yl)ethoxy)ethoxy) -N-methylacetamide, N-(2-(2-(2-(4-(2-amino-8-((4-chlorobenzyl)oxy)-5,6-dih ydrobenzo[h]quinazolin-4-yl)piperazin-1-yl)ethoxy)etho xy)ethyl)methanesulfonamide,
   methyl 2-(2-(2-(4-(2-amino-8-((4-chlorobenzyl)oxy)-5,6-dihydr obenzo[h]quinazolin-4-yl)piperazin-1-yl)ethoxy)ethoxy) acetate, 2-(2-(2-(4-(2-amino-8-((4-chlorobenzyl)oxy)-5,6-dihydr obenzo[h]quinazolin-4-yl)piperazin-1-yl)ethoxy)ethoxy) ethan-1-ol, 2-(2-(2-(4-(2-amino-8-((4-chlorobenzyl)oxy)-5,6-dihydr obenzo[h]quinazolin-4-yl)piperazin-1-yl)ethoxy)ethoxy) ethyl sulfamate, 2-(2-(2-(4-(2-amino-8-((4-chlorobenzyl)oxy)-5,6-dihydr obenzo[h]quinazolin-4-yl)piperazin-1-yl)ethoxy)ethoxy) -N-(methylsulfonyl)acetamide, 2-(2-(2-(2-(4-(2-amino-8-((4-chlorobenzyl)oxy)-5,6-dih ydrobenzo[h]quinazolin-4-yl)piperazin-1-yl)ethoxy)etho xy)ethyl)isoindoline-1,3-dione, 8-((4-bromobenzyl)oxy)-4-(4-methylpiperazin-1-yl)-5,6-dihydrobenzo[h]quinazolin-2-amine, 4-(4-(2-(2-(2-aminoethoxy)ethoxy)ethyl)piperazin-1-yl) -8-((4-chlorobenzyl)oxy)-5,6-dihydrobenzo[h]quinazolin -2-amine,
   methyl 3-((2-(4-(2-amino-8-((4-chlorobenzyl)oxy)-5,6-dihydrob enzo[h]quinazolin-4-yl)piperazin-1-yl)ethoxy)methyl)be nzoate,
   methyl 4-((2-(4-(2-amino-8-((4-chlorobenzyl)oxy)-5,6-dihydrob enzo[h]quinazolin-4-yl)piperazin-1-yl)ethoxy)methyl)be nzoate,
   methyl 5-((2-(4-(2-amino-8-((4-chlorobenzyl)oxy)-5,6-dihydrob enzo[h]quinazolin-4-yl)piperazin-1-yl)ethoxy)methyl)fu ran-2-carboxylate, 5-((2-(4-(2-amino-8-((4-chlorobenzyl)oxy)-5,6-dihydrob enzo[h]quinazolin-4-yl)piperazin-1-yl)ethoxy)methyl)fu ran-2-carboxylic acid, N-(2-amino-4-(4-methylpiperazin-1-yl)-5,6-dihydrobenzo [h]quinazolin-8-yl)-4-(2,2,2-trifluoroethoxy)benzenesu Ifonamide,
   methyl 5-((2-(4-(2-amino-8-((4-(trifluoromethoxy)benzyl)oxy)-5,6-dihydrobenzo[h]quinazolin-4-yl)piperazin-1-yl)etho xy)methyl)furan-2-carboxylate, 3-((2-(4-(2-amino-8-((4-chlorobenzyl)oxy)-5,6-dihydrob enzo[h]quinazolin-4-yl)piperazin-1-yl)ethoxy)methyl)be nzoic acid, 4-((2-(4-(2-amino-8-((4-chlorobenzyl)oxy)-5,6-dihydrob enzo[h]quinazolin-4-yl)piperazin-1-yl)ethoxy)methyl)be nzoic acid, 5-((2-(4-(2-amino-8-((4-(trifluoromethoxy)benzyl)oxy)-5,6-dihydrobenzo[h]quinazolin-4-yl)piperazin-1-yl)etho xy)methyl)furan-2-carboxylic acid,
   methyl 2-(2-(4-(2-amino-8-((4-chlorobenzyl)oxy)-5,6-dihydrobe nzo[h]quinazolin-4-yl)piperazin-1-yl)ethoxy) acetate, 8-((4-chlorobenzyl)oxy)-4-(4-(3-(ethylsulfonyl)propyl) piperazin-1-yl)-5,6-dihydrobenzo[h]quinazolin-2-amine, methyl 3-((2-(4-(2-amino-8-((4-(trifluoromethoxy)benzyl)oxy)-5,6-dihydrobenzo[h]quinazolin-4-yl)piperazin-1-yl)etho xy)methyl)benzoate,
   methyl 6-((2-(4-(2-amino-8-((4-chlorobenzyl)oxy)-5,6-dihydrob enzo[h]quinazolin-4-yl)piperazin-1-yl)ethoxy)methyl)pi colinate, 3-((2-(4-(2-amino-8-((4-(trifluoromethoxy)benzyl)oxy)-5,6-dihydrobenzo[h]quinazolin-4-yl)piperazin-1-yl)etho xy)methyl)benzoic acid, 6-((2-(4-(2-amino-8-((4-chlorobenzyl)oxy)-5,6-dihydrob enzo[h]quinazolin-4-yl)piperazin-1-yl)ethoxy)methyl)pi colinic acid, N-(2-amino-4-(4-methyl-1,4-diazepan-1-yl)-5,6-dihydrob enzo[h]quinazolin-8-yl)-4-(trifluoromethoxy)benzenesul fonamide, 2-(2-(2-(4-(2-amino-8-((4-chlorobenzyl)oxy)-5,6-dihydr obenzo[h]quinazolin-4-yl)piperazin-1-yl)ethoxy)ethoxy) acetonitrile,
   methyl 3-((2-(4-(2-amino-8-((4-chlorobenzyl)oxy)-5,6-dihydrob enzo[h]quinazolin-4-yl)piperazin-1-yl)ethoxy)methyl)-4 -fluorobenzoate,
   methyl 5-((2-(4-(2-amino-8-((4-chlorobenzyl)oxy)-5,6-dihydrob enzo[h]quinazolin-4-yl)piperazin-1-yl)ethoxy)methyl)-2 -fluorobenzoate, 3-((2-(4-(2-amino-8-((4-chlorobenzyl)oxy)-5,6-dihydrob enzo[h]quinazolin-4-yl)piperazin-1-yl)ethoxy)methyl)-4 -fluorobenzoic acid, 5-((2-(4-(2-amino-8-((4-chlorobenzyl)oxy)-5,6-dihydrob enzo[h]quinazolin-4-yl)piperazin-1-yl)ethoxy)methyl)-2 -fluorobenzoic acid, 4-(4-(2-((tetrazol-5-yl)methoxy)ethyl)piperazin-1-yl)-8-((4-chlorobenzyl)oxy)-5,6-dihydrobenzo[h]quinazolin-2-amine, 4-(4-(2-(2-((1H-tetrazol-5-yl)methoxy)ethoxy)ethyl)pip erazin-1-yl)-8-((4-chlorobenzyl)oxy)-5,6-dihydrobenzo[ h]quinazolin-2-amine,
   methyl 4-(2-(4-(2-amino-8-((4-chlorobenzyl)oxy)-5,6-dihydrobe nzo[h]quinazolin-4-yl)piperazin-1-yl)ethoxy)benzoate, methyl 3-(3-(4-(2-amino-8-((4-chlorobenzyl)oxy)-5,6-dihydrobe nzo[h]quinazolin-4-yl)piperazin-1-yl)propoxy)benzoate, 2-(3-(2-(4-(2-amino-8-((4-chlorobenzyl)oxy)-5,6-dihydr obenzo[h]quinazolin-4-yl)piperazin-1-yl)ethoxy)phenyl) acetic acid, 4-(2-(4-(2-amino-8-((4-chlorobenzyl)oxy)-5,6-dihydrobe nzo[h]quinazolin-4-yl)piperazin-1-yl)ethoxy)benzoic acid, 3-(3-(4-(2-amino-8-((4-chlorobenzyl)oxy)-5,6-dihydrobe nzo[h]quinazolin-4-yl)piperazin-1-yl)propoxy)benzoic acid, 1-(4-(4-(2-amino-8-((4-chlorobenzyl)oxy)-5,6-dihydrobe nzo[h]quinazolin-4-yl)piperazin-1-yl)butyl)-1H-pyrazol e-4-carboxylic acid, 1-(2-(2-(4-(2-amino-8-((4-chlorobenzyl)oxy)-5,6-dihydr obenzo[h]quinazolin-4-yl)piperazin-1-yl)ethoxy)ethyl)-1H-pyrazole-4-carboxylic acid, 4-(4-(4-(2-amino-8-((4-chlorobenzyl)oxy)-5,6-dihydrobe nzo[h]quinazolin-4-yl)piperazin-1-yl)butoxy)benzoic acid, 4-(4-(3-((tetrazol-5-yl)methoxy)propyl)piperazin-1-yl) -8-((4-chlorobenzyl)oxy)-5,6-dihydrobenzo[h]quinazolin -2-amine, 4-(4-(4-((tetrazol-5-yl)methoxy)butyl)piperazin-1-yl)-8-((4-chlorobenzyl)oxy)-5,6-dihydrobenzo[h]quinazolin-2-amine, 4-(4-(2-(2-((tetrazol-5-yl)oxy)ethoxy)ethyl)piperazin-1-yl)-8-((4-chlorobenzyl)oxy)-5,6-dihydrobenzo[h]quina zolin-2-amine, 2-((2-(4-(2-amino-8-((4-(trifluoromethoxy)benzyl)oxy)-5,6-dihydrobenzo[h]quinazolin-4-yl)piperazin-1-yl)etho xy)methyl)thiazole-4-carboxylic acid, 2-(2-(2-(4-(2-amino-8-((4-chlorobenzyl)oxy)-5,6-dihydr obenzo[h]quinazolin-4-yl)piperazin-1-yl)ethoxy)phenyl) acetic acid, 2-(4-(2-(4-(2-amino-8-((4-chlorobenzyl)oxy)-5,6-dihydr obenzo[h]quinazolin-4-yl)piperazin-1-yl)ethoxy)phenyl) acetic acid, 1-(2-(2-(4-(2-amino-8-((4-chlorobenzyl)oxy)-5,6-dihydr obenzo[h]quinazolin-4-yl)piperazin-1-yl)ethoxy)ethyl)-1H-pyrazole-3-carboxylic acid, 2-(3-(2-(4-(2-amino-8-((4-chlorobenzyl)oxy)-5,6-dihydr obenzo[h]quinazolin-4-yl)piperazin-1-yl)ethyl)phenoxy) acetic acid, 2-(4-(2-(4-(2-amino-8-((4-chlorobenzyl)oxy)-5,6-dihydr obenzo[h]quinazolin-4-yl)piperazin-1-yl)ethyl)phenoxy) acetic acid, 4-((2-(4-(2-amino-8-((4-chlorobenzyl)oxy)-5,6-dihydrob enzo[h]quinazolin-4-yl)piperazin-1-yl)ethoxy)methyl)cy clohexane-1-carboxylic acid, 7-(4-methylpiperazin-1-yl)-2-(4-phenylbutyl)-2,4,5,3-( 4-(4-(2-amino-8-((4-chlorobenzyl)oxy)-5,6-dihydrobenzo [h]quinazolin-4-yl)piperazin-1-yl)butoxy)cyclobutane-1 -carboxylic acid, 3-(2-(4-(2-amino-8-((4-chlorobenzyl)oxy)-5,6-dihydrobe nzo[h]quinazolin-4-yl)piperazin-1-yl)ethoxy)cyclobutan e-1-carboxylic acid, N-(2-amino-4-(4-methylpiperazin-1-yl)-5,6-dihydrobenzo [h]quinazolin-8-yl)-4,4-difluoropiperidine-1-sulfonami de, 4-(2-(4-(2-amino-8-((4-chlorobenzyl)oxy)-5,6-dihydrobe nzo[h]quinazolin-4-yl)piperazin-1-yl)ethoxy)cyclohexan e-1-carboxylic acid, 4-((2-(4-(2-amino-8-((4-(trifluoromethoxy)benzyl)oxy)-5,6-dihydrobenzo[h]quinazolin-4-yl)piperazin-1-yl)etho xy)methyl)cyclohexane-1-carboxylic acid, 3-(2-(4-(2-amino-8-((4-(trifluoromethoxy)benzyl)oxy)-5 ,6-dihydrobenzo[h]quinazolin-4-yl)piperazin-1-yl)ethox y)cyclobutane-1-carboxylic acid, 4-((2-(4-(2-amino-8-((4-chlorobenzyl)amino)-5,6-dihydr obenzo[h]quinazolin-4-yl)piperazin-1-yl)ethoxy)methyl) cyclohexane-1-carboxylic acid, 4-((2-(4-(2-amino-8-(benzylamino)-5,6-dihydrobenzo[h]q uinazolin-4-yl)piperazin-1-yl)ethoxy)methyl)cyclohexan e-1-carboxylic acid, 4-((2-(4-(2-amino-8-((4-(trifluoromethoxy)benzyl)amino )-5,6-dihydrobenzo[h]quinazolin-4-yl)piperazin-1-yl)et hoxy)methyl)cyclohexane-1-carboxylic acid, 4-((2-(4-(2-amino-8-((4-cyanobenzyl)oxy)-5,6-dihydrobe nzo[h]quinazolin-4-yl)piperazin-1-yl)ethoxy)methyl)cyc lohexane-1-carboxylic acid, 4-((2-(4-(2-amino-8-((4-chlorobenzyl)(methyl)amino)-5, 6-dihydrobenzo[h]quinazolin-4-yl)piperazin-1-yl)ethoxy )methyl)cyclohexane-1-carboxylic acid, 3-(2-(4-(2-amino-8-((2-fluoro-4-(trifluoromethoxy)benz yl)oxy)-5,6-dihydrobenzo[h]quinazolin-4-yl)piperazin-1 -yl)ethoxy)cyclobutane-1-carboxylic acid, 3-(2-(4-(2-amino-8-((3-fluoro-4-(trifluoromethoxy)benz yl)oxy)-5,6-dihydrobenzo[h]quinazolin-4-yl)piperazin-1 -yl)ethoxy)cyclobutane-1-carboxylic acid, 3-(2-(4-(2-amino-8-((2-methoxy-4-(trifluoromethoxy)ben zyl)oxy)-5,6-dihydrobenzo[h]quinazolin-4-yl)piperazin-1-yl)ethoxy)cyclobutane-1-carboxylic acid, 3-(2-(4-(2-amino-8-((2-methyl-4-(trifluoromethoxy)benz yl)oxy)-5,6-dihydrobenzo[h]quinazolin-4-yl)piperazin-1 -yl)ethoxy)cyclobutane-1-carboxylic acid, 3-(2-(4-(2-amino-8-(1-(4-(trifluoromethoxy)phenyl)etho xy)-5,6-dihydrobenzo[h]quinazolin-4-yl)piperazin-1-yl) ethoxy)cyclobutane-1-carboxylic acid, 3-(2-(4-(2-amino-8-((4-cyclopropylbenzyl)oxy)-5,6-dihy drobenzo[h]quinazolin-4-yl)piperazin-1-yl)ethoxy)cyclo butane-1-carboxylic acid, 3-(2-(4-(2-amino-8-((4-(trifluoromethyl)cyclohexyl)met hoxy)-5,6-dihydrobenzo[h]quinazolin-4-yl)piperazin-1-y 1)ethoxy)cyclobutane-1-carboxylic acid, 3-(2-(4-(2-amino-8-((4-((trifluoromethyl)thio)benzyl)o xy)-5,6-dihydrobenzo[h]quinazolin-4-yl)piperazin-1-yl) ethoxy)cyclobutane-1-carboxylic acid, 3-(2-(4-(2-amino-8-((4-(difluoromethoxy)benzyl)oxy)-5, 6-dihydrobenzo[h]quinazolin-4-yl)piperazin-1-yl)ethoxy )cyclobutane-1-carboxylic acid, 3-(2-(4-(2-amino-8-((4-((trifluoromethyl)sulfonyl)benz yl)oxy)-5,6-dihydrobenzo[hlquinazolin-4-yl)piperazin-1 -yl)ethoxy)cyclobutane-1-carboxylic acid, N-(2-amino-4-(4-methylpiperazin-1-yl)-5,6-dihydrobenzo [h]quinazolin-8-yl)-4,4-difluoropiperidine-1-sulfonami de, 4-(2-(4-(2-amino-8-((4-chlorobenzyl)oxy)-5,6-dihydrobe nzo[h]quinazolin-4-yl)piperazin-1-yl)ethoxy)cyclohexan e-1-carboxylic acid,
   ethyl 3-(2-(4-(2-amino-8-((4-chlorobenzyl)oxy)-5,6-dihydrobe nzo[h]quinazolin-4-yl)piperazin-1-yl)ethoxy)-1-methylc yclobutane-1-carboxylate, 3-(2-(4-(2-amino-8-((4-chlorobenzyl)oxy)-5,6-dihydrobe nzo[h]quinazolin-4-yl)piperazin-1-yl)ethoxy)-1-methylc yclobutane-1-carboxylic acid,
   ethyl 4-((2-(4-(2-amino-8-((4-chlorobenzyl)oxy)-5,6-dihydrob enzo[h]quinazolin-4-yl)piperazin-1-yl)ethoxy)methyl)-1 -cyanocyclohexane-1-carboxylate, 4-((2-(4-(2-amino-8-((4-chlorobenzyl)oxy)-5,6-dihydrob enzo[h]quinazolin-4-yl)piperazin-1-yl)ethoxy)methyl)-1 -cyanocyclohexane-1-carboxylic acid,
   ethyl 4-(2-(4-(2-amino-8-((4-chlorobenzyl)oxy)-5,6-dihydrobe nzo[h]quinazolin-4-yl)piperazin-1-yl)ethoxy)-1-methylc yclohexane-1-carboxylate, 4-(2-(4-(2-amino-8-((4-chlorobenzyl)oxy)-5,6-dihydrobe nzo[h]quinazolin-4-yl)piperazin-1-yl)ethoxy)-1-methylc yclohexane-1-carboxylic acid, 4-((2-(4-(2-amino-8-((4-chlorobenzyl)oxy)-5,6-dihydrob enzo[h]quinazolin-4-yl)piperazin-1-yl)ethoxy)methyl)-1 -methylcyclohexane-1-carboxylic acid,
   ethyl 4-((2-(4-(2-amino-8-((4-chlorobenzyl)oxy)-5,6-dihydrob enzo[h]quinazolin-4-yl)piperazin-1-yl)ethoxy)methyl)-4 -hydroxycyclohexane-1-carboxylate, 4-((2-(4-(2-amino-8-((4-chlorobenzyl)oxy)-5,6-dihydrob enzo[h]quinazolin-4-yl)piperazin-1-yl)ethoxy)methyl)-4 -hydroxycyclohexane-1-carboxylic acid, 4-(2-(4-(2-amino-8-((4-chlorobenzyl)oxy)-5,6-dihydrobe nzo[h]quinazolin-4-yl)piperazin-1-yl)ethoxy)-4-(hydrox ymethyl)cyclohexane-1-carboxylic acid, 4-(2-(4-(2-amino-8-((4-chlorobenzyl)oxy)-5,6-dihydrobe nzo[h]quinazolin-4-yl)piperazin-1-yl)ethoxy)-1-methylc yclohexane-1-carboxamide, 3-((2-(4-(2-amino-8-((4-chlorobenzyl)oxy)-5,6-dihydrob enzo[h]quinazolin-4-yl)piperazin-1-yl)ethoxy)methyl)cy clobutane-1-carboxylic acid, 3-(2-(4-(2-amino-8-((4-(trifluoromethoxy)benzyl)oxy)-5 ,6-dihydrobenzo[h]quinazolin-4-yl)piperazin-1-yl)ethox y)-1-methylcyclobutane-1-carboxylic acid, 3-(2-(4-(2-amino-8-((4-(trifluoromethoxy)benzyl)oxy)-5 ,6-dihydrobenzo[h]quinazolin-4-yl)piperazin-1-yl)ethox y)cyclobutane-1-carboxamide, 4-((2-(4-(2-amino-8-((4-(trifluoromethoxy)benzyl)oxy)-5,6-dihydrobenzo[h]quinazolin-4-yl)piperazin-1-yl)etho xy)methyl)-1-cyanocyclohexane-1-carboxylic acid, 4-((2-(4-(2-amino-8-((4-(trifluoromethoxy)benzyl)oxy)-5,6-dihydrobenzo[h]quinazolin-4-yl)piperazin-1-yl)etho xy)methyl)-1-cyanocyclohexane-1-carboxamide, 3-(2-(4-(2-amino-8-((4-chlorobenzyl)oxy)-5,6-dihydrobe nzo[h]quinazolin-4-yl)piperazin-1-yl)ethoxy)isoxazole-5-carboxylic acid,
   methyl 3-((2-(4-(2-amino-8-((4-chlorobenzyl)oxy)-5,6-dihydrob enzo[h]quinazolin-4-yl)piperazin-1-yl)ethoxy)methyl)cy clopentane-1-carboxylate, 3-((2-(4-(2-amino-8-((4-chlorobenzyl)oxy)-5,6-dihydrob enzo[h]quinazolin-4-yl)piperazin-1-yl)ethoxy)methyl)cy clopentane-1-carboxylic acid, 2-(2-(4-(2-amino-8-((4-chlorobenzyl)oxy)-5,6-dihydrobe nzo[h]quinazolin-4-yl)piperazin-1-yl)ethoxy)cyclopropa ne-1-carboxylic acid, 3-(2-(4-(2-amino-8-((4-chlorobenzyl)oxy)-5,6-dihydrobe nzo[h]quinazolin-4-yl)piperazin-1-yl)ethoxy)cyclopenta ne-1-carboxylic acid,
   methyl 3-((2-(4-(2-amino-8-((4-(trifluoromethoxy)benzyl)oxy)-5,6-dihydrobenzo[h]quinazolin-4-yl)piperazin-1-yl)etho xy)methyl)cyclopentane-1-carboxylate, 3-((2-(4-(2-amino-8-((4-(trifluoromethoxy)benzyl)oxy)-5,6-dihydrobenzo[h]quinazolin-4-yl)piperazin-1-yl)etho xy)methyl)cyclopentane-1-carboxylic acid,
   ethyl 2-((2-(4-(2-amino-8-((4-chlorobenzyl)oxy)-5,6-dihydrob enzo[h]quinazolin-4-yl)piperazin-1-yl)ethoxy)methyl)cy clopropane-1-carboxylate,
   ethyl 3-(2-(4-(2-amino-8-((4-chlorobenzyl)oxy)-5,6-dihydrobe nzo[h]quinazolin-4-yl)piperazin-1-yl)ethoxy)cyclopenta ne-1-carboxylate, 2-((2-(4-(2-amino-8-((4-chlorobenzyl)oxy)-5,6-dihydrob enzo[h]quinazolin-4-yl)piperazin-1-yl)ethoxy)methyl)cy clopropane-1-carboxylic acid,
   ethyl 2-((2-(4-(2-amino-8-((4-(trifluoromethoxy)benzyl)oxy)-5,6-dihydrobenzo[hlquinazolin-4-yl)piperazin-1-yl)etho xy)methyl)cyclopropane-1-carboxylate, 2-((2-(4-(2-amino-8-((4-(trifluoromethoxy)benzyl)oxy)-5,6-dihydrobenzo[h]quinazolin-4-yl)piperazin-1-yl)etho xy)methyl)cyclopropane-1-carboxylic acid, 4-((2-(4-(2-amino-8-((4-chlorobenzyl)oxy)-5,6-dihydrob enzo[h]quinazolin-4-yl)piperazin-1-yl)ethoxy)methyl)bi cyclo[2.2.2]octane-1-carboxylic acid, 4-(4-(2-((3-(benzyloxy)isoxazol-5-yl)methoxy)ethyl)pip erazin-1-yl)-8-((4-chlorobenzyl)oxy)-5,6-dihydrobenzo[ h]quinazolin-2-amine, 3-(2-(4-(2-amino-8-((4-(trifluoromethoxy)benzyl)oxy)-5 ,6-dihydrobenzo[h]quinazolin-4-yl)piperazin-1-yl)ethox y)-N-hydroxycyclobutane-1-carboxamide, 6-(benzyloxy)-4-(3-(methylamino)pyrrolidin-1-yl)-5H-in deno[1,2-d]pyrimidin-2-amine, 7-(benzyloxy)-4-(4-methylpiperazin-1-yl)-5H-indeno[1,2 -d]pyrimidin-2-amine, 7-(benzyloxy)-4-(3-(methylamino)pyrrolidin-1-yl)-5H-in deno[1,2-d]pyrimidin-2-amine, 8-(benzyloxy)-4-(4-methylpiperazin-1-yl)-5H-indeno[1,2 -d]pyrimidin-2-amine, 8-(benzyloxy)-4-(3-(methylamino)pyrrolidin-1-yl)-5H-in deno[1,2-d]pyrimidin-2-amine, 8-(benzyloxy)-4-(4-methylpiperazin-1-yl)-5,6-dihydrobe nzo[h]quinazolin-2-amine, 8-(benzyloxy)-4-(3-(methylamino)pyrrolidin-1-yl)-5,6-d ihydrobenzo[h]quinazolin-2-amine, 9-(benzyloxy)-4-(4-methylpiperazin-1-yl)-5,6-dihydrobe nzo[h]quinazolin-2-amine, 9-(benzyloxy)-4-(3-(methylamino)pyrrolidin-1-yl)-5,6-d ihydrobenzo[h]quinazolin-2-amine, 10-(benzyloxy)-4-(4-methylpiperazin-1-yl)-6,7-dihydro-5H-benzo[6,7]cyclohepta[1,2-d]pyrimidin-2-amine, 10-(benzyloxy)-4-(3-(methylamino)pyrrolidin-1-yl)-6,7-dihydro-5H-benzo[6,7]cyclohepta[1,2-d]pyrimidin-2-amin e, 10-(benzyloxy)-4-(4-methylpiperazin-1-yl)-5,6-dihydrob enzo[2,3]oxepino[4,5-d]pyrimidin-2-amine, 10-(benzyloxy)-4-(3-(methylamino)pyrrolidin-1-yl)-5,6-dihydrobenzo[2,3]oxepino[4,5-d]pyrimidin-2-amine, 10-(benzyloxy)-4-(piperazin-1-yl)-5,6-dihydrobenzo[2,3 ]oxepino[4,5-d]pyrimidin-2-amine, 9-(benzyloxy)-4-(4-methylpiperazin-1-yl)-5,6-dihydrobe nzo[2,3]oxepino[4,5-d]pyrimidin-2-amine, 9-(benzyloxy)-4-(3-(methylamino)pyrrolidin-1-yl)-5,6-d ihydrobenzo[2,3]oxepino[4,5-d]pyrimidin-2-amine, 9-(benzyloxy)-4-(piperazin-1-yl)-5,6-dihydrobenzo[2,3] oxepino[4,5-d]pyrimidin-2-amine, 4-(4-methylpiperazin-1-yl)-8-((4-(trifluoromethoxy)ben zyl)oxy)-5,6-dihydrobenzo[h]quinazolin-2-amine, 2-(2-(4-(2-amino-8-((4-chlorobenzyl)oxy)-5,6-dihydrobe nzo[h]quinazolin-4-yl)piperazin-1-yl)ethoxy)ethan-1-ol , 2-(2-(2-(4-(2-amino-8-((4-(trifluoromethoxy)benzyl)oxy )-5,6-dihydrobenzo[h]quinazolin-4-yl)piperazin-1-yl)et hoxy)ethoxy)acetic acid, 2-((2-(2-(4-(2-amino-8-((4-(trifluoromethoxy)benzyl)ox y)-5,6-dihydrobenzo[h]quinazolin-4-yl)piperazin-1-yl)e thoxy)ethyl)thio)acetic acid, 5-(2-(4-(2-amino-8-((4-(trifluoromethoxy)benzyl)oxy)-5 ,6-dihydrobenzo[h]quinazolin-4-yl)piperazin-1-yl)ethox y)tetrahydro-2H-pyran-2-carboxylic acid, 3-(2-(4-(2-amino-8-((4-(trifluoromethoxy)benzyl)oxy)-5 ,6-dihydrobenzo[h]quinazolin-4-yl)piperazin-1-yl)ethox y)-6-fluorobicyclo[3.1.0]hexane-6-carboxylic acid, 2-(2-(4-(2-amino-8-((4-(trifluoromethoxy)benzyl)oxy)-5 ,6-dihydrobenzo[h]quinazolin-4-yl)piperazin-1-yl)ethox y)bicyclo[3.1.0]hexane-6-carboxylic acid, 5-((2-(4-(2-amino-8-((4-(trifluoromethoxy)benzyl)oxy)-5,6-dihydrobenzo[h]quinazolin-4-yl)piperazin-1-yl)etho xy)methyl)thiophene-2-carboxylic acid, or 4-(2-(4-(2-amino-8-((4-(trifluoromethoxy)benzyl)oxy)-5 ,6-dihydrobenzo[h]quinazolin-4-yl)piperazin-1-yl)ethox y)thiophene-2-carboxylic acid.
[8] A pharmaceutical composition comprising at least one selected from the group consisting of the compound and a pharmacologically acceptable salt thereof according to any one of [1] to [7].
[9] The pharmaceutical composition according to [8], for dually modulating a histamine H1 receptor and a histamine H4 receptor.
[10] The pharmaceutical composition according to [8], for treating a disease attributable to a histamine H1 receptor and/or a histamine H4 receptor.
[11] A method for dually modulating a histamine H1 receptor and a histamine H4 receptor by using at least one selected from the group consisting of the compound and a pharmacologically acceptable salt thereof according to any one of [1] to [7].
[12] A method for treating a disease attributable to a histamine H1 receptor and/or a histamine H4 receptor, the method comprising:
   administering at least one selected from the group consisting of the compound and a pharmacologically acceptable salt thereof according to any one of [1] to [7] and the pharmaceutical composition according to any one of [8] to [10] to a patient.
[13] A use of the compound or a pharmacologically acceptable salt thereof according to any one of [1] to [7], for dually modulating a histamine H1 receptor and a histamine H4 receptor.
[14] A use of the compound or a pharmacologically acceptable salt thereof according to any one of [1] to [7], for treating a disease attributable to a histamine H1 receptor and/or a histamine H4 receptor.
[15] A use of the compound or a pharmacologically acceptable salt thereof according to any one of [1] to [7], for producing a dual modulator for a histamine H1 receptor and a histamine H4 receptor.
[16] A use of the compound or a pharmacologically acceptable salt thereof according to any one of [1] to [7], for producing a therapeutic agent for a disease attributable to a histamine H1 receptor and/or a histamine H4 receptor.

### [Advantageous Effects of Invention]

According to the present invention, a compound with an excellent dual modulatory function for the histamine H1 receptor and the histamine H4 receptor, and a pharmacologically acceptable salt thereof, can be provided.

The compound and a pharmacologically acceptable salt thereof as well as the pharmaceutical compositions containing the same, of the present invention, are useful in treating and/or preventing various allergies and inflammatory diseases involving the histamine H1 and/or H4 receptors. Diseases that involve the histamine H1 and/or H4 receptor include, for example, skin diseases such as hives, pruritus, insect bites, atopic dermatitis, allergic dermatitis, contact dermatitis, and psoriasis vulgaris, respiratory diseases such as allergic rhinitis, nonallergic rhinitis, bronchial asthma, and pulmonary fibrosis, eye diseases such as allergic conjunctivitis and atopic conjunctivitis, nervous system diseases such as dizziness and vestibular disorder, inflammatory diseases such as arthritis, rheumatism, and Crohn's disease, and the like, but are not limited to these.

### [Description of Embodiments]

Hereinafter, the present invention is described in detail with reference to preferred Embodiments thereof. Note that in the following description, the same or corresponding elements are denoted with the same signs, and repetitive descriptions are omitted.

In a compound or a pharmacologically acceptable salt thereof of the present invention, the above-described compound is represented by the following general formula (I):

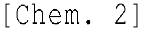

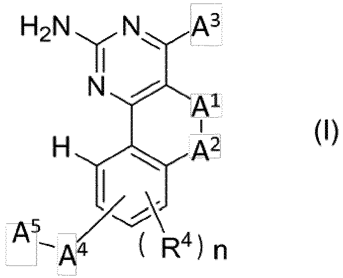

In the general formula (I), A¹ represents a single bond, a methylene group, an ethylene group, or a sulfur atom; A² represents a methylene group or an oxygen atom; and A³ represents an azetidinyl group, a pyrrolidinyl group, a piperidinyl group, a piperazinyl group, or a homopiperazinyl group which is optionally substituted with at least one substituent selected from the group consisting of -N(R¹)R², -N(R¹)R³, and -R³. Here, R¹ and R² may be the same or different and each represents a hydrogen atom, a C₁₋₆ alkyl group, or an optionally substituted C₆₋₁₀ monocyclic or polycyclic arylalkyl group.

In addition, R³ represents -an optionally substituted C₁₋₆ alkylene group-D⁵-D⁶ [D⁵ represents a single bond, -O-, -OC(O)-, -S-, -S(O)₂-, -N(R¹)S(O)₂-, -O-N=, -C(O)-, or -C(O)N(R¹)-; D⁶ represents a hydrogen atom, an optionally substituted C₁₋₆ alkyl group, an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₁₋₆ alkyloxyalkyl group, a 3- to 10-membered monocyclic or bicyclic heterocyclyloxyalkyl group, an optionally substituted C₁₋₆ alkylthioalkyl group, or an optionally substituted C₁₋₆ alkylsulfonylalkyl group], in other words, R³ represents a group represented by the following formula: -D¹-D²-D³ or a group represented by the following formula: -D¹-ON=D⁴ (except where -N(R¹)R³ is -N(R¹)R²), where D¹ represents an optionally substituted C₁₋₆ alkylene group; D² represents a single bond, -O-, -OC(O)-, -S-, -S(O)₂-, -N(R¹)S(O)₂-, -C(O)-, or -C(O)N(R¹)-; and D³ represents a hydrogen atom, an optionally substituted C₁₋₆ alkyl group, an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₁₋₆ alkyloxyalkyl group, a 3- to 10-membered monocyclic or bicyclic heterocyclyloxyalkyl group, an optionally substituted C₁₋₆ alkylthioalkyl group, or an optionally substituted C₁₋₆ alkylsulfonylalkyl group. Moreover, D⁴ represents an optionally substituted C₃₋₁₀ cycloalkylene group or an optionally substituted 3- to 10-membered monocyclic or bicyclic divalent heterocyclyl group.

In addition, in the general formula (I), A⁴ represents -O-, -S-, -S(O)-, -S(O)₂-, or -N(R¹)-; A⁵ represents an optionally substituted C₃₋₁₀ cycloalkylalkyl group, an optionally substituted C₆₋₁₀ monocyclic or polycyclic arylalkyl group, an optionally substituted 3-to 10-membered monocyclic or bicyclic heterocyclylalkyl group, an optionally substituted C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, an optionally substituted C₆₋₁₀ monocyclic or polycyclic arylalkylsulfonyl group, an optionally substituted C₆₋₁₀ monocyclic or polycyclic aryloxyalkylsulfonyl group, or an optionally substituted C₆₋₁₀ monocyclic or polycyclic arylaminocarbonyl group; and R⁴ represents a halogen atom, a hydroxyl group, a C₁₋₆ alkyloxy group, or a cyano group, and n represents an integer of 0 or 1.

Note that excluded from the compounds represented by the general formula (I), is a compound in which A¹ is a methylene group, an ethylene group, or a sulfur atom; A² is a methylene group or an oxygen atom; A³ is an azetidinyl group, a pyrrolidinyl group, or a piperidinyl group which is substituted with at least one substituent selected from the group consisting of -N(R¹)R², -N(R¹)R³, and -R³, a piperazinyl group in which one carbon atom on the ring is substituted with a hydroxyalkyl group or a C₁₋₆ alkyloxyalkyl group, or a homopiperazinyl group in which a nitrogen atom on the ring is optionally substituted with a C₁₋₆ alkyl group, a hydroxyalkyl group, a C₁₋₆ alkyloxyalkyl group, or a C₃₋₁₀ cycloalkyloxyalkyl group; and A⁴ is -N (R¹)- and A⁵ is a C₃₋₁₀ cycloalkylsulfonyl group, a C₆₋₁₀ monocyclic or polycyclic arylsulfonyl group, or a 3- to 10-membered monocyclic or bicyclic heterocyclylsulfonyl group.

In the present invention, "-" represents a single bond and "=" represents a double bond in each substituent. In addition, in the present invention, when a substituent is represented by a formula corresponding to the following formula: -a(b)_{c}- or -a(b)_{c}d- ["a", "b", and "d" are either an atom or a substituent, and "c" is 1 or 2], this indicates "c" piece(s) of the "b"('s) bind to the "a" only.

In the compound represented by the general formula (I), the halogen atom means a fluorine atom, a chlorine atom, a bromine atom, or an iodine atom, and is preferably a fluorine atom, a chlorine atom, or a bromine atom.

In the compound represented by the general formula (I), the "C₁₋₆ alkyl group" means a linear or branched alkyl group having 1 to 6 carbon atoms. The C₁₋₆ alkyl group includes, for example, a methyl group, an ethyl group, a n-propyl group, an isopropyl group, a n-butyl group, an isobutyl group, a tert-butyl group, a n-pentyl group, an isoamyl group, a n-hexyl group, and the like, and is preferably, a methyl group, an ethyl group, a n-propyl group, an isopropyl group, a n-butyl group, or a n-pentyl group.

In the compound represented by the general formula (I), the "C₁₋₆ alkylene group" means a divalent group obtained by removing one hydrogen atom from the above-described C₁₋₆ alkyl group. The C₁₋₆ alkylene group includes, for example, a methylene group, an ethylene group, a propylene group, a trimethylene group, a tetramethylene group, a pentamethylene group, and the like, and is preferably a methylene group, an ethylene group, a trimethylene group, or a tetramethylene group.

The substituent in the "optionally substituted C₁₋₆ alkyl group" and the "optionally substituted C₁₋₆ alkylene group" includes one or more of a halogen atom, an amino group, a cyano group, a hydroxyl group, a carboxy group, a sulfo group, an alkyloxycarbonyl group (preferably having 1 to 6 carbon atoms), -C(O)N(R¹)R², -S(O)₂N(R¹)R², an alkyloxysulfonyl group (preferably having 1 to 6 carbon atoms), and the like.

The "optionally substituted C₁₋₆ alkyl group" and the "optionally substituted C₁₋₆ alkylene group" are preferably a C₁₋₆ alkyl group unsubstituted or substituted with a hydroxyl group, a carboxy group, or an alkyloxycarbonyl group (preferably having 1 to 6 carbon atoms) and a C₁₋₆ alkylene group unsubstituted or substituted with a halogen atom or a hydroxyl group, respectively.

In the compound represented by the general formula (I), the "C₃₋₁₀ cycloalkyl group" means a cyclic alkyl group having 3 to 10 carbon atoms, and may have a bonding portion at any position if possible. The C₃₋₁₀ cycloalkyl group includes, for example, a cyclopropyl group, a cyclobutyl group, a cyclopentyl group, a cyclohexyl group, a cycloheptyl group, and the like, and is preferably a cyclopropyl group or a cyclohexyl group. In addition, this cycloalkyl group may be fused or crosslinked, or may form a spiro ring. Examples of the fused cycloalkyl group include, for example, bicyclo[3.1.0]hexane, bicyclo[3.2.0]heptane, bicyclo[4.1.0]heptane, octahydropentalene, bicyclo[4.2.0]octane, octahydro-1H-indane, decahydronaphthalene, and pentacyclo[4.2.0.0^{2,5}.0^{3,8}.0^{4,7}]octane (cubane). Examples of the crosslinked cycloalkyl group include, for example, bicyclo[1.1.1]pentane, bicyclo[2.1.1]hexane, bicyclo[2.2.1]heptane, and bicyclo[2.2.2]octane. Examples of the spirocycloalkyl group include, for example, spiro[3.3]heptane, spiro[3.4]octane, spiro[3.5]nonane, spiro[4.4]nonane, and the like, and among these, bicyclo[3.1.0]hexane, pentacyclo[4.2.0.0^{2,5}.0^{3,8}.0^{4,7}]octane (cubane), bicyclo[1.1.1]pentane, bicyclo[2.1.1]hexane, bicyclo[2.2.1]heptane, bicyclo[2.2.2]octane, and spiro[3.3]heptane are preferable.

In the compound represented by the general formula (I), the "C₃₋₁₀ cycloalkylene group" means a divalent group obtained by removing one hydrogen atom from the above-described C₃₋₁₀ cycloalkyl group.

The substituent in the "optionally substituted C₃₋₁₀ cycloalkyl group" and the "optionally substituted C₃₋₁₀ cycloalkylene group" includes one or more of a halogen atom, an amino group, a cyano group, a hydroxyl group, an oxo group, an alkyl group (preferably having 1 to 6 carbon atoms), an alkyloxy group (preferably having 1 to 6 carbon atoms), a carboxy group, an alkyloxycarbonyl group (preferably having 1 to 6 carbon atoms), -C(O)N(R¹)R², -C(O)N(R¹)OH, -N(R¹)C(O)N(R¹)R², -N(R¹)C(=NH)S(O)₂N(R¹)R², and the like. Moreover, the alkyl group and the alkyloxy group substituting these cycloalkyl groups may be further substituted with one or more of a halogen atom, a hydroxyl group, a carboxy group, and a sulfo group.

The "optionally substituted C₃₋₁₀ cycloalkyl group" and the "optionally substituted C₃₋₁₀ cycloalkylene group" are preferably a C₃₋₁₀ cycloalkyl group unsubstituted or substituted with a halogen atom, an amino group, a cyano group, a hydroxyl group, an oxo group, an alkyl group (preferably having 1 to 6 carbon atoms), an alkyl group substituted with one or more hydroxyl groups (preferably having 1 to 6 carbon atoms), an alkyl group substituted with one or more carboxy groups (preferably having 1 to 6 carbon atoms), an alkyl group substituted with one or more halogen atoms (preferably having 1 to 6 carbon atoms), a carboxy group, an alkyloxycarbonyl group (preferably having 1 to 6 carbon atoms), -C(O)N(R¹)R², or -C(O)N(R¹)OH, and a C₃₋₁₀ cycloalkylene group unsubstituted or substituted with a halogen atom, an oxo group, or a carboxy group, respectively.

In the compound represented by the general formula (I), the "C₆₋₁₀ monocyclic or polycyclic aryl group" means a monocyclic aromatic hydrocarbyl group having 6 to 10 carbon atoms or a polycyclic aromatic hydrocarbyl group having 6 to 10 carbon atoms. These aryl groups include, for example, a phenyl group, a naphthyl group, and the like, and a phenyl group is preferable.

The substituent in the "optionally substituted C₆₋₁₀ monocyclic or polycyclic aryl group" includes one or more of a halogen atom, a cyano group, a hydroxyl group, a nitro group, an alkyl group, a cycloalkyl group, a cycloalkylalkyl group, an alkyloxy group, a cycloalkyloxy group, an alkyloxyalkyl group, a carboxy group, a sulfo group, an alkyloxycarbonyl group, -C(O)N(R¹)R², -S(O)₂N(R¹)R², an alkyloxysulfonyl group, an alkylsulfonyl group, an alkylthio group, a pentafluorosulfanyl group, and the like. In the alkyl group, the cycloalkyl group, the cycloalkylalkyl group, the alkyloxy group, the cycloalkyloxy group, the alkyloxyalkyl group, and the alkyloxysulfonyl group substituting these aryl groups, the number of carbon atoms in the alkyl group is preferably 1 to 6, and the alkyl group may be further substituted with one or more of a halogen atom, a hydroxyl group, a cyano group, a carboxy group, and a sulfo group.

The "optionally substituted C₆₋₁₀ monocyclic or polycyclic aryl group" is preferably a C₆₋₁₀ monocyclic or polycyclic aryl group unsubstituted or substituted with a halogen atom, an alkyloxy group, a carboxy group, or an alkyloxycarbonyl group.

In the compound represented by the general formula (I), the "3- to 10-membered monocyclic or bicyclic heterocyclyl group" means a 3- to 10-membered monocyclic heterocyclyl group or a 3- to 10-membered bicyclic heterocyclyl group that contains 1 to 4 hetero atoms selected from an oxygen atom, a nitrogen atom, and a sulfur atom, and may have a bonding portion at any position if possible. These heterocyclyl groups include, for example, an oxetanyl group, a tetrahydrofuranyl group, a tetrahydropyranyl group, a 1,3-dioxolanyl group, a 1,3-dioxanyl group, a 1,4-dioxanyl group, an aziridinyl group, an azetidinyl group, a pyrrolidyl group, a piperidyl group, a piperazyl group, a morpholinyl group, a pyrrolyl group, a pyrazolyl group, a pyrrolidinyl group, a furanyl group, a thienyl group, an oxazolyl group, an isoxazolyl group, a thiazolyl group, an isothiazolyl group, a thiadiazolyl group, a 1H-tetrazolyl group, a 2H-tetrazolyl group, a 1,2,3-oxadiazolyl group, a 1,2,4-oxadiazolyl group, an imidazolyl group, a 1H-1,2,3-triazolyl group, a 1H-1,2,4-triazolyl group, a pyridyl group, a pyrimidyl group, a quinolyl group, an isoquinolyl group, a 1H-indolyl group, a 2H-isoindolyl group, a 4,5,6,7-tetrahydro-2H-isoindolyl group, a 3-azabicyclo[3.1.0]hexyl group, and the like, and an oxetanyl group, a tetrahydrofuranyl group, a tetrahydropyranyl group, an azetidinyl group, a piperidyl group, a pyrrolyl group, a pyrazolyl group, a furanyl group, a thienyl group, an isoxazolyl group, a thiazolyl group, a 1,2,3-thiadiazolyl group, a 1,2,5-thiadiazolyl group, a tetrazolyl group, a pyridyl group, and a 4,5,6,7-tetrahydro-2H-isoindolyl group are preferable.

In the compound represented by the general formula (I), the "3- to 10-membered monocyclic or bicyclic divalent heterocyclyl group" means a divalent group obtained by removing one hydrogen atom from the above-described 3- to 10-membered monocyclic or bicyclic heterocyclyl group.

The substituent in the "optionally substituted 3-to 10-membered monocyclic or bicyclic heterocyclyl group" and the "optionally substituted 3- to 10-membered monocyclic or bicyclic divalent heterocyclyl group" includes one or more of a halogen atom, a cyano group, a hydroxyl group, a nitro group, an oxo group, an alkyl group, an alkenyl group, a cycloalkenyl group, an alkyloxy group, a cycloalkyloxy group, an arylalkyloxy group, a carboxy group, a sulfo group, an alkyloxycarbonyl group, a carbamoyl group, an alkylthio group, an amidino group, a heterocyclyl group, and the like. In the alkyl group, the alkenyl group, the cycloalkenyl group, the alkyloxy group, the cycloalkyloxy group, the arylalkyloxy group, the alkyloxycarbonyl group, and the alkylthio group substituting these heterocyclyl groups, the number of carbon atoms in the alkyl group or the alkenyl group is preferably 1 to 6, and the heterocyclyl group substituting these heterocyclyl groups is preferably a 3- to 10-membered monocyclic or bicyclic heterocyclyl group. In addition, the alkyl group, the alkenyl group, the cycloalkyloxy group, the carbamoyl group, and the amidino group substituting these heterocyclyl groups may be further substituted with one or more of a halogen atom, a hydroxyl group, an amino group, a nitro group, a cyano group, a carboxy group, an alkyloxycarbonyl group, a carbamoyl group, a sulfamoyl group, an amidino group, an alkyloxy group, an oxo group, and a sulfo group, and the heterocyclyl group and the cycloalkenyl group may be further substituted with a hydroxyl group, an alkyloxy group, an amino group, or an oxo group.

The "optionally substituted 3- to 10-membered monocyclic or bicyclic heterocyclyl group" and the "optionally substituted 3- to 10-membered monocyclic or bicyclic divalent heterocyclyl group" are preferably a 3-to 10-membered monocyclic or bicyclic heterocyclyl group unsubstituted or substituted with a halogen atom, an oxo group, an alkyl group, an alkyl group substituted with one or more halogen atoms, an alkyl group substituted with one or more alkyloxycarbonyl groups, an alkyloxy group, an alkyloxy group substituted with one or more halogen atoms, an arylalkyloxy group, a carboxy group, an alkyloxycarbonyl group, an alkylthio group, or an alkylthio group substituted with one or more halogen atoms and a 3- to 10-membered monocyclic or bicyclic divalent heterocyclyl group unsubstituted or substituted with a halogen atom, an oxo group, an alkyl group, or a carboxy group, respectively.

In the compound represented by the general formula (I), the "C₃₋₁₀ cycloalkylalkyl group" means the above-described C₁₋₆ alkyl group substituted with one or more of the above-described C₃₋₁₀ cycloalkyl groups, and the cycloalkyl group may be bound at any position thereof to the alkyl group if possible. The C₃₋₁₀ cycloalkylalkyl group includes, for example, a cyclopropylmethyl group, a methylcyclopropan-1-yl group, a cyclopropylethyl group, a cyclobutylmethyl group, a cyclopentylmethyl group, a cyclohexylmethyl group, a cycloheptylmethyl group, a bicyclo[3.1.0]hexan-3-ylmethyl group, a bicyclo[1.1.1]pentan-1-ylmethyl group, a bicyclo[2.1.1]hexan-1-ylmethyl group, a bicyclo[2.2.1]heptan-1-ylmethyl group, a bicyclo[2.2.2]octan-1-ylmethyl group, a pentacyclo[4.2.0.0^{2,5}.0^{3,8}.0^{4,7}]octan-1-ylmethyl group, a spiro[3.3]heptan-1-ylmethyl group, and the like, and is preferably a cyclopropylmethyl group, a methylcyclopropan-1-yl group, a cyclobutylmethyl group, a cyclopentylmethyl group, a cyclohexylmethyl group, a bicyclo[1.1.1]pentan-1-ylmethyl group, a bicyclo[2.1.1]hexan-1-ylmethyl group, a bicyclo[2.2.1]heptan-1-ylmethyl group, a bicyclo[2.2.2]octan-1-ylmethyl group, a pentacyclo[4.2.0.0^{2,5}.0^{3,8}.0^{4,7}]octan-1-ylmethyl group, or a spiro[3.3]heptan-2-ylmethyl group.

In addition, the "optionally substituted C₃₋₁₀ cycloalkylalkyl group" means that any of the cycloalkyl group and the alkyl group may be substituted, and the substituent in this cycloalkyl group and the substituent in this alkyl group include the same as those given as the substituents of the "optionally substituted C₃₋₁₀ cycloalkyl group" and the "optionally substituted C₁₋₆ alkyl group", respectively.

The "optionally substituted C₃₋₁₀ cycloalkylalkyl group" is preferably a C₃₋₁₀ cycloalkylalkyl group unsubstituted or substituted with a halogen atom, an amino group, a cyano group, a hydroxyl group, an oxo group, an alkyl group (preferably having 1 to 6 carbon atoms), an alkyloxy group (preferably having 1 to 6 carbon atoms), a carboxy group, an alkyloxycarbonyl group (preferably having 1 to 6 carbon atoms), -C(O)N(R¹)R², -N(R¹)C(O)N(R¹)R², or -N(R¹)C(=NH)S(O)₂N(R¹)R².

In the compound represented by the general formula (I), the "C₆₋₁₀ monocyclic or polycyclic arylalkyl group" means the above-described C₁₋₆ alkyl group substituted with one or more of the above-described C₆₋₁₀ monocyclic or polycyclic aryl groups, and the aryl group may be bound at any position thereof to the alkyl group if possible. These arylalkyl groups include, for example, a benzyl group, a benzhydryl group, a phenethyl group, a 1-phenylethyl group, a 3-phenylpropyl group, a 4-phenylbutyl group, and a naphthylmethyl group, and the like, and a benzyl group, a phenethyl group, a 1-phenyl-ethyl group, and a 3-phenylpropyl group are preferable.

In addition, the "optionally substituted C₆₋₁₀ monocyclic or polycyclic arylalkyl group" means that any of the aryl group and the alkyl group may be substituted, and the substituent in this aryl group and the substituent in this alkyl group include the same as those given as the substituents of the "optionally substituted C₆₋₁₀ monocyclic or polycyclic aryl group" and the "optionally substituted C₁₋₆ alkyl group", respectively.

The "optionally substituted C₆₋₁₀ monocyclic or polycyclic arylalkyl group" is preferably a C₆₋₁₀ monocyclic or polycyclic arylalkyl group unsubstituted or substituted with a halogen atom, a cyano group, an alkyl group, an alkyl group substituted with one or more cyano groups, an alkyl group substituted with one or more hydroxyl groups, an alkyl group substituted with one or more hydroxyl groups and one or more halogen atoms, an alkyloxy group, an alkyloxy group substituted with one or more halogen atoms, a carboxy group, an alkyloxycarbonyl group, an alkyloxysulfonyl group, an alkylsulfonyl group, an alkylsulfonyl group substituted with one or more halogen atoms, an alkylthio group, or a pentafluorosulfanyl group.

In the compound represented by the general formula (I), the "3- to 10-membered monocyclic or bicyclic heterocyclylalkyl group" means the above-described C₁₋₆ alkyl group substituted with one or more of the above-described 3- to 10-membered monocyclic or bicyclic heterocyclyl groups, and the heterocyclyl group may be bound at any position thereof to the alkyl group if possible. These heterocyclylalkyl groups include, for example, a pyridylmethyl group, a pyridin-3-ylmethyl group, a thienylmethyl group, a furan-2-ylmethyl group, a thiophen-2-ylmethyl group, a thiophen-3-ylmethyl group, a (1H-pyrrol-2-yl)methyl group, a thiazol-2-ylmethyl group, an isoxazol-3-ylmethyl group, an isoxazol-5-ylmethyl group, an oxetan-2-ylmethyl group, an azetidin-3-ylmethyl group, a tetrahydrofuran-2-ylmethyl group, a tetrahydrofuran-3-ylmethyl group, a tetrahydrofuran-4-ylmethyl group, a 1,3-dioxan-5-yl group, a 1,3-dioxolan-2-ylmethyl group, a pyrrolidin-2-ylmethyl group, a 2-(1H-pyrazol-1-yl)ethyl group, a 4-(1H-pyrazol-1-yl)butyl group, a (tetrazol-5-yl)methyl group, and the like, and a pyridylmethyl group, a furan-2-ylmethyl group, a thiophen-2-ylmethyl group, a (1H-pyrrole-2-yl)methyl group, athiazol-2-ylmethyl group, an isoxazol-5-ylmethyl group, an oxetan-2-ylmethyl group, an azetidin-3-ylmethyl group, a tetrahydrofuran-2-ylmethyl group, a tetrahydrofuran-4-ylmethyl group, a pyrrolidin-2-ylmethyl group, a 2-(1H-pyrazol-1-yl)ethyl group, a 4-(1H-pyrazol-1-yl)butyl group, and a (tetrazol-5-yl)methyl group are preferable.

In addition, the "optionally substituted 3- to 10-membered monocyclic or bicyclic heterocyclylalkyl group" means that any of the heterocyclyl group and the alkyl group may be substituted, and the substituent in this heterocyclic ring and the substituent in this alkyl group include the same as those given as the substituents of the "optionally substituted 3- to 10-membered monocyclic or bicyclic heterocyclyl group" and the "optionally substituted C₁₋₆ alkyl group", respectively.

The "optionally substituted 3- to 10-membered monocyclic or bicyclic heterocyclylalkyl group" is preferably a 3- to 10-membered monocyclic or bicyclic heterocyclylalkyl group unsubstituted or substituted with a halogen atom, an alkyl group, an alkyloxy group, an alkyloxy group substituted with one or more halogen atoms, a carboxy group, an alkyloxycarbonyl group, an alkylthio group, an alkylthio group substituted with one or more halogen atoms, or an arylalkyloxy group.

In the compound represented by the general formula (I), the "C₁₋₆ alkyloxy group" means a group containing an oxygen atom substituted with the above-described C₁₋₆ alkyl group, and the alkyl group may be bound at any position thereof to the oxygen atom if possible. The C₁₋₆ alkyloxy group includes, for example, a methoxy group, an ethoxy group, a n-propoxy group, an isopropoxy group, a n-butoxy group, an isobutoxy group, and the like, and is preferably a methoxy group, an ethoxy group, or a n-propoxy group.

In the compound represented by the general formula (I), the "C₁₋₆ alkyloxyalkyl group" means a group containing the above-described C₁₋₆ alkyl group substituted with one or more of the above-described C₁₋₆ alkyloxy groups. The alkyloxyalkyl group includes, for example, a 2-methoxyethyl group, a 3-methoxy-n-propyl group, a 4-methoxybutyl group, a 5-methoxypentyl group, a 2-ethoxyethyl group, a 4-ethoxybutyl group, and a 2-(n-butoxy)ethyl group, and is preferably a 2-methoxyethyl group, a 4-methoxybutyl group, a 5-methoxypentyl group, a 2-ethoxyethyl group, a 4-ethoxybutyl group, or a 2-(n-butoxy)ethyl group.

The "optionally substituted C₁₋₆ alkyloxyalkyl group" means that the terminal alkyl group (the alkyl group of the C₁₋₆ alkyloxy group) may be substituted with one or more of a halogen atom, a cyano group, a hydroxyl group, an amino group, a carboxy group, a sulfo group, an alkyloxycarbonyl group (preferably having 1 to 6 carbon atoms), a carbamoyl group, a sulfamoyloxy group, and a heterocyclyl group. Moreover, the carbamoyl group, the amino group, and the heterocyclyl group substituting these terminal alkyl groups may be further substituted with an alkyl group (preferably having 1 to 6 carbon atoms), an alkylsulfonyl group (preferably having 1 to 6 carbon atoms), or an oxo group.

The "optionally substituted C₁₋₆ alkyloxyalkyl group" is preferably a C₁₋₆ alkyloxyalkyl group unsubstituted or substituted with a cyano group, a hydroxyl group, an amino group, an amino group substituted with one or more alkylsulfonyl groups, a carboxy group, an alkyloxycarbonyl group (preferably having 1 to 6 carbon atoms), a carbamoyl group, a carbamoyl group substituted with one or more alkyl groups, a carbamoyl group substituted with one or more alkylsulfonyl groups, a sulfamoyloxy group, or a heterocyclyl group.

In the compound represented by the general formula (I), the "3- to 10-membered monocyclic or bicyclic heterocyclyl oxyalkyl group" means the above-described C₁₋₆ alkyl group substituted with a group containing an oxygen atom and substituted with the above-described 3-to 10-membered monocyclic or bicyclic heterocyclyl group, that is, a 3- to 10-membered monocyclic or bicyclic heterocyclyl oxy group. These heterocyclyl oxyalkyl groups include, for example, a 2-(azetidin-3-yloxy)ethyl group, a 2-(tetrahydropyran-4-yloxy)ethyl group, a 2-(tetrahydrofuran-3-yloxy)ethyl group, a 2-(isoxazol-3-yloxy)ethyl group, and the like, and a 2-(isoxazol-3-yloxy)ethyl group is preferable.

In the compound represented by the general formula (I), the "C₁₋₆ alkylthioalkyl group" means a group containing an alkyl group having 1 to 6 carbon atoms substituted with one or more of a group containing a sulfur atom substituted with the above-described C₁₋₆ alkyl groups, that is, a C₁₋₆ alkylthio group. The C₁₋₆ alkylthioalkyl group includes, for example, 2-methylthioethyl group, a 3-methylthiopropyl group, a 4-methylthiobutyl group, a 2-ethylthioethyl group, a 4-ethylthiobutyl group, and the like.

In addition, the "optionally substituted C₁₋₆ alkylthioalkyl group" means that the terminal alkyl group (the alkyl group of the C₁₋₆ alkylthio group) may be substituted with one or more of a halogen atom, a cyano group, a hydroxyl group, an amino group, a carboxy group, a sulfo group, an alkyloxycarbonyl group (preferably having 1 to 6 carbon atoms), a carbamoyl group, a sulfamoyloxy group, and a heterocyclyl group.

The "optionally substituted C₁₋₆ alkylthioalkyl group" is preferably a C₁₋₆ alkylthioalkyl group unsubstituted or substituted with a carboxy group.

In the compound represented by the general formula (I), the "C₁₋₆ alkylsulfonylalkyl group" means a group containing the above-described C₁₋₆ alkyl group substituted with one or more of a group containing a sulfonyl group substituted with the above-described C₁₋₆ alkyl groups, that is, a C₁₋₆ alkylsulfonyl group. The C₁₋₆ alkylsulfonylalkyl group includes, for example, a 2-methylsulfonylethyl group, a 3-methylsulfonylpropyl group, a 4-methylsulfonylbutyl group, a 2-ethylsulfonylethyl group, a 4-ethylsulfonylbutyl group, and the like.

In addition, the "optionally substituted C₁₋₆ alkylsulfonylalkyl group" means that the terminal alkyl group (the alkyl group of the C₁₋₆ alkylsulfonyl group) may be substituted with one or more of a halogen atom, a cyano group, a hydroxyl group, an amino group, a carboxy group, a sulfo group, an alkyloxycarbonyl group (preferably having 1 to 6 carbon atoms), a carbamoyl group, a sulfamoyloxy group, and a heterocyclyl group.

The "optionally substituted C₁₋₆ alkylsulfonylalkyl group" is preferably a C₁₋₆ alkylsulfonylalkyl group unsubstituted or substituted with a carboxy group.

In the compound represented by the general formula (I), the "C₃₋₁₀ cycloalkylsulfonyl group" means a sulfonyl group substituted with the above-described C₃₋₁₀ cycloalkyl group, and the cycloalkyl group may be bound at any position thereof to the sulfonyl group if possible. The C₃₋₁₀ cycloalkylsulfonyl group includes, for example, a cyclopropylsulfonyl group, a cyclobutylsulfonyl group, a cyclohexylsulfonyl group, and the like, and is preferably a cyclohexylsulfonyl group.

The "optionally substituted C₃₋₁₀ cycloalkylsulfonyl group" means that the C₃₋₁₀ cycloalkyl group bound to the sulfonyl group may be substituted, and the substituent in this cycloalkyl group includes the same as those given as the substituents of the "optionally substituted C₃₋₁₀ cycloalkyl group". The "optionally substituted C₃₋₁₀ cycloalkylsulfonyl group" is preferably a C₃₋₁₀ cycloalkylsulfonyl group unsubstituted or substituted with a halogen atom.

In the compound represented by the general formula (I), the "C₆₋₁₀ monocyclic or polycyclic arylsulfonyl group" means a group containing a sulfonyl group substituted with the above-described C₆₋₁₀ monocyclic or polycyclic aryl group, and the aryl group may be bound at any position thereof to a sulfonyl group if possible. These arylsulfonyl groups include, for example, a phenylsulfonyl group, a 1-naphthylsulfonyl group, a 2-naphthylsulfonyl group, and the like, and a phenylsulfonyl group is preferable.

The "optionally substituted C₆₋₁₀ monocyclic or polycyclic arylsulfonyl group" means that the aryl group bound to the sulfonyl group may be substituted, and the substituent in this aryl group includes the same as those given as the substituents of the "optionally substituted C₆₋₁₀ monocyclic or polycyclic aryl group". The "optionally substituted C₆₋₁₀ monocyclic or polycyclic arylsulfonyl group" is preferably a C₆₋₁₀ monocyclic or polycyclic arylsulfonyl group unsubstituted or substituted with a halogen atom, a cyano group, a nitro group, an alkyl group, an alkyloxy group, an alkyloxy group substituted with one or more halogen atoms, or an alkyloxycarbonyl group.

In the compound represented by the general formula (I), the "3- to 10-membered monocyclic or bicyclic heterocyclylsulfonyl group" means a group containing a sulfonyl group substituted with the above-described 3- to 10-membered monocyclic or bicyclic heterocyclyl group, and the heterocyclyl group may be bound at any position thereof to the sulfonyl group if possible. These heterocyclyl sulfonyl groups include, for example, a pyridin-2-ylsulfonyl group, a pyridin-3-ylsulfonyl group, a thiophen-2-ylsulfonyl group, a tetrahydrofuran-4-ylsulfonyl group, a piperidin-4-ylsulfonyl group, a piperidin-1-ylsulfonyl group, and a piperazin-1-ylsulfonyl group, and the like, and a pyridin-2-ylsulfonyl group, a thiophen-2-ylsulfonyl group, and a piperidin-1-ylsulfonyl group are preferable.

The "optionally substituted 3- to 10-membered monocyclic or bicyclic heterocyclylsulfonyl group" means that the heterocyclyl group bound to the sulfonyl group may be substituted, and the substituent in this heterocyclyl group includes the same as those given as the substituents of the "optionally substituted 3- to 10-membered monocyclic or bicyclic heterocyclyl group". The "optionally substituted 3- to 10-membered monocyclic or bicyclic heterocyclylsulfonyl group" is preferably a 3- to 10-membered monocyclic or bicyclic heterocyclylsulfonyl group unsubstituted or substituted with a halogen atom, a cyano group, or an alkyloxy group.

In the compound represented by the general formula (I), the "C₆₋₁₀ monocyclic or polycyclic arylalkylsulfonyl group" means a group containing a sulfonyl group substituted with the above-described C₆₋₁₀ monocyclic or polycyclic arylalkyl group. These arylalkylsulfonyl groups include, for example, a benzylsulfonyl group, a phenethylsulfonyl group, a 3-phenylpropylsulfonyl group, a 1-naphthylmethylsulfonyl group, a 2-naphthylmethylsulfonyl group, and the like, and a benzylsulfonyl group, a phenethylsulfonyl group, and a 3-phenylpropylsulfonyl group are preferable.

The "optionally substituted C₆₋₁₀ monocyclic or polycyclic arylalkylsulfonyl group" means that the C₆₋₁₀ monocyclic or polycyclic arylalkyl group bound to the sulfonyl group may be substituted, and the substituent in this arylalkyl group includes the same as those given as the substituents of the "optionally substituted C₆₋₁₀ monocyclic or polycyclic arylalkyl group". The "optionally substituted C₆₋₁₀ monocyclic or polycyclic arylalkylsulfonyl group" is preferably a C₆₋₁₀ monocyclic or polycyclic arylalkylsulfonyl group unsubstituted or substituted with a halogen atom, an alkyloxy group, a halogenated alkyloxy group, or a cyano group.

In the compound represented by the general formula (I), the "C₆₋₁₀ monocyclic or polycyclic aryloxyalkylsulfonyl group" means an alkylsulfonyl group having 1 to 6 carbon atoms substituted with a group containing an oxygen atom substituted with the above-described C₆₋₁₀ monocyclic or polycyclic aryl group, that is, a C₆₋₁₀ monocyclic or polycyclic aryloxy group. These aryloxyalkylsulfonyl groups include, for example, a (2-phenyloxyethyl)sulfonyl group, a (3-phenyloxypropyl)sulfonyl group, a (4-phenyloxybutyl)sulfonyl group, a (5-phenyloxypentyl)sulfonyl group, a 2-(naphthalen-1-yloxy)ethylsulfonyl group, and a 2-(naphthalen-2-yloxy)ethylsulfonyl group, and a 2-phenyloxyethylsulfonyl group and a (3-phenyloxypropyl)sulfonyl group are preferable.

The "optionally substituted C₆₋₁₀ monocyclic or polycyclic aryloxyalkylsulfonyl group" means that the alkyl group of the alkylsulfonyl group and the aryl group of the C₆₋₁₀ monocyclic or polycyclic aryloxy group may be substituted, and the substituents in these alkyl group and aryl group include the same as those given as the substituents of the "optionally substituted C₁₋₆ alkyl group" and the "optionally substituted C₆₋₁₀ monocyclic or polycyclic aryl group", respectively. The "optionally substituted C₆₋₁₀ monocyclic or polycyclic aryloxyalkylsulfonyl group" is preferably a C₆₋₁₀ monocyclic or polycyclic aryloxyalkylsulfonyl group unsubstituted or substituted with a halogen atom, an alkyloxy group, an alkyloxy group substituted with one or more halogen atoms, or a cyano group.

In the compound represented by the general formula (I), the "C₆₋₁₀ monocyclic or polycyclic arylaminocarbonyl group" means a group containing an aminocarbonyl group substituted with the above-described C₆₋₁₀ monocyclic or polycyclic aryl group. These arylaminocarbonyl groups include, for example, a phenylaminocarbonyl group, a naphthylaminocarbonyl group, and the like, and a phenylaminocarbonyl group is preferable.

The "optionally substituted C₆₋₁₀ monocyclic or polycyclic arylaminocarbonyl group" means that the C₆₋₁₀ monocyclic or polycyclic aryl group bound to the aminocarbonyl group may be substituted, and the substituent in this aryl group includes the same as those given as the substituents of the "optionally substituted C₆₋₁₀ monocyclic or polycyclic aryl group". The "optionally substituted C₆- monocyclic or polycyclic arylaminocarbonyl group" is preferably a C₆- monocyclic or polycyclic arylaminocarbonyl group unsubstituted or substituted with a halogen atom, an alkyloxy group, an alkyloxy group substituted with one or more halogen atoms, or a cyano group.

In the general formula (I), as A¹ and A², A¹ preferably represents a single bond, a methylene group, or an ethylene group, and both A¹ and A² are more preferably methylene groups. In addition, in the general formula (I), n is preferably 0.

In addition, A³ is more preferably a pyrrolidinyl group, a piperidinyl group, a piperazinyl group, or a homopiperazinyl group which may be substituted with preferably any one substituent selected from the group consisting of -N(R¹)R², -N(R¹)R³, and -R³, further preferably a piperazinyl group which may be substituted with one of them, and still further preferably a piperazinyl group which may be substituted particularly preferably with one -R³. In addition, in a case where A³ is a group substituted with -N(R¹)R³ or -R³, in R³ that is a group represented by the following formula: -D¹-D²-D³ or a group represented by the following formula: -D¹-ON=D⁴, D¹ is preferably a C₁₋₆ alkylene group, and R³ is preferably a group represented by the following formula: -D¹-D²-D³.

Alternatively, A³ is preferably such that the substituent in the azetidinyl group, the pyrrolidinyl group, the piperidinyl group, the piperazinyl group, or the homopiperazinyl group be -N(R¹)R² and/or -R³.

In addition, the group represented by -A⁴-A⁵ is preferably such that A⁴ be -O- or -N(R¹)- and A⁵ be a group containing a benzene ring or a 5- to 6-membered monocyclic heterocyclic ring. Such A⁵ is particularly preferably a group containing a benzene ring, or a pyridine ring or a thiophene ring. Moreover, as the compound represented by the general formula (I) of the present invention, it is particularly preferable that the group represented by -A⁴-A⁵ be such a group and A³ be an azetidinyl group, a pyrrolidinyl group, a piperidinyl group, a piperazinyl group, or a homopiperazinyl group which is substituted with the above-described substituent, from the viewpoint that such compound exhibits a more excellent dual modulatory action against the histamine H1 receptor and the histamine H4 receptor.

In addition, in the present invention, more preferable embodiments of the compound represented by the general formula (I) include, for example, the following embodiments.
[1] A compound, wherein
   in the formula (I),
   A³ represents an azetidinyl group, a pyrrolidinyl group, a piperidinyl group, a piperazinyl group, or a homopiperazinyl group which is optionally substituted with at least one substituent selected from the group consisting of -N(R¹)R³ and -R³,
      R³ represents a group represented by the following formula: -D¹-D²-D³ or a group represented by the following formula: -D¹-ON=D⁴ (except where -N(R¹)R³ is -N(R¹)R²),
      D¹ represents a C₁₋₆ alkylene group,
      D² represents a single bond, -O-, -OC(O)-, -S(O)₂-, -N(R¹)S(O)₂-, -C(O)-, or -C(O)N(R¹)-,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
   n represents 0.
[2] A compound, wherein
   in the formula (I),
   both A¹ and A² represent methylene groups,
   A³ represents an azetidinyl group, a pyrrolidinyl group, a piperidinyl group, a piperazinyl group, or a homopiperazinyl group which is optionally substituted with at least one substituent selected from the group consisting of -N(R¹)R³ and -R³,
      R³ represents a group represented by the following formula: -D¹-D²-D³ or a group represented by the following formula: -D¹-ON=D⁴ (except where -N(R¹)R³ is -N(R¹)R²),
      D¹ represents a C₁₋₆ alkylene group,
      D² represents a single bond, -O-, -OC(O)-, -S(O)₂-, -N(R¹)S(O)₂-, -C(O)-, or -C(O)N(R¹)-,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
   n represents 0.
[3] A compound, wherein
   in the formula (I),
   both A¹ and A² represent methylene groups,
   A³ represents an azetidinyl group, a pyrrolidinyl group, a piperidinyl group, a piperazinyl group, or a homopiperazinyl group which is optionally substituted at least one -N(R¹)R²,
      R¹ and R² may be the same or different and each represents a hydrogen atom or a C₁₋₆ alkyl group,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
   n represents 0.
[4] A compound, wherein
   in the formula (I),
   both A¹ and A² represent methylene groups,
   A³ represents a piperazinyl group optionally substituted with at least one -R³,
      R³ represents a group represented by the following formula: -D¹-D²-D³ or a group represented by the following formula: -D¹-ON=D⁴,
      D¹ represents a C₁₋₆ alkylene group,
      D² represents a single bond, -O-, -OC(O)-, -S(O)₂-, -N(R¹)S(O)₂-, -C(O)-, or -C(O)N(R¹)-,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
   n represents 0.
[6] A compound, wherein
   in the formula (I),
   A¹ represents a single bond, a methylene group, or an ethylene group,
   A³ represents a pyrrolidinyl group, a piperidinyl group, piperazinyl group, or a homopiperazinyl group which is optionally substituted with at least one substituent selected from the group consisting of -N(R¹)R² and -R³,
      R¹ and R² may be the same or different and each represent a hydrogen atom or a C₁₋₆ alkyl group,
      R³ represents a group represented by the following formula: -D¹-D²-D³,
      D¹ represents a C₁₋₆ alkylene group,
      D² represents a single bond or -O-,
      D³ represents a hydrogen atom, an optionally substituted C₁₋₆ alkyl group, an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₁₋₆ alkyloxyalkyl group, a 3- to 10-membered monocyclic or bicyclic heterocyclyloxyalkyl group, or an optionally substituted C₁₋₆ alkylthioalkyl group,
   A⁴ represents -O- or -N(R¹)-,
   A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
   n represents 0.

The compound represented by the general formula (I) of the present invention may be a salt thereof (preferably a pharmacologically acceptable salt). Such a salt includes medically acceptable non-toxic salts, which include, for example, salts of alkali metals or alkaline earth metals, such as sodium salts, potassium salts, and calcium salts; salts of hydrohalic acids, such as hydrofluoric acid, hydrochloric acid, hydrobromic acid, and hydroiodic acid; salts of inorganic acids, such as sulfuric acid, nitric acid, phosphoric acid, hydrogen peroxide acid, perchloric acid, and carbonic acid; salts of organic carboxylic acids, such as acetic acid, trichloroacetic acid, trifluoroacetic acid, hydroxyacetic acid, lactic acid, citric acid, tartaric acid, oxalic acid, benzoic acid, mandelic acid, butyric acid, fumaric acid, succinic acid, maleic acid, propionic acid, formic acid, and malic acid; salts of acidic amino acids, such as aspartic acid and glutamic acid; salts of alkyl sulfonic acids, such as methanesulfonic acid and ethanesulfonic acid; salts of arylsulfonic acids such as benzenesulfonic acid and p-toluenesulfonic acid, and the like.

In the compound represented by the general formula (I), in a case where stereoisomers, geometric isomers, or tautomers exist, the compound may be a mixture of two or more of the isomers, or may be any one of them.

In addition, although the compound represented by the general formula (I) of the present invention may have one or two or more asymmetric carbon atoms depending on the types of substituents in some cases, optically active isomers, diastereoisomers, any mixtures of these, racemates, and the like based on one or two or more asymmetric carbon atoms are all encompassed by the scope of the compound represented by the general formula (I) of the present invention. Moreover, the compound represented by the general formula (I) of the present invention or a pharmacologically acceptable salt thereof also encompasses their corresponding hydrates or solvates, and crystal polymorphisms.

Furthermore, the compound represented by the general formula (I) of the present invention and a salt thereof encompass compounds obtained by labeling these with radioisotopes and nonradioisotopes, and also encompass hydrates, solvates, and crystal polymorphisms with these.

In the present Specification, when a compound in which the above-described isomers or isotopes are present is not mentioned regarding its name, the compound may be one or a mixture of two or more of these isomers and isotopes.

### Production of Compound Expressed by General Formula (I) and Salt Thereof

Although the compound represented by the general formula (I) of the present invention (hereinafter, sometimes, referred to simply as the "compound of the formula (I)") and a salt thereof can be produced by the production methods described below, the method for producing a compound of the present invention is not limited to these, and the scope of the compound of the present invention is also not limited to compounds produced by the production methods described below. In addition, since the following Examples illustrate more specific examples of the method for producing a compound and a salt thereof of the present invention, a person skilled in the art can produce compounds encompassed by the compound of the formula (I) and a salt thereof by appropriately selecting starting materials, reaction conditions, reagents, and the like, and conducting appropriate modifications and improvements as necessary, while referring to the description of general production methods, the specific description of Examples and Reference Examples as well as publicly-known techniques of generally known documents, books, and the like (for example, "YUUKI JINMEI HANNOU" by Katsuyuki Ogura, or "JIKKEN KAGAKU KOUZA" by Maruzen Publishing Co., Ltd., and the like) as described below. Note that the method for producing the compound of the present invention encompasses all methods for producing compounds through publicly-known means based on the properties of the compounds revealed by the present invention.

The compound of the formula (I) and a salt thereof can be produced by employing publicly-known techniques upon utilizing characteristics achieved by the basic skeleton or the structures of substituents . In this event, depending on the type of a functional group, it is possible to obtain a target intermediate or final product by protecting the functional group, bringing a starting material into an intermediate, and removing a protecting group. Such a protecting group includes protecting groups described in PROTECTIVE GROUPS in ORGANIC SYNTHESIS (by Greene Wuts) and the like, and may be selected as appropriate in accordance with reactions to be used.

For production, a microwave reaction device (manufactured by Biotage, and the like), a microreactor reaction device, and the like may be used as appropriate.

The compound obtained by the method for producing the compound of the present invention sometimes forms a salt, and this salt includes the same as those given as the salts of the compound of the formula (I) described above.

Symbols in structural formulae shown below represent the same meanings defined in the formula (I) unless otherwise specified. In addition, R⁶ to R¹⁴, X¹, X², and X³ shown below mean a partial structure of the formula (I) respectively, and are thus assumed to be not beyond the ranges defined in the formula (I). Moreover, regarding letters newly appearing occasionally, the meaning of such letter is defined every time when it is particularly desirable to be described because of the properties of the compound, reaction conditions, and the like, and the same definition applies also when the letter appears later.

R⁶ to R¹⁴ and substituents thereof can be changed into other functional groups by methods described in generally known documents (regarding organic synthesis) and books (JIKKEN KAGAKU KOUZA, Maruzen Publishing Co., Ltd., and the like), reactions obvious to a person skilled in the art, or improved and modified methods of these. Also by appropriately combining steps that can be adopted by a person skilled in the art using methods which can be usually learned, R⁶ to R¹⁴ and substituents thereof can be changed into other functional groups (such steps include, for example, oxidation, reduction, hydrolysis, amidation, sulfonylation, alkylation, and the like)

L, L¹, and L² described in the following reaction formulae represent leaving groups, and the leaving groups include, for example, a hydroxyl group, alkyloxy groups (for example, a methoxy group, an ethoxy group, a propoxy group, a tert-butoxy group, and the like), optionally substituted aryloxy groups (for example, a phenyloxy group, a naphthyloxy group, an anthracenyloxy group, and the like), optionally substituted heterocyclic aryloxy groups (for example, a pyridyloxy group, a pyrimidyloxy group, an N-methyl pyridiniumoxy group, and the like), halogen atoms (for example, fluorine, chlorine, bromine, iodine, and the like), C₁₋₆ alkylsulfonyloxy groups optionally substituted with halogens (for example, a methanesulfonyloxy group, a trifluoromethanesulfonyloxy group, an ethanesulfonyloxy group, a pentafluoroethanesulfonyloxy group, and the like), optionally substituted arylsulfonyloxy groups (for example, a toluenesulfonyloxy group, a 4-trifluoromethyl phenylsulfonyloxy group, an ortho-nitrophenylsulfonyloxy group, a para-nitrophenylsulfonyloxy group, and the like), C₁₋₆ alkylthio groups optionally substituted with halogens (for example, a methylthio group, an ethylthio group, and the like), an optionally substituted benzothiazol-2-ylthio group, and the like.

General organic solvents described herein include acetic ester-based solvents such as methyl acetate, ethyl acetate, butyl acetate, and isopropyl acetate; ether-based solvents such as diethyl ether, cyclopropyl methyl ether, diphenyl ether, tetrahydrofuran (abbreviated as THF), 2-methyltetrahydrofuran, dioxane, dimethoxyethane, and tert-butyl methyl ether; aromatic hydrocarbon-based solvents such as benzene, toluene, xylene, trifluoromethylbenzene, and chlorobenzene; aliphatic hydrocarbon-based solvents such as hexane, cyclohexane, pentane, and heptane; halogen-based solvents such as methylene chloride, 1,2-dichloroethane, and chloroform; polar aprotic solvents such as N,N-dimethylformamide (abbreviated as DMF), dimethyl sulfoxide (abbreviated as DMSO), 1-methylpyrrolidone (abbreviated as NMP), acetonitrile, pyridine, acetone, methyl ethyl ketone, and methyl isobutyl ketone; polar protic solvents such as methanol, ethanol, propanol, 2-propanol, n-butanol, sec-butanol, and tert-butanol, and the like.

Generally known inorganic bases described herein include basic salts such as potassium carbonate, sodium hydrogen carbonate, sodium carbonate, cesium carbonate, potassium phosphate, potassium hydrogen phosphate, and sodium hydrogen phosphate; metal hydroxides such as sodium hydroxide, potassium hydroxide, barium hydroxide, magnesium hydroxide, and lithium hydroxide; metal hydrides such as sodium hydride, lithium hydride, and calcium hydride; metal amides such as sodium amide, lithium diisopropylamide, and lithium hexamethyldisilazide; metal alkoxides such as sodium methoxide, sodium ethoxide, and sodium tert-butoxide, and the like.

In addition, generally known organic bases include aliphatic amines such as methylamine, dimethylamine, trimethylamine, ethylamine, diethylamine, triethylamine, N,N-diisopropylethylamine, ethanolamine, 2-(dimethylamino)ethanol, cyclohexylamine, dicyclohexylamine, dicyclohexylmethylamine, and N,N,N',N'-tetramethylethane-1,2-diamine; heterocyclic amines such as pyridine, picoline, dimethylaminopyridine (abbreviated as DMAP), 2,6-lutidine, 2,4,6-collidine, 1,5-diazabicyclo[4.3.0]non-5-ene (abbreviated as DBN), 1,8-diazabicyclo[5.4.0]undec-7-ene (abbreviated as DBU), 7-methyl-1,5,7-triazabicyclo-[4.4.0]dec-5-ene, 1,4-diazabicyclo[2.2.2]octane (abbreviated as DABCO), N-methylpiperidine, N-methylpyrrolidine, N,N'-dimethylpiperazine, N-methylmorpholine, and the like.

Moreover, generally known sulfonylation reagents include C₁₋₆ alkylsulfonyl chlorides optionally substituted with a halogen atom (for example, methanesulfonyl chloride, ethanesulfonyl chloride, pentafluoroethanesulfonyl chloride, and the like), C₁₋₆ alkylsulfonic anhydrides optionally substituted with a halogen atom (for example, methanesulfonic anhydride, ethanesulfonic anhydride, trifluoromethanesulfonic anhydride, and the like), optionally substituted arylsulfonyl chlorides (for example, toluenesulfonyl chloride, 4-trifluoromethylphenylsulfonyl chloride, ortho-nitrophenylsulfonyl chloride, para-nitrophenylsulfonyl chloride, and the like), and the like.

Regarding reactions shown below, it is advantageous to carry out the reactions using a solvent inert to the reaction, some reactions may be carried out without any solvent, and it is desirable to change such condition as appropriate in accordance with the type of the reaction.

Firstly, the group of compounds represented by the formula (I) can be produced by Scheme 1 based on the general methods as described below, for example. A compound of the formula (1), its commercial product can be easily obtained, and the compound itself can be produced using publicly-known techniques or using a method based on the publicly-known techniques.

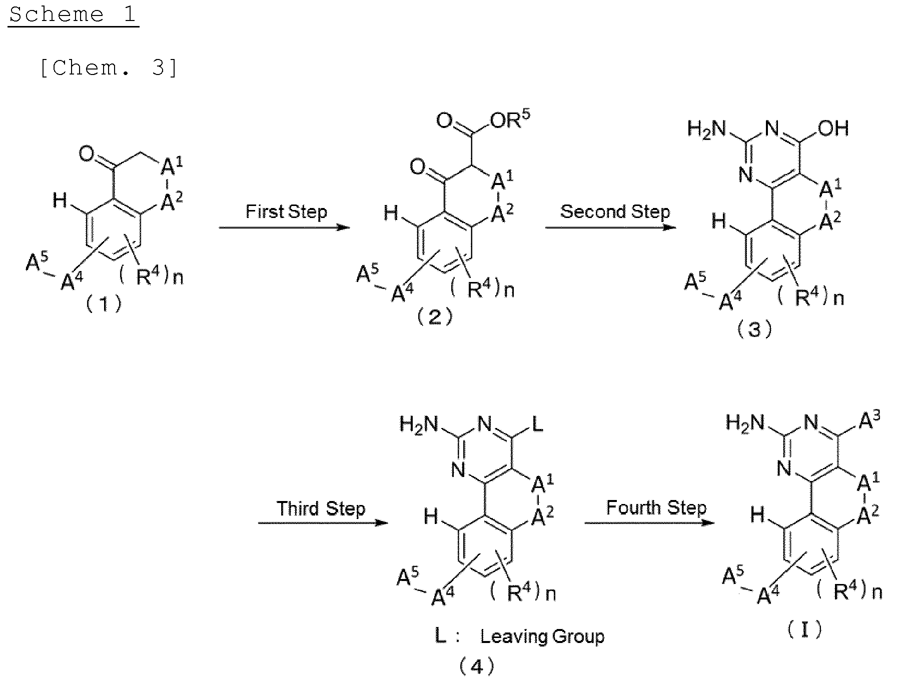

In the formulae, R⁵ represents an optionally substituted alkyl group, an optionally substituted cycloalkyl group, an optionally substituted arylalkyl group, an optionally substituted allyl group, an optionally substituted phenyl group, or the like.

First, in the first step, conversion from the compound of the formula (1) to a compound of a formula (2) can be carried out using the following method, for example. Specifically, the compound of the formula (2) can be produced by reacting the compound of the formula (1) with a reagent represented by C(O)(OR⁵)₂, in a THF solvent, in the presence of a base. The reaction solvent in this reaction may be any of general organic solvents inert to the reaction instead of THF, or a mixed solvent of these may be used. The reaction solvent is preferably THF, diethyl ether, cyclopropyl methyl ether, diphenyl ether, dioxane, dimethoxyethane, benzene, toluene, xylene, DMF, or pyridine. The reaction solvent is further preferably THF or dioxane. As the base, 1 to 10 equivalents of an alkali metal hydride such as lithium hydride, sodium hydride, or calcium hydride may be used, and sodium hydride is preferably used. As the dialkyl carbonate, 1 to 20 equivalents of dimethyl carbonate, diethyl carbonate, or the like may be used, 1 to 10 equivalents thereof may be preferably used, and 1 to 5 equivalents thereof may further preferably used. The reaction temperature is within the range of -30 to 150 °C, and the reaction time is 0.5 to 24 hours.

Next, in the second step, conversion from the compound of the formula (2) to a compound of a formula (3) can be carried out by the following method, for example. Specifically, the compound of the formula (3) can be produced by reacting the compound of the formula (2) with guanidine or a salt thereof, in a DMF solvent, in the presence of a base. The reaction solvent in this reaction may be any of general organic solvents inert to the reaction, or a mixed solvent of these may be used. The reaction solvent is preferably diethyl ether, cyclopropyl methyl ether, diphenyl ether, THF, dioxane, dimethoxyethane, DMF, DMSO, pyridine, or NMP. The reaction solvent is further preferably DMF, DMSO, pyridine, or NMP. The base is a generally known inorganic base or organic base, and is preferably potassium carbonate, sodium hydrogen carbonate, cesium carbonate, potassium hydroxide, sodium hydroxide, triethylamine, N,N-diisopropylethylamine, or pyridine, and 1 to 10 equivalents thereof may be used. Further preferably, 1 to 5 equivalents of potassium carbonate may be used. The reaction temperature is within the range of 0 to 200°C, and the reaction time is 0.5 to 24 hours.

Next, in the third step, conversion from the compound of the formula (3) to a compound of a formula (4) can be carried out by the following method, for example. Specifically, the compound of the formula (4) can be produced by any of the following methods (i) and (ii). Specifically,
(i) : The compound of the formula (4) can be produced by carrying out reaction to substitute a hydroxyl group of the compound of the formula (3) with a halogen atom in a toluene solvent in the presence of phosphorus oxychloride. The reaction solvent in this reaction may be any of general organic solvents inert to the reaction instead of toluene, or a mixed solvent of these may be used. The reaction solvent is preferably toluene, benzene, xylene, methylene chloride, acetonitrile, cyclopropyl methyl ether, or diethyl ether. The reaction solvent is further preferably toluene, benzene, or xylene. Instead of phosphorus oxychloride, 1 to 20 equivalents of phosphorus oxybromide, thionyl chloride, thionyl bromide, or the like may be used, 5 to 15 equivalents of phosphorus oxychloride may be preferably used, and 5 to 10 equivalents thereof may be further preferably used. The reaction temperature is within the range of -78 to 150°C, and the reaction time is 0.5 to 24 hours.
(ii) : The compound of the formula (4) can be produced by reacting the compound of the formula (3) in a solvent inert to the reaction in the presence of a base and a sulfonylation reagent. The reaction solvent in this reaction may be any of general organic solvents inert to the reaction, or a mixed solvent of these may be used. The reaction solvent is preferably carbon tetrachloride, chloroform, 1,2-dichloroethane, methylene chloride and benzene, toluene, xylene, or mesitylene. The reaction solvent is further preferably chloroform or methylene chloride. As the base, 1 to 10 equivalents of triethylamine, N,N-diisopropylethylamine, DBN, DBU, 7-methyl-1,5,7-triazabicyclo-[4.4.0]dec-5-ene, DABCO, or the like may be used, and triethylamine and N,N-diisopropylethylamine are preferable. The sulfonylation reagent may be a generally known sulfonylation reagent, and 1 to 10 equivalents of methanesulfonyl chloride, toluenesulfonyl chloride, or trifluoromethanesulfonic anhydride may be used, and toluenesulfonyl chloride is further preferable. The reaction temperature is within the range of -50 to 150°C, and the reaction time is 0.5 to 24 hours.

Next, in the fourth step, conversion from the compound of the formula (4) to the compound of the formula (I) can be carried out by the following method, for example. Specifically, the compound of the formula (I) can be produced by reacting the compound of the formula (4) with an amine compound for introducing the substituent A³ in a DMF solvent in the presence of an additive. The reaction solvent in this reaction may be any of general organic solvents inert to the reaction instead of DMF, or a mixed solvent of these may be used. The reaction solvent is preferably diethyl ether, cyclopropyl methyl ether, diphenyl ether, THF, 2-methyltetrahydrofuran, dioxane, dimethoxyethane, tert-butyl methyl ether, benzene, toluene, xylene, trifluoromethylbenzene, chlorobenzene, methylene chloride, 1,2-dichloroethane, chloroform, DMF, DMSO, NMP, acetonitrile, or pyridine. The reaction solvent is further preferably THF, dioxane, DMF, DMSO, or NMP. The above-described amine compound may be used in an amount of 1 to 30 equivalents, and may be combined with, but does not have to be combined with, an additive, which can be selected as appropriate. As the additive, 1 to 30 equivalents of a generally known organic base or inorganic base inert to the reaction may be used, and sodium hydrogen carbonate, sodium carbonate, potassium phosphate, potassium carbonate, cesium carbonate or triethylamine, N,N-diisopropylethylamine, DBN, DBU, 7-methyl-1,5,7-1riazabicyclo-[4.4.0]dec-5-ene, or DABCO may be preferably used. Triethylamine and N,N-diisopropylethylamine are further preferable. The reaction temperature is within the range of 0 to 150°C, and the reaction time is 0.5 to 24 hours.

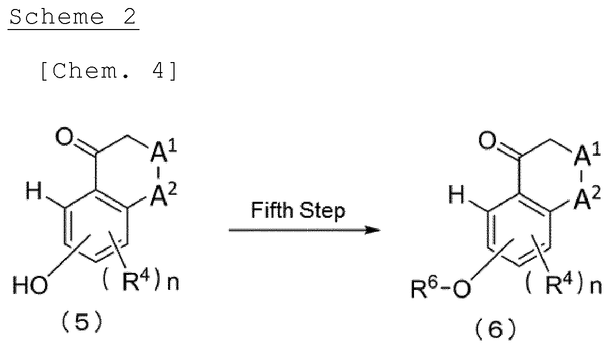

Secondly, a compound in which the group corresponding to -A⁴-A⁵ in the formula (1) is -OH is represented by a formula (5). In addition, a compound of a formula (6) is a compound encompassed by the compound of the formula (1), and R⁶ means the same as A⁵. The compound of the formula (6) can be derivatized into the compound of the formula (I) by the above-described reactions from the first step to the fourth step. In the fifth step, conversion from the compound of the formula (5) to the compound of the formula (6) can be carried out by any of the following methods (i), (ii), or (iii), for example. Specifically,
(i): The compound of the formula (6) can be produced by reacting the compound of the formula (5) in an acetone solvent in the presence of a base and a compound represented by R⁶L. The reaction solvent in this reaction may be any of general organic solvents inert to the reaction instead of acetone, or a mixed solvent of these may be used. The reaction solvent is preferably diethyl ether, cyclopropyl methyl ether, diphenyl ether, THF, 2-methyltetrahydrofuran, dioxane, dimethoxyethane, tert-butyl methyl ether, benzene, toluene, xylene, trifluoromethylbenzene, chlorobenzene, DMF, DMSO, NMP, acetonitrile, pyridine, acetone, methyl ethyl ketone, or methyl isobutyl ketone. The reaction solvent is further preferably THF or acetone. As the base, 1 to 10 equivalents of a carbonate-based inorganic base such as potassium carbonate, calcium carbonate, sodium carbonate, or cesium carbonate may be used, and potassium carbonate or cesium carbonate is preferably used. The reaction temperature is within the range of 0 to 160°C, and the reaction time is 0.5 to 24 hours.
(ii) : The compound of the formula (6) can be produced by reacting the compound of the formula (5) in a DMF solvent in the presence of a base and a compound represented by R⁶L. The reaction solvent in this reaction may be any of general organic solvents inert to the reaction instead of DMF, or a mixed solvent of these may be used. The reaction solvent is preferably diethyl ether, cyclopropyl methyl ether, diphenyl ether, THF, 2-methyltetrahydrofuran, dioxane, dimethoxyethane, tert-butyl methyl ether, benzene, toluene, xylene, trifluoromethylbenzene, chlorobenzene, DMF, or NMP. The reaction solvent is further preferably THF, dioxane, DMF, or NMP. As the base, 1 to 10 equivalents of a metal hydride such as sodium hydride, calcium hydride, or lithium hydride may be used, and sodium hydride may be preferably used. The reaction temperature is within the range of -10 to 160°C, and the reaction time is 0.5 to 24 hours.
(iii) : The compound of the formula (6) can be produced by reacting the compound of the formula (5) in a THF solvent in the presence of triphenylphosphine (a phosphine compound) and diethyl azodicarboxylate (an azo compound) and in the presence of a compound represented by R⁶L. The reaction solvent in this reaction may be any of general organic solvents inert to the reaction instead of THF, or a mixed solvent of these may be used. The reaction solvent is preferably THF, diethyl ether, cyclopropyl methyl ether, diphenyl ether, 2-methyltetrahydrofuran, dioxane, dimethoxyethane, tert-butyl methyl ether, benzene, toluene, xylene, trifluoromethylbenzene, or chlorobenzene. The reaction solvent is further preferably THF, dioxane, or toluene. The phosphine reagent may be a phosphine compound known in the literature and the like relating to the Mitsunobu reaction instead of triphenylphosphine, and is preferably triphenylphosphine, tri(ortho-tolyl)phosphine, tri-n-butylphosphine, or tri-tert-butylphosphine, and, 1 to 10 equivalents thereof are preferably used. The azo compound may be a general azo compound known in the literature and the like relating to the Mitsunobu reaction instead of diethyl azodicarboxylate, and is preferably diisopropyl azodicarboxylate, 1,1'-(azodicarbonyl)dipiperidine, N,N,N',N'-tetramethylazodicarboxamide, N,N,N',N'-tetraisopropylazodicarboxamide, or 1,6-dimethyl-1,5,7-hexahydro-1,4,6,7-tetrazocin-2,5-di one, and 1 to 10 equivalents thereof are preferably used. The reaction temperature is within the range of 0 to 50°C, and the reaction time is 0.5 to 24 hours. When heating at 50°C or higher is necessary, (cyanomethylene)-tri-n-butylphosphorane, (cyanomethylene)trimethylphosphorane, or the like is desirably used in place of the combination of a phosphine compound and an azo compound.

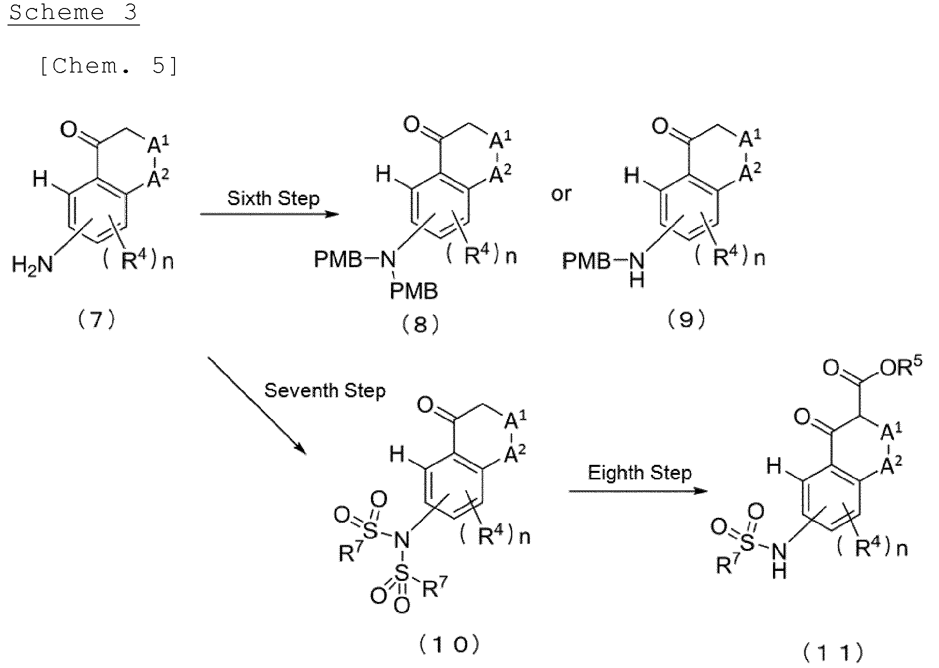

Thirdly, a compound in which the group corresponding to -A⁴-A⁵ in the formula (1) is -NH₂ is represented by a formula (7). In addition, a compound of a formula (8), a compound of a formula (9), and a compound of a formula (10) are compounds encompassed by the compound of the formula (1), and R⁷ represents an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₆₋₁₀ monocyclic or polycyclic arylalkyl group, or an optionally substituted C₆₋₁₀ monocyclic or polycyclic aryloxyalkyl group respectively in the optionally substituted C₃₋₁₀ cycloalkylsulfonyl group, the optionally substituted C₆₋₁₀ monocyclic or polycyclic arylsulfonyl group, the optionally substituted 3- to 10-membered monocyclic or bicyclic heterocyclylsulfonyl group, the optionally substituted C₆₋₁₀ monocyclic or polycyclic arylalkylsulfonyl group, or the optionally substituted C₆₋₁₀ monocyclic or polycyclic aryloxyalkylsulfonyl group represented by A⁵ in the formula (I). In addition, a compound of a formula (11) is a compound encompassed by the compound of the formula (2).

First, in the sixth step, conversion from the compound of the formula (7) to the compound of the formula (8) or the compound of the formula (9) having a paramethoxybenzyl (abbreviated as PMB) group can be carried out by the following method, for example. Specifically, the compound of the formula (8) or the compound of the formula (9) can be produced by reacting the compound of the formula (7) with 0.1 to 20 equivalents of 1-(chloromethyl)-4-methoxybenzene in an acetonitrile solvent in the presence of an inorganic base. The reaction solvent in this reaction may be any of general organic solvents inert to the reaction instead of acetonitrile, or a mixed solvent of these may be used. The reaction solvent is preferably acetonitrile, diethyl ether, cyclopropyl methyl ether, dioxane, dimethoxyethane, THF, DMF, NMP, or DMSO. The reaction solvent is further preferably acetonitrile, THF, dioxane, or DMF. The inorganic base may be a generally known inorganic base, and 1 to 20 equivalents of cesium carbonate, sodium carbonate, sodium hydrogen carbonate, potassium phosphate, potassium carbonate, lithium hydride, sodium hydride, or calcium hydride may be preferably used, and cesium carbonate or potassium carbonate may be further preferably used. The reaction temperature is within the range of -30 to 150°C, and the reaction time is 0.5 to 200 hours.

Next, in the seventh step, conversion from the compound of the formula (7) to the compound of the formula (10) can be carried out by the following method, for example. Specifically, the compound of the formula (10) can be produced by reacting the compound of the formula (7) with 0.5 to 10 equivalents of any of various sulfonylation reagents in a THF solvent in the presence of a base. The reaction solvent in this reaction may be any of general organic solvents inert to the reaction instead of THF, or a mixed solvent of these may be used. The reaction solvent is preferably diethyl ether, cyclopropyl methyl ether, dioxane, dimethoxyethane, or THF. The reaction solvent is further preferably THF. The base may be a generally known organic base, and 1 to 20 equivalents of pyridine, DMAP, DBU, DBN, or N,N-diisopropylethylamine may be preferably used, and DMAP may be further preferably used. The reaction temperature is within the range of -30 to 100°C, and the reaction time is 0.5 to 24 hours.

Next, in the eighth step, conversion from the compound of the formula (10) to a compound of a formula (11) can be carried out by the following method, for example. Specifically, the compound of the formula (11) can be produced by reacting the compound of the formula (10) with 0.5 to 30 equivalents of dialkyl carbonate in a DMF solvent in the presence of a base. The reaction solvent in this reaction may be any of general organic solvents inert to the reaction instead of DMF, or a mixed solvent of these may be used. The reaction solvent is preferably DMF, DMSO, NMP, acetonitrile, diethyl ether, cyclopropyl methyl ether, dioxane, dimethoxyethane, or THF. The reaction solvent is further preferably DMF, DMSO, dioxane, or THF. The base may be a generally known inorganic base, 1 to 50 equivalents of sodium hydride, lithium hydride, or calcium hydride may be preferably used, and sodium hydride may be further preferably used. The dialkyl carbonate may be dimethyl carbonate, diethyl carbonate, dipropyl carbonate, diisopropyl carbonate, di-n-butyl carbonate, di-tert-butyl carbonate, di-sec-butyl carbonate, diphenyl carbonate, dibenzyl carbonate, or the like, is preferably dimethyl carbonate, diethyl carbonate, or dipropyl carbonate, and is further preferably dimethyl carbonate or diethyl carbonate. The reaction temperature is within the range of -10 to 150°C, and the reaction time is 0.5 to 48 hours.

### Scheme 4

### [Chem. 6]

In a case where A⁴ is an oxygen atom and A⁵ is E¹ in the formula (I),

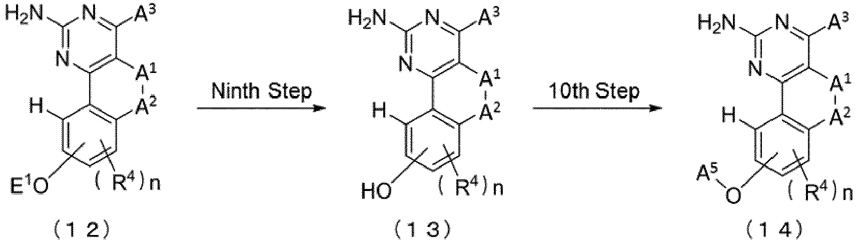

Fourthly, in a case where A⁴ is an oxygen atom and A⁵ is E¹ in the formula (I), a compound in which E¹ is an optionally substituted benzyl group is represented by a formula (12).

First, in the ninth step, conversion from the compound of the formula (12) to a compound of a formula (13) can be carried out by the following method, for example. Specifically, the compound of the formula (13) can be produced by reacting the compound of the formula (12) with hydrogen in a methanol solvent in the presence of a palladium-carbon catalyst. The reaction solvent in this reaction may be any of general organic solvents inert to the reaction instead of methanol, or a mixed solvent of these may be used. The reaction solvent is preferably methanol, ethanol, propanol, butanol, or tert-butanol. The reaction solvent is further preferably methanol or ethanol. In addition, depending on the situation, an acid such as hydrochloric acid, acetic acid, formic acid, or sulfuric acid may be added as appropriate, and these may be used as a solvent. Moreover, a metal-carbon catalyst such as a platinum-carbon catalyst or a rhodium-carbon catalyst may be used instead of a palladium-carbon catalyst. The reaction may be carried out under pressurized hydrogen as appropriate. The reaction temperature is within the range of -30 to 150°C, and the reaction time is 0.5 to 48 hours.

Next, in the 10th step, conversion from the compound of the formula (13) to a compound of a formula (14) can be carried out by a method similar to that described in the fifth step, for example.

### Scheme 5

### [Chem. 7]

In a case where -A⁴-A⁵ is -N(PMB)₂ in the formula (I),

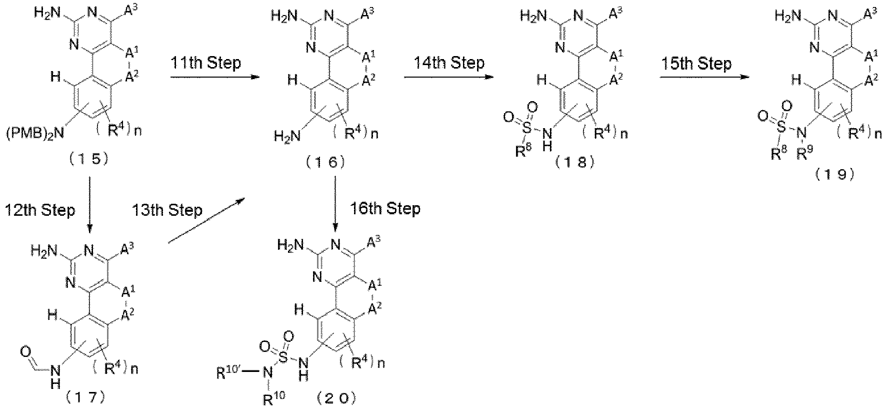

In the formulae, R⁸ represents an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₆₋₁₀ monocyclic or polycyclic arylalkyl group, or an optionally substituted C₆₋₁₀ monocyclic or polycyclic aryloxyalkyl group respectively in the optionally substituted C₃₋₁₀ cycloalkylsulfonyl group, the optionally substituted C₆₋₁₀ monocyclic or polycyclic arylsulfonyl group, the optionally substituted 3- to 10-membered monocyclic or bicyclic heterocyclylsulfonyl group, the optionally substituted C₆₋₁₀ monocyclic or polycyclic arylalkylsulfonyl group, or the optionally substituted C₆₋₁₀ monocyclic or polycyclic aryloxyalkylsulfonyl group represented by A⁵ in the formula (I), R⁹ represents the same group as R¹ in the formula (I), and R¹⁰ and R^{10'} together with N represent an optionally substituted 3- to 10-membered monocyclic or bicyclic heterocyclyl group in the optionally substituted 3- to 10-membered monocyclic or bicyclic heterocyclylsulfonyl group represented by A⁵ in the formula (I).

Fifthly, a compound in which -A⁴-A⁵ is -N(PMB)₂ in the formula (I) is represented by the formula (15). First, in the 11th step, conversion from the compound of the formula (15) to a compound of a formula (16) can be carried out by a method similar to that described in the ninth step, for example.

Next, in the 12th step, conversion from the compound of the formula (15) to a compound of a formula (17) can be produced by the following method, for example. Specifically, the compound of the formula (17) can be produced by reacting the compound of the formula (15) with hydrogen in an ethanol solvent in the presence of formic acid and a palladium-carbon catalyst. The reaction solvent in this reaction may be any of general organic solvents inert to the reaction instead of ethanol, or a mixed solvent of these may be used. The reaction solvent is preferably methanol, ethanol, propanol, butanol, or tert-butanol. The reaction solvent is further preferably methanol or ethanol. Formic acid may be used also as a solvent. In addition, a metal-carbon catalyst such as a platinum-carbon catalyst or a rhodium-carbon catalyst may be used instead of a palladium-carbon catalyst. The reaction may be carried out under hydrogen pressure as appropriate. The reaction temperature is within the range of -30 to 150°C, and the reaction time is 0.5 to 48 hours.

Next, in the 13th step, conversion from the compound of the formula (17) to a compound of a formula (16) can be carried out by the following method, for example. Specifically, the compound of the formula (16) can be produced by reacting the compound of the formula (17) with hydrochloric acid in an ethanol solvent. The reaction solvent in this reaction may be any of general organic solvents inert to the reaction instead of ethanol, or a mixed solvent of these may be used. The reaction solvent is preferably methanol, ethanol, propanol, butanol, or tert-butanol. The reaction solvent is further preferably methanol or ethanol. The reaction temperature is within the range of 0 to 100°C, and the reaction time is 0.5 to 48 hours.

Next, in the 14th step, conversion from the compound of the formula (16) to a compound of a formula (18) can be carried out by the following method, for example. Specifically, the compound of the formula (18) can be produced by reacting the compound of the formula (16) with 0.5 to 10 equivalents of any of various sulfonylation reagents in a chloroform solvent in the presence of a base. The reaction solvent in this reaction may be any of general organic solvents inert to the reaction instead of chloroform, or a mixed solvent of these may be used. The reaction solvent is preferably chloroform, 1,2-dichloroethane, methylene chloride, diethyl ether, cyclopropyl methyl ether, dioxane, dimethoxyethane, or THF. The reaction solvent is further preferably chloroform, 1,2-dichloroethane, dioxane, or THF. The base may be a generally known organic base, 1 to 20 equivalents of pyridine, DMAP, DBU, DBN, triethylamine, or N,N-diisopropylethylamine may be preferably used, and pyridine or DMAP may be further preferably used. The reaction temperature is within the range of -30 to 100°C, and the reaction time is 0.5 to 24 hours. In addition, a bis-sulfonamide generated by excess reaction can be converted to a mono-sulfonamide (the compound of the formula (18)) by adding 0.5 to 10 equivalents of tetra-n-butylammonium fluoride depending on the situation.

Next, in the 15th step, conversion from the compound of the formula (18) to a compound of a formula (19) can be carried out by a method similar to that described in the fifth step, for example.

Next, in the 16th step, conversion from the compound of the formula (16) to a compound of a formula (20) can be carried out by the following method, for example. Specifically, the compound of the formula (20) can be produced by reacting the compound of the formula (16) with 1 to 10 equivalents of a sulfonylation reagent represented by R¹⁰(R^{10'})NS(O)₂Cl in a chloroform solvent in the presence of a base. The reaction solvent in this reaction may be any of general organic solvents inert to the reaction instead of chloroform, or a mixed solvent of these may be used. The reaction solvent is preferably chloroform, 1,2-dichloroethane, methylene chloride, diethyl ether, cyclopropyl methyl ether, dioxane, dimethoxyethane, THF, pyridine, DMF, NMP, DMSO, or acetonitrile. The base may be a generally known organic base, 1 to 20 equivalents of pyridine, DMAP, DBU, DBN, triethylamine, or N,N-diisopropylethylamine may be preferably used, and pyridine or DMAP may be further preferably used. The reaction temperature is within the range of -30 to 100°C, and the reaction time is 0.5 to 48 hours.

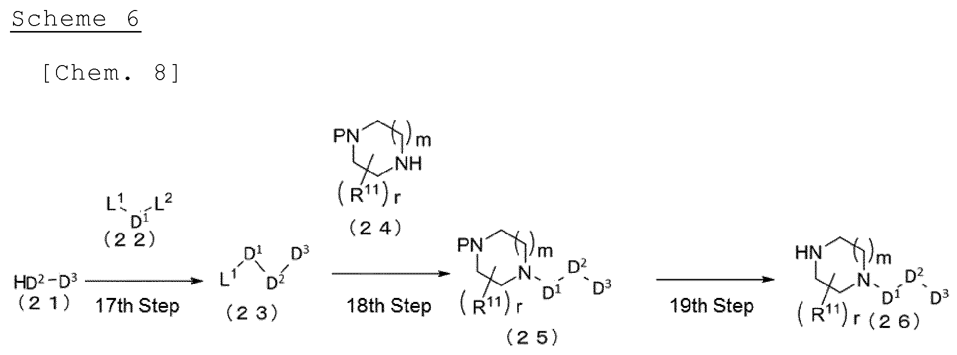

In the formulae, R¹¹ represents at least one of -N(R¹)R², -N(R¹)R³, and -R³, r represents 0 or an integer capable of binding to carbon atoms in the cyclic amine moiety of piperazine or homopiperazine which is part of the substituent A³ in the formula (I), and m represents 1 or 2. P is a protecting group for nitrogen atoms, which has been known in PROTECTIVE GROUPS in ORGANIC SYNTHESIS by Greene Wuts, and the like, and represents, Boc, Cbz, or the like, for example. In addition, in the formulae, the group represented by -D²-D³ may be a group represented by -ON=D⁴.

Sixthly, a compound in which A³ is a group obtained by removing a hydrogen atom from the compound of the formula (26) in the formula (I) is represented by a formula (27) described in Scheme 7 described below. These compounds include compounds (the compounds of the formula (27)) that are difficult to efficiently produce because of its production, purification, or the like. Regarding these compounds (the compounds of the formula (27)), the compounds of the formula (27), which are encompassed by the compound of the formula (I), can be produced by the methods described in Schemes 6 and 7 as alternatives of the Scheme 1. In addition, a compound of a formula (21), a compound of a formula (22), and a compound of a formula (30) are compounds which can be obtained by purchase or by employing publicly-known techniques known in documents, books, and the like.

First, in the 17th step, conversion from the compound of the formula (21) to a compound of a formula (23) can be carried out by a method similar to that described below, for example. Specifically, the compound of the formula (23) can be produced by reacting the compound of the formula (21) with 0.5 to 50 equivalents of the compound of the formula (22) in a toluene solvent in the presence of a base. The reaction solvent in this reaction may be any of general organic solvents inert to the reaction instead of toluene, or a mixed solvent of these may be used. The reaction solvent is preferably toluene, benzene, xylene, diethyl ether, cyclopropyl methyl ether, dioxane, dimethoxyethane, or THF. The base may be a generally known organic base, 1 to 50 equivalents of pyridine, DMAP, DBU, DBN, or N,N-diisopropylethylamine may be preferably used, and N,N-diisopropylethylamine may be further preferably used. The reaction temperature is within the range of -30 to 150°C, and the reaction time is 0.5 to 48 hours.

Next, in the 18th step, conversion from the compound of the formula (23) to a compound of a formula (25) can be carried out by the following method, for example. Specifically, the compound of the formula (25) can be produced by reacting the compound of the formula (23) with 0.5 to 50 equivalents of a compound of a formula (24) in a DMF solvent in the presence of a base. The reaction solvent in this reaction may be any of general organic solvents inert to the reaction instead of DMF, or a mixed solvent of these may be used. The reaction solvent is preferably DMF, DMSO, NMP, benzene, xylene, diethyl ether, cyclopropyl methyl ether, dioxane, dimethoxyethane, or THF. The reaction solvent is further preferably DMF, DMSO, or NMP. The base may be a generally known organic base, 1 to 50 equivalents of pyridine, DMAP, DBU, DBN, or N,N-diisopropylethylamine may be preferably used, and N, N-diisopropylethylamine may be further preferably used. The reaction temperature is within the range of -30 to 150°C, and the reaction time is 0.5 to 48 hours.

Next, in the 19th step, conversion from the compound of the formula (25) to a compound of a formula (26) can be carried out by a method for removing a protecting group for nitrogen atoms, which has been known in documents, books (JIKKEN KAGAKU KOUZA or PROTECTIVE GROUPS in ORGANIC SYNTHESIS), and the like as publicly-known techniques, for example. If an example is given, in a case where P is a Boc group, the compound can be produced by removing the Boc group in the formula (25) in a methylene chloride solvent in the presence of trifluoroacetic acid. The reaction solvent in this reaction may be any of general organic solvents inert to the reaction instead of methylene chloride, or a mixed solvent of these may be used. The reaction solvent is preferably methylene chloride, DMSO, NMP, methanol, ethanol, benzene, xylene, diethyl ether, dioxane, dimethoxyethane, THF, 1,2-dichloroethane, or methylene chloride. The acid may be hydrochloric acid, sulfuric acid, or the like instead of trifluoroacetic acid. The reaction temperature is within the range of -30 to 50°C, and the reaction time is 0.5 to 48 hours.

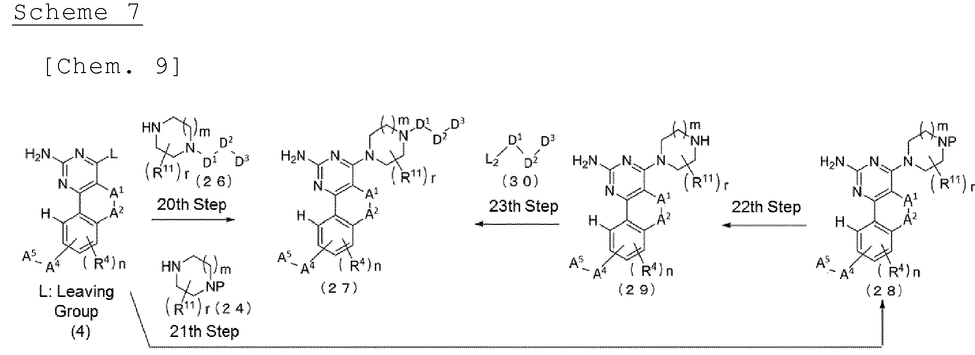

Next, in the 20th step, conversion from the compound of the formula (4) to a compound of a formula (27) can be carried out by a method similar to that described in the fourth step, for example. Next, in the 21st step, conversion from the compound of the formula (4) to a compound of a formula (28) can be carried out by a method similar to that described in the fourth step, for example. Next, in the 22nd step, conversion from the compound of the formula (28) to a compound of a formula (29) can be carried out by a method similar to that described in the 19th step, for example. Next, in the 23rd step, conversion from the compound of the formula (29) to a compound of a formula (27) can be carried out by a method similar to that described in the 18th step, for example.

### Scheme 8

### [Chem. 10]

In a case where -D³ is -X¹-C(O)O-R¹² in the formula (27),

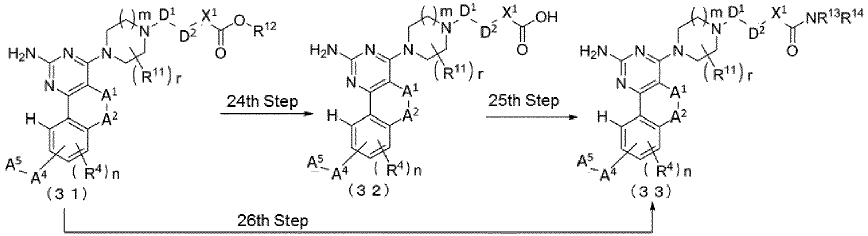

Seventhly, a compound in which -D³ is represented by -X¹-C(O)O-R¹² in the formula (27) is represented by a formula (31) described in Scheme 8, and X¹ represents a group substituted with -C(O)O-R¹² among an optionally substituted C₁₋₆ alkyl group, an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₁₋₆ alkyloxyalkyl group, a 3- to 10-membered monocyclic or bicyclic heterocyclyloxyalkyl group, an optionally substituted C₁₋₆ alkylthioalkyl group, and an optionally substituted C₁₋₆ alkylsulfonylalkyl group represented by D³ in the formula (I), R¹² represents a protecting group for a carboxyl group, and R¹³ and R¹⁴ may be the same or different and each represents a hydrogen atom, an alkyl group, a hydroxyl group, an alkylsulfonyl group, or the like.

First, in the 24th step, conversion from the compound of the formula (31) to a compound of a formula (32) can be carried out by the following method, for example. Specifically, the compound of the formula (32) can be produced by hydrolyzing the compound of the formula (31) in a mixed solvent of THF, methanol, and water in the presence of a base. The reaction solvent in this reaction may be a mixed solvent obtained by appropriately selecting one or two from polar solvents such as DMF, DMSO, NMP, dimethylacetamide, methanol, ethanol, propanol, n-butanol, sec-butanol, tert-butanol, diethyl ether, diisopropyl ether, THF, dioxane, diphenyl ether, and cyclopropyl methyl methyl ether and combining the selected polar solvents with water instead of a mixed solvent of THF, methanol, and water, and is preferably THF/methanol/water, THF/water, methanol/water, dioxane/methanol/water, dioxane/water, ethanol/water, or dioxane/ethanol/water. The reaction solvent is further preferably THF/methanol/water, THF/water, methanol/water, or dioxane/methanol/water. The base may be sodium hydroxide, lithium hydroxide, potassium hydroxide, barium hydroxide, calcium hydroxide, or the like, and is preferably sodium hydroxide, lithium hydroxide, or potassium hydroxide, and 1 to 20 equivalents thereof may be used. The reaction temperature is within the range of -30 to 150°C, and the reaction time is 0.5 to 48 hours.

Next, in the 25th step, conversion from the compound of the formula (32) to a compound of a formula (33) can be carried out by the following method, for example. The compound of the formula (33) can be produced by reacting the compound of the formula (32) with 1 to 20 equivalents of an amine represented by HN(R¹³)R¹⁴ in a DMF solvent in the presence of 1-ethyl-3-(3-dimethylaminopropyl)carbodiimide hydrochloride as a condensation reagent and 1-hydroxybenzotriazole as an additive. The reaction solvent in this reaction may be a general solvent instead of DMF, or a mixed solvent of these may be used. The reaction solvent is preferably DMSO, NMP, DMF, THF, diethyl ether, or dioxane. In addition, as the condensation reagent, 1 to 10 equivalents of N,N'-dicyclohexylcarbodiimide, N,N'-diisopropylcarbodiimide, N-cyclohexyl-N' (-2-morpholinoethyl)carbodiimide-p-tolu enesulfonate, 4-(4,6-dimethoxy-1,3,5-triazin-2-yl)-4-methylmorpholin ium chloride · n-hydrate, (4,6-dimethoxy-1,3,5-triazin-2-yl)-(2-octyloxy-2-oxoet hyl)dimethylammonium trifluoromethanesulfonate, 1H-benzotriazol-1-yloxytris(dimethylamino)phosphonium hexafluorophosphate, 1H-benzotriazol-1-yloxytripyrrolizinophosphonium hexafluorophosphate, O-(7-azabenzotriazol-1-yl)-N,N,N',N'-tetramethyluroniu m hexafluorophosphate, O-(7-azabenzotriazol-1-yl)-N,N,N',N'-tetramethyluroniu m hexafluorophosphate, O-(N-succinimidyl)-N,N,N',N'-tetramethyluronium tetrafluoroborate, or O-(3,4-dihydro-4-oxo-1,2,3-benzotriazin-3-yl)-N,N,N',N '-tetramethyluronium tetrafluoroborate may be used instead of 1-ethyl-3-(3-dimethylaminopropyl)carbodiimide hydrochloride. As the additive, 1 to 10 equivalents of 3H-1,2,3-triazolo[4,5-b]pyridin-3-ol, or the like may be used instead of 1-hydroxybenzotriazole. The reaction temperature is within the range of 0 to 150°C, and the reaction time is 0.5 to 48 hours.

Next, in the 26th step, conversion from the compound of the formula (31) to a compound of a formula (33) can be carried out by the following method, for example. Specifically, the compound of the formula (33) can be produced by reacting the compound of the formula (31) with 1 to 100 equivalents of an amine corresponding to HN(R¹³)R¹⁴ in a methanol solvent. The reaction solvent in this reaction may be any of general organic solvents inert to the reaction instead of methanol, or a mixed solvent of these may be used. The reaction solvent is preferably DMF, DMSO, NMP, methanol, ethanol, propanol, diethyl ether, dioxane, or THF, and further preferably DMSO, NMP, methanol, ethanol, or propanol. The reaction temperature is within the range of -30 to 150°C, and the reaction time is 0.5 to 48 hours.

### Scheme 9

### [Chem. 11]

In a case where -D³ is represented by an azetidin-3-ylmethyl group having a protecting group (P) in the formula (27),

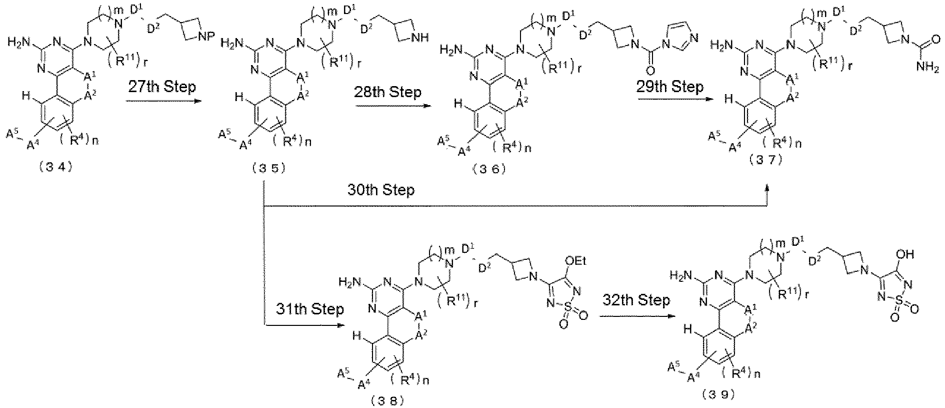

Eighthly, a compound in which -D³ is represented by an azetidin-3-ylmethyl group having a protecting group (P) in the formula (27) is represented by a formula (34) described in Scheme 9. First, in the 27th step, conversion from the compound of the formula (34) to a compound of a formula (35) can be carried out by a method similar to that described in the 19th step, for example.

Next, in the 28th step, conversion from the compound of the formula (35) to a compound of a formula (36) can be carried out by the following method, for example. Specifically, the compound of the formula (36) can be produced by reacting the compound of the formula (35) with 1 to 10 equivalents of 1,1'-carbonyldiimidazole in a DMF solvent. The reaction solvent in this reaction may be a general polar solvent inert to the reaction instead of DMF, and is preferably DMSO, NMP, DMF, THF, dioxane, or diethyl ether. The reaction temperature is within the range of 0 to 100°C, and the reaction time is 0.5 to 24 hours.

Next, in the 29th step, conversion from the compound of the formula (36) to a compound of a formula (37) can be carried out by the following method, for example. Specifically, the compound of the formula (37) can be produced by reacting the compound of the formula (36) in a DMF solvent in the presence of ammonia water. The reaction solvent in this reaction may be a general polar solvent inert to the reaction instead of DMF, and is preferably DMSO, NMP, DMF, THF, dioxane, or diethyl ether. The reaction temperature is within the range of 0 to 100°C, and the reaction time is 0.5 to 24 hours.

Next, in the 30th step, conversion from the compound of the formula (35) to a compound of a formula (37) can be carried out by the following method, for example. Specifically, the compound of the formula (37) can be produced by reacting the compound of the formula (35) with 1 to 10 equivalents of trimethylsilyl isocyanate in a methylene chloride solvent in the presence of a base. The reaction solvent in this reaction may be any of general organic solvents inert to the reaction instead of DMF, may also be a mixed solvent, and is preferably 1,2-dichloroethane, chloroform, DMSO, NMP, DMF, THF, dioxane, or diethyl ether. The base may be a general organic base inert to the reaction, and is preferably DBU, DBN, triethylamine, diisopropylethylamine, pyridine, or DMAP. The reaction temperature is within the range of 0 to 100°C, and the reaction time is 0.5 to 24 hours.

Next, in the 31st step, conversion from the compound of the formula (35) to a compound of a formula (38) can be carried out by the following method, for example. Specifically, the compound of the formula (38) can be produced by reacting the compound of the formula (35) with 1 to 10 equivalents of 3,4-ethoxy-1,2,5-thiadiazo1-1,1-dioxide in a THF solvent. The reaction solvent in this reaction may be any of general organic solvents inert to the reaction instead of THF, or may be a mixed solvent of these, and is preferably THF, diethyl ether, dioxane, or dimethoxyethane. The reaction temperature is within the range of 0 to 100°C, and the reaction time is 0.5 to 24 hours.

Next, in the 32nd step, conversion from the compound of the formula (38) to a compound of a formula (39) can be carried out by the following method, for example. Specifically, the compound of the formula (39) can be produced by reacting the compound of the formula (38) in a mixed solvent of acetonitrile, THF, and water. The reaction solvent in this reaction may be a mixed solvent of a polar aprotic solvent, an ether-based solvent, and water instead of the mixed solvent of acetonitrile, THF, and water, and is preferably acetonitrile/THF/water. The reaction temperature is within the range of 0 to 150°C, and the reaction time is 0.5 to 48 hours.

### Scheme 10

In a case where -D³ is represented by -X²-O-CH₂-CN in the formula (27),

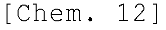

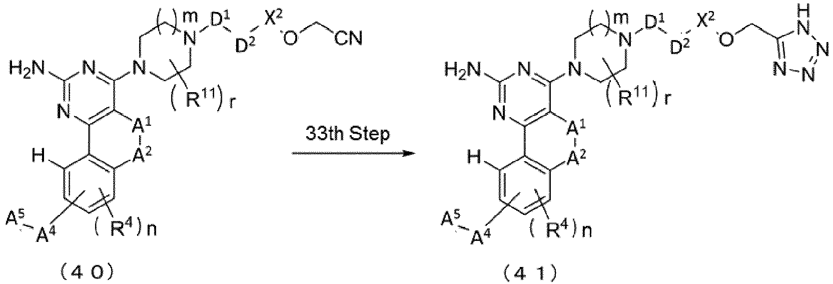

Ninthly, a compound in which -D³ is represented by -X²-O-CH₂-CN in the formula (27) is represented by a formula (40) described in Scheme 10, and X² represents a C₁₋₆ alkyl group or a C₁₋₆ alkyloxyalkyl group of an optionally substituted C₁₋₆ alkyl group or an optionally substituted C₁₋₆ alkyloxyalkyl group represented by D³ in the formula (I). In the 33rd step, conversion from the compound of the formula (40) to a compound of a formula (41) can be carried out by the following method, for example. Specifically, the compound of the formula (40) can be produced by reacting the compound of the formula (40) with 1 to 10 equivalents of sodium azide in a DMF solvent in the presence of an ammonium salt. The reaction solvent in this reaction may be any of general organic solvents inert to the reaction instead of DMF, or may be a mixed solvent, and is preferably DMSO, NMP, or DMF. As the ammonium salt, 1 to 10 equivalents of triethylamine hydrochloride, ammonium chloride, or the like may be used. The reaction temperature is within the range of 0 to 150°C, and the reaction time is 0.5 to 48 hours.

### Scheme 11

### [Chem. 13]

In a case where -D³ is represented by -X³-phthalimide in the formula (27),

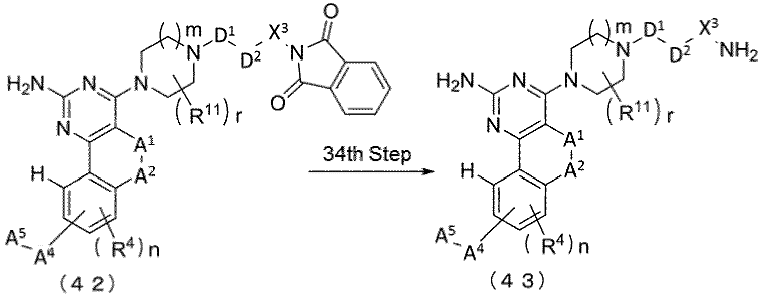

Tenthly, a compound in which -D³ is represented by -X³-phthalimide in the formula (27) is represented by a formula (42) described in Scheme 11, and X³ is a C₁₋₆ alkyl group or a C₁₋₆ alkyloxyalkyl group of an optionally substituted C₁₋₆ alkyl group or an optionally substituted C₁₋₆ alkyloxyalkyl group represented by D³ in the formula (I). In the 34th step, conversion from the compound of the formula (42) to a compound of a formula (43) can be carried out by the following method, for example. Specifically, the compound of the formula (43) can be produced by reacting the compound of the formula (42) in an ethanol solvent in the presence of hydrazine monohydrate. The reaction solvent in this reaction may be any of general organic solvents inert to the reaction instead of ethanol, or may be a mixed solvent, and is preferably methanol, ethanol, propanol, or butanol. The reaction temperature is within the range of 0 to 150°C, and the reaction time is 0.5 to 48 hours.

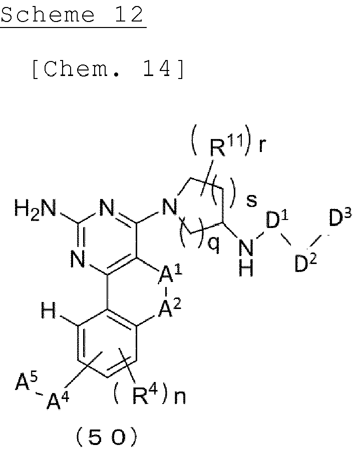

In the formula, q represents an integer of 1 or 2; when q is 1, s represents an integer of 1 or 2; when q is 2, s represents an integer of 1.

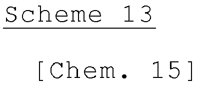

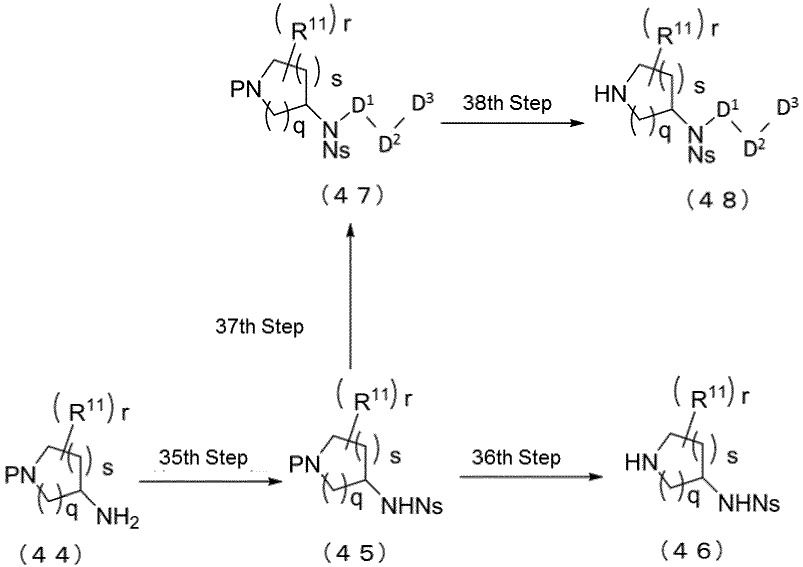

Eleventhly, there are some compounds of a formula (50) that are hard to efficiently prepare related to its production, purification, or the like, among the compounds (the compounds of the formula (50)). Regarding such compounds (the compounds of the formula (50)), the compounds of the formula (50), which are encompassed by the compound of the formula (I), can be produced by the methods described in Schemes 13 to 14 as alternatives. The compound of the formula (44) is a compound which can be obtained by purchase or by employing publicly-known techniques known in documents, books, and the like. The compound of the formula (50) can be produced by producing the compound of the formula (46) or the compound of the formula (48) using the compound of the formula (44) as a starting material, then producing the compound of the formula (49) by a method similar to that described in Scheme 7 using the compound of the formula (46) and the compound of the formula (48) respectively in place of the compound of the formula (24) and the compound of the formula (26) described in Scheme 7, and removing a nosyl group (Ns group) from the compound of the formula (49).

First, in the 35th step, conversion from the compound of the formula (44) to the compound of the formula (45) can be carried out by the following method, for example. Specifically, the compound of the formula (45) can be produced by reacting the compound of the formula (44) with 1 to 10 equivalents of o-nosylchloride in a methylene chloride solvent in the presence of a base. The reaction solvent in this reaction may be any of general organic solvents inert to the reaction instead of methylene chloride, or may be a mixed solvent thereof. The reaction solvent is preferably methylene chloride, 1,2-dichloroethane, chloroform, diethyl ether, dioxane, THF, DMSO, DMF, NMP, or acetonitrile. The reaction temperature is within the range of 0 to 100°C, and the reaction time is 0.5 to 48 hours.

Next, in the 36th step, conversion from the compound of the formula (45) to the compound of the formula (46) can be carried out by a method similar to that described in the 19th step, for example. Next, in the 37th step, conversion from the compound of the formula (45) to a compound of a formula (47) can be carried out by a method similar to that described in the 18th step, for example. Next, in the 38th step, conversion from the compound of the formula (47) to a compound of a formula (48) can be carried out by a method similar to that described in the 19th step, for example.

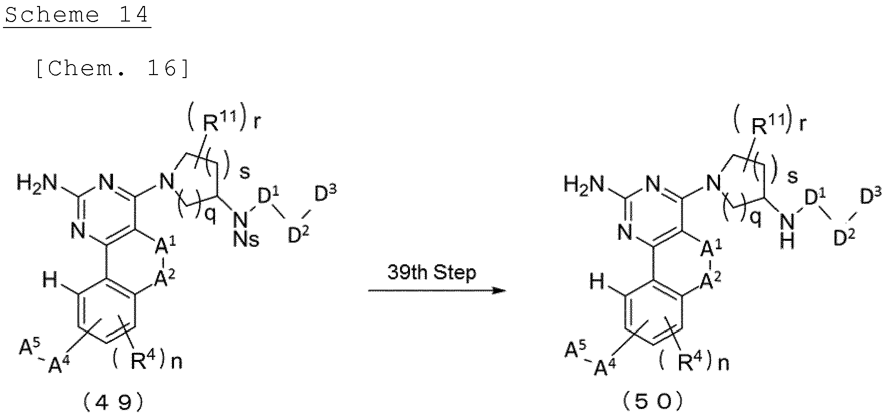

Next, in the 39th step, conversion from the compound of the formula (49) to the compound of the formula (50) can be carried out by the following method, for example. Specifically, the compound of the formula (50) can be produced by reacting the compound of the formula (49) with 1 to 20 equivalents of a generally known thiol compound in an acetonitrile solvent in the presence of a base. The reaction solvent in this reaction may be any of general organic solvents inert to the reaction instead of acetonitrile, or may be a mixed solvent thereof. The reaction solvent is preferably acetonitrile, DMSO, DMF, NMP, 1,2-dichloroethane, methylene chloride, chloroform, diethyl ether, dioxane, or THF. The base may be a generally known organic base or inorganic base, and is preferably, DBU, DBN, triethylamine, diisopropylethylamine, potassium carbonate, sodium carbonate, sodium hydrogen carbonate, or cesium carbonate. The above-described thiol compound may be any thiol compound known for its usage for the removal of the nosyl group in the literature and the like, and 1 to 20 equivalents of 2-mercaptoacetic acid, alkyl 2-mercaptoacetate, an optionally substituted benzenethiol, or an optionally substituted pyridinethiol is preferably used. The reaction temperature is within the range of -50 to 150°C, and the reaction time is 0.5 to 48 hours.

### Scheme 15

### [Chem. 17]

In a case where -D³ in the formula (50) is represented by -X¹-C(O)O-R¹²,

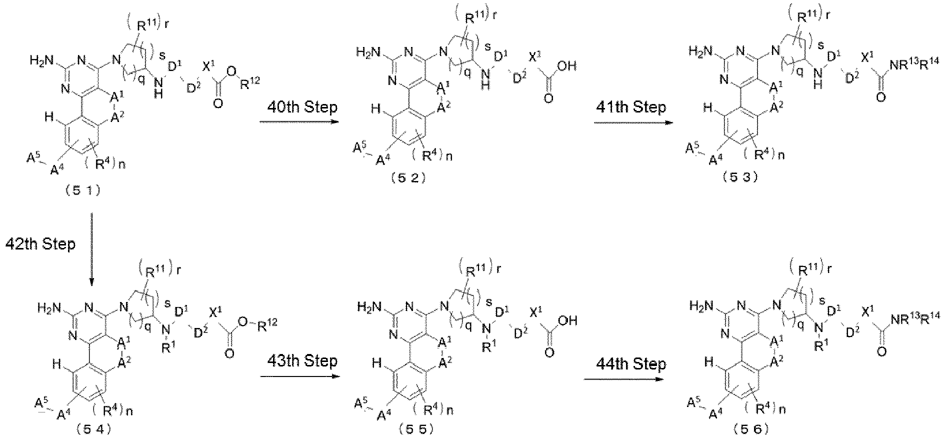

Twelfthly, a compound in which -D³ is represented by -X¹-C(O)O-R¹² in the formula (50) is represented by a formula (51) described in Scheme 15. Compounds of the formula (51) to the formula (56), which are described in Scheme 15, are compounds encompassed by the formula (I) .

First, in the 40th step, conversion from the compound of the formula (51) to a compound of a formula (52) can be carried out by a method similar to that described in the 24th step, for example. Next, in the 41st step, conversion from the compound of the formula (52) to a compound of a formula (53) can be carried out by a method similar to that described in the 25th step, for example.

Next, in the 42nd step, conversion from the compound of the formula (51) to a compound of a formula (54) can be carried out by using and reacting 1 to 10 equivalents of a corresponding ketone or aldehyde with the compound of the formula (51) in a chloroform solvent in the presence of an acid and a reducing agent, for example. The reaction solvent in this reaction may be any of general organic solvents inert to the reaction instead of chloroform, or may be a mixed solvent thereof. The reaction solvent is preferably methylene chloride, 1,2-dichloroethane, chloroform, THF, diethyl ether, dioxane, methanol, ethanol, or butanol. As the acid, acetic acid or hydrochloric acid is preferably used. The reducing agent may be a generally known reducing agent, and preferably sodium triacetoxyborohydride, 1 to 10 equivalents of which may be used. The reaction temperature is within the range of 0 to 120°C, and the reaction time is 0.5 to 10 hours.

Next, in the 43rd step, conversion from the compound of the formula (54) to a compound of a formula (55) can be carried out by a method similar to that described in the 24th step, for example. Next, in the 44th step, conversion from the compound of the formula (55) to a compound of a formula (56) can be carried out by a method similar to that described in the 25th step, for example.

Thirteenthly, a compound in which A³ is a cyclic amine other than a piperazinyl group or a pyrrolidinyl group in the formula (I) can also be produced by the above-described methods.

The intermediates and the compounds of the formula (I) synthesized by the above-described production methods can sometimes be used for the next steps in a state of a reaction solution or as a crude product. Alternatively, the intermediates and the compounds of the formula (I) may be isolated by a normal purification method. The purification can be carried out easily, for example, by appropriately selecting or combining recrystallization, reprecipitation, solvent extraction, distillation, various types of filtration (Celite, ultrafiltration membrane, and the like) various types of column chromatography (normal-phase chromatography, reversed-phase chromatography, hydrophilic interaction chromatography (abbreviated as HILIC), various types of silica gel with a chiral column and the like, and adsorptive resins), centrifugation, and the like.

The compound represented by the general formula (I) and a pharmacologically acceptable salt thereof of the present invention have excellent affinity to the histamine H1 receptor and the histamine H4 receptor, and have a dual modulatory function capable of modulating the activity of both of the histamine H1 receptor and the histamine H4 receptor. For this reason, the compound represented by the general formula (I) and a pharmacologically acceptable salt thereof of the present invention are capable of functioning as a dual modulator for the histamine H1 receptor and the histamine H4 receptor, that is, capable of dually modulating the histamine H1 receptor and the histamine H4 receptor (that is, dually modulating the behavior of the histamine H1 receptor and the histamine H4 receptor to histamine) . Note that the term "modulator" in the present invention indicates a compound that can function as an agonist capable of bonding to a receptor (the histamine H1 receptor and the histamine H4 receptor in the present invention) to partially or fully activate the receptor, a compound that can function as an antagonist capable of partially or fully inhibiting the activated state of the receptor, and/or a compound that can function as an inverse agonist capable of inhibiting the constitutive activity of the receptor.

Moreover, the compound represented by the general formula (I) and a pharmacologically acceptable salt thereof of the present invention can function as an excellent antagonist against the histamine H1 receptor and the histamine H4 receptor.

Accordingly, the compound represented by the general formula (I) and a pharmacologically acceptable salt thereof of the present invention are useful in treating and/or preventing various allergies and inflammatory diseases involving the histamine H1 and/or H4 receptors. Diseases that involve the histamine H1 and/or H4 receptors includes, for example, skin diseases such as hives, pruritus, insect bites, atopic dermatitis, allergic dermatitis, contact dermatitis, and psoriasis vulgaris, respiratory diseases such as allergic rhinitis, nonallergic rhinitis, bronchial asthma, and pulmonary fibrosis, eye diseases such as allergic conjunctivitis and atopic conjunctivitis, nervous system diseases such as dizziness and vestibular disorder, and inflammatory diseases such as arthritis, rheumatism, and Crohn's disease, and the like, but are not limited to only these.

Hence, the present invention provides a pharmaceutical composition containing at least one selected from the group consisting of compounds represented by the general formula (I) and pharmacologically acceptable salts thereof (hereinafter, sometimes referred to simply as "compounds represented by the general formula (I) and/or salts thereof"), preferably, a pharmaceutical composition for dually modulating the histamine H1 receptor and the histamine H4 receptor, and/or, a pharmaceutical composition for treating diseases attributable to the histamine H1 receptor and/or the histamine H4 receptor, as well as a modulation method and a treatment method using these.

The pharmaceutical composition of the present invention contains a compound represented by the general formula (I) and/or a salt thereof as an active ingredient, and may be administered through any route of oral or parenteral administration, such as for example, inhalation administration, intranasal administration, eye drop, subcutaneous administration, intravenous injection, intramuscular injection, rectal administration, and transdermal administration, and can be administered to a human or an animal other than a human. Accordingly, the pharmaceutical composition of the present invention can be formed into pharmaceutical preparations formulated appropriately for the route of administration.

Examples of the above-described formulation specifically include oral preparations such as tablets, pills, capsules, granules, powders, subtle granules, troche tablets, elixirs, suspensions, emulsion concentrates, and syrups; solutions for external application such as inhalants, nasal drops, and eye drops; injections such as intravenous injections and intramuscular injections; rectally administered agent; suppositories; liniments such as lotions, sprays, ointments, and creams; and parenteral preparations such as patches.

These various pharmaceutical preparations can be produced by conventional methods using additives normally used in the field of pharmacy, such as excipients, expanders, wetting agents, surfactants, collapsing agents, binders, lubricants, dispersants, buffers, preservatives, solubilizers, antiseptics, flavoring deodorants, soothing agents, stabilizers, lubricants, and colorants. Accordingly, the pharmaceutical composition of the present invention may further contain these additives. The above-described additives are preferably nontoxic, and include lactose, fructose, glucose, starch, gelatin, magnesium carbonate, synthetic magnesium silicate, talc, magnesium stearate, methyl cellulose, carboxymethyl cellulose or a salt thereof, gum Arabic, olive oil, propylene glycol, polyethylene glycol, syrup, petrolatum, glycerine, ethanol, citric acid, sodium chloride, sodium sulfite, sodium phosphate, and the like, for example.

In the pharmaceutical composition of the present invention, the content of a compound represented by the general formula (I) and/or a salt thereof (the total content of the compounds in the case of a mixture of two or more) varies depending on its formulation, but usually 0.01 to 70% by mass, and preferably 0.05 to 50% by mass, based on the total mass of the pharmaceutical composition when converted into a free base. The dosage is determined as appropriate depending on each individual case in consideration of the usage, the age, the weight, the sex, the difference in disease, and the degree of symptoms of the patient, and the like. Usually, the dosage is 0.1 to 2000 mg, and preferably 1 to 1000 mg, per day for an adult, and this dosage is administered once or separately several times a day.

### [Examples]

Hereinafter, the present invention is described in further detail based on Examples; however these Examples are mere illustrations, the present invention is not limited to these Examples, and it goes without saying that various changes and modifications may be made without departing from the scope of the present invention. In addition, methods of producing starting material compounds used in Examples are described as Reference Examples.

Hereinafter, abbreviations used in Examples and Reference Examples mean as follows:
HPLC: High-Performance Liquid Chromatography,
ESI: Electrospray Ionization,
MS: Mass Spectrum,
MW: Microwave, and
¹H-NMR: Nuclear Magnetic Resonance Spectrum (internal standard: tetramethylsilane (TMS)). In addition, in the following structural formulae, a part with no description of a group at the end of a single bond and Me both represent a methyl group, Et represents an ethyl group, Ts represents a tosyl group, Tf represents a trifluoromethanesulfonyl group, and tBu represents a tert-butyl group.

### (Reference Example 1)

### (a) 6-((4-chlorobenzyl)oxy)-3,4-dihydronaphthalen-1(2H)-on e

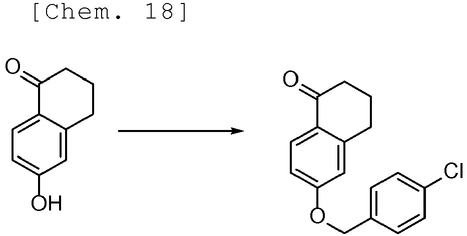

6-Hydroxy-3,4-dihydronaphthalen-1(2H)-one(2.0 g, 12.3 mmol) and 1-(bromomethyl)-4-chlorobenzene (3.0 g, 14.8 mmol, 1.2 equivalents) were dissolved into acetone (25 mL), and potassium carbonate (3.4 g, 24.66 mmol, 2.0 equivalents) was added, followed by heating under reflux for 4 hours. Water was added to the reaction liquid, and the product was extracted with ethyl acetate. The organic layer was then washed with brine and dried over anhydrous sodium sulfate. Thereafter, the solvent was distilled off under reduced pressure to obtain a crude product. This crude product was purified through a silica gel column chromatography (eluent, ethyl acetate : hexane) to obtain 3.50 g of the title compound. ¹H-NMR (400 MHz, DMSO-d₆) δ: 2.06-2.15 (2H, m), 2.58-2.64 (2H, m), 2.92 (2H, t, J=6.1 Hz), 5.08 (2H, s), 6.76 (1H, d, J=2.6 Hz), 6.87 (1H, dd, J=2.6, 8.7 Hz), 7.33-7.38 (4H, m), 8.01 (1H, d, J=8.7 Hz). MS (ESI) m/z: 287 [M+H]⁺

### (b) ethyl 6-((4-chlorobenzyl)oxy)-1-oxo-1,2,3,4-tetrahydronaphth alene-2-carboxylate

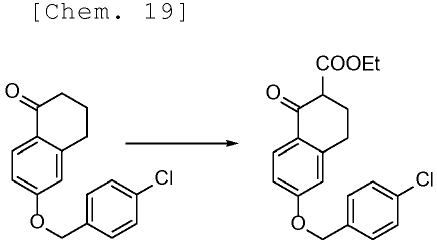

The compound (3.45 g, 12.0 mmol) obtained in Reference Example 1(a) was dissolved into THF (24 mL), and sodium hydride (60%, dispersed in liquid paraffin) (1.44 g, 36.1 mmol, 3.0 equivalents) was added, followed by stirring at room temperature for 1 hour. Thereafter, dimethyl carbonate (2.53 mL, 30.1 mmol, 2.5 equivalents) was added, followed by heating under reflux for 2 hours. Under ice cooling, a saturated ammonium chloride aqueous solution was added to the reaction liquid, and the product was extracted with ethyl acetate. The organic layer was then washed with brine and dried over anhydrous sodium sulfate. Thereafter, the solvent was distilled off under reduced pressure to obtain a crude product. This crude product was subjected to a silica gel column chromatography (eluent, ethyl acetate:hexane) to obtain 3.89 g of the title compound.
¹H-NMR (400 MHz, CDCl₃) δ: 2.27-2.37 (0.71H, m), 2.42-2.59 (1.29H, m), 2.74-2.81 (0.71H, m), 2.85-3.07 (1.29H, m), 3.59 (0.36H, dd, J=4.9, 10.1 Hz), 3.78 (1.9H, s), 3.81 (1.1H, s), 5.06 (0.7H, s), 5.09 (1.3H, s), 5.06 (0.7H, s), 5.09 (1.3H, s), 6.74-6.78 (1H, m), 6.83-6.92 (1H, m), 7.33-7.40 (4H, m), 7.74 (0.36H, d, J=8.6 Hz), 8.03 (6, 4H, d, J=8.6 Hz).
MS (ESI) m/z: 359 [M+H]⁺

### (c) 2-amino-8-((4-chlorobenzyl)oxy)-5,6-dihydrobenzo[h]qui nazolin-4-ol

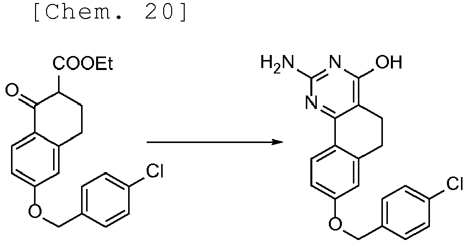

The compound (3.0 g, 8.70 mmol) obtained in Reference Example 1(b) was dissolved into NMP (17.4 mL), and guanidine hydrochloride (2.49 g, 26.1 mmol, 3.0 equivalents), potassium carbonate (4.21 g, 30.5 mmol, 3.5 equivalents), and N,N-diisopropylethylamine (1.52 mL, 8.70 mmol) were added, followed by stirring in an oil bath at 165°C for 2 hours. Water was added to the reaction liquid and insolubles thus generated were collected by filtration, followed by washing with diethyl ether and water and drying under reduced pressure to obtain 1.66 g of title compound.
¹H-NMR (400 MHz, DMSO-d₆) δ: 2.43-2.52 (2H, m), 2.71-2.80 (2H, m), 5.14 (2H, s), 6.36 (2H, brs), 6.88-6.96 (2H, m), 7.44-7.54 (4H, m), 7.91 (1H, d, J=8.1 Hz).
MS (ESI) m/z: 354 [M+H]⁺

### (d) 4-chloro-8-((4-chlorobenzyl)oxy)-5,6-dihydrobenzo[h]qu inazolin-2-amine

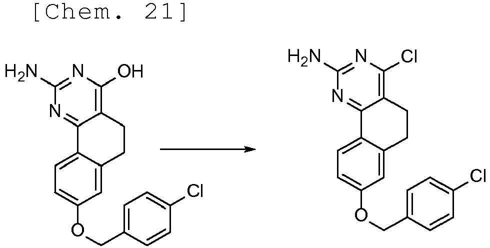

The compound (7.48 g, 20.1 mmol) obtained in Reference Example 1(c) was suspended into toluene (150 mL), and phosphorus oxychloride (19.7 mL, 211 mmol, 10.5 equivalents) was added, followed by stirring in an oil bath at 90°C for 2 hours. The solvent was distilled off under reduced pressure and the residue was dissolved into chloroform. After a saturated sodium hydrogen carbonate aqueous solution was added, insolubles were removed by filtration. The organic layer of the filtrate was separated, washed with brine, and dried over anhydrous sodium sulfate. The solvent was distilled off under reduced pressure to obtain 4.15 g of the title compound. ¹H-NMR (400 MHz, DMSO-d₆) δ: 2.52-2.59 (2H, m), 2.73-2.80 (2H, m), 3.30 (3H, s), 5.16 (2H, s), 6.76 (2H, brs), 6.89-6.96 (2H, m), 7.44-7.54 (4H, m), 7.91 (1H, d, J=8.1 Hz).
MS (ESI) m/z: 372 [M+H]⁺

### (e) 2-amino-8-((4-chlorobenzyl)oxy)-5,6-dihydrobenzo[h]qui nazolin-4-yl 4-methylbenzenesulfonate

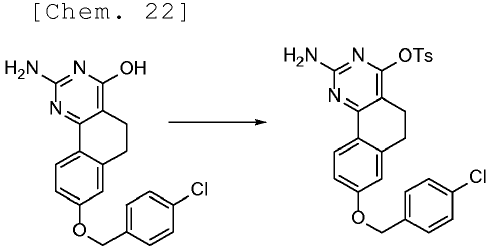

The compound (500 mg, 1.41 mmol) obtained in Reference Example 1(c) was suspended into chloroform, and p-toluenesulfonyl chloride (323 mg, 1.70 mmol, 1.2 equivalents), triethylamine (296 mL, 2.12 mmol, 1.5 equivalents), and N,N-dimethyl-4-aminopyridine (17 mg, 0.141 mmol, 10 mol%) were added, followed by stirring in a hot water bath at 60°C for 6 hours. Water was added, and insolubles were collected by filtration, followed by washing with water and ethyl acetate and drying under reduced pressure to obtain 410 mg of the title compound. ¹H-NMR (400 MHz, DMSO-d₆) δ: 2.52-2.59 (2H, m), 2.73-2.80 (2H, m), 3.30 (3H, s), 5.16 (2H, s), 6.76 (2H, brs), 6.94-7.01 (2H, m), 7.44-7.52 (6H, m), 7.99-8.04 (3H, m). MS (ESI) m/z: 508 [M+H]⁺.

### (Reference Example 2)

### 2-amino-8-(benzyloxy)-5,6-dihydrobenzo[h]quinazolin-4-yl 4-methylbenzenesulfonate

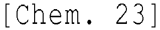

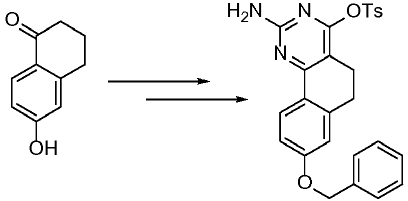

The title compound was obtained by the same method as in Reference Examples 1(a), (b), (c), and (e) except that 1-(bromomethyl)benzene was used in place of 1-(bromomethyl)-4-chlorobenzene in Reference Example 1(a).
¹H-NMR (400 MHz, DMSO-d₆) δ: 2.52-2.59 (2H, m), 2.74-2.81 (2H, m), 3.30 (3H, s), 5.16 (2H, s), 6.77 (2H, brs), 6.95-7.02 (2H, m), 7.32-7.52 (7H, m) 7.98-8.04 (3H, m). MS (ESI) m/z: 474 [M+H]⁺.

### (Reference Example 3)

### 4-(4-methylpiperazin-1-yl)-5,6-dihydrobenzo[h]quinazol ine-2,8-diamine

### (a) 6-(bis(4-methoxybenzyl)amino)-3,4-dihydronaphthalene-1 (2H)-one

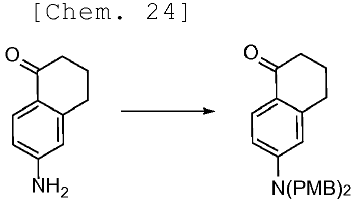

6-Amino-3,4-dihydronaphthalen-1(2H)-one (1.00 g) was dissolved into acetonitrile (20 mL), followed by stirring in an oil bath at 80°C for 112 hours while stepwisely adding 1-(chloromethyl)-4-methoxybenzene (4.18 mL, 4.6 equivalents) and cesium carbonate (9.09 g, 4.5 equivalents) little by little. The reaction liquid was cooled down to room temperature, and filtered through Celite, and then, a saturated sodium hydrogen carbonate aqueous solution was added and the product was extracted with ethyl acetate. The organic layer was washed with a saturated sodium chloride aqueous solution and dried over anhydrous magnesium sulfate. Thereafter, the solvent was distilled off reduced pressure to obtain a crude product. This crude product was purified through a silica gel column chromatography (eluent, ethyl acetate:hexane) to obtain 2.31 g of the title compound.
¹H-NMR (400 MHz, CDCl₃) δ: 2.00-2.10 (2H, m), 2.55 (2H, t, J=6.5 Hz), 2.80 (2H, t, J=6.1 Hz), 3.80 (6H, s), 4.61 (4H, s), 6.49 (1H, d, J=2.6 Hz), 6.67 (1H, dd, J=8.9, 2.6 Hz), 6.84-6.89 (4H, m), 7.08-7.15 (4H, m), 7.90 (1H, d, J=8.9 Hz).
MS (ESI) m/z: 402 [M+H]⁺

### (b) N⁸,N⁸-bis(4-methoxybenzyl)-4-(4-methylpiperazin-1-yl)-5,6-dihydrobenzo[h]quinazoline-2,8-diamine

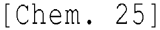

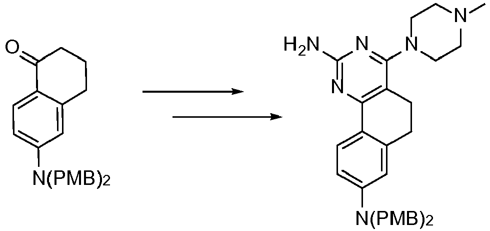

By using the compound obtained in Reference Example 3(a), the title compound was obtained by the same methods as in Reference Examples 1(b) to (d), and subsequently Example 1.
¹H-NMR (400 MHz, CDCl₃) δ: 2.30-2.36 (3H, m), 2.52 (4H, t, J=4.3 Hz), 2.58-2.65 (2H, m), 2.65-2.72 (2H, m), 3.29 (4H, t, J=4.3 Hz), 3.79 (6H, s), 4.58 (4H, s), 6.55 (1H, d, J=2.6 Hz), 6.71 (1H, dd, J=8.7, 2.6 Hz), 6.83-6.89 (4H, m), 7.14 (4H, m), 7.91 (1H, d, J=8.7 Hz).
MS (ESI) m/z: 551 [M+H]⁺

### (c) 4-(4-methylpiperazin-1-yl)-5,6-dihydrobenzo[h]quinazol in-2,8-diamine

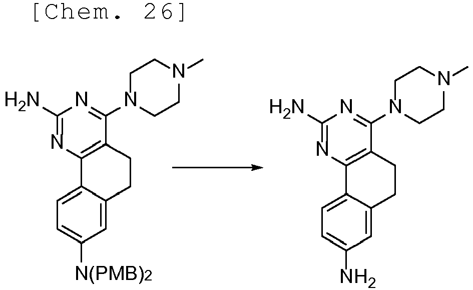

The compound (1.90 g) obtained in Reference Example 3(b) was dissolved into ethanol (19 mL) and formic acid (19 mL), and 10% palladium-carbon catalyst (1.90 g) was added. The resultant was heated in an oil bath at 80°C for 11 hours in an argon atmosphere. The reaction liquid was cooled down to room temperature and filtered through Celite, and thereafter the solvent was distilled off. The residue was dissolved into chloroform:2-propanol=4:1, and then a saturated sodium hydrogen carbonate aqueous solution was added to extract the product. The organic layer was dried over anhydrous magnesium sulfate, and then the solvent was distilled off under reduced pressure to obtain a crude product. This crude product was purified through a silica gel column chromatography (eluent, ethyl acetate : hexane). The compound thus obtained was dissolved into ethanol (27.4 mL). Then, 5N hydrochloric acid (2.43 mL) was added stepwisely, followed by gradually heating up and heating in an oil bath at 70°C for 30 minutes. The solution was cooled down to room temperature, and dissolved into chloroform. Thereafter, a saturated sodium hydrogen carbonate aqueous solution was added and the product was extracted with chloroform: 2-propanol=4:1. The organic layer was dried over anhydrous magnesium sulfate and the solvent was distilled off under reduced pressure to obtain a crude product. This crude product was purified through a silica gel column chromatography (eluent, chloroform:methanol) to obtain 573.3 mg of the title compound.
¹H-NMR (400 MHz, CDCl₃) δ: 2.34 (3H, s), 2.52 (4H, t, J=4.7 Hz), 2.60-2.67 (2H, m), 2.68-2.76 (2H, m), 3.30 (4H, t, J=4.7 Hz), 3.78-3.87 (2H, brs), 4.64-4.72 (2H, brs), 6.49 (1H, d, J=2.3 Hz), 6.61 (1H, dd, J=8.30, 2.3 Hz), 7.91 (1H, d, J=8.3 Hz).
MS (ESI) m/z: 311 [M+H]⁺.

### (Reference Example 4)

### methyl cis-3-(2-(piperazin-1-yl)ethoxy)cyclobutane-1-carboxyl ate

### (a) methyl cis-3-(2-chloroethoxy)cyclobutanecarboxylate

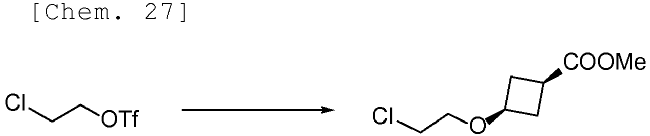

Methyl cis-3-hydroxycyclobutane-1-carboxylate (1 g, 7.68 mmol) was dissolved into toluene (10 mL), and N,N-diisopropylethylamine (2.67 mL, 15.36 mmol, 2 equivalents) and 2-chloroethyl trifluoromethanesulfonate (2.05 mL, 15.36 mmol, 2 equivalents) were added, followed by stirring at 90°C for 6 hours in an argon atmosphere. Ethyl acetate and a saturated ammonium chloride aqueous solution were added to the reaction liquid to extract. The organic layer was washed with water. The organic layer was dried over anhydrous sodium sulfate and the solvent was distilled off under reduced pressure to obtain 1.33 g of the title target product.
¹H-NMR (400 MHz, CDCl₃) δ: 2.19-2.33 (2H, m), 2.45-2.57 (2H, m), 2.58-2.71 (1H, m), 3.57-3.65 (4H, m), 3.69 (3H, s), 3.92-3.98 (1H, m).
MS (ESI) m/z: 193 [M+H]⁺

### (b) tert-butyl cis-4-(2-(-3-(methoxycarbonyl)cyclobutoxy)ethyl)pipera zin-1-carboxylate

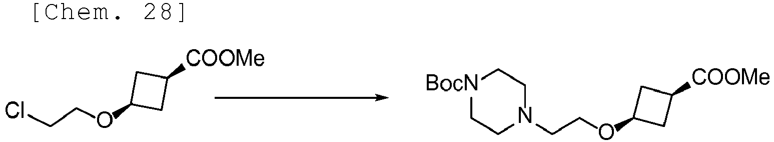

The compound (788 mg, 4.1 mmol) obtained in Reference Example 4(a) was dissolved in DMF (10 mL), and 1-(tert-butoxycarbonyl)piperazine (764 mg, 4.1 mmol, 1.0 equivalent), potassium iodide (681 mg, 4.1 mmol, 1.0 equivalent), and N,N-diisopropylethylamine (1.43 mL, 2 equivalents) were added, followed by stirring at 120°C for 4 hours under MW irradiation. Ethyl acetate (80 mL), a saturated sodium hydrogen carbonate aqueous solution (40 mL), and methanol (5 mL) were added to the reaction liquid to extract. The organic layer was washed with brine. The water layer was extracted again with ethyl acetate, and the organic layer combined was dried over anhydrous magnesium sulfate and the solvent was distilled off under reduced pressure to obtain a crude product. This crude product was purified through a silica gel column chromatography (eluent, ethyl acetate : hexane → methanol:ethyl acetate) to obtain the title compound (1.44 g).
H-NMR (400 MHz, CDCl₃) δ: 1.46 (9H, s), 2.17-2.25 (2H, m), 2.42-2.45 (4H, m), 2.46-2.53 (2H, m), 2.55-2.58 (2H, t, J=5.9 Hz), 3.42-3.49 (6H, m), 3.68 (3H, s), 3.83-3.91 (1H, m).
MS (ESI) m/z: 343 [M+H]⁺

### (c) methyl cis-3-(2-(piperazin-1-yl)ethoxy)cyclobutane-1-carboxyl ate

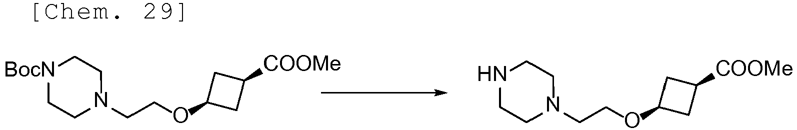

The compound (1.44 g, 4.21 mmol) obtained in Reference Example 4(b) was dissolved into methanol (14.4 mL), and a 4M hydrochloric acid/1,4-dioxane solution (10.5 mL, 42 mmol, 10 equivalents) was added, followed by stirring overnight at room temperature. The solvent was distilled off under reduced pressure, and then the residue was dissolved into chloroform (100 mL), and a saturated sodium hydrogen carbonate aqueous solution (50 mL) and a 1M sodium hydroxide aqueous solution (10 mL) were added to extract an organic layer. Chloroform (50 mL) and a 1M sodium hydroxide aqueous solution (5 mL) were again added to the water layer to extract an organic layer. The organic layer combined was dried over anhydrous magnesium sulfate and the solvent was distilled off under reduced pressure to obtain the title compound (845.0 mg).
¹H-NMR (400 MHz, CDCl₃) δ: 2.18-2.27 (2H, m), 2.46-2.67 (9H, m), 2.88-2.91 (4H, m), 3.48 (2H, t, J=6.1 Hz), 3.68 (3H, s), 3.85-3.91 (1H, m).
MS (ESI) m/z: 243 [M+H]⁺.

### (Reference Example 5)

### methyl 2-(2-(2-(piperazin-1-yl)ethoxy)ethoxy)acetate

### (a) tert-butyl 4-(2-(2-(2-(tert-butoxy)-2-oxoethoxy)ethoxy)ethyl)pipe razine-1-carboxylate

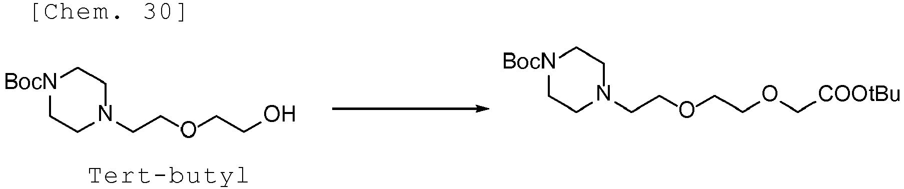

4-(2-(2-hydroxyethoxy)ethyl)piperazine-1-carboxylate(2 74 mg, 1.0 mmol) was dissolved into methylene chloride (3.3 mL), and tetrabutylammonium chloride (28 mg, 0.1 mmol) was added and a 35% sodium hydroxide aqueous solution (3.3 mL) was added under ice cooling. Tert-butyl 2-bromoacetate (220 µL, 1.5 mmol, 1.5 equivalents) was added to the solution at the same temperature, followed by vigorously stirring for 3 hours. A saturated ammonium chloride aqueous solution was added to neutralize under ice cooling, the product was extracted with methylene chloride, the organic layer was dried with anhydrous sodium sulfate, and the solvent was distilled off under reduced pressure to obtain a crude product. This crude product was subjected to a silica gel column chromatography (eluent, ethyl acetate:methanol) to obtain 284 mg of the title compound. ¹H-NMR (400 MHz, CDCl₃) δ: 1.47 (9H, d, J=8.1 Hz), 2.43-2.48 (4H, m), 2.61 (2H, t, J=5.8 Hz), 3.41-3.47 (4H, m), 3.61-3.73 (6H, m), 4.02 (2H, s).
MS (ESI) m/z: 389 [M+H]⁺

### (b) methyl 2-(2-(2-(piperazin-1-yl)ethoxy)ethoxy)acetate

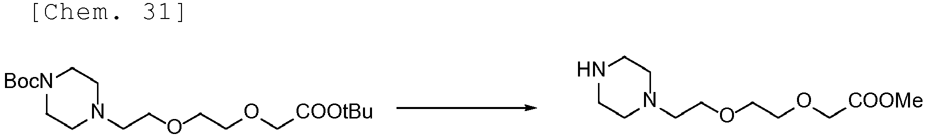

The compound (284 mg, 0.839 mmol) obtained in Reference Example 5(a) was dissolved into 2M hydrochloric acid/methanol (1.68 mL), followed by stirring overnight at room temperature. The solvent was distilled off under reduced pressure to obtain 207 mg of the title compound. H-NMR (400 MHz, CD₃OD) δ: 3.51-3.61 (6H, m), 3.68-3.77 (9H, m), 3.92-4.05 (4H, m), 4.20 (2H, s).
MS (ESI) m/z: 247 [M+H]⁺.

### (Example 1)

### 8-((4-chlorobenzyl)oxy)-4-(4-methylpiperazin-1-yl)-5,6 -dihydrobenzo[h]quinazolin-2-amine

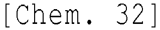

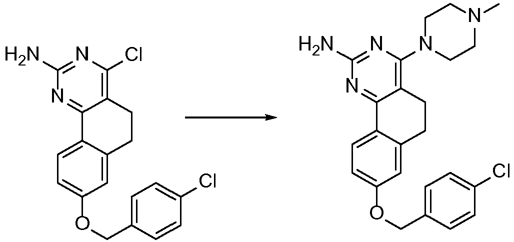

The compound (771 mg, 2.07 mmol) obtained in Reference Example 1(d) was dissolved into 2-propanol (10 mL), and 1-methylpiperazine (892 µL, 8.23 mmol, 4.0 equivalents) and N,N-diisopropylethylamine (1.44 mL, 8.23 mmol, 4.0 equivalents) were added, followed by stirring at 140°C for 30 minutes under MW irradiation. Water was added to the reaction liquid, followed by extracting with ethyl acetate. The organic layer was washed with brine and dried with anhydrous sodium sulfate. The solvent was distilled off under reduced pressure to obtain a crude product. This crude product was subjected to a silica gel column chromatography (eluent, chloroform:methanol) to obtain 724 mg of the title compound.
¹H-NMR (400 MHz, CDCl₃) δ: 2.34 (3H, s), 2.48-2.56 (4H, m), 2.63-2.68 (2H, m), 2.74-2.81 (2H, m), 3.28-3.36 (4H, m), 4.75 (2H, brs), 5.06 (2H, s), 6.79 (1H, d, J=2.4 Hz), 6.89 (1H, dd, J=2.6, 8.7 Hz), 7.33-7.40 (4H, m), 8.04 (1H, d, J=8.6 Hz).
MS (ESI) m/z: 436 [M+H]⁺.

### (Example 2)

### 8-(benzyloxy)-4-(4-methylpiperazin-1-yl)-5,6-dihydrobe nzo[h]quinazolin-2-amine

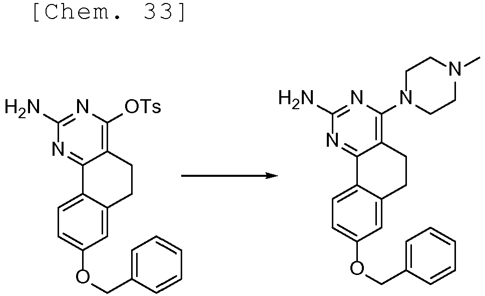

The title compound was obtained by the same method as in Example 1 from the compound obtained in Reference Example 2.
¹H-NMR (400 MHz, CDCl₃) δ: 2.35 (3H, s), 2.50 (4H, t, J=4.0 Hz), 2.62-2.67 (2H, m), 2.75-2.81 (2H, m), 3.35 (4H, t, J=4.0 Hz), 4.70 (2H, s), 5.15 (3H, s), 6.85 (1H, d, J=2.0 Hz), 6.93 (1H, dd, J=2.0, 8.0 Hz), 7.30-7.46 (5H, m), 8.05 (1H, d, J=8.0 Hz).
MS (ESI) m/z: 402 [M+H]⁺.

### (Example 3)

### 4-(4-methylpiperazin-1-yl)-8-(thiophen-3-ylmethoxy)-5, 6-dihydrobenzo[h]quinazolin-2-amine

### (a) 2-amino-4-(4-methylpiperazin-1-yl)-5,6-dihydrobenzo[h] quinazolin-8-ol

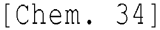

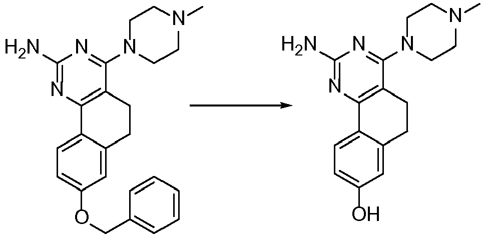

The compound (650 mg, 1.62 mmol) obtained in Example 2 was dissolved into methanol, and a 10% palladium-carbon catalyst (wetted with ca. 50% water) was added, followed by stirring at room temperature for 24 hours under a hydrogen gas flow. The reaction solution was filtered through Celite and the solvent was concentrated under reduced pressure to obtain a crude product. This crude product was subjected to a silica gel column chromatography (eluent, chloroform:methanol) to obtain 229 mg of the title compound.
H-NMR (400 MHz, CDCl₃) δ: 2.35 (3H, s), 2.51-2.63 (6H, m), 2.66-2.73 (2H, m), 3.29-3.36 (4H, m), 3.28-3.36 (4H, m), 4.94 (2H, brs), 6.60-6.68 (2H, m), 7.85 (1H, J=8.6 Hz). MS (ESI) m/z: 312 [M+H]⁺

### (b) 4-(4-methylpiperazin-1-yl)-8-(thiophen-3-ylmethoxy)-5, 6-dihydrobenzo[h]quinazolin-2-amine

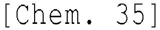

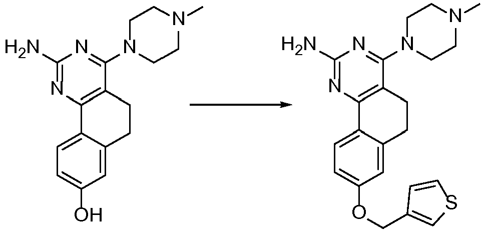

The compound (30 mg, 0.096 mmol) obtained in Example 3 (a) was dissolved in THF (480 µL), and triphenylphosphine (50.5 mg, 0.193 mmol, 2.0 equivalents) and thiophen-3-yl methanol (13.6 µL, 0.145 mmol, 1.5 equivalents) were added, followed by stirring. Thereafter, diethyl azodicarboxylate (40% toluene solution, 2.2 mol/L) (88 µL, 0.193 mmol, 2.0 equivalents) was added, followed by stirring at room temperature for 1 hour. Water was added to the reaction liquid, followed by extracting with ethyl acetate. The organic layer was washed with brine and dried over anhydrous sodium sulfate. The solvent was distilled off under reduced pressure to obtain a crude product. This crude product was subjected to a silica gel column chromatography (eluent, chloroform : methanol) to obtain 12.6 mg of 4-(4-methylpiperazin-1-yl)-8-(thiophen-3-ylmethoxy)-5, 6-dihydrobenzo[h]quinazolin-2-amine.
¹H-NMR (400 MHz, CDCl₃) δ: 2.34 (3H, s), 2.48-2.56 (4H, m), 2.63-2.68 (2H, m), 2.75-2.82 (2H, m), 3.28-3.36 (4H, m), 4.78 (2H, brs), 5.11 (2H, s), 6.80 (1H, d, J=2.7 Hz), 6.91 (1H, dd, J=2.7, 8.6 Hz), 7.16 (1H, dd, J=1.6, 4.8 Hz), 7.32-7.37 (2H, m), 8.04 (1H, d, J=8.6 Hz).
MS (ESI) m/z: 408 [M+H]⁺.

### (Example 4)

### 8-((4-chlorobenzyl)oxy)-4-(piperazin-1-yl)-5,6-dihydro benzo[h]quinazolin-2-amine

### (a) tert-butyl 4-(2-amino-8-((4-chlorobenzyl)oxy)-5,6-dihydrobenzo[h] quinazolin-4-yl)piperazine-1-carboxylate

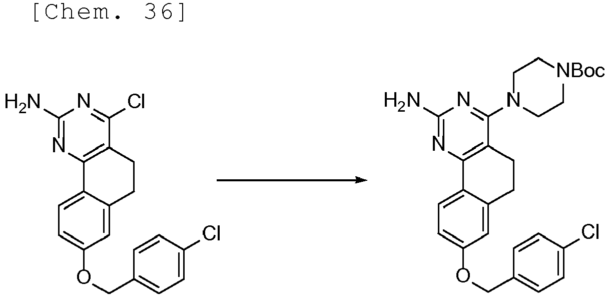

The title compound was obtained in the same method as in Example 1 from the compound obtained in Reference Example 1(d) except that tert-butyl piperazine-1-carboxylate was used in place of N-methylpiperazine.
¹H-NMR (400 Hz, CDCl₃) δ: 1.48 (9H, s), 2.62-2.69 (2H, m), 2.75-2.82 (2H, m), 3.20-3.27 (4H, m), 3.47-3.56 (4H, m), 4.87 (2H, brs), 5.07 (2H, s), 6.80 (1H, d, J=2.4 Hz), 6.89 (1H, dd, J=2.4, 8.6 Hz), 7.34-7.40 (4H, m), 8.02 (1H, d, J=8.6 Hz).
MS (ESI) m/z: 522 [M+H]⁺

### (b) 8-((4-chlorobenzyl)oxy)-4-(piperazin-1-yl)-5,6-dihydro benzo[h]quinazolin-2-amine

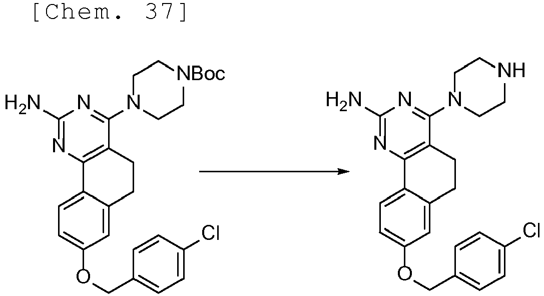

The title compound was obtained in the same method as in Reference Example 4(c) from the compound obtained in Example 4(a).
¹H-NMR (400 MHz, CDCl₃) δ: 2.63-2.69 (2H, m), 2.75-2.82 (2H, m), 2.96-3.01 (4H, m), 3.24-3.27 (4H, m), 4.74 (2H, brs), 5.07 (2H, s), 6.80 (1H, d, J=2.7 Hz), 6.90 (1H, dd, J=8.6 Hz), 7.34-7.39 (4H, m), 8.04 (1H, d, J=8.6 Hz).
MS (ESI) m/z: 422 [M+H]⁺.

### (Example 5)

### 8-(benzyloxy)-4-(piperazin-1-yl)-5,6-dihydrobenzo[h]qu inazolin-2-amine

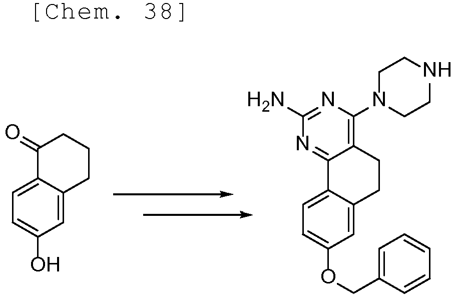

The title compound was obtained by the same method as in Example 4 except that 1-(bromomethyl)benzene was used in place of 1-(bromomethyl)-4-chlorobenzene in Reference Example 1(a).
¹H-NMR (400 MHz, CDCl₃) δ: 2.62-2.70 (2H, m), 2.76-2.83 (2H, m) , 2.96-3.02 (4H, m), 3.21-3.27 (4H, m), 4.71 (2H, brs), 5.11 (2H, s), 6.82 (1H, dd, J=2.6 and 8.7 Hz), 6.92 (1H, d, J=2.44 Hz), 7.31-7.47 (5H, m), 8.04 (1H, d, J=8.6 Hz).
MS (ESI) m/z: 388 [M+H]⁺.

### (Example 6)

### 4-(piperazin-1-yl)-8-((4-(trifluoromethoxy)benzyl)oxy) -5,6-dihydrobenzo[h]quinazolin-2-amine

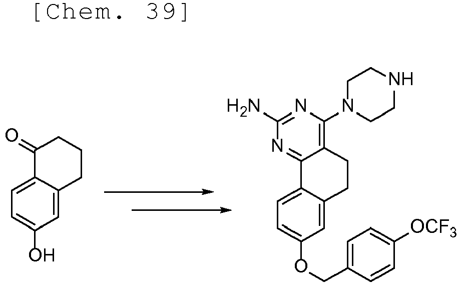

The title compound was obtained by the same method as in Example 5 except that 1-(bromomethyl)-4-(trifluoromethoxy)benzene was used in place of 1-(bromomethyl)-4-chlorobenzene in Reference Example 1(a).
¹H-NMR (400 MHz, CDCl₃) δ: 2.63-2.70 (2H, m), 2.75-2.84 (2H, m), 2.95-3.03 (4H, m), 3.21-3.29 (4H, m), 4.63-4.75 (1H, m), 5.10 (2H, s), 6.81 (1H, d, J=2.6 Hz), 6.90 (1H, dd, J=2.6, 8.6Hz), 7.24 (2H, d, J=7.9 Hz), 7.42-7.50 (2H, m), 8.05 (1H, d, J=8.6 Hz) .
MS (ESI) m/z: 472 [M+H]⁺.

### (Examples 7 to 10, 101 to 117)

Compounds described in Tables 1 to 3 given below were each obtained using a corresponding cyclic amine in place of N-methylpiperazine in the same method as in Example 1 from the compound obtained in Reference Example 1(e) or Reference Example 2. Tables 1 to 3 show the structural formula, the compound name, the nuclear magnetic resonance spectrum (¹H-NMR) and its solvent, and the result of the electrospray ionization mass spectrum (ESI MS) of each compound. Note that in Tables below, when there is no particular description on the structure in the name of compound whose structural isomers can exists, it indicates the compound is a mixture of these (the same applies hereinbelow).

**[Table 1]**

| Ex. No. | Structural formula | Compound name | ¹H-NMR (400 MHz) δ (ppm) from TMS | Solvent | ESI MS m/z |
|---|---|---|---|---|---|
| 7 | 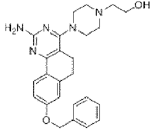 | 2-(4-(2-amino-8-(benzyloxy)-5,6-dihydrobenzo[h]quinazolin-4-yl)piperazin-1-yl)ethan-1-ol | 2.58-2.70(8H, m), 2.75-2.83(2H, m), 3.25-3.35(4H, m), 3.66(2H, t, J=5.3 Hz), 4.75(2H, brs), 5.11(2H, s), 6.82(1H, d, J=2.4 Hz), 6.93(1H, dd, J=2.4, 8.8Hz), 7.30-7.47(5H, m), 8.04(1H, d, J=8.5 Hz). | CDCl₃ | 432 [M+H]⁺ |
| 8 | 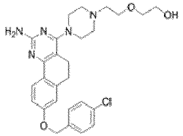 | 2-(2-(4-(2-amino-8-((4-chlorobenzyl)oxy)-5,6-dihydrobenzo[h]quinazolin-4-yl)piperazin-1-yl)ethoxy)ethan-1-ol | 2.55-2.67(8H, m), 2.73-2.81(2H, m), 3.28-3.39(4H, m), 3.58-3.69(7H, m), 4.83(2H, s), 5.05(2H, s), 6.78(1H, s), 6.88(1H, d, J=8.6 Hz), 7.31-7.38(4H, m), 8.03(1 H, d, J=8.6 Hz). | CDCl₃ | 510 [M+H]⁺ |
| 9 | 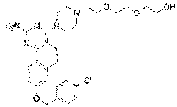 | 2-(2-(2-(4-(2-amino-8-((4-chlorobenzyl)oxy)-5,6-dihydrobenzo[h]quinazolin-4-yl)piperazin-1-yl)ethoxy)ethoxy)ethan-1-ol | 2.57-2.70(8H, m), 2.72-2.81(2H, m), 3.30-3.38(4H, m), 3.60-3.77(10H. m), 4.83(2H, brs), 5.06(2H, s), 6.79(1H, d, J=2.5 Hz), 6.89(1H, dd, J=2.5. 8.6 Hz), 7.33-7.42(4H, m), 8.03(1H, d, J=8.6 Hz). | CDCl₃ | 554 [M+H]⁺ |
| 10 | 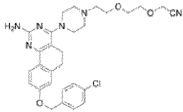 | 2-(2-(2-(4-(2-amino-8-((4-chlorobenzyl)oxy)-5,6-dihydrobenzo[h]quinazolin-4-yl)piperazin-1-yl)ethoxy)ethoxy)acetonitr ile | 2.59-2.69(8H, m), 2.74-2.81(2H, m), 3.29-3.35(4H, m), 3.64-3.71(4H, m), 3.75-3.79(2H, m), 4.34(2H, s), 4.68-4.77(2H, m), 5.07(2H, s), 6.79(1 H, d, J=2.6 Hz), 6.89(1 H, dd, J=2.6, 8.6 Hz), 7.33-7.39(4H, m), 8.03(1 H, d, J=8.6 Hz). | CDCl₃ | 549 [M+H]⁺ |
| 101 | 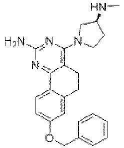 | (S)-8-(benzyloxy)-4-(3-(methylamino)pyrrolidin-1-yl)-5,6-dihydrobenzo[h]quinazolin-2-amine | 1.72-1.82(1H, m), 2.02-2.12(1H, m), 2.45(3H, s), 2.74-2.81(2H, m), 2.85-2.92(2H, m), 3.23-330(1H, m), 3.38-3.45(1H, m), 3.58-3.68(1H, m), 3.70-3.80(2H, m), 4.65(2H, s), 5.15(2H, s), 680(1H, d, J=2.0 Hz), 692(1H, dd, J=2.0, 8.0 Hz), 7.31-7.45(5H, m), 8.05(1H, d, J=8.0 Hz). | CDCl₃ | 402 [M+H]⁺ |
| 102 | 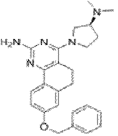 | (S)-8-(benzyloxy)-4-(3-(dimethylamino)pyrrolidin-1-yl)-5,6-dihydrobenzo[h]quinazolin-2-amine | 1.73-1.85(1H, m), 2.06-2.14(1H, m), 2.30(6H, s), 2.61-2.94(5H, m), 3.50(1H, t), 3.62-3.75(3H, m), 4.66(2H, brs), 5.10(2H, s), 680(1H, d, J=2.4 Hz), 6.91(1H, dd, J=2.4, 8.6 Hz), 7.30-7.47(5H, m), 8.03(1H, d, J=8.6 Hz). | CDCl₃ | 416 [M+H]⁺ |
| 103 | 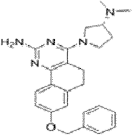 | (R)-8-(benzyloxy)-4-(3-(dimethylamino)pyrrolidin-1-yl)-5,6-dihydrobenzo[h]quinazolin-2-amine | 1.73-1.85(1H, m), 2.06-2.14(1H, m), 2.30(6H, s), 2.61-2.94(5H, m), 3.50(1H, t), 3.62-3.75(3H, m), 4.66(2H, brs), 5.10(2H, s), 680(1H, d, J=2.4 Hz), 6.91(1H, dd, J=2.4, 8.6 Hz), 7.30-7.47(5H, m), 8.03(1H, d, J=8.6 Hz). | CDCl₃ | 416 [M+H]⁺ |
| 104 | 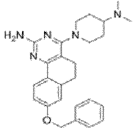 | 8-(benzyloxy)-4-(4-(dimethylamino)piperidin-1-yl)-5,6-dihydrobenzo[h]quinazolin-2-amine | 1.53-1.64(2H, m), 1.90(2H, d, J=12.5 Hz), 2.26-2.36(7H, m), 2.63-2.69(2H, m), 2.72-2.82(4H, m), 3.77(2H, d, J=12.5 Hz), 471(2H, s), 5.11(2H, s), 6.82(1 H, d, J=2.5 Hz), 6.92(1 H, dd, J=2.5, 8.3 Hz), 7.30-7.46(5H, m), 8.28(1 H, d, J=8.3 Hz). | CDCl₃ | 430 [M+H]⁺ |

**[Table 2]**

| Ex. No. | Structural formula | Compound name | ¹H-NMR (400 MHz) *δ* (ppm) from TMS | Solvent | ESI MS m/z |
|---|---|---|---|---|---|
| 105 | 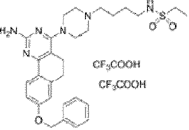 | N-(4-(4-(2-amino-8-(benzyloxy)-5,6-dihydrobenzo[h]quinazolin-4-yl)piperazin-1-yl)butyl)ethanesulfonamide bis(trifluoroacetate) | 1.32(3H, t, J=7.3 Hz), 1.58-1.68(2H, m), 1.82-1.94(2H, m), 2.72-2.80(2H, m), 2.83-2.92(2H, m), 3.01-3.14(4H, m), 3.20-3.28(2H, m), 5.20(2H, m), 7.04-7.11(2H, m), 7.30-7.48(5H, m), 7.72(1H, d, J=8.8 Hz). | CDCl₃ | 551 [M+H]⁺ |
| 106 | 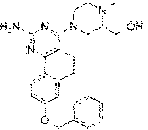 | (4-(2-amino-8-(benzyloxy)-5,6-dihydrobenzo[h]quinazolin-4-yl)-1-methylpiperazin-2-yl)methanol | 2.43-2.52(2H, m), 2.62-2.88(6H, m), 3.03-3.13(1 H, m), 3.16-3.28(1 H, dd, J=9.5, 13.2Hz), 3.31-3.39(1H, m), 3.42-3.48(1H, m), 3.52-3.64(4H, m), 3.85(1 H, dd, J=5.3, 11.1 Hz), 4.78(2H, brs), 5.10(2H, s), 6.82(1H, d, J=1.8 Hz), 6.92(1H, dd, J=1.8, 8.5Hz), 7.30-7.47(5H, m), 8.03(1 H, J=8.5 Hz). | CDCl₃ | 432 [M+H]⁺ |
| 107 | 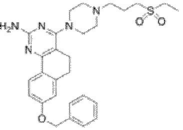 | 8-(benzyloxy)-4-(4-(3-(ethylsulfonyl)propyl)pipera zin-1-yl)-5,6-dihydrobenzo[h]quinazolin-2-amine | 1.34(3H, t, J=1.3 Hz), 2.28-2.39(2H, m), 2.55-2.63(2H, m), 2.73-2.82(2H, m), 3.02(2H, q, J=7.5 Hz), 3.06-3.13(4H, t, J=6.2 Hz), 3.20-3.44(6H, m), 3.65-4.30(4H, m), 5.07(2H, s), 6.21(1H, d, J=2.4 Hz), 6.96(1H, dd, J=2.4, 8.8 Hz), 7.42-7.71 (5H, m), 791(1H, d, J=8.8 Hz),. | CDCl₃ | 522 [M+H]⁺ |
| 108 | 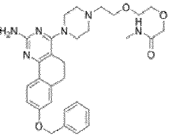 | 2-(2-(2-(4-(2-amino-8-(benzyloxy)-5,6-dihydrobenzo[h]quinazolin-4-yl)piperazin-1-yl)ethoxy)ethoxy)-N-methylacetamide | 2.60-2.68(8H, m), 2.75-2.80(2H, m), 2.86(3H, d, J=5.0 Hz), 3.28-3.35(4H, m), 3.62-3.69(6H m), 4.00(2H, s), 4.75(2H, brs), 5.10(2H, s), 6.81 (1H, d, J=2.6 Hz), 6.92(1H, dd, J=2.6, 8.6 Hz), 7.05(1H, brs), 7.31-7.36(1H, m), 7.36-7.42(2H, m), 7.42-7.45(2H, m), 8.03(1H, d, J=8.6 Hz). | CDCl₃ | 547 [M+H]⁺ |
| 109 | 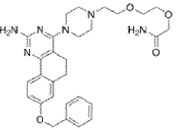 | 2-(2-(2-(4-(2-amino-8-(benzyloxy)-5,6-dihydrobenzo[h]quinazolin-4-yl)piperazin-1-yl)ethoxy)ethoxy)acetamid e | 2.55-2.68(8H, m), 2.73-2.79(2H, m), 3.26-3.24(4H, m), 3.62-3.68(4H, m), 3.68-3.73(2H, m), 402(2H. s), 4.76(2H, brs), 5.10(2H, s), 5.63(1H, brs), 6.81(1H, d, J=2.6 Hz), 6.92(1H, dd, J=2.6, 8.7 Hz), 7.15(1H, brs), 7.31-7.35(1H, m), 7.36-7.42(2H, m), 7.42-7.45(2H, m), 8.03(1H, d, J=8.7 Hz). | CDCl₃ | 533 [M+H]⁺ |
| 110 | 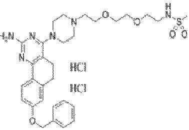 | N-(2-(2-(2-(4-(2-amino-8-(benzyloxy)-5,6-dihydrobenzo[h]quinazolin-4-yl)piperazin-1-yl)ethoxy)ethoxy)ethyl)me thanesulfonamide dihydrochloride | 2.77-2.82(2H, m), 2.88-2.94(2H, m), 2.97(3H, s), 3.24-3.29(4H, m), 3.45-3.49(2H, m), 3.57-3.75(12H, m), 3.93-3.98(2H, m), 5.21(2H, s), 7.04-7.10(2H, m), 7.31-7.45(5H, m), 7.92(1 H, d, J=8.6 Hz). | CD₃OD | 597 [M+H]⁺ |
| 111 | 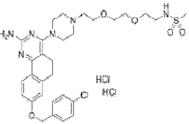 | N-(2-(2-(2-(4-(2-amino-8-((4-chlorobenzyl)oxy)-5,6-dihydrobenzo[h]quinazolin-4-yl)piperazin-1-yl)ethoxy)ethoxy)ethyl)me thanesulfonamide dihydrochloride | 2.55-2.68(8H, m), 2.72-2.80(2H, m), 2.95(3H, s), 3.23-3.36(6H, m), 3.55-3.67(8H, m), 4.93(2H, brs), 5.05(2H, s), 5.38(1H, brs), 6.78(1H, d, J=2.3 Hz), 6.88(1H, dd, J=2.3, 8.6 Hz), 7.27-7.39(4H, m), 8.02(1H, d, J=8.6 Hz). | CDCl₃ | 631 [M+H]⁺ |
| 112 | 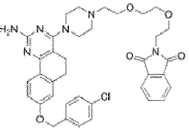 | 2-(2-(2-(2-(4-(2-amino-8-((4-chlorobenzyl)oxy)-5,6-dihydrobenzo[h]quinazolin-4-yl)piperazin-1-yl)ethoxy)ethoxy)ethyl)isoi ndoline-1,3-dione | 2.53-2.69(8H, m), 2.74-2.83(2H, m), 3.26-3.35(4H, m), 3.56-3.68(6H, m), 3.75(2H, t, J=5.7 Hz), 3.90(2H, t, J=5.7 Hz), 4.74(2H, brs), 5.07(2H, s), 6.79(1 H, dd, J=2.3 Hz), 6.89(1 H, dd, J=2.3, 8.6Hz), 7.33-7.41(4H, m), 7.67-7.73(2H, m), 7.81-7.89(2H, m), 8.04(1H, d, J=8.6 Hz). | CDCl₃ | 683 [M+H]⁺ |

**[Table 3]**

| Ex. No. | Structural formula | Compound name | ¹H-NMR (400 MHz) *δ* (ppm) from TMS | Solvent | ESI MS m/z |
|---|---|---|---|---|---|
| 113 | 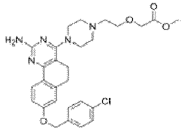 | methyl 2-(2-(4-(2-amino-8-((4-chlorobenzyl)oxy)-5,6-dihydrobenzo[h]quinazolin-4-yl)piperazin-1-yl)ethoxy)acetate | 2.61-2.71(8H, m), 2.74-2.80(2H, m), 3.31-3.37(4H, m), 3.72(2H, t, J=5.6 Hz), 3.76(3H, s), 4.14(2H, s), 4.75-4.82(2H, m), 5.06(2H, s), 6.79(1 H, d, J=2.5 Hz), 6.89(1 H, dd, J=2.5, 8.6 Hz), 7.33-7.39(4H, m), 8.03(1 H, d, J=8.6 Hz). | CDCl₃ | 538 [M+H]⁺ |
| 114 | 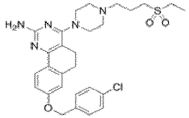 | 8-((4-chlorobenzyl)oxy)-4-(4-(3-(ethylsulfonyl)propyl)pipera zin-1-yl)-5,6-dihydrobenzo[h]quinazolin-2-amine | 1.42(3H, t, J=7.5 Hz), 2.01-2.10(2H, m), 2.51-2.59(6H, m), 2.62-2.68(2H, m), 2.75-2.81(2H, m), 2.97-3.11(4H, m), 3.25-3.33(4H, m), 4.72-4.81(2H, m), 5.07(2H, s), 6.79(1H, d, J=2.5 Hz), 6.90(1H, dd, J=2.5, 8.6 Hz), 7.33-7.39(4H, m), 8.03(1H, d, J=8.6 Hz). | CDCl₃ | 556 [M+H]⁺ |
| 115 | 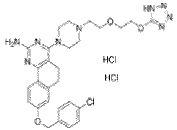 | 4-(4-(2-(2-((tetrazol-5-yl)oxy)ethoxy)ethyl)piperaz in-1-yl)-8-((4-chlorobenzyl)oxy)-5,6-dihydrobenzo[h]quinazolin-2-amine dihydrochloride | 2.63-2.70(2H, m), 2.77-2.83(2H, m), 3.30-3.50(10H, m), 3.83-3.89(4H, m), 4.55-4.60(2H, m), 5.22(2H, s), 7.05-7.13(2H, m), 7.45-7.52(4H, m), 7.86-7.92(1 H, m). | DMSO-d₆ | 578 [M+H]⁺ |
| 116 | 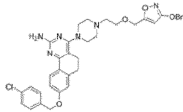 | 4-(4-(2-((3-(benzyloxy)isoxazol-5-yl)methoxy)ethyl)piperazin -1-yl)-8-((4-chlorobenzyl)oxy)-5,6-dihydrobenzo[h]quinazolin-2-amine | 2.52-2.62(8H, m), 2.68-2.77(2H, m), 3.17(4H, brs), 3.62(2H, t, J=5.6 Hz), 4.53(2H, s), 5.15(2H, s), 5.25(2H, s), 6.02(2H, s), 6.31(1H, s), 6.90-6.98(2H, m), 7.35-7.43(3H, m), 7.44-7.52(6H, m), 7.93(1H, d, J=8.2 Hz). | DMSO-d₆ | 653 [M+H]⁺ |
| 117 | 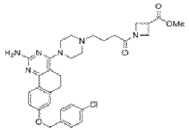 | methyl 1-(4-(4-(2-amino-8-((4-chlorobenzyl)oxy)-5,6-dihydrobenzo[h]quinazolin-4-yl)piperazin-1-yl)butanoyl)azetidine-3-carboxylate | 1.79-1.90(2H, m), 2.06-2.21(2H, m), 2.41(2H, t, J=7.1 Hz), 2.54(4H, t, J=4.4 Hz), 2.61-2.70(2H, m), 2.73-2.82(2H, m), 3.28(4H, t, J=4.4 Hz), 3.35-3.46(1 H, m), 3.76(3H, s), 4.09-4.16(1 H, m), 4.16-4.23(1 H, m), 4.26-4.33(1 H, m), 4.33-4.39(1 H, m), 4.67-4.75(2H, m), 5.06(2H, s), 6.79(1 H, d, J=2.4 Hz), 689(1H, dd, J=2.4, 8.6 Hz), 7.31-7.42(4H, m), 8.03(1H, d, J=8.6 Hz). | CDCl₃ | 605 [M+H]⁺ |

### (Example 118)

### 4-(4-(2-(2-(2-aminoethoxy)ethoxy)ethyl)piperazin-1-yl) -8-((4-chlorobenzyl)oxy)-5,6-dihydrobenzo[h]quinazolin -2-amine

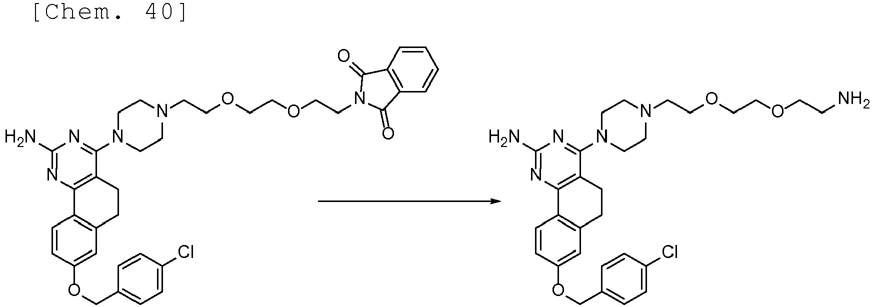

2-(2-(2-(2-(4-(2-amino-8-((4-chlorobenzyl)oxy)-5 ,6-dihydrobenzo[h]quinazolin-4-yl)piperazin-1-yl)ethox y)ethoxy)ethyl)isoindoline-1,3-dione (10 mg, 0.015 mmol) obtained in Example 112 was dissolved in ethanol (1 mL), and hydrazine monohydrate (7.1 µL, 0.15 mmol, 10 equivalents) was added, followed by stirring at 50°C for 3 hours. Insolubles were filtered, and the filtrate was concentrated to obtain a crude product. This crude product was purified through an HPLC (mobile phase, water : acetonitrile) to obtain 5.3 mg of the title compound.
¹H-NMR (400 MHz, CDCl₃) δ: 2.58-2.68 (8H, m), 2.74-2.80 (2H, m), 2.87 (2H, t, J=5.2 Hz), 3.28-3.34 (4H, m), 3.52 (2H, t, J=5.2 Hz), 3.62-3.69 (6H, m), 4.69 (2H, brs), 5.07 (2H, s), 6.79 (1H, d, J=2.6 Hz), 6.89 (1H, dd, J=2.6, 8.6 Hz), 7.33-7.38 (4H, m), 8.03 (1H, d, J=8.6 Hz).
MS (ESI) m/z: 553 [M+H]⁺.

### (Example 11)

### 2-(2-(2-(4-(2-amino-8-((4-chlorobenzyl)oxy)-5,6-dihydr obenzo[h]quinazolin-4-yl)piperazin-1-yl)ethoxy)ethoxy) acetic acid

### (a) methyl 2-(2-(2-(4-(2-amino-8-((4-chlorobenzyl)oxy)-5,6-dihydr obenzo[h]quinazolin-4-yl)piperazin-1-yl)ethoxy)ethoxy) acetate

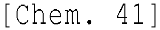

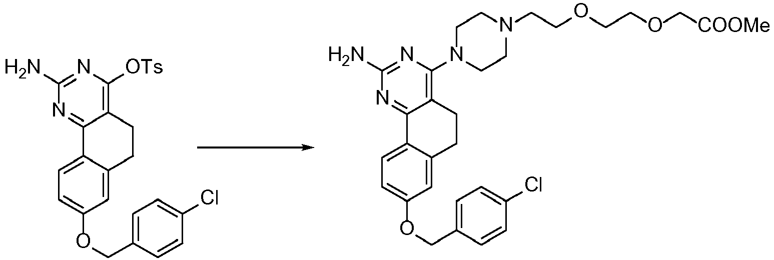

The title compound was obtained using the compound of Reference Example 5(b) in place of N-methylpiperazine in the same method as in Example 1 from the compound obtained in Example 1(e).
¹H-NMR (400 MHz, CDCl₃) δ: 2.59-2.68 (8H, m), 2.75-2.82 (2H, m), 3.29-3.35 (4H, m), 3.64-3.79 (9H, m), 4.18 (2H, s), 4.71 (2H, brs), 5.07 (2H, s), 6.79 (1H, dd, J=2.6 Hz), 6.90 (1H, dd, J=2.6, 8.6 Hz), 7.33-7.40 (4H, m), 8.03 (1H, d, J=8.6 Hz).
MS (ESI) m/z: 582 [M+H]⁺

### (b) 2-(2-(2-(4-(2-amino-8-((4-chlorobenzyl)oxy)-5,6-dihydr obenzo[h]quinazolin-4-yl)piperazin-1-yl)ethoxy)ethoxy) acetic acid

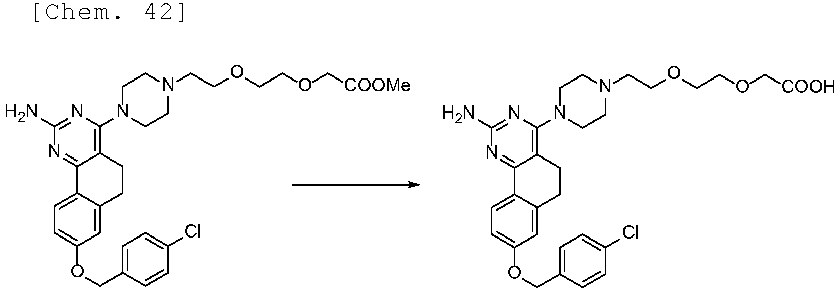

The compound (15 mg, 0.026 mmol) obtained in Example 11(a) was dissolved in methanol (1 mL), and a 5N sodium hydroxide aqueous solution (1 mL) was added, followed by stirring at room temperature for 30 minutes. The reaction liquid was neutralized using 5N hydrochloric acid and subjected to an octadecylsilylated silica gel column chromatography (eluent, water:acetonitrile) to obtain 14.3 mg of the title compound.
¹H-NMR (400 MHz, D₂O) δ: 2.33-2.61 (4H, m), 3.10-3.26 (2H, m), 3.33-3.46 (4H, m), 3.62-3.92 (8H, m), 4.18 (2H, s), 4.32-4.48 (2H, m), 4.75 (2H, s), 6.43 (1H, brs), 6.55 (1H, d, J=7.6 Hz), 7.04 (2H, d, J=7.8 Hz), 7.11 (2H, d, J=7.8 Hz), 7.38 (1H, d, J=7.6 Hz).
MS (ESI) m/z: 568 [M+H]⁺.

### (Examples 12 to 20, 119 to 143)

Compounds described in Tables 4 to 9 given below were each obtained using a corresponding cyclic amine in the same method as in Example 11 from the compound obtained in Reference Example 1(e) or 2-amino-8-((4-(trifluoromethoxy)benzyl)oxy)-5,6-dihydr obenzo[h]quinazolin-4-yl 4-methylbenzenesulfonate synthesized in a method similar to that in Reference Example 1 (e) using the same starting material as of Example 6. Tables 4 to 9 show the structural formula, the compound name, the nuclear magnetic resonance spectrum (¹H-NMR) and its solvent, and the result of the electrospray ionization mass spectrum (ESI MS) of each compound. Note that in Tables below, (a) and (b) described in the Example number indicate that the examples were obtained by the method (a) and the method (b) in the production method of Example 11, respectively.

**[Table 4]**

| Ex. No. | Structural formula | Compound name | ¹H-NMR (400 MHz) *δ* (ppm) from TMS | Solvent | ESI MS m/z |
|---|---|---|---|---|---|
| 12 | 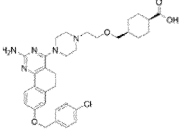 | cis-4-((2-(4-(2-amino-8-((4-chlorobenzyl)oxy)-5,6-dihydrobenzo[h]quinazolin-4-yl)piperazin-1-yl)ethoxy)methyl)cyclohex ane-1-carboxylic acid | 0.93-1.01(2H, m), 1.26-1.33(2H, m), 1.75-1.80(2H, m), 1.87-1.94(2H, m), 2.08-2.17(1H, m), 2.50-2.64(9H, m), 2.72-2.78(2H, m), 3.13-3.24(6H, m), 3.48-3.55(2H, m), 5.17(2H, s), 6.04(2H, brs), 6.93-6.98(2H, m), 7.45-7.53(4H, m), 7.95(1H, d, J=8.2 Hz). | DMSO-d₆ | 606 [M+H]⁺ |
| 13 | 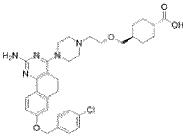 | trans-4-((2-(4-(2-amino-8-((4-chlorobenzyl)oxy)-5,6-dihydrobenzo[h]quinazolin-4-yl)piperazin-1-yl)ethoxy)methyl)cyclohex ane-1-carboxylic acid | 1.16-1.28(4H, m), 1.43-1.58(2H, m), 1.82-1.92(2H, m), 2.45-2.65(10H, m), 2.71-2.78(2H, m), 3.14-3.26(6H, m), 3.52(2H, t, J=5.2 Hz), 5.17(2H, s), 6.04 2H, brs), 6.93-6.98(2H, m), 7.45-7.53(4H, m), 7.95(1H, d, J=8.2 Hz). | DMSO-d₆ | 606 [M+H]⁺ |
| 14 | 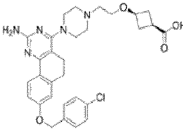 | cis-3-(2-(4-(2-amino-8-((4-chlorobenzyl)oxy)-5,6-dihydrobenzo[h]quinazolin-4-yl)piperazin-1-yl)ethoxy)cyclobutane-1-carboxylic acid | 2.12-2.22(2H, m), 2.48-2.60(3H, m), 2.66-2.77(2H, m), 2.82-2.89(2H, m), 3.05-3.16(4H, m), 3.57-3.75(6H, m), 3.90-4.02(3H, m), 5.17(2H, s), 7.06-7.11(2H, m), 7.38-7.47(4H, m), 773(1H, d, J=8.3 Hz). | CD₃OD | 564 [M+H]⁺ |
| 15(a) | 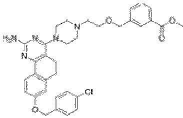 | methyl 3-((2-(4-(2-amino-8-((4-chlorobenzyl)oxy)-5,6-dihydrobenzo[h]quinazolin-4-yl)piperazin-1-yl)ethoxy)methyl)benzoate | 2.56-2.71(8H, m), 2.73-2.80(2H, m), 3.27-3.37(4H, m), 3.63(2H, t, J=5.7 Hz), 3.91(3H, s), 4.59(2H, s), 4.78-4.86(2H, m), 5.05(2H, s), 6.78(1H, d, J=2.4 Hz), 6.88(1H, dd, J=2.6, 8.6 Hz), 7.32-7.38(4H, m), 7.42(1 H, t, J=7.7 Hz), 7.53-7.58(1H, m), 7.94-7.96(1H, dt, J=1.4, 7.8 Hz), 8.00-8.05(2H. m). | CDCl₃ | 614 [M+H]⁺ |
| 15(b) | 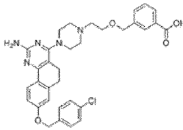 | 3-((2-(4-(2-amino-8-((4-chlorobenzyl)oxy)-5,6-dihydrobenzo[h]quinazolin-4-yl)piperazin-1-yl)ethoxy)methyl)benzoic acid | 2.61-2.71(8H,m), 2.73-2.79(2H, m), 3.28-3.34(4H, m), 3.67(2H, t, J=5.5 Hz), 4.57(2H, s), 5.11(2H, s), 6.85-6.94(2H, m), 7.32-7.46(6H, m), 7.88-7.94(3H, m). | CD₃OD | 600 [M+H]⁺ |
| 16(a) | 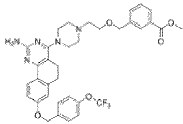 | methyl 3-((2-(4-(2-amino-8-((4-(trifluoromethoxy)benzyl)o xy)-5,6-dihydrobenzo[h]quinazolin-4-yl)piperazin-1-vl)ethoxy)methyl)benzoate | 2.56-2.71(8H, m), 2.74-2.80(2H, m), 3.28-3.36(4H, m), 3.63(2H, t, J=5.7 Hz), 3.91(3H, s), 4.59(2H, s), 4.81(2H, brs), 5.08(2H, s), 680(1H, d, J=2.4 Hz), 6.89(1H, dd, J=2.5, 8.6 Hz), 7.23(2H, d, J=8 Hz), 7.40-7.48(3H, m), 7.53-7.58(1 H, m), 7.94-7.97(1H, m), 8.00-8.06(2H, m). | CDCl₃ | 664 [M+H]⁺ |
| 16(b) | 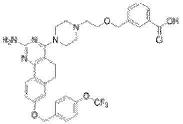 | 3-((2-(4-(2-amino-8-((4-(trifluoromethoxy)benzyl)o xy)-5,6-dihydrobenzo[h]quinazolin-4-yl)piperazin-1-yl)ethoxy)methyl)benzoic acid | 2.57-2.65(2H, m), 2.70-2.80(8H, m), 3.34-3.38(4H, m), 3.69(2H, t, J=5.5 Hz), 4.58(2H, s), 5.14(2H, s), 6.88(1H, d, J=2.4 Hz), 6.94(1H. dd, J=2.5, 8.6 Hz), 7.28(2H, d, J=7.8 Hz), 7.35(1 H, t, J=7.3 Hz), 7.41-7.45(1 H, m), 7.52-7.57(2H, m), 7.87-7.92(2H, m), 7.95-7.98(1 H, m). | CD₃OD | 650 [M+H]⁺ |

**[Table 5]**

| Ex. No. | Structural formula | Compound name | ¹H-NMR (400 MHz) *δ* (ppm) from TMS | Solvent | ESI MS m/z |
|---|---|---|---|---|---|
| 17(a) | 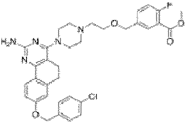 | methyl 5-((2-(4-(2-amino-8-((4-chlorobenzyl)oxy)-5,6-dihydrobenzo[h]quinazolin-4-yl)piperazin-1-yl)ethoxy)methyl)-2-fluorobenzoate | 2.58-2.71(8H, m), 2.74-2.81(2H, m), 3.29-3.36(4H, m), 3.62(2H, t, J=5.7 Hz), 3.93(3H, s), 4.54(2H, s), 4.67-4.73(2H, m), 5.07(2H, s), 6.79(1H, d, J=2.4 Hz), 6.89(1H, dd, J=2.7, 8.6 Hz), 7.13(1H, dd, J=8.5, 10.5 Hz), 7.33-7.40(4H, m), 7.49-7.55(1H, m), 7.90(1H, dd, J=2.3, 6.9 Hz), 8.03(1H. d. J=8.6 Hz). | CDCl₃ | 632 [M+H]⁺ |
| 17(b) | 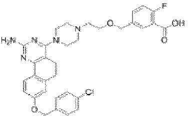 | 5-((2-(4-(2-amino-8-((4-chlorobenzyl)oxy)-5,6-dihydrobenzo[h]quinazolin-4-yl)piperazin-1-yl)ethoxy)methyl)-2-fluorobenzoic acid | 2.65-2.70(2H, m), 2.77-2.83(2H, m), 3.00-3.09(6H, m), 3.54-3.61(4H, m), 3.74(2H, t, J=5.2 Hz), 4.56(2H, s), 5.14(2H, s), 6.92(1 H, d, J=2.5 Hz), 6.97(1 H. dd, J=2.5, 8.6 Hz), 7.08(1 H, dd, J=8.4, 10.2 Hz), 7.35-7.41(3H, m), 7.42-7.46(2H, m), 7.73(1H, dd, J=2.3, 6.9 Hz), 7.86(1H, d, J=8.6 Hz). | CDCl₃ | 618 [M+H]⁺ |
| 18(a) | 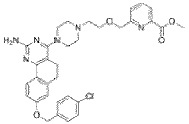 | methyl6-((2-(4-(2-amino-8-((4-chlorobenzyl)oxy)-5,6-dihydrobenzo[h]quinazolin-4-yl)piperazin-1-yl)ethoxy)methyl)picolinate | 2.61-2.81(10H, m), 3.31-3.36(4H, m), 3.74(2H, t, J=5.6 Hz), 4.01(3H, s), 4.67-4.74(2H, m), 4.79(2H, s), 5.07(2H, s), 6.79(1H, d J=2.6 Hz), 6.89(1H, dd, J=2.6, 8.6 Hz), 7.33-7.39(4H, m), 770-774(1H, m), 7.89(1 H, t, J=7.8 Hz), 8.02-8.06(2H, m). | CDCl₃ | 615 [M+H]⁺ |
| 18(b) | 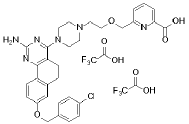 | 6-((2-(4-(2-amino-8-((4-chlorobenzyl)oxy)-5,6-dihydrobenzo[h]quinazolin-4-yl)piperazin-1-yl)ethoxy)methyl)picolinic acid bis(trifluoroacetate) | 2.74-2.81(2H, m), 2.86-2.94(2H, m), 3.50-372(6H, m), 3.97-4.05(6H, m), 4.81(2H, s), 5.19(2H, s), 6.97(1H, d, J=7.7 Hz), 7.04-7.12(2H, m), 7.37-7.47(4H, m), 7.72(1H, d, J=7.4 Hz), 8.01(1H, t, J=7.7 Hz), 8.10(1H, d, J=7.4 Hz). | CD₃OD | 601 [M+H]⁺ |
| 19(a) | 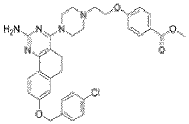 | methyl 4-(2-(4-(2-amino-8-((4-chlorobenzyl)oxy)-5,6-dihydrobenzo[h]quinazolin-4-yl)piperazin-1-yl)ethoxy)benzoate | 2.63-2.72(6H, m), 2.73-2.82(2H, m), 2.88(2H, t, J=5.8 Hz), 3.30-3.36(4H, m), 3.88(s, 3H), 4.19(2H, t, J=5.7 Hz), 4.73-4.81(m, 2H), 5.06(2H, s), 6.79(1H, d, J=2.6 Hz), 6.89(1H, dd, J=2.6, 8.6 Hz), 6.91-6.96(2H, m), 7.33-7.39(4H, m), 7.96-8.01(2H, m), 8.04(1H, d, J=8.6 Hz). | CDCl₃ | 600 [M+H]⁺ |
| 19(b) | 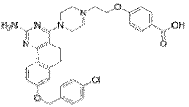 | 4-(2-(4-(2-amino-8-((4-chlorobenzyl)oxy)-5,6-dihydrobenzo[h]quinazolin-4-yl)piperazin-1-yl)ethoxy)benzoic acid | 255-283(10H, m), 3.50-3.85(4H, m), 4.05-4.38(2H, m), 5.17(2H, s), 6.94-7.15(4H, m), 7.45-7.52(4H, m), 7.85-7.98(3H, m). | DMSO-d₆ | 586 [M+H]⁺ |
| 20(a) | 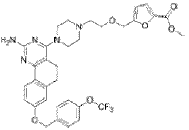 | methyl 5-((2-(4-(2-amino-8-((4-(trifluoromethoxy)benzyl)o xy)-5,6-dihydrobenzo[h]quinazolin-4-yl)piperazin-1-yl)ethoxy)methyl)furan-2-carboxylate | 2.57-2.68(8H, m), 2.73-2.81(2H, m), 3.27-3.35(4H, m), 3.63-3.69(2H, t, J=5.7 Hz), 3.88(3H, s), 4.55(2H, s), 4.77-4.84(2H, m), 5.09(2H, s), 6.44(1H, d, J=3.4 Hz), 6.80(1H, d, J=2.5 Hz), 6.89(1H, dd, J=2.5, 8.6 Hz), 7.14(1H, d, J=3.4 Hz), 7.21-7.26(2H, m), 7.44-7.54(2H, m), 8.03(1H, d, J=8.6 Hz). | CDCl₃ | 654 [M+H]⁺ |
| 20(b) | 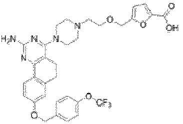 | 5-((2-(4-(2-amino-8-((4-(trifluoromethoxy)benzyl)o xy)-5,6-dihydrobenzo[h]quinazolin-4-yl)piperazin-1-yl)ethoxy)methyl)furan-2-carboxylic acid | 2.56-2.65(8H, m), 2.68-2.76(2H, m), 3.25-3.32(4H, m), 3.67(2H, t, J=5.5 Hz), 4.50(2H, s), 512(2H, s), 6.42(1H, d, J=3.2 Hz), 6.85(1H, d, 6.92(1Hz), 6.92(1H, d, J=3.2 Hz), 6.92(1H, dd, J=25, 8.6 Hz), 7.24-7.29(2H, m), 7.51-7.56(2H, m). | CD₃OD | 640 [M+H]⁺ |

**[Table 6]**

| Ex. No. | Structural formula | Compound name | ¹H-NMR (400 MHz) *δ* (ppm) from TMS | Solvent | ESI MS m/z |
|---|---|---|---|---|---|
| 119 | 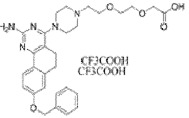 | 2-(2-(2-(4-(2-amino-8-(benzyloxy)-5,6-dihydrobenzo[h]quinazolin-4-yl)piperazin-1-yl)ethoxy)ethoxy)acetic acid bis(trifluoroacetate) | 2.56-2.63(2H, m), 2.71-2.79(2H, m), 3.33-3.39(2H, m), 3.12-3.24(2H, m), 3.41-3.54(2H, m), 3.64-3.64(6H, m), 3.82-3.87(2H, m), 4.17(2H, s), 4.23-4.37(2H, m), 5.07(2H, s), 692(1H, d, J=2.4 Hz), 6.96(1H, dd, J=2.4, 8.6 Hz), 7.33-7.45(5H, m), m 7.57(1H, d, J=8.6 Hz). | D₂O | 534 [M+H]⁺ |
| 120 | 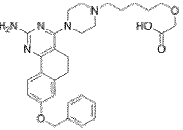 | 2-((5-(4-(2-amino-8-(benzyloxy)-5,6-dihydrobenzo[h]quinazolin-4-yl)piperazin-1-yl)pentyl)oxy)acetic acid | 1.35-1.48(2H, m), 1.55-1.72(4H, m), 2.48-294(10H, m), 3.42-3.58(6H, m), 3.95(2H, brs), 5.06(2H, s), 5.50-5.80(2H, m), 6.77(1H, d, J=1.7 Hz), 6.90(1H, dd, J=1.7, 8.6 Hz), 7.29-7.43(5H, m), 8.03(1H, d, J=8.6 Hz). | CDCl₃ | 532 [M+H]⁺ |
| 121 | 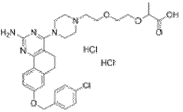 | 2-(2-(2-(4-(2-amino-8-((4-chlorobenzyl)oxy)-5,6-dihydrobenzo[h]quinazolin-4-yl)piperazin-1-yl)ethoxy)ethoxy)propanoi c acid dihydrochloride | 1.41(3H, d, J=6.0 Hz), 2.75-2.82(2H, m), 2.87-2.95(2H, m), 3.45-4.10(17H, m), 5.19(2H, s), 7.05-7.12(2H, m), 7.39-7.52(4H, m), 8.22(1H, d, J=7.9 Hz). | CD₃OD | 582 [M+H]⁺ |
| 122 | 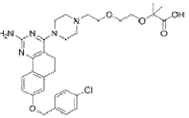 | 2-(2-(2-(4-(2-amino-8-((4-chlorobenzyl)oxy)-5,6-dihydrobenzo[h]quinazolin-4-yl)piperazin-1-yl)ethoxy)ethoxy)-2-methylpropanoic acid | 1.79(6H, s), 2.48-2.62(2H, m), 2.64-2.76(2H, m), 3.30-3.45(6H, m), 3.60-3.74(4H, m), 3.95-4.18(6H, m), 4.97(2H, brs), 6.71(1H, m), 6.97(1H, d, J=8.0 Hz), 7.39-7.52(4H, m), 8.22(1 H, d, J=8.0 Hz). | D₂O | 596 [M+H]⁺ |
| 123 | 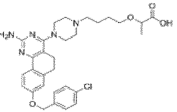 | 2-(4-(4-(2-amino-8-((4-chlorobenzyl)oxy)-5,6-dihydrobenzo[h]quinazolin-4-yl)piperazin-1-yl)butoxy)propanoic acid | 1.76(3H, d, J=6.6 Hz), 1.95-2.15(4H, m), 2.50-2.63(2H, m), 2.66-2.76(2H, m), 3.19-3.45(6H, m), 3.56-3.75(4H, m), 3.81-3.40(2H, m), 4.22-4.32(1H, m), 4.96(2H, brs), 6.73(1H, brs), 6.96(1H, d, J=7.8 Hz), 7.40-7.53(4H, m), 8.23(1 H, d, J=7.8 Hz). | D₂O | 566 [M+H]⁺ |
| 124 | 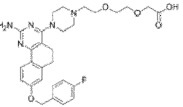 | 2-(2-(2-(4-(2-amino-8-((4-fluorobenzyl)oxy)-5,6-dihydrobenzo[h]quinazolin-4-yl)piperazin-1-yl)ethoxy)ethoxy)acetic acid | 2.47-2.60(2H, m), 2.66-2.74(2H, m), 3.26-3.40(6H, m), 3.59-3.78(4H, m), 4.07-4.18(6H, m), 4.40(2H, s), 4.99(2H, brs), 6.72(1H, brs), 7.00(1H d, J=8.8 Hz), 7.16-7.26(2H, m), 7.45-7.55(2H, m), 8.22(1 H, d, J=8.8 Hz). | D₂O | 552 [M+H]⁺ |
| 125 | 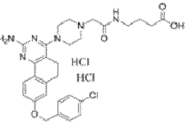 | 4-(2-(4-(2-amino-8-((4-chlorobenzyl)oxy)-5,6-dihydrobenzo[h]quinazolin-4-yl)piperazin-1-yl)acetamido)butanoic acid dihydrochloride | 2.33-2.42(2H, m), 2.91-2.97(2H, t, J=7.3 Hz), 3.19-3.24(2H, m), 3.33-3.39(2H, m), 3.84(2H, t, J=7.0 Hz), 4.02-4.09(4H, m), 4.49-4.57(6H, m), 5.67(2H, s), 7.51-7.60(2H, m), 7.92-7.98(4H, m), 8.20(1H, d, J=8.5 Hz). | D₂O | 565 [M+H]⁺ |
| 126 | 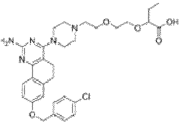 | 2-(2-(2-(4-(2-amino-8-((4-chlorobenzyl)oxy)-5,6-dihydrobenzo[h]quinazolin-4-yl)piperazin-1-yl)ethoxy)ethoxy)butanoic acid | 1.35(3H, t, J=7.5 Hz), 2.04-2.23(2H, m), 2.48-2.79(4H, m), 3.16-3.42(6H, m), 3.56-3.74(4H, m), 3.93-4.22(7H, m), 5.01(2H, brs), 6.73-6.77 (1H, m), 6.98-7.06(1 H, m), 7.43-7.55(4H, m), 8.22-8.28(1H, m). | D₂O | 596 [M+H]⁺ |

**[Table 7]**

| Ex. No. | Structural formula | Compound name | ¹H-NMR (400 MHz) δ (ppm) from TMS | Solvent | ESI MS m/z |
|---|---|---|---|---|---|
| 127(a) | 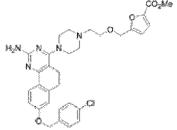 | methyl 5-((2-(4-(2-amino-8-((4-chlorobenzyl)oxy)-5,6-dihydrobenzo[h]quinazolin-4-yl)piperazin-1-yl)ethoxy)methyl)furan-2-carboxvlate | 2.57-2.68(8H, m), 2.73-2.81(2H, m), 3.29-3.36(4H, m), 3.67(2H, t, J=5.7 Hz), 3.89(3H, s), 4.55(2H, s), 4.75-4.82(2H, m), 5.06(2H, s), 6.44(1H, d, J=3.5 Hz), 6.79(1H, d, J=2.4 Hz), 6.89(1H, dd, J=2.6, 8.5 Hz), 7.14(1H, d, J=3.5 Hz), 7.33-7.39(4H, m), 8.03(1H, d, J=8.6 Hz). | CDCl₃ | 604 [M+H]⁺ |
| 127(b) | 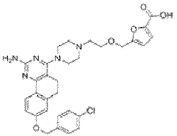 | 5-((2-(4-(2-amino-8-((4-chlorobenzyl)oxy)-5,6-dihydrobenzo[h]quinazolin-4-yl)piperazin-1-yl)ethoxy)methyl)furan-2-carboxylic acid | 2.51-2.62(8H, m), 2.64-2.70(2H, m), 3.20-3.25(4H, m), 3.57(2H, t, J=5.5 Hz), 4.47(2H, s), 5.01(2H, s), 6.42(1H, d, J=3.3 Hz), 6.86-6.89(2H, m), 692(1H, dd, J=2.5, 8.6 Hz), 7.35-7.47(4H, m), 7.90(1H, d, J=3.3 Hz). | DMSO-d₆ | 590 [M+H]⁺ |
| 128(a) | 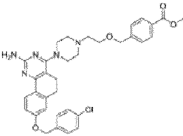 | methyl 4-((2-(4-(2-amino-8-((4-chlorobenzyl)oxy)-5,6-dihydrobenzo[hquinazolin-4-yl)p,peraz,n-1-yl)ethoxy)methyl)benzoate | 2.57-2.71(8H, m), 2.73-2.81(2H, m), 3.28-3.36(4H, m), 3.64(2H, t, J=5.7 Hz), 3.91(3H, s), 4.61(2H, s), 4.72-4.79(2H, m), 5.06(2H, s), 6.78(1H, d, J=2.4 Hz), 6.89(1H, dd, J=2.7, 8.6 Hz), 7.33-7.44(6H, m), 7.99-8.05(3H, m). | CDCl₃ | 614 [M+H]⁺ |
| 128(b) | 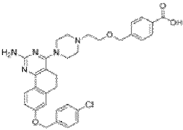 | 4-((2-(4-(2-amino-8-((4-chlorobenzyl)oxy)-5,6-dihydrobenzo[h]quinazolin-4-yl)piperazin-1-yl)ethoxy)methyl)benzoic acid | 2.61-2.71(8H,m), 2.73-2.79(2H, m), 3.28-3.34(4H, m), 3.67(2H, t, J=5.5 Hz), 4.57(2H, s), 5.10(2H, s), 6.85-6.86(1 H, m), 6.89-6.94(1 H, m), 7.32-7.46(6H, m), 7.86-7.96(3H, m). | CD₃OD | 600 [M+H]⁺ |
| 129(a) | 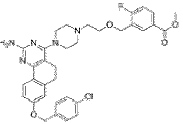 | methyl 3-((2-(4-(2-amino-8-((4-chlorobenzyl)oxy)-5,6-dihydrobenzo[h]quinazolin-4-yl)piperazin-1-yl)ethoxy)methyl)-4-fluorobenzoate | 2.59-2.72(8H, m), 2.74-2.81(2H, m), 3.30-3.36(4H, m), 3.68(2H, t, J=5.8 Hz), 3.91(3H, s), 4.63(2H, s), 4.69-4.78(2H, m), 5.06(2H, s), 6.79(1H, d, J=2.6 Hz), 6.89(1H, dd, J=2.6, 8.6 Hz), 7.10(1 H, t, J=8.6 Hz), 7.33-7.39(4H, m), 7.96-8.02(1H, m), 8.04(1H, d, J=8.6 Hz), 8.15(1H, dd, J=2.2, 7.1 Hz). | CDCl₃ | 632 [M+H]⁺ |
| 129(b) | 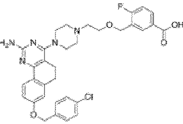 | 3-((2-(4-(2-amino-8-((4-chlorobenzyl)oxy)-5,6-dihydrobenzo[h]quinazolin-4-yl)piperazin-1-yl)ethoxy)methyl)-4-fluorobenzoic acid | 2.65-2.70(2H, m), 2.77-2.83(2H, m), 2.91-3.00(6H, m), 3.46-3.53(4H, m), 3.78(2H, t, J=5.3 Hz), 4.66(2H, s), 5.14(2H, s), 6.90-6.98(2H, m), 709-716(1H, m), 7.37-7.47(4H, m), 787(1H, d, J=8.6 Hz), 7.93-8.00(1 H, m), 8.07-8.13(1 H, m). | CD₃OD | 618 [M+H]⁺ |
| 130 | 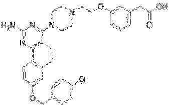 | 2-(3-(2-(4-(2-amino-8-((4-chlorobenzyl)oxy)-5,6-dihydrobenzo[h]quinazolin-4-yl)piperazin-1-yl)ethoxy)phenyl)acetic acid | 2.55-2.69(6H, m), 2.71-2.81(4H, m), 3.13-3.23(4H, m), 3.53(2H, s), 4.12(2H, t, J=5.4 Hz), 5.16(2H,s), 6.03(2H, brs), 6.86-7.03(4H, m), 7.18(1 H, dd, J=1.7, 7.2 Hz), 7.21 (1H, td, J=7.9, 1.7 Hz), 7.44-7.52(4H, m), 7.94(1H, d, J=8.4 Hz). | DMSO-d₆ | 600 [M+H]⁺ |
| 131(a) | 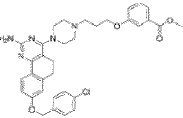 | methyl 3-(3-(4-(2-amino-8-((4-chlorobenzyl)oxy)-5,6-dihydrobenzo[h]quinazolin-4-yl)piperazin-1-yl)propoxy)benzoate | 1.97-2.04(2H, m), 2.54-2.61 (6H, m), 2.63-2.69(2H, m), 2.74-2.81(2H, m), 3.28-3.35(4H, m), 3.91(3H, s), 4.06-4.11(2H, m), 4.75-4.79(2H, m), 5.06(2H, s), 6.79(1H, d, J=2.4 Hz), 6.89(1H, dd, J=2.4, 8.6 Hz), 7.10(1H, ddd, J=0.9, 2.3, 8.6 Hz), 7.30-7.30(5H, m), 7.55-7.58(1 H, m), 7.60-7.64(1H. m). 8.04(1H. d. J=8.6Hz). | CDCl₃ | 614 [M+H]⁺ |
| 131(b) | 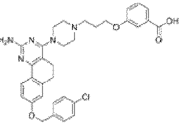 | 3-(3-(4-(2-amino-8-((4-chlorobenzyl)oxy)-5,6-dihydrobenzo[h]quinazolin-4-yl)piperazin-1-yl)propoxy)benzoic acid | 1.89-1.97(2H, m), 2.46-2.56(6H, m), 2.57-2.63(2H, m), 2.70-2.77(2H, m) 3.16-3.24(4H, m), 4.09(2H, t, J=6.4 Hz), 5.16(2H, s), 6.03(2H, brs), 6.92-6.97(2H, m), 7.20(1H, ddd, J=1.0, 2.6, 8.2 Hz), 7.38-7.55(7H, m),7.93(1 H, d, J=8.4 Hz). | DMSO-d₆ | 600 [M+H]⁺ |

**[Table 8]**

| Ex. No. | Structural formula | Compound name | ¹H-NMR (400 MHz) δ (ppm) from TMS | Solvent | ESI MS m/z |
|---|---|---|---|---|---|
| 132 | 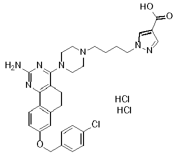 | 1-(4-(4-(2-amino-8-((4-chlorobenzyl)oxy)-5,6-dihydrobenzo[h]quinazolin-4-yl)piperazin-1-yl)butyl)-1H-pyrazole-4-carboxylic acid dihydrochloride | 1.30-1.70(2H, m) 1.78-1.88(2H, m) 2.56-2.64(2H, m) 2.70-2.77(2H, m) 3.02-3.22,(6H, m) 3.40-3.83(4H, m) 4.11-4.23(2H, m) 5.15(2H, s) 6.10-6.23(1.5H, m) 6.91-6.98(2H, m) 7.44-7.51(4H, m) 7.82(1 H, brs) 7.93(1 H, d, J=8.4 Hz) 8.28(1 H, brs) 9.75-9.92(0.5H, m) 12.31(1H, brs). | DMSO-d₆ | 588 [M+H]⁺ |
| 133 | 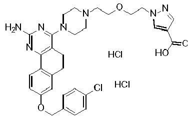 | 1-(2-(2-(4-(2-amino-8-((4-chlorobenzyl)oxy)-5,6-dihydrobenzo[h]quinazolin-4-yl)piperazin-1-yl)ethoxy)ethyl)-1H-pyrazole-4-carboxylic acid dihydrochloride | 2.62-2.70(2H, m), 2.76-2.85(2H, m), 3.05-3.17(2H, m), 3.25-3.35(4H, m), 3.35-3.55(4H, m), 3.76-3.87(4H, m), 4.36(2H, t, J=5.0 Hz), 5.22(2H, s), 7.07-7.14(2H, m), 7.45-7.53(4H, m), 7.83(1 H, s), 7.96(1H, d, J=9.5 Hz), 8.33(1H, s), 10.65-1095(1H, m), 1210-1260(1H, m), 13.10-13.40(1H, m). | DMSO-d₆ | 604 [M+H]⁺ |
| 134 | 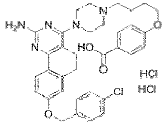 | 4-(4-(4-(2-amino-8-((4-chlorobenzyl)oxy)-5,6-dihydrobenzo[h]quinazolin-4-yl)piperazin-1-yl)butoxy)benzoic acid dihydrochloride | 1.88-2.05(4H, m), 2.73-2.89(2H, m), 2.87-2.94(2H, m), 3.26-3.75(10H, m), 4.15(2H, t, J=5.7 Hz), 5.19(2H, s), 6.98-7.02(2H, m), 7.04-7.10(2H, m), 7.37-7.47(4H, m), 772(1H, d, J=8.4 Hz), 7.94-8.00(2H, m). | CD₃OD | 614 [M+H]⁺ |
| 135(a) | 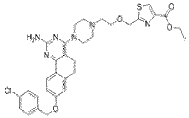 | ethyl 2-((2-(4-(2-amino-8-((4-chlorobenzyl)oxy)-5,6-dihydrobenzo[h]quinazolin-4-yl)piperazin-1-yl)ethoxy)methyl)thiazole-4-carboxylate | 1.41(3H, t, J=7.1 Hz), 257-284(10H, m), 3.27-3.39(4H, m), 3.77(2H, t, J=5.5 Hz), 4.43(2H, q, J=7.1 Hz), 4.69(2H, s), 4.88(2H, s), 5.07(2H, s), 6.79(1H, d, J=2.4 Hz), 6.89(1H, dd, J=2.4, 8.6 Hz), 7.33-7.41 (4H, m), 8.03(1 H, d, J=8.5 Hz), 8.18(1 H, s). | CDCl₃ | 635 [M+H]⁺ |
| 135(b) | 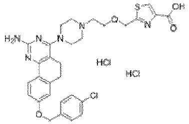 | 2-((2-(4-(2-amino-8-((4-chlorobenzyl)oxy)-5,6-dihydrobenzo[h]quinazolin-4-yl)piperazin-1-yl)ethoxy)methyl)thiazole-4-carboxylic acid dihydrochloride | 2.53-2.75(10H, m), 3.12-3.20(4H, m), 3.72(2H, t, J=5.6 Hz), 4.81(2H, s), 5.14(2H, s), 6.01(2H, brs), 6.90-6.91(2H, m), 7.43-7.51(4H, m), 7.92(1 H, d, J=8.6 Hz), 8.42(1H, s). | DMSO-d₆ | 607 [M+H]⁺ |
| 136 | 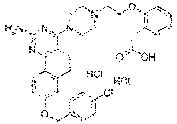 | 2-(2-(2-(4-(2-amino-8-((4-chlorobenzyl)oxy)-5,6-dihydrobenzo[h]quinazolin-4-yl)piperazin-1-yl)ethoxy)phenyl)acetic acid dihydrochloride | 2.44-2.50(6H, m), 2.75-2.74(2H, m), 2.77-2.84(2H, m), 3.25-3.68(8H, m), 4.42(2H, brs), 5.22(2H, s), 6.92-6.98(1H, m), 7.03(1H, d, J=8.6 Hz), 7.07-7.14(2H, m), 7.20-7.30(2H, m), 7.45-7.52(4H, m), 791(1H, d, J=8.6 Hz). | DMSO-d₆ | 600 [M+H]⁺ |

**[Table 9]**

| Ex. No. | Structural formula | Compound name | ¹H-NMR (400 MHz) δ (ppm) from TMS | Solvent | ESI MS m/z |
|---|---|---|---|---|---|
| 137 | 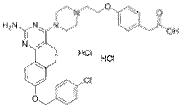 | 2-(4-(2-(4-(2-amino-8-((4-chlorobenzyl)oxy)-5,6-dihydrobenzo[h]quinazolin-4-yl)piperazin-1-yl)ethoxy)phenyl)acetic acid dihydrochloride | 2.63-2.74(2H, m), 2.77-2.85(2H, m), 3.22-3.72(14H, m), 4.36-4.44(2H, m), 5.22(2H, s), 6.96(2H, d, J=7.0 Hz), 7.06-7.14(2H, m), 7.22(2H, d, J=7.0 Hz), 7.44-7.52(4H, m), 788-794(1H, m). | DMSO-d₆ | 600 [M+H]⁺ |
| 138 | 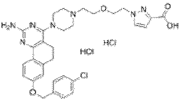 | 1-(2-(2-(4-(2-amino-8-((4-chlorobenzyl)oxy)-5,6-dihydrobenzo[h]quinazolin-4-yl)piperazin-1-yl)ethoxy)ethyl)-1 H-pyrazole-3-carboxylic acid dihydrochloride | 2.44-2.50(6H, m), 2.52-2.62(2H, m), 2.68-2.76(2H,m), 3.14-3.19(4H, m), 3.51(2H, t, J=5.6 Hz), 3.73(2H, t, J=5.6 Hz), 4.68(2H, t, J=5.6 Hz), 5.15(2H, s), 6.05(2H, brs), 6.73(1H, d, J=1.9 Hz), 6.92-6.96(2H, m), 7.43-7.52(5H, m),7.93(1H, d, J=8.5 Hz). | DMSO-d₆ | 604 [M+H]⁺ |
| 139 | 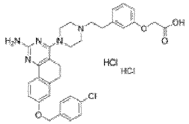 | 2-(3-(2-(4-(2-amino-8-((4-chlorobenzyl)oxy)-5,6-dihydrobenzo[h]quinazolin-4-yl)piperazin-1-yl)ethyl)phenoxy)acetic acid dihydrochloride | 2.62-2.73(2H, m), 2.76-2.82(2H, m), 2.98-3.08(2H, m), 3.12-3.28(4H, m), 3.30-3.45(4H, m), 3.55-3.68(2H, m), 4.68(2H, s), 5.20(2H, s), 6.88-684(1H, m), 6.86-6.90(2H, m), 6.95-7.12(2H, m), 7.24-7.31 (1H, m), 7.40-7.52(4H, m), 7.90-7.99(1H, m). | DMSO-d₆ | 600 [M+H]⁺ |
| 140 | 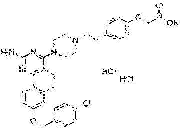 | 2-(4-(2-(4-(2-amino-8-((4-chlorobenzyl)oxy)-5,6-dihydrobenzo[h]quinazolin-4-yl)piperazin-1-yl)ethyl)phenoxy)acetic acid dihydrochloride | 2.65-2.73(2H, m), 2.78-2.85(2H, m), 2.95-304(2H, m), 3.14-3.27(2H, m), 3.28-3.39(4H, m), 3.57-3.70(4H, m), 4.66(2H, s), 4.47-4.57(2H, m), 5.23(2H, s), 6.91(2H, d), 7.06-7.16(2H, m), 7.21(2H, d, J=8.7 Hz), 7.46-7.54(4H, m), 7.88-795(1H, m). | DMSO-d₆ | 600 [M+H]⁺ |
| 141 | 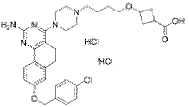 | 3-(4-(4-(2-amino-8-((4-chlorobenzyl)oxy)-5,6-dihydrobenzo[h]quinazolin-4-yl)piperazin-1-yl)butoxy)cyclobutane-1-carboxylic acid dihydrochloride | 1.61-1.70(2H, m), 1.84-1.94(2H, m), 2.05-2.13(2H, m), 2.47-2.56(2H, m), 2.64-2.74(1H, m), 2.75-2.81(2H, m), 2.88-2.94(2H, m), 3.13-3.80(12H, m), 3.89-3.97(1H, m), 5.19(2H, s), 7.06-7.11(2H, m), 7.38-7.47(4H, m), 7.73(1H, d, J=8.3 Hz). | CD₃OD | 592 [M+H]⁺ |
| 142 | 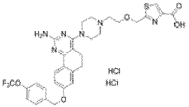 | 2-((2-(4-(2-amino-8-((4-(trifluoromethoxy)benzyl)o xy)-5,6-dihydrobenzo[h]quinazolin-4-yl)piperazin-1-yl)ethoxy)methyl)thiazole-4-carboxylic acid dihydrochloride | 2.58-4.07 (16H, m, superimposed by DMSO, H2O), 4.90 (2H, brs), 5.20 (2H, s), 6.18 (1 H, brs), 6.94-7.06 (2H, m), 7.41 (2H, d, J=8.3 Hz), 7.60 (2H, d, J=8.3 Hz), 7.90-7.98 (1H, m), 8.49 (1H, s), 9.97 (1H, s), 13.08 (1H, s) | DMSO-d₆ | 657 [M+H]⁺ |
| 143 | 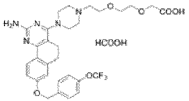 | 2-(2-(2-(4-(2-amino-8-((4-(trifluoromethoxy)benzyl)o xy)-5,6-dihydrobenzo[h]quinazolin-4-yl)piperazin-1-yl)ethoxy)ethoxy)acetic acid formate | 2.52-2.63(8H, m), 2.68-2.78(2H, m), 3.17(4H, brs), 3.52-3.60(6H, m), 4.02(2H, s), 5.18(2H, s), 6.02(2H, s), 6.90-6.99(2H, m), 7.40(2H, d, J=7.8 Hz), 7.55-7.64(2H, m), 7.93(1H, d, J=8.4 Hz), 8.15(0.6H, s). | DMSO-d₆ | 618 [M+H]⁺ |

### (Example 21)

### 4-(4-(2-(2-((tetrazol-5-yl)methoxy)ethoxy)ethyl)pipera zin-1-yl)-8-((4-chlorobenzyl)oxy)-5,6-dihydrobenzo[h]q uinazolin-2-amine

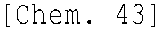

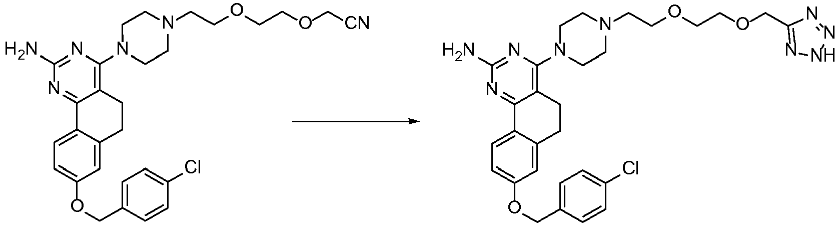

The compound (15 mg, 0.027 mmol) obtained in Example 10 was dissolved in DMF (0.3 mL), and sodium azide (5.3 mg, 0.082 mmol, 3.0 equivalents) and ammonium chloride (4.4 mg, 0.082 mmol, 3.0 equivalents) were added, followed by stirring at 100°C for 2 hours. Water was added to the reaction liquid, followed by extracting with ethyl acetate. The organic layer was washed with brine and dried over anhydrous sodium sulfate. The solvent was distilled off under reduced pressure to obtain a crude product. This crude product was purified through an HPLC (mobile phase, water : acetonitrile) to obtain 17.3 mg of the title compound.
¹H-NMR (400 MHz, CDCl₃) δ: 2.55-2.86 (8H, m), 3.32-3.37 (2H, m), 3.42-3.49 (4H, m), 3.57-3.68 (6H, m), 4.93 (2H, s), 5.03 (2H, s), 6.74-6.81 (1H, m), 6.86-6.94 (1H, m), 7.31-7.39 (4H, m), 8.06 (1H, d, J=8.6 Hz).
MS (ESI) m/z: 592 [M+H]⁺.

### (Example 22)

### 4-(4-(2-((tetrazol-5-yl)methoxy)ethyl)piperazin-1-yl)-8-((4-chlorobenzyl)oxy)-5,6-dihydrobenzo[h]quinazolin-2-amine

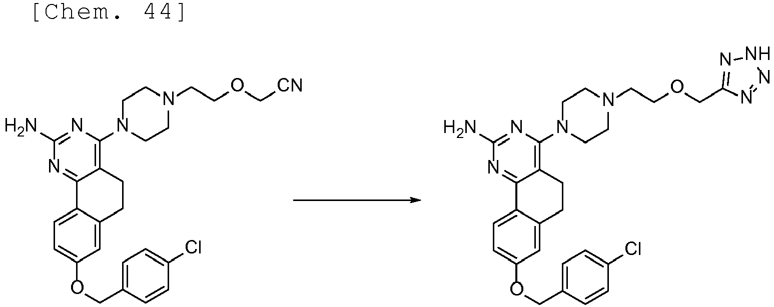

The title compound was obtained in the same method as in Example 21 except that 2-(2-(4-(2-amino-8-((4-chlorobenzyl)oxy)-5,6-dihydrobe nzo[h]quinazolin-4-yl)piperazin-1-yl)ethoxy)acetonitri le obtained in the same method as in Example 10 was used as a starting material.
¹H-NMR (400 MHz, CDCl₃) δ: 2.60-2.68 (2H, m), 2.76-2.92 (8H, m), 3.58-3.64 (4H, m), 3.83-3.89 (2H, m), 4.99 (2H, s), 5.07 (2H, s), 6.79 (1H, d, J=2.3 Hz), 6.92 (1H, dd, J=2.3, 8.6 Hz), 7.32-7.39 (4H, m), 8.09 (1H, d, J=8.6 Hz). MS (ESI) m/z: 548 [M+H]⁺.

### (Examples 144 to 145)

Compounds in Table 10 were obtained using the same methods as in Examples 21, 22. Table 10 shows the structural formula, the compound name, the nuclear magnetic resonance spectrum (¹H-NMR) and its solvent, and the result of the electrospray ionization mass spectrum (ESI MS) of each compound.

**[Table 10]**

| Ex. No. | Structural formula | Compound name | ¹H-NMR (400 MHz) δ (ppm) from TMS | Solvent | ESI MS m/z |
|---|---|---|---|---|---|
| 144 | 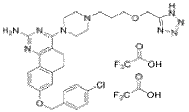 | 4-(4-(3-((tetrazol -5-yl)methoxy)propyl)piperazi n-1 -yl)-8-((4-chlorobenzyl)oxy)-5,6-dihydrobenzo[h]quinazolin-2-amine bis(trifluoroacetate) | 2.11-2.19(2H, m), 2.74-2.81(2H, m), 2.87-2.94(2H, m), 3.39(2H, d, J=7.2 Hz), 3.45-3.58(4H, m), 3.79(2H, t, J=5.5 Hz), 3.89-4.25(4H, m), 4.93(2H, s), 5.19(2H, s), 7.05-7.10(2H, m), 7.38-7.46(4H, m), 7.71(1H, d, J=8.8 Hz) | CD₃OD | 562 [M+H]⁺ |
| 145 | 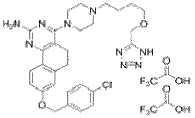 | 4-(4-(4-((tetrazol-5-yl)methoxy)butyl)piperazin -1-yl)-8-((4-chlorobenzyl)oxy)-5,6-dihydrobenzo[h]quinazolin-2-amine bis(trifluoroacetate) | 1.70-1.78(2H, m), 1.85-1.95(2H, m), 2.73-2.80(2H, m), 2.86-2.93(2H, m), 3.34-3.37(4H, m), 3.20-3.28(2H, m), 3.37-3.53(4H, m), 3.66(2H, t, J=5.8 Hz), 3.82-4.15(2H, m), 5.19(2H, s), 7.05-7.10(2H, m), 7.37-7.47(4H, m), 7.70(1 H, d, J=8.6 Hz) | CD₃OD | 576 [M+H]⁺ |

### (Example 23)

### cis-3-(2-(4-(2-amino-8-((4-(trifluoromethoxy)benzyl)ox y)-5,6-dihydrobenzo[h]quinazolin-4-yl)piperazin-1-yl)e thoxy)cyclobutane-1-carboxylic acid

### (a) methyl cis-3-(2-(4-(2-amino-8-((4-(trifluoromethoxy)benzyl)ox y)-5,6-dihydrobenzo[h]quinazolin-4-yl)piperazin-1-yl)e thoxy)cyclobutane-1-carboxylate

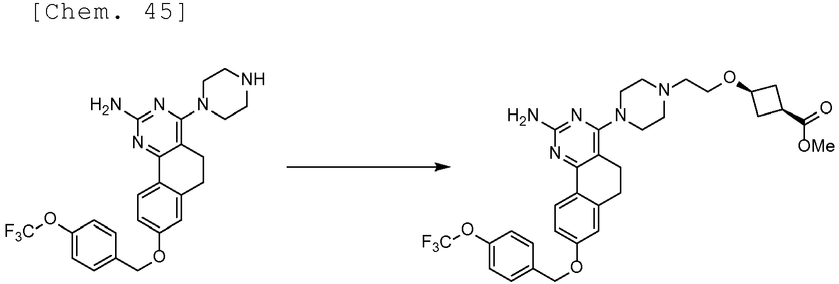

The compound (1.98 g, 4.2 mmol, 1.0 equivalent) obtained in Example 6 and potassium iodide (1.39 g, 8.4 mmol, 2.0 equivalents) were suspended into a DMF (4 mL) solution of methyl cis-3-(2-chloroethoxy)cyclobutanecarboxylate (1.62 g, 8.4 mmol, 2.0 equivalents) obtained in Reference Example 4(a). Thereafter, N,N-diisopropylethylamine (2.19 mL, 12.6 mmol, 3.0 equivalents) was added, followed by stirring at 70°C for 6 hours in an argon atmosphere. A crude product obtained by distilling off the solvent under reduced pressure was purified through an amino silica gel column chromatography (eluent, ethyl acetate : hexane) to obtain the title compound (2.01 g).
¹H-NMR (400 MHz, CDCl₃) δ: 2.17-2.30 (2H, m), 2.46-2.56 (2H, m), 2.59-2.71 (8H, m), 2.73-2.84 (2H, m), 3.25-3.37 (4H, m), 3.51 (2H, t, J=5.9 Hz), 3.68 (3H, s), 3.83-3.98 (1H, m), 4.63-4.72 (1H, m), 5.10 (2H, s), 6.80 (1H, d, J=2.5 Hz), 6.90 (1H, dd, J=8.6, 2.5 Hz), 7.24 (2H, d, J=8.4 Hz), 7.47 (2H, d, J=8.4 Hz), 8.04 (1H, d, J=8.6 Hz).
MS (ESI) m/z: 628 [M+H]⁺

### (b) cis-3-(2-(4-(2-amino-8-((4-(trifluoromethoxy)benzyl)ox y)-5,6-dihydrobenzo[h]quinazolin-4-yl)piperazin-1-yl)e thoxy)cyclobutane-1-carboxylic acid

The title compound was obtained in the same method as in Example 11(b) using the compound obtained in Example 23(a). Table 11 described below shows the structural formula, the nuclear magnetic resonance spectrum (¹H-NMR) and its solvent, and the result of the electrospray ionization mass spectrum (ESI MS) of this compound.

### (Examples 24 to 32, 35 to 37, 146 to 186)

Compound described in Tables 11 to 19 below were obtained in the same method as or a method similar to that in Example 23. Tables 11 to 19 show the structural formula, the compound name, the nuclear magnetic resonance spectrum (¹H-NMR) and its solvent, and the result of the electrospray ionization mass spectrum (ESI MS) of each compound. Note that in Tables below, (a) and (b) described in the Example number indicate the examples were obtained by the method (a) and the method (b) in the production method of Example 23, respectively.

**[Table 11]**

| Ex. No. | Structural formula | Compound name | ¹H-NMR (400 MHz) δ (ppm) from TMS | Solvent | ESI MS m/z |
|---|---|---|---|---|---|
| 23(b) | 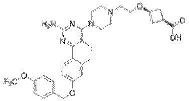 | cis-3-(2-(4-(2-amino-8-(trifluoromethoxy)benzyl)oxy)-5,6-dihydrobenzo[h]quinazolin-4-yl)piperazin-1-yl)ethoxy)cyclobutane-1-carboxylic acid | 1.91-2.00(2H, m), 2.41-2.52(2H, m), 2.58-2.62(3H overlapped with DMSO, m) 2.72-2.75(2H, m), 3.17(4H, brs), 3.32(4H overlapped with DHO, s), 3.37-3.49(2H, m), 3.82-3.89(1H, m), 5.17-5.22(2H, m), 6.02(2H, s), 6.93-6.98(2H, m), 7.42(2H, d, J=7.7 Hz), 7.60(2H, d, J=7.7 Hz), 7.94(1 H, d, J=8.6 Hz), 8.32(1 H, s). | DMSO-d₆ | 614 [M+H]⁺ |
| 24 | 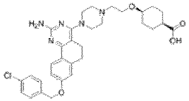 | cis-4-(2-(4-(2-amino-8-((4-chlorobenzyl)oxy)-5,6-dihydrobenzo[h]quinazolin-4-yl)piperazin-1-yl)ethoxy)cyclohexane-1-carboxylic acid | 1.45-1.58(4H, m), 1.63-1.75(4H, m), 2.24-2.33(1H, m), 2.49-2.59(8H, m), 2.70-2.74(2H, m), 3.16(4H, brs), 3.43(1 H, brs), 3.50(2H, t, J=5.9 Hz), 5.15(2H, s), 6.04(2H, brs), 6.93-6.98(2H, m), 7.45-7.53(4H, m), 7.95(1 H, d, J=8.2 Hz). | DMSO-d₆ | 592 [M+H]⁺ |
| 25(a) | 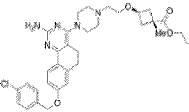 | ethyl trans-3-(2-(4-(2-amino-8-((4-chlorobenzyl)oxy)-5,6-dihydrobenzo[h]quinazolin-4-yl)piperazin-1-yl)ethoxy)-1-methylcyclobutane-1-carboxylate | 1.27(3H, t, J=7.1 Hz), 1.40(3H, s), 1.87-1.95(2H, m), 2.55-2.69(8H, m), 2.69-2.82(4H, m), 3.26-3.38(4H, m), 3.50(2H, t, J=6.0 Hz), 4.03(1H, quin, J=7.1 Hz), 4.15(2H, q, J=7.1 Hz), 4.72(2H, s), 5.06(2H, s), 6.79(1H, brd, J=2.5 Hz), 6.89(1H, dd, J=2.5, 8.6 Hz), 7.33-7.39(4H, m), 8.03(1H, d, J=8.6 Hz). | CDCl₃ | 606 [M+H]⁺ |
| 25(b) | 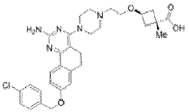 | trans-3-(2-(4-(2-amino-8-((4-chlorobenzyl)oxy)-5,6-dihydrobenzo[h]quinazolin-4-yl)piperazin-1-yl)ethoxy)-1-methylcyclobutane-1-carboxylic acid | 1.42(3H, s), 1.86-1.96(2H, m), 2.58-2.70(8H, m), 2.72-2.85(4H, m), 3.29-3.42(4H, m), 3.52(2H, t, J=5.9 Hz), 4.08(1H, quin, J=7.1 Hz), 5.09(2H, s), 6.82(1H, brd, J=2.5 Hz), 6.92(1H, dd, J=8.6, 2.5 Hz), 7.34-7.43(4H, m), 7.94(1H, d, J=8.6 Hz). | CD₃OD | 578 [M+H]⁺ |
| 26(a) | 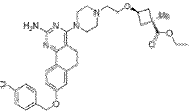 | ethyl cis-3-(2-(4-(2-amino-8-((4-chlorobenzyl)oxy)-5,6-dihydrobenzo[h]quinazolin-4-yl)piperazin-1-yl)ethoxy)-1-Hz), methylcyclobutane-1 - carboxylate | 1.27(3H, t, J=7.1 Hz), 1.38(3H, s), 2.16-2.26(2H, m), 2.36-2.49(2H, m), 2.54-2.70(8H, m), 2.73-2.85(2H, m), 3.23-3.38(4H, m), 3.51(2H, t, J=5.9 Hz), 4.04(1 H, quin, J=7.2 Hz), 4.15(2H, q, J=7.1 Hz), 4.71(2H, s), 5.07(2H, s), 6.80(1H, brd, J=2.6 Hz), 6.90(1 H, dd, J=2.6, 8.6 Hz), 7.33(4H, m), 8.04(1H, d, J=8.6 Hz). | CDCl₃ | 606 [M+H]⁺ |
| 26(b) | 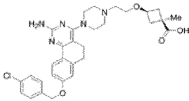 | cis-3-(2-(4-(2-amino-8-((4-chlorobenzyl)oxy)-5,6-dihydrobenzo[h]quinazolin-4-yl)piperazin-1-yl)ethoxy)-1-methylcyclobutane-1-carboxylic acid | 1.38(3H, s), 2.13-2.24(2H, m), 2.41-2.53(2H, m), 2.53-2.65(2H, m), 2.65-2.85(8H, m), 3.41(4H, m), 3.52(2H, t, J=5.4 Hz), 4.01(1 H, quin, J=7.0 Hz), 5.05(2H, s), 5.30-5.90(2H, brs), 6.77(1 H, brd, J=2.6 Hz), 6.89(1H, dd, J=2.6, 8.6 Hz), 7.32-7.39(4H, m), 8.03(1 H, d, J=8.6 Hz). | CDCl₃ | 578 [M+H]⁺ |
| 27 | 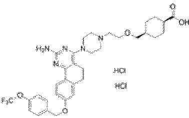 | cis-4-((2-(4-(2-amino-8-((4-(trifluoromethoxy)benzyl)oxy)-5,6-dihydrobenzo[h]quinazolin-4-yl)piperazin-1-yl)ethoxy)methyl)cyclohex ane-1-carboxylic acid dihydrochloride | 117-127(2H, m), 1.44-1.55(4H, m), 1.66(1 H, m), 1.84-1.88(2H, m), 2.60-2.68(2H, m), 2.81(2H, m), 3.23-3.40(10H, m), 3.53-3.56(3H, m), 3.77(2H, brs), 5.24(2H, m), 7.09 (2H, brs), 7.41(2H, d, J=8.4 Hz), 7.60(2H, d, J=8.4 Hz), 7.98(1H, d, J=9.0 Hz). | DMSO-d₆ | 656 [M+H]⁺ |

**[Table 12]**

| Ex. No. | Structural formula | Compound name | ¹H-NMR (400 MHz) δ (ppm) from TMS | Solvent | ESI MS m/z |
|---|---|---|---|---|---|
| 28 | 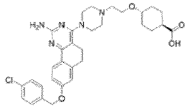 | trans-4-(2-(4-(2-amino-8-((4-chlorobenzyl)oxy)-5,6-dihydrobenzo[h]quinazolin-4-yl)piperazin-1-yl)ethoxy)cyclohexane-1-carboxylic acid | 1.11-1.23(3H, m), 1.28-1.39(2H, m), 1.87-1.91(2H, m), 1.95-1.99(2H, m), 2.11-2.22(1H, m), 2.45-2.59(5H, m, overlapped with DMSO), 2.70-2.74(2H, m), 3.09-3.27(6H, m, overlapped with DHO), 3.42-3.48(1 H, m), 3.54(2H, t, J=6.1 Hz), 5.14(2H, s), 6.01(2H, brs), 6.92-6.95(2H, m), 7.43-7.52(4H. m). 7.92(1 H. d. J=8.3 Hz). | DMSO-d₆ | 592 [M+H]⁺ |
| 29 | 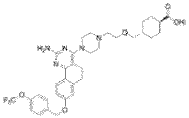 | trans-4-((2-(4-(2-amino-8-((4-(trifluoromethoxy)benzyl)o xy)-5,6-dihydrobenzo[h]quinazolin-4-yl)piperazin-1-yl)ethoxy)methyl)cyclohexane-1-carboxylic acid | 0.87-1.01(2H, m), 1.22-1.34(2H, m), 1.43-1.54(1H, m), 1.72-1.79(2H, m), 1.85-1.93(2H, m), 207-215(1H, m), 2.52-2.61(8H, m), 2.66-2.78(2H, m), 3.12-3.23(6H, m), 3.49(2H, t, J=5.9 Hz), 5.18(2H, s), 6.01(2H, s), 6.92-6.97(2H , m), 7.40(2H, d, J=8.0 Hz), 7.60(2H, d, J=8.0 Hz), 7.93(1H, d, J=8.3 Hz). | DMSO-d₆ | 656 [M+H]⁺ |
| 30 | 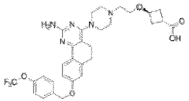 | trans-3-(2-(4-(2-amino-8-((4-(trifluoromethoxy)benzyl)o xy)-5,6-dihydrobenzo[h]quinazolin-4-yl)piperazin-1-yl)ethoxy)cyclobutane-1-carboxylic acid | 2.05-2.18(2H, m), 2.31-2.41(2 H, m), 2.52-2.64(8H, m), 2.72-2.75(2H, m), 2.86-2.96(1 H, m), 3.17(4H, brs), 3.43(2H, t, J=5.9 Hz,), 4.03-4.16(1H, m), 5.19(2H, s), 6.02(2H, s), 6.91-6.98(2H, m), 7.41(2H, d, J=8.2 Hz), 7.61(2H, d, J=8.2 Hz), 7.94(H, d, J=8.6 Hz), 11.96-12.33(1H, m). | DMSO-d₆ | 614 [M+H]⁺ |
| 31 | 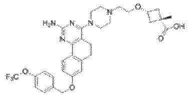 | cis-3-(2-(4-(2-amino-8-((4-(trifluoromethoxy)benzyl)oxy)-5,6-dihydrobenzo[h]quinazolin-4-yl)piperazin-1-yl)ethoxy)-1-methylcyclobutane-1-carboxylic acid | 1.28(3H, s), 2.06-2.14(2H, m), 2.15-2.24(2H, m), 2.46-2.56(6H, m), 2.56-2.64(2H, m), 2.69-2.77(2H, m), 3.10-3.22(4H, m), 3.41(2H, t, J=5.9 Hz), 4.02(1 H, quin, J=7.2 Hz), 5.18(2H, s), 6.02(2H, s), 6.92-6.98(2H, m), 7.37-7.43(2H, m), 7.57-7.65(2H, m), 7.93(1H, d, J=8.3 Hz). | DMSO-d₆ | 628 [M+H]⁺ |
| 32 | 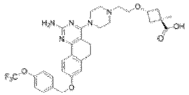 | trans-3-(2-(4-(2-amino-8-((4-(trifluoromethoxy)benzyl)oxy)-5,6-dihydrobenzo[h]quinazolin-4-yl)piperazin-1-yl)ethoxy)-1-methylcyclobutane-1 - carboxylic acid | 1.31(3H, s), 1.72-1.82(2H, m), 2.47-2.56(6H, m), 2.56-2.65(4H, m), 2.69-2.77(2H, m), 3.09-3.21(4H, m), 3.41(2H, t, J=5.9 Hz), 395(1H, quin, J=7.0 Hz), 5.18(2H, s), 6.02(2H, s), 6.91-6.98(2H, m), 7.37-7.44(2H, m), 7.57-7.63(2H, m), 7.93(1 H, d, J=8.4 Hz). | DMSO-d₆ | 628 [M+H]⁺ |
| 35 | 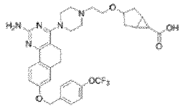 | 3-(2-(4-(2-amino-8-((4-(trifluoromethoxy)benzyl)o xy)-5,6-dihydrobenzo[h]quinazolin-4-yl)piperazin-1-yl)ethoxy)bicyclo[3.1.0]hexane-6-carboxylic acid | Mixture of isomers: 1.29(1 H, m), 1.58(0.4H, t, J=8.3 Hz), 1.78-1.91(3H, m), 2.05-2.10(0.4H, m), 2.25-2.34(1.4H, m), 2.64-2.74(2H, m), 2.77-2.83(2H, m), 2.83-3.01(6H, m), 3.46-3.55(4H, m), 3.55-3.64(2H, m), 3.66-3.77(1H, m), 3.98(0.3H, m), 4.05-4.1 2(0.5H, m), 5.17(2H, s), 6.92-7.00(2H, m), 7.29(2H, d, J=7.9 Hz), 7.56(2H, d, J=7.9 Hz), 7.87(1H. dd. J=8.6. 2.3 Hz). | CD₃OD | 640 [M+H]⁺ |

**[Table 13]**

| Ex. No. | Structural formula | Compound name | ¹H-NMR (400 MHz) δ (ppm) from TMS | Solvent | ESI MS m/z |
|---|---|---|---|---|---|
| 146 | 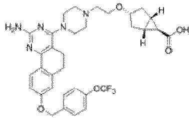 | (1R,3r,5S,6r)-3-(2-(4-(2-amino-8-((4-(trifluoromethoxy)benzyl)o xy)-5,6-dihydrobenzo[h]quinazolin-4-yl)piperazin-1-yl)ethoxy)bicyclo[3.1.0]hex ane-6-carboxylic acid | 1.74-1.77(3H, m), 1.95-2.08(4H, m), 2.64-281(10H, m), 3.32-3.43(4H, m), 3.52(2H, t, J=5.8 Hz), 3.95(1 H, t, J=5.8 Hz), 5.16(2H, s), 6.90(1 H, d, J=2.4 Hz), 6.95(1H, dd, J=2.4, 8.5 Hz), 7.29(2H, d, J=7.6 Hz), 7.56(2H, d, J=7.6 Hz), 7.91 (1 H, d, J=8.5 Hz). | CD₃OD | 640 [M+H]⁺ |
| 147 | 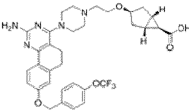 | (1R,3s,5S,6r)-3-(2-(4-(2-amino-8-((4-(trifluoromethoxy)benzyl)o xy)-5,6-dihydrobenzo[h]quinazolin-4-yl)piperazin-1-yl)ethoxy)bicyclo[3.1.0]hex ane-6-carboxvlic acid | 1.22(1 H, t, J=2.5 Hz), 1.72-1.79(4H, m), 2.24(2H, dd, J=7.1, 2.7 Hz), 2.64-2.74(8H, m), 2.74-2.85(2H, m), 3.32-3.41(4H, m), 3.56(2H, t, J=5.5 Hz), 362-375(1H, m), 5.16(2H, s), 6.91(1H, d, J=2.6 Hz), 6.95(1H, dd, J=2.6, 8.5 Hz), 7.29(2H, d, J=7.6 Hz), 7.56(2H, d, J=7.6 Hz), 7.91(1 H, d, J=8.5 Hz). | CD₃OD | 640 [M+H]⁺ |
| 148 | 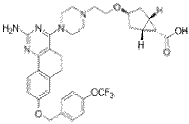 | (1R,3s,5S,6s)-3-(2-(4-(2-amino-8-((4-(trifluoromethoxy)benzyl)o xy)-5,6-dihydrobenzo[h]quinazolin-4-yl)piperazin-1-yl)ethoxy)bicyclo[3.1.0]hexane-6-carboxylic acid | 1.52(1H, t, J=7.0 Hz), 1.59-1.71(2H, m), 1.75-1.94(2H, m), 2.37(2H, dd, J=7.0, 13.5 Hz), 2.62-281(10H, m), 3.36(4H, brs), 3.54(2H, t, J=5.6 Hz), 3.89-4.04(1H, m), 5.15(2H, s), 6.86-6.91(1H, m), 6.94(1H, dd, J=2.6, 8.5 Hz), 7.29(2H, d, J=8.2 Hz), 7.55(2H, d, J=8.5 Hz), 7.90(1 H, d, J=8.6 Hz). | CD₃OD | 640 [M+H]⁺ |
| 36 | 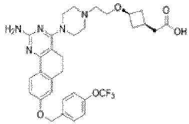 | cis-2-(3-(2-(4-(2-amino-8-((4-(trifluoromethoxy)benzyl)o xy)-5,6-dihydrobenzo[h]quinazolin-4-yl)piperazin-1-yl)ethoxy)cyclobutyl)acetic acid | 155-166(2H, m), 2.10-2.25(1H, m), 2.32(2H, d, J=7.5 Hz), 2.39-2.50(2H, m), 2.60-2.68(2H, m), 2.68-2.81(8H, m), 3.37(4H, t, J=4.5 Hz), 3.54(2H, t, J=5.6 Hz), 3.80-3.90(1H, m), 5.15(2H, s), 6.89(1H, d, J=2.5 Hz), 6.94(1H, dd, J=2.5, 8.6 Hz), 7.26-7.33(2H, m), 7.52-7.55(1 H, m), 7.55-7.58(1H, m), 7.90(1H, d, J=8.6 Hz). | CD₃OD | 628 [M+H]⁺ |
| 37 | 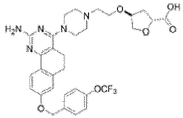 | (2R,4S)-4-(2-(4-(2-amino-8-((4-(trifluoromethoxy)benzyl)o xy)-5,6-dihydrobenzo[h]quinazolin-4-yl)piperazin-1-yl)ethoxy)tetrahydrofuran-2-carboxylic acid | 1.93-2.04(1H, m), 2.40(1H, m), 2.64-2.71(2H, m), 2.77-2.86(2H, m), 2.98(6H, brs), 3.53(4H, brs), 3.64-3.79(2H, m), 3.90(1H, m), 4.01(1H, m), 4.16-4.22(1 H, m), 4.39(1 H, m), 5.1 7(2H, s), 6.93(1 H, d, J=2.3 Hz), 6.97(1H, dd, J=2.3, 8.6 Hz), 7.26-7.33(2H, m), 7.53-7.59(2H, m), 7.88(1H, d, J=8.6 Hz). | CD₃OD | 630 [M+H]⁺ |
| 149 | 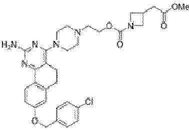 | 2-(4-(2-amino-8-((4-chlorobenzyl)oxy)-5,6-dihydrobenzo[h]quinazolin-4-yl)piperazin-1-yl)ethyl 3-(2-methoxy-2-oxoethyl)azetidine-1-carboxylate | 2.57-2.72(10H, m), 2.78(2H, t, J=7.0 Hz), 2.85-3.03(1H, m), 3.30(4H, t, J=4.6 Hz), 3.63-3.74(5H, m), 4.17(2H, t, J=8.6 Hz), 4.22(2H, t, J=5.9 Hz), 4.71(2H, brs), 5.07(2H, s), 6.79(1H, d, J=2.4 Hz), 690(1H, dd, J=2.4, 8.6 Hz), 7.32-7.42(4H, m), 8.03(1H, d, J=8.6 Hz). | CDCl₃ | 621 [M+H]⁺ |
| 150 | 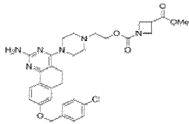 | 1-(2-(4-(2-amino-8-((4-chlorobenzyl)oxy)-5,6-dihydrobenzo[h]quinazolin-4-yl)piperazin-1-yl)ethyl) 3-methyl azetidine-1,3-dicarboxylate | 2.59-2.72(8H, m), 2.78(2H, t, J=7.0 Hz), 3.30(4H, t, J=4.4 Hz), 3.41(1H, q, J=7.5 Hz), 3.76(3H, s), 4.17(4H, d, J=7.5 Hz), 4.23(2H, t, J=5.9 Hz), 4.72(2H, brs), 5.07(2H, s), 6.79(1H, d, J=2.4 Hz), 6.90(1H, dd, J=2.4, 8.6 Hz), 7.33-7.42(4H, m), 8.03(1H, d, J=8.6 Hz). | CDCl₃ | 607 [M+H]⁺ |

**[Table 14]**

| Ex. No. | Structural formula | Compound name | ¹H-NMR (400 MHz) δ (ppm) from TMS | Solvent | ESI MS m/z |
|---|---|---|---|---|---|
| 151 | 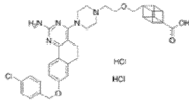 | 4-((2-(4-(2-amino-8-((4-chlorobenzyl)oxy)-5,6-dihydrobenzo[h]quinazolin-4-yl)piperazin-1-yl)ethoxy)methyl)pentacyclo[4.2.0.0^{2,5}.0^{3,8}.0^{4,7}]octane-1-carboxylic acid dihydrochloride | 2.61-2.72(2H, m), 2.81(2H, brs), 3.12-3.34(superimposed by H₂O, s), 3.46-3.63(6H, m),3.66-3.89(3H, m), 4.18(1H, brs), 5.21(3H, s), 5.39(1H, dd, J=3.2. 4.6 Hz), 5.87(1H, brs), 7.09(2H, brs), 7.43-7.53(4H, m), 7.95(1H, d, J=9.4 Hz),10.74(1H, brs), 1218(1H, brs), 1318(1H, s). | DMSO-d₆ | 626 [M+H]⁺ |
| 152 | 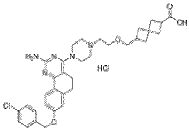 | 6-((2-(4-(2-amino-8-((4-chlorobenzyl)oxy)-5,6-dihydrobenzo[h]quinazolin-4-yl)piperazin-1-yl)ethoxy)methyl)spiro[3.3]heptane-2-carboxylic acid hydrochloride | 167(1H, dd, J=7.7, 11.5 Hz), 171-181(1H, m), 1.92-2.02(1H, m), 2.03-2.26(5H, m), 2.29-2.43(2H, m), 2.63-2.72(2H, m), 2.80(2H, d, J=7.7 Hz), 2.89(1H, quin, J=8.4 Hz), 3.11-3.44(superimposed by H₂O, m), 3.54(4H, d, J=10.9 Hz), 3.75(2H, brs), 4.20(2H, brs), 5.22(2H, s), 7.10(2H, brs), 7.44-7.53(4H, m), 7.95(1H, d, J=9.4 Hz), 1079(1H, brs), 1199(1H, brs), 1318(1H, brs). | DMSO-d₆ | 618 [M+H]⁺ |
| 153(a) | 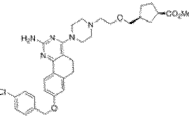 | methyl cis-3-((2-(4-(2-amino-8-((4-chlorobenzyl)oxy)-5,6-dihydrobenzo[h]quinazolin-4-yl)piperazin-1-yl)ethoxy)methyl)cyc lopentane-1-carboxylate | 1.27-1.45(2H, m), 1.60-1.88(3H, m), 1.93-2.04(1H, m), 2.09-2.22(1H, m), 2.51-2.64(8H, m), 2.67-2.81(3H, m), 3.16(4H, brs), 3.28-3.31(2H, m), 3.51(2H, t, J=5.8 Hz), 3.58(3H, s), 5.15(2H, s), 6.01(2H, s), 6.91-6.96(2H, m), 7.36-7.57(4H, m), 7.92(1H, d, J=8.4 Hz). | DMSO-d₆ | 606 [M+H]⁺ |
| 153(b) | 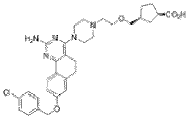 | cis-3-((2-(4-(2-amino-8-((4-chlorobenzyl)oxy)-5,6-dihydrobenzo[h]quinazolin-4-yl)piperazin-1-yl)ethoxy)methyl)cyclopentane-1-carboxvlic acid | 1.28-1.47(3H, m), 1.60-1.83(4H, m), 1.90-2.04(1H, m), 2.07-2.20(1H, m), 2.54-2.70(9H, m), 2.70-2.75(2H, m), 3.17(4H, brs), 3.52(2H, t, J=5.6 Hz), 5.16(2H, s), 6.02(2H, s), 6.89-6.99(2H, m), 7.45-7.54(4H, m), 7.93(1 H, d, J=8.4 Hz). | DMSO-d₆ | 592 [M+H]⁺ |
| 154 | 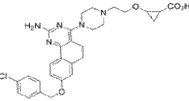 | 2-(2-(4-(2-amino-8-((4-chlorobenzyl)oxy)-5,6-dihydrobenzo[h]quinazolin-4-yl)piperazin-1-yl)ethoxy)cyclopropane-1-carboxylic acid | 103-110(1H, m), 1.12-1.21(2H, m), 1.60-1.69(1H, ddd, J=1.8, 5.9, 9.4 Hz), 2.56-2.61 (5H, m), 2.71-2.75(2H, m), 3.17(4H, brs), 3.46-3.72(5H, m), 5.16(2H, s), 6.02(2H, s), 6.91-6.98(2H, m), 7.45-7.53(4H, m), 7.93(1 H, d, J=8.3 Hz) | DMSO-d₆ | 550 [M+H]⁺ |
| 155 | 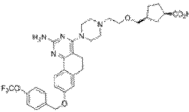 | cis-3-((2-(4-(2-amino-8-((4-(trifluoromethoxy)benzyl)o xy)-5,6-dihydrobenzo[h]quinazolin-4-yl)piperazin-1-yl)ethoxy)methyl)cyclopentane-1-carboxvlic acid | 1.27-1.46(2H, m), 1.66-1.85(3H, m), 1.94-2.04(1H, m), 2.10-2.25(1H, m), 2.56-2.87(5H, m), 3.09-3.41(9H, m), 3.45-3.64(3H, m), 3.78(2H, brs), 5.23(2H, brs), 6.84-7.16(2H, m), 7.41(2H, d, J=8.3 Hz), 7.60(2H, d, J=8.3 Hz), 7.97(1H, d, J=9.4 Hz), 12.01(1H, brs). | DMSO-d₆ | 642 [M+H]⁺ |
| 156 | 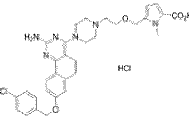 | 4-((2-(4-(2-amino-8-((4-chlorobenzyl)oxy)-5,6-dihydrobenzo[h]quinazolin-4-yl)piperazin-1-yl)ethoxy)methyl)-1-methyl-1H-pyrrole-2-carboxylic acid hydrochloride | 2.61-2.72(2H, m), 2.80(2H, d, J=7.5 Hz), 3.19(3H, d, J=9.7 Hz), 3.36(superimposed by HDO, m), 3.53(4H, d, J=10.3 Hz), 3.72(2H, brs), 3.82(3H, s), 4.36(2H, s), 5.22(2H, s), 6.81(1H, d, J=2.0 Hz), 7.09(3H, d, J=2.0 Hz), 7.44-7.54(4H, m), 7.89-7.99(1H, m), 10.55(1H, brs), 12.24(1H, brs), 1315(1H, brs). | DMSO-d₆ | 603 [M+H]⁺ |

**[Table 15]**

| Ex. No. | Structural formula | Compound name | ¹H-NMR (400 MHz) δ (ppm) from TMS | Solvent | ESI MS m/z |
|---|---|---|---|---|---|
| 157 | 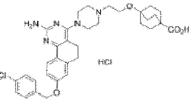 | 4-(2-(4-(2-amino-8-((4-chlorobenzyl)oxy)-5,6-dihydrobenzo[h]quinazolin-4-yl)piperazin-1-yl)ethoxy)bicyclo[2.2.2]octane-1-carboxylic acid hydrochloride | 1.57-1.71(6H, m), 1.77-1.90(6H, m), 2.69(2H, d, J=6.7 Hz), 2.76-2.90(2H, m), 3.11-3.47(superimposed by HDO, m), 3.56(4H, d, J=11.7 Hz), 3.72(2H, brs), 4.19(2H, brs), 5.23(2H, s), 7.05-7.16(2H, m), 7.45-7.55(4H, m), 7.99(1H, d, J=9.3 Hz), 1094(1H, brs), 1210(1H, brs), 13.31(1H, brs). | DMSO-d₆ | 618 [M+H]⁺ |
| 158 | 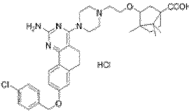 | 3-(2-(4-(2-amino-8-((4-chlorobenzyl)oxy)-5,6-dihydrobenzo[h]quinazolin-4-yl)piperazin-1-yl)ethoxy)-4,7,7-trimethylbicyclo[2.2.1]hept ane-1-carboxylic acid hydrochloride | 0.83(3H, s), 0.89(3H, s), 0.94(3H, s), 1.05-1.16(1H, m), 1.26-1.37(1H, m), 1.53(1H, dt, J=4.2, 12.1 Hz), 188(1H, dd, J=7.6, 13.2 Hz), 1.93-2.04(1H, m), 2.05-2.15(1H, m), 2.62-2.74(2H, m), 2.76-2.90(2H, m), 3.13-3.46(5H, m), 3.49-3.73(5H, m), 3.78-3.93(1H, m), 4.21(2H, brs), 5.23(2H, s), 7.06-7.15(2H, m), 7.45-7.55(4H, m), 8.00(1 H, d, J=9.3 Hz), 11.23(1 H, brs), 12.10(1 H, brs). 13.35(1 H. brs). | DMSO-d₆ | 646 [M+H]⁺ |
| 159(a) | 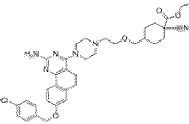 | ethyl cis- or trans-4-((2-(4-(2-amino-8-((4-chlorobenzyl)oxy)-5,6-dihydrobenzo[h]quinazolin-4-yl)piperazin-1-yl)ethoxy)methyl)-1-cyanocyclohexane-1-carboxvlate | 1.32(3H, t, J=7.1 Hz), 1.36-1.47(2H, m), 1.59-1.72(1 H, m), 1.81(2H, dt, J=13.5, 3.4 Hz), 1.86-1.96(2H, m), 2.15-2.23(2H, m), 2.57-2.70(8H, m), 2.73-2.82(2H, m), 3.26-3.37(6H, m), 3.59(2H, t, J=5.7 Hz), 4.26(2H, q, J=7.1 Hz), 4.73(2H, brs), 5.07(2H, s), 6.79(1H, brd, J=2.5 Hz), 6.89(1H, dd, J=2.5. 8.6 Hz), 7.33-7.40(4H, m), 8.03(1H, d, J=8.6 Hz). | CDCl₃ | 659 [M+H]⁺ |
| 159(b) | 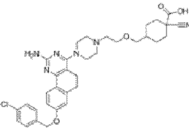 | cis- or trans-4-((2-(4-(2-amino-8-((4-chlorobenzyl)oxy)-5,6-dihydrobenzo[h]quinazolin-4-yl)piperazin-1-yl)ethoxy)methyl)-1-cyanocyclohexane-1-carboxylic acid hydrochloride | 1.40-1.54(2H, m), 1.64-1.77(1H, m), 1.81-1.94(4H, m), 2.11 -2.20(2H, m), 2.73-2.81(2H, m), 2.86-2.94(2H, m), 3.30-3.42(2H, m), 3.42-3.51(4H, m), 3.55-3.68(2H, m), 3.68-3.78(2H, m), 3.82-3.90(2H, m), 4.36-4.52(2H, m), 5.19(2H, s), 7.05-7.11(2H, m), 7.37-7.42(2H, m), 7.43-7.48(2H, m), 7.72-7.78(1 H, m). | CD₃OD | 631 [M+H]⁺ |
| 160(a) | 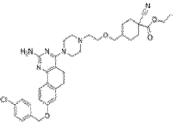 | ethyl trans- or cis-4-((2-(4-(2-amino-8-((4-chlorobenzyl)oxy)-5,6-dihydrobenzo[h]quinazolin-4-yl)piperazin-1-yl)ethoxy)methyl)-1-cyanocyclohexane-1-carboxvlate | 1.32(3H, t, J=7.1 Hz), 1.34-1.45(2H, m), 1.75-1.92(5H, m), 2.24-2.37(2H, m), 2.54-2.70(8H, m), 2.72-2.83(2H, m), 3.23-3.38(6H, m), 3.57(2H, t, J=5.7 Hz), 4.27(2H, q, J=7.1 Hz), 4.77(2H, brs), 5.07(2H, s), 6.79(1 H, d, J=2.5 Hz), 6.89(1 H, dd, J=2.5, 8.6 Hz), 7.32-7.41(4H, m), 8.04(1H, d, J=8.6 Hz). | CDCl₃ | 659 [M+H]⁺ |
| 160(b) | 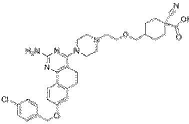 | trans- or cis-4-((2-(4-(2-amino-8-((4-chlorobenzyl)oxy)-5,6-dihydrobenzo[h]quinazolin-4-yl)piperazin-1-yl)ethoxy)methyl)-1-cyanocyclohexane-1-carboxylic acid | 1.46-1.92(7H, m), 2.00-2.35(2H, m), 2.33-2.83(10H, m), 3.10-3.62(8H, m), 5.00(2H, s), 6.73(1 H, brs), 6.86(1 H, brd, J=7.5 Hz), 7.25-7.44(4H, m), 8.04(1 H, brd, J=7.5 Hz). | CDCl₃ | 631 [M+H]⁺ |
| 161 | 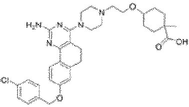 | 4-(2-(4-(2-amino-8-((4-chlorobenzyl)oxy)-5,6-dihydrobenzo[h]quinazolin-4-yl)piperazin-1-yl)ethoxy)-1-methylcyclohexane-1-carboxylic acid | 1.23(3H, s), 1.55-1.74(6H, m), 1.78-1.90(2H, m), 2.59-2.73(8H, m), 2.74-2.84(2H, m), 3.27-3.45(5H, m), 3.55-3.68(2H, m), 5.07(4H, m), 679(1H, brd, J=2.5 Hz), 690(1H, dd, J=2.5, 8.6 Hz), 7.32-7.42(4H, m), 8.03(1H, d, J=8.6 Hz). | CDCl₃ | 606 [M+H]⁺ |

**[Table 16]**

| Ex. No. | Structural formula | Compound name | ¹H-NMR (400 MHz) δ (ppm) from TMS | Solvent | ESI MS m/z |
|---|---|---|---|---|---|
| 162 | 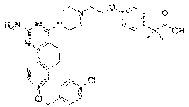 | 2-(4-(2-(4-(2-amino-8-((4-chlorobenzyl)oxy)-5,6-dihydrobenzo[h]quinazolin-4-yl)piperazin-1-yl)ethoxy)phenyl)-2-methylpropanoic acid | 1.49(6H, s), 2.63-2.70(2H, m), 2.72-2.81(6H, m), 2.88(2H, t, J=5.4 Hz), 3.37(4H, t, J=4.3 Hz), 4.16(2H, t, J=5.4 Hz), 5.12(2H, s), 6.85-6.90(3H, m), 6.94(1 H, dd, J=2.6, 8.6 Hz), 7.29-7.31 (1H, m), 7.31-7.34(1H, m), 7.36-7.41(2H, m), 7.43-7.47(2H, m), 7.86-7.93(1 H, m). | CD₃OD | 628 [M+H]⁺ |
| 163 | 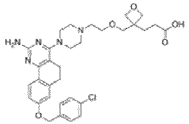 | 3-(3-((2-(4-(2-amino-8-((4-chlorobenzyl)oxy)-5,6-dihydrobenzo[h]quinazolin-4-yl)piperazin-1-yl)ethoxy)methyl)oxetan-3-yl)propanoic acid | 2.10(2H, t, J=7.3 Hz), 2.24(2H, t, J=7.3 Hz), 2.60-2.67(2H, m), 2.73-2.80(2H, m), 2.96-3.05(6H, m), 3.53(4H, t, J=4.6 Hz), 3.69(2H, s), 3.74(2H, t, J=5.1 Hz), 4.40-4.47(4H, m), 5.10(2H, s), 6.87(1 H, d, J=2.6 Hz), 694(1H, dd, J=2.6, 8.6 Hz), 7.35-7.39(2H, m), 7.40-7.45(2H, m), 7.88(1H, d, J=8.6 Hz). | CD₃OD | 608 [M+H]⁺ |
| 164 | 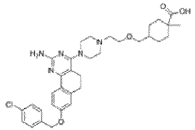 | cis- or trans-4-((2-(4-(2-amino-8-((4-chlorobenzyl)oxy)-5,6-dihydrobenzo[h]quinazolin-4-yl)piperazin-1-yl)ethoxy)methyl)-1-methylcyclohexane-1-carboxylic acid | 1.10-1.29(5H, m), 1.55-1.82(7H, m), 2.58-282(10H, m), 3.28(2H, d, J=6.4 Hz), 3.33(4H, m), 3.59(2H, t, J=5.6 Hz), 5.06(2H, s), 5.12-5.28(2H, m), 6.79(1H, brd, J=2.5 Hz), 6.89(1H, dd, J=2.5, 8.6 Hz), 7.32-7.41(4H, m), 8.02(1H, d, J=8.6 Hz). | CDCl₃ | 620 [M+H]⁺ |
| 165 | 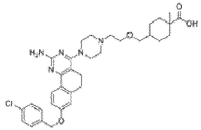 | trans- or cis-4-((2-(4-(2-amino-8-((4-chlorobenzyl)oxy)-5,6-dihydrobenzo[h]quinazolin-4-yl)piperazin-1-yl)ethoxy)methyl)-1-methylcyclohexane-1-carboxylic acid | 1.07-1.22(5H, m), 1.18(3H, s), 1.62-1.74(2H, m), 2.14-2.25(2H, m), 2.53-2.63(2H, m), 2.66-2.82(8H, m), 3.22(2H, d, J=6.2 Hz), 3.35-3.46(4H, m), 3.55-3.64(2H, m), 5.05(2H, s), 5.40(2H, brs), 6.76(1H, brd, J=2.5 Hz), 6.90(1H, dd, J=2.5, 8.6 Hz), 7.31-7.41(4H, m), 8.03(1H, d, J=8.6 Hz). | CDCl₃ | 620 [M+H]⁺ |
| 166(a) | 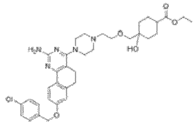 | ethyl cis- or trans-4-((2-(4-(2-amino-8-((4-chlorobenzyl)oxy)-5,6-dihydrobenzo[h]quinazolin-4-yl)piperazin-1-yl)ethoxy)methyl)-4-hydroxycyclohexane-1-carboxylate | 1.24(3H, t, J=7.1 Hz), 1.43-1.53(2H, m), 1.58-1.69(2H, m), 1.70-1.79(2H, m), 1.87-1.98(2H, m), 236-247(1H, m), 2.58-2.69(8H, m), 2.72-2.81(2H, m), 3.26-3.39(4H, m), 3.45(2H, s), 3.70(2H, t, J=5.3 Hz), 4.12(2H, q, J=7.1 Hz), 4.78(2H, brs), 5.06(2H, s), 6.78(1H, d, J=2.5 Hz), 6.89(1H, dd, J=2.5, 8.6 Hz), 7.32-7.40(4H, m), 8.03(1H. d. J=8.6 Hz). | CDCl₃ | 650 [M+H]⁺ |
| 166(b) | 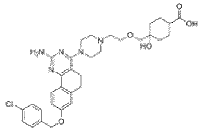 | cis- or trans-4-((2-(4-(2-amino-8-((4-chlorobenzyl)oxy)-5,6-dihydrobenzo[h]quinazolin-4-yl)piperazin-1-yl)ethoxy)methyl)-4-hydroxycyclohexane-1-carboxylic acid | 1.22-1.33(2H, m), 1.49-1.67(4H, m), 1.71-1.82(2H, m), 2.27-2.35(1 H, m), 2.52-2.63(8H, m), 2.68-2.76(2H, m), 3.10-3.28(6H, m), 3.56(2H, t, J=5.7 Hz), 5.15(2H, s), 6.02(2H, brs), 6.90-6.96(2H, m), 7.43-7.51(4H, m), 7.92(1H, d, J=8.4 Hz). | DMSO-d₆ | 622 [M+H]⁺ |
| 167 | 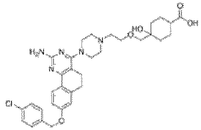 | trans- or cis-4-((2-(4-(2-amino-8-((4-chlorobenzyl)oxy)-5,6-dihydrobenzo[h]quinazolin-4-yl)piperazin-1-yl)ethoxy)methyl)-4-hydroxycyclohexane-1-carboxylic acid | 1.28-1.42(2H, m), 1.45-1.54(2H, m), 1.56-1.75(4H, m), 201-214(1H, m), 2.49-2.63(8H, m), 2.68-2.78(2H, m), 3.10-3.23(6H, m), 3.55(2H, t, J=5.8 Hz), 5.14(2H, s), 6.01(2H, s), 6.89-6.97(2H, m), 7.43-7.53(4H, m), 7.93(1H, d, J=8.4 Hz). | DMSO-d₆ | 622 [M+H]⁺ |

**[Table 17]**

| Ex. No. | Structural formula | Compound name | ¹H-NMR (400 MHz) *δ* (ppm) from TMS | Solvent | ESI MS m/z |
|---|---|---|---|---|---|
| 168 | 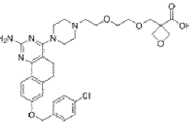 | 3-((2-(2-(4-(2-amino-8-((4-chlorobenzyl)oxy)-5,6-dihydrobenzo[h]quinazolin-4-yl)piperazin-1-yl)ethoxy)ethoxy)methyl)o xetane-3-carboxvlic acid | 2.59-2.67(2H, m), 2.71-2.79(2H, m), 3.10(2H, t, J=5.0 Hz), 3.18(3H, t, J=4.2 Hz), 3.58-3.64(6H, m), 3.65-3.69(2H, m), 3.78(2H, t, J=5.0 Hz), 3.92(2H, s), 4.48(2H, d, J=5.9 Hz), 4.88(2H, d, J=5.9 Hz), 5.08(2H, s), 6.84-6.87(1 H, m), 6.93(1 H, m), 7.34-7.38(2H, m), 7.39-7.45(2H, m), 7.89(1 H, m). | CD₃OD | 624 [M+H]⁺ |
| 169 | 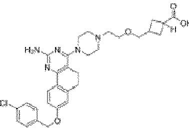 | cis- or trans-3-((2-(4-(2-amino-8-((4-chlorobenzyl)oxy)-5,6-dihydrobenzo[h]quinazolin-4-yl)piperazin-1-yl)ethoxy)methyl)cyclobut ane-1-carboxylic acid | 1.80-1.94(2H, m), 2.09-2.23(2H, m), 2.35-2.47(1H, m), 2.50-2.64(8H, m), 2.69-2.77(2H, m), 2.86-2.98(1H, m), 3.06(4H, m), 3.20-3.40(2H, m), 3.50(2H, t, J=5.9 Hz), 5.15(2H, s), 6.01(2H, s), 6.89-6.97(2H, m), 7.40-7.54(4H, m), 7.92(1 H, d, J=8.4 Hz). | DMSO-d₆ | 578 [M+H]⁺ |
| 170 | 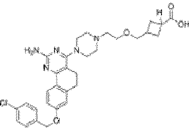 | trans- or cis-3-((2-(4-(2-amino-8-((4-chlorobenzyl)oxy)-5,6-dihydrobenzo[h]quinazolin-4-yl)piperazin-1-yl)ethoxy)methyl)cyclobut ane-1-carboxylic acid | 1.85-1.94(2H, m), 2.14-2.24(2H, m), 2.39-2.47(1H, m), 2.51-2.62(8H, m), 2.68-2.77(2H, m), 2.95-3.05(1H, m), 3.10-3.22(4H, m), 3.42(2H, d, J=6.8 Hz), 3.53(2H, t, J=5.9 Hz), 5.15(2H, s), 6.01(2H, s), 6.90-6.96(2H, m), 7.44-7.52(4H, m), 7.92(1H, d, J=8.4 Hz). | DMSO-d₆ | 578 [M+H]⁺ |
| 171 | 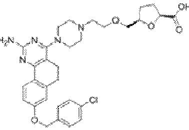 | cis-5-((2-(4-(2-amino-8-((4-chlorobenzyl)oxy)-5,6-dihydrobenzo[h]quinazolin-4-yl)piperazin-1-yl)ethoxy)methyl)tetrahydr ofuran-2-carboxylic acid | 1.65-1.78(1H, m), 1.88-1.99(1H, m), 2.02-213(1H, m), 2.18-2.31(1H, m), 2.63-2.71(2H, m), 2.74-2.82(2H, m), 3.18-3.42(6H, m), 3.52-3.69(6H, m), 3.83-3.95(2H, m), 4.21(1H, qd, J=3.1, 6.9 Hz), 436(1H, dd, J=6.0, 8.1 Hz), 5.12(2H, s), 6.88(1H, d, J=2.5 Hz), 6.94(1H, dd, J=2.5, 8.6 Hz), 734-741(2H, m), 7.41-7.47(2H, m), 7.92(1H, d, J=8.6 Hz) | CD₃OD | 594 [M+H]⁺ |
| 172 | 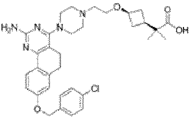 | cis-2-(3-(2-(4-(2-amino-8-((4-chlorobenzyl)oxy)-5,6-dihydrobenzo[h]quinazolin-4-yl)piperazin-1-yl)ethoxy)cyclobutyl)-2-methylpropanoic acid | 1.10(4H, s), 1.12(2H, s), 1.70-1.81(2H, m), 1.98(3H, s), 2.01-2.13(2H, m), 2.18-2.28(2H, m), 2.65(4H, s), 2.69(2H, d, J=7.58 Hz), 2.77-2.83(2H, m), 2.85-2.99(6H, m), 3.52(4H, brs), 3.58(2H, d, J=4.65 Hz), 3.76-3.88(1H, m), 5.14(2H, s), 6.89-6.93(1H, m), 6.94-7.01(1H, m), 7.35-7.42(2H, m), 7.42-749(2H, m). 7.84-7.90(1H, m). | CD₃OD | 606 [M+H]⁺ |
| 173 | 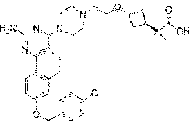 | trans-2-(3-(2-(4-(2-amino-8-((4-chlorobenzyl)oxy)-5,6-dihydrobenzo[h]quinazolin-4-yl)piperazin-1-yl)ethoxy)cyclobutyl)-2-methylpropanoic acid | 1.07(1H, s), 1.08(5H, s), 1.98-2.11(2H, m), 2.12-2.26(2H, m), 2.62-2.73(9H, m), 2.73-2.83(2H, m), 3.27-3.38(4H, m), 3.49-3.55(2H, m), 3.93-3.99(1H, m), 5.13(2H, s), 6.86-6.90(1H, m), 6.91-6.96(1H. m). 7.35-7.41(2H, m), 7.42-7.47(2H, m), 7.88-7.93(1H, m). | CD₃OD | 606 [M+H]⁺ |
| 174 | 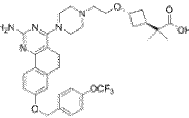 | trans-2-(3-(2-(4-(2-amino-8-((4-(trifluoromethoxy)benzyl)oxy)-5,6-dihydrobenzo[h]quinazolin-4-yl)piperazin-1-yl)ethoxy)cyclobutyl)-2-methylpropanoic acid | 1.09(1H, s), 1.10(5H, s), 1.99-2.10(2H, m), 2.13-2.24(2H, m), 2.59-2.76(9H, m), 2.76-2.84(2H, m), 3.34-3.42(4H, brs), 3.53(2H, t, J=5.5 Hz), 3.92-4.00(1H, m), 5.16(2H, s), 6.90(1H, d, J=2.5 Hz), 6.95(1H, dd, J=2.5, 8.6 Hz), 7.26-7.33(2H, m), 7.53-7.60(2H, m), 7.91(1H, d, J=8.6 Hz). | CD₃OD | 656 [M+H]⁺ |

**[Table 18]**

| Ex. No. | Structural formula | Compound name | ¹H-NMR (400 MHz) *δ* (ppm) from TMS | Solvent | ESI MS m/z |
|---|---|---|---|---|---|
| 175 | 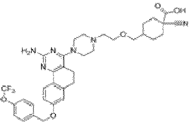 | 4-((2-(4-(2-amino-8-((4-(trifluoromethoxy)benzyl)oxy)-5,6-dihydrobenzo[h]quinazolin-4-yl)piperazin-1-yl)ethoxymethyl)-1-cyanocyclohexane-1-carboxylic acid | 1.11-1.33(3H, m), 1.66-1.83(4H, m), 2.03(2H, d, J=13.7 Hz), 2.56-2.69(8H, m), 2.69-2.78(2H, m), 3.14-3.27(6H, m), 3.49-3.58(2H, m), 5.18(2H, s), 6.04(2H, brs), 6.91-6.99(2H, m), 7.40(2H, d, J=7.9 Hz), 7.60(2H, d, J=8.8 Hz), 7.93(1H, d, J=8.8 Hz). | DMSO-d₆ | 681 [M+H]⁺ |
| 176 | 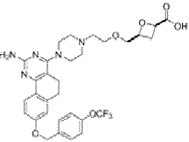 | cis-4-((2-(4-(2-amino-8-((4-(trifluoromethoxy)benzyl)oxy)-5,6-dihydrobenzo[h]quinazolin-4-yl)piperazin-1-yl)ethoxy)methyl)oxetane-2-carboxylic acid | 2.53-2.67(3H, m), 2.70-2.80(2H, m), 2.88-2.97(1H, m), 3.20-3.40(2H, m), 3.43-3.53(3H, m), 3.54-3.78(7H, m), 3.78-3.88(1H, m), 3.93-4.02(1H, m), 4.76-4.90(2H, m), 5.13(2H, s), 6.86(1 H, d, J=2.5 Hz), 6.94(1 H, dd, J=2.5, 8.6 Hz), 7.24-7.32(2H, m), 7.50-7.57(2H, m), 7.92(1 H, d, J=8.6 Hz). | CD₃OD | 630 [M+H]⁺ |
| 177 | 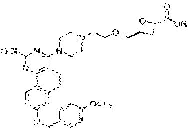 | trans-4-((2-(4-(2-amino-8-((4-(trifluoromethoxy)benzyl)oxy)-5,6-dihydrobenzo[h]quinazolin-4-yl)piperazin-1-yl)ethoxy)methyl)oxetane-2-carboxylic acid | 2.56-2.81(6H, m), 3.05-3.18(5H, brs), 3.47-3.60(4H, m), 3.64-3.72(3H, m), 3.85(2H, m), 4.77-4.90(2H, m), 5.08(2H, s), 6.83(1H, d, J=2.4 Hz), 6.93(1H, dd, J=2.4, 8.6 Hz), 7.22-7.30(2H, m), 7.46-7.56(2H, m), 7.87(1 H, d, J=8.6 Hz). | CDCl₃ | 630 [M+H]⁺ |
| 178 | 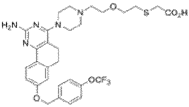 | 2-((2-(2-(4-(2-amino-8-((4-(trifluoromethoxy)benzyl)oxy)-5,6-dihydrobenzo[h]quinazolin-4-yl)piperazin-1-yl)ethoxy)ethyl)thio)acetic acid | 2.51-2.62(8H, m), 2.68-2.77(4H, m), 3.17(4H, brs), 3.29(2H, s), 3.54(2H, t, J=5.7 Hz), 3.58(2H, t, J=6.4 Hz), 5.18(2H, s), 6.02(2H, brs), 6.90-6.99(2H, m), 7.40(2H, d, J=8.4 Hz), 7.60(2H, d, J=8.4 Hz), 7.93(1H, d, J=8.6 Hz). | DMSO-d₆ | 634 [M+H]⁺ |
| 179 | 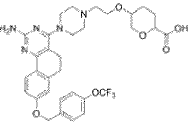 | 5-(2-(4-(2-amino-8-((4-(trifluoromethoxy)benzyl)oxy)-5,6-dihydrobenzo[h]quinazolin-4-yl)piperazin-1-yl)ethoxy)tetrahydro-2H-pyran-2-carboxylic acid | 1.37-1.49(1H, m), 1.50-1.70(1H, m), 1.89(1H, s), 2.11(1H, dd, J=13.4, 2.9 Hz), 2.15(1H, s), 2.17-2.26(1H, m), 2.57-2.72(8H, m), 2.73-2.82(2H, m), 3.09-3.18(1H, m), 3.48(1H, s), 3.53-3.65(1H, m), 3.65-3.70(2H, m), 3.70-3.79(1H, m), 3.87-3.94(1H, m), 4.11(1H, t, J=7.4 Hz), 5.16(2H, s), 6.90(1H, s), 6.94(1H, dd, J=8.6, 2.6 Hz), 7.29(2H, d, J=8.3 Hz), 7.56(2H, d, J=8.3 Hz), 7.91(1H, d, J=8.6 Hz). | CD₃OD | 644 [M+H]⁺ |
| 180 | 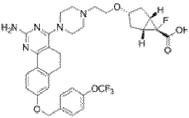 | (1R,3r,5S,6s)-3-(2-(4-(2-amino-8-((4-(trifluoromethoxy)benzyl)o xy)-5,6-dihydrobenzo[h]quinazolin-4-yl)piperazin-1-yl)ethoxy)-6-fluorobicyclo[3.1.0]hexane-6-carboxylic acid | 1.52-1.62(2H, m), 1.82-1.89(2H, m), 2.26-2.39(2H, m), 2.56-2.71(8H, m), 2.72-2.81(2H, t, J=70 Hz), 3.28-3.36(4H, m), 3.58(2H, t, J=5.7 Hz), 4.22-4.34(1H, m), 4.85(10H, s), 5.14(2H, s), 6.86-6.90(1H, m), 6.91-6.98(1H, m), 7.25-7.32(2H, m), 7.51-7.59(2H, m), 7.88-7.93(1 H, m). | CD₃OD | 658 [M+H]⁺ |

**[Table 19]**

| Ex. No. | Structural formula | Compound name | ¹H-NMR (400 MHz) *δ* (ppm) from TMS | Solvent | ESI MS m/z |
|---|---|---|---|---|---|
| 181 | 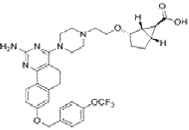 | (1S,2S,5R,6S)-2-(2-(4-(2-amino-8-((4-(trifluoromethoxy)benzyl)o xy)-5,6-dihydrobenzo[h]quinazolin-4-yl)piperazin-1-yl)ethoxy)bicyclo[3.1.0]hexane-6-carboxylic acid | 1.08-1.21(1 H, m), 1.66(1 H, t, J=3.1 Hz), 1.69-1.75(1H, m), 1.77-1.85(2H, m), 1.85-1.95(1H, m), 1.96-2.02(1H, m), 2.61-2.74(8H, m), 2.74-2.81(2H, t, J=6.9 Hz), 3.32-3.40(4H, m), 3.61-3.68(1 H, m), 3.72-3.80(1 H, m), 4.22-4.30(1 H, m), 5.16(2H, s), 6.87-6.91(1H, m), 6.92-6.97(1H, m), 7.26-7.32(2H, m), 7.52-7.59(2H, m), 7.88-7.94(1H. m). | CD₃OD | 640 [M+H]⁺ |
| 182 | 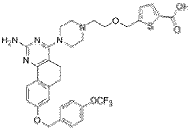 | 5-((2-(4-(2-amino-8-((4-(trifluoromethoxy)benzyl)oxy)-5,6-dihydrobenzo[h]quinazolin-4-yl)piperazin-1-yl)ethoxy)methyl)thiophene-2-carboxylic acid | 2.57-2.71(8H, m), 2.71-2.79(2H, m), 3.27-3.35(4H, m), 3.67(2H, t, J=5.4 Hz), 4.65(2H, s), 5.13(2H, s), 6.86-6.89(1H, m), 6.90-6.97(2H, m), 7.25-7.31(2H, m), 7.42(1H, d, J=3.5 Hz), 7.52-7.58(2H, m), 7.88-7.93(1H, m). | CD₃OD | 656 [M+H]⁺ |
| 183 | 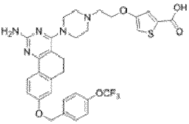 | 4-(2-(4-(2-amino-8-((4-(trifluoromethoxy)benzyl)o xy)-5,6-dihydrobenzo[h]quinazolin-4-yl)piperazin-1-yl)ethoxy)thiophene-2-carboxylic acid | 2.61-2.69(2H, m), 2.73(4H, t, J=4.5 Hz), 2.75-2.82(2H, m), 2.85(2H, t, J=5.4 Hz), 3.33-3.40(4H, m), 4.15(2H, t, J=5.4 Hz), 5.16(2H, s), 6.52(1 H, d, J=1.8 Hz), 6.88-6.92(1H, m), 6.92-6.97(1H, m), 7.20(1H, d, J=1.8 Hz), 7.26-7.32(2H, m), 7.53-7.60(2H, m), 7.88-7.95(1 H, m). | CD₃OD | 642 [M+H]⁺ |
| 184 | 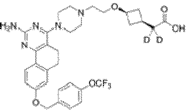 | cis-2-(3-(2-(4-(2-amino-8-((4-(trifluoromethoxy)benzyl)oxy)-5,6-dihydrobenzo[h]quinazolin-4-yl)piperazin-1-yl)ethoxy)cyclobutyl)acetic-2.2-d2 acid | 1.54-1.66(2H, m), 2.10-2.23(1H, m), 2.37-2.50(2H, m), 2.55-2.64(2H, m), 2.64-2.80(8H, m), 3.32-3.40(4H, m), 3.52(2H, t, J=5.6 Hz), 3.78-3.89(1H, m), 5.12(2H, s), 6.83-6.88(1H, m), 6.90-6.96(1H, m), 7.24-7.31(2H, m), 7.50-7.58(2H, m), 7.87-7.94(1H, m). | CD₃OD | 630 [M+H]⁺ |
| 185 | 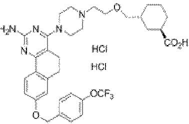 | trans-3-((2-(4-(2-amino-8-((4-(trifluoromethoxy)benzyl)oxy)-5,6-dihydrobenzo[h]quinazolin-4-yl)piperazin-1-yl)ethoxy)methyl)cyclohexane-1-carboxylic acid dihydrochloride | 1.10(1H, q, J=9.2 Hz), 1.28-1.43(2H, m), 1.44-1.66(3H, m), 1.72-1.91(3H, m), 2.54-2.63(1H, m), 2.65-2.75(2H, m), 2.77-2.88(2H, m), 3.13-3.36(5H, m), 3.50-3.69(5H, m), 3.79(2H, brs), 4.22(2H, brs), 5.27(2H, s), 7.06-7.19(2H, m), 7.42(2H, d, J=7.9 Hz), 7.56-7.65(2H, m), 8.01(1H, d, J=8.9 Hz), 11.03(1H, brs), 12.06(1H, brs), 13.37(1 H, brs). | DMSO-d₆ | 656 [M+H]⁺ |
| 186 | 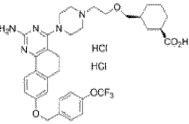 | cis-3-((2-(4-(2-amino-8-((4-(trifluoromethoxy)benzyl)oxy)-5,6-dihydrobenzo[h]quinazolin-4-yl)piperazin-1-yl)ethoxy)methyl)cyclohexane-1-carboxylic acid dihydrochloride | 0.75-0.92(1H, m). 0.99(1H, q, J=12.3 Hz), 1.13-1.34(2H, m), 1.54-1.81(3H, m), 1.82-2.01(2H, m), 2.12-2.28(1H, m), 2.64-2.77(2H, m), 2.82(2H, d, J=7.6 Hz), 3.12-3.33(6H, m),3.57(4H, d, J=11.1 Hz), 3.77(2H, brs), 4.22(2H, brs), 5.27(2H, s),7.06-7.19(2H, m),7.42(2H, d, J=7.8Hz), 7.61(2H, d, J=8.7 Hz), 7.99(1H, d, J=9.3 Hz), 10.88(1H, brs), 12.04(1 H, d, J=14.1 Hz), 13.27(1 H, brs), | DMSO-d₆ | 656 [M+H]⁺ |

### (Example 187)

### 2-(2-(2-(4-(2-amino-8-((4-chlorobenzyl)oxy)-5,6-dihydr obenzo[h]quinazolin-4-yl)piperazin-1-yl)ethoxy)ethoxy) -N-(methylsulfonyl)acetamide

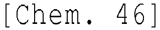

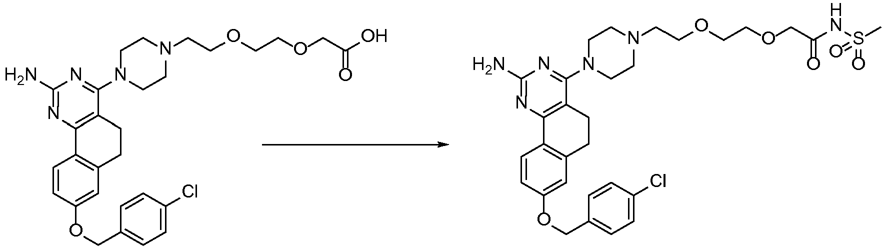

2-(2-(2-(4-(2-amino-8-((4-chlorobenzyl)oxy)-5,6-dihydrobenzo[h]quinazolin-4-yl)piperazin-1-yl)ethoxy)e thoxy)acetic acid (20 mg, 0.031 mol) obtained in Example 11 was dissolved in tetrahydrofuran (1 mL), and 1,1'-carbonyldiimidazole (15 mg, 0.094 mmol, 3.0 equivalents) and triethylamine (13 µL, 0.094 mmol, 3.0 equivalents) were added, followed by heating under reflux for 2 hours. The reaction liquid was cooled down to room temperature, and sodium hydride (in the form of oil, 60%)(3.6 mg, 0.09 mml, 3.0 equivalents), methane sulfonamide (8.9 mg, 0.094 mmol, 3.0 equivalents) were added, followed by stirring for 24 hours. A saturated ammonium chloride aqueous solution was added to the reaction liquid, and the product was extracted with ethyl acetate. The organic layer was washed with brine and dried over anhydrous sodium sulfate. The solvent was distilled off under reduced pressure to obtain a crude product. This crude product was purified through an HPLC (mobile phase, water : acetonitrile) to obtain 15.3 mg of the title compound.
¹H-NMR (400 MHz, CD₃OD) δ: 2.61-2, 79 (10H, m), 3.00 (3H, s), 3.25-3.37 (4H, m), 3.61-3.71 (6H, m), 3.95 (2H, s), 5.11 (2H, s), 6.87 (1H, d, J=2.6 Hz), 6.93 (1H, dd, J=2.6, 8.6 Hz), 7.35-7.46 (4H, m), 7.90 (1H, d, J=8.6 Hz).
MS (ESI) m/z: 645 [M+H]⁺.

### (Examples 33 to 34, 188 to 189)

Compounds described in Table 20 were obtained by reacting corresponding carboxylic acids or esters with various amines in the same method as or a method similar to that in Example 187. Table 20 shows the structural formula, the compound name, the nuclear magnetic resonance spectrum (¹H-NMR) and its solvent, and the result of the electrospray ionization mass spectrum (ESI MS) of each compound.

**[Table 20]**

| Ex. No. | Structural formula | Compound name | ¹H-NMR (400 MHz) *δ* (ppm) from TMS | Solvent | ESI MS m/z |
|---|---|---|---|---|---|
| 188 | 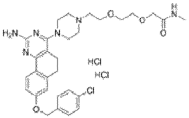 | 2-(2-(2-(4-(2-amino-8-((4-chlorobenzyl)oxy)-5,6-dihydrobenzo[h]quinazolin-4-yl)piperazin-1-yl)ethoxy)ethoxy)-N-methylacetamide dihydrochloride | 2.55-2.69(8H, m), 2.74-2.82(2H, m), 2.86(3H, d, J=4.9 Hz), 3.31(4H, m), 3.60-3.70(6H, m), 4.01(2H, s), 4.69(2H, s), 5.07(2H, s), 6.79(1H, d, J=2.7 Hz), 6.90(1H, dd, J=2.7, 8.6 Hz), 6.96-7.07(1H, m), 7.33-7.40(4H, m), 8.04(1H, d, J=8.6 Hz). | CDCl₃ | 581 [M+H]⁺ |
| 33 | 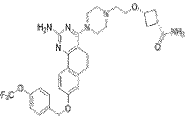 | cis-3-(2-(4-(2-amino-8-((4-(trifluoromethoxy)benzyl)oxy)-5,6-dihydrobenzo[h]quinazolin-4-yl)piperazin-1-yl)ethoxy)cyclobutane-1-carboxamide | 2.17-2.28(2H, m), 2.46-2.70(11 H, m), 2.73-2.85(2H, m), 3.26-3.39(4H, m), 3.52(2H, t, J=5.7 Hz), 3.85-3.95(1 H, m), 4.83(2H, brs), 5.09(2H, s), 5.42-5.65(2H, m), 6.80(1 H, d, J=2.5 Hz), 6.90(1 H, dd, J=2.5, 8.6 Hz), 7.20-7.28(2H, m), 7.44-7.51(2H, m), 8.04(1H, d, J=8.6 Hz). | CDCl₃ | 613 [M+H]⁺ |
| 34 | 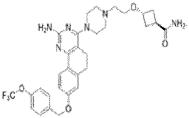 | trans-3-(2-(4-(2-amino-8-((4-(trifluoromethoxy)benzyl)oxy)-5,6-dihydrobenzo[h]quinazolin-4-yl)piperazin-1-yl)ethoxy)cyclobutane-1-carboxamide | 2.19-2.31(2H, m), 2.42-2.57(2H, m), 2.57-2.70(8H, m), 2.74-2.83(2H, m), 2.88-2.98(1H, m), 3.26-3.39(4H, m), 3.52(2H, t, J=5.9 Hz), 4.18-4.30(1H, m), 4.81(2H, brs), 5.09(2H, s), 5.35-5.59(2H, m), 6.80(1H, d, J=2.5 Hz), 6.90(1H, dd, J=2.5, 8.6 Hz), 7.19-7.28(2H, m), 7.43-7.50(2H, m), 8.04(1 H, d, J=8.6 Hz). | CDCl₃ | 613 [M+H]⁺ |
| 189 | 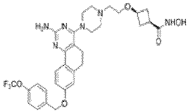 | cis-3-(2-(4-(2-amino-8-((4-(trifluoromethoxy)benzyl)oxy)-5,6-dihydrobenzo[h]quinazolin-4-yl)piperazin-1-yl)ethoxy)-N-hydroxycyclobutane-1-carboxamide | 1.86-2.04(2H, m), 2.20-2.46(3H, m), 2.55-2.61(8H, m), 2.73(2H, m), 3.17(4H, brs), 3.43(2H, t, J=5.9 Hz), 3.84(1H, m), 5.19(2H, s), 6.02(2H, s), 6.90-7.01(2H, m), 7.41(2H, d, J=8.5 Hz), 7.61(2H, d, J=8.5 Hz), 7.94(1 H, d, J=8.6 Hz), 8.71(1 H, s), 10.39(1H, s). | DMSO-d₆ | 629 [M+H]⁺ |

### (Example 38)

### 4-(4-(2-(azetidin-3-ylmethoxy)ethyl)piperazin-1-yl)-8-((4-(trifluoromethoxy)benzyl)oxy)-5,6-dihydrobenzo[h]q uinazolin-2-amine

### (a) tert-butyl 3-((2-(4-(2-amino-8-((4-(trifluoromethoxy)benzyl)oxy)-5,6-dihydrobenzo[h]quinazolin-4-yl)piperazin-1-yl)etho xy)methyl)azetidine-1-carboxylate

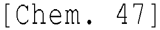

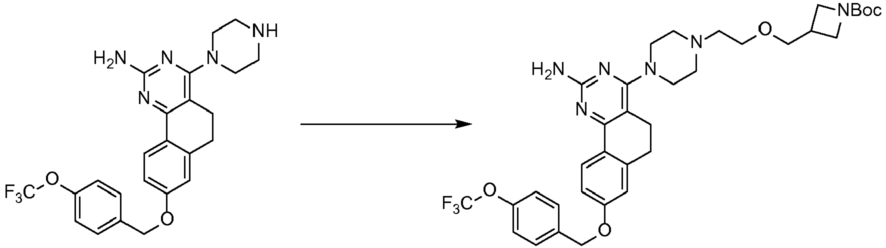

The title compound was obtained in the same method as in Example 23(a) except that tert-butyl 3-((2-chloroethoxy)methyl)azetidine-1-carboxylate was used in place of methyl cis-3-(2-chloroethoxy)cyclobutanecarboxylate.
¹H-NMR (400 MHz, CDCl₃) δ: 1.44 (9H, s), 2.57-2.70 (8H, m), 2.71-2.84 (3H, m), 3.27-3.38 (4H, m), 3.55-3.69 (6H, m), 3.99 (2H, t, J=8.5 Hz), 4.63-4.74 (2H, m), 5.10 (2H, s), 6.80 (1H, d, J=2.6 Hz), 6.90 (1H, dd, J=2.6 and 8.6 Hz), 7.24 (2H, d, J=7.9 Hz), 7.44-7.50 (2H, m), 8.04 (1H, d, J=8.6 Hz).
MS (ESI) m/z: 685 [M+H]⁺

### (b) 4-(4-(2-(azetidin-3-ylmethoxy)ethyl)piperazin-1-yl)-8-((4-(trifluoromethoxy)benzyl)oxy)-5,6-dihydrobenzo[h]q uinazolin-2-amine

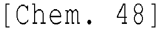

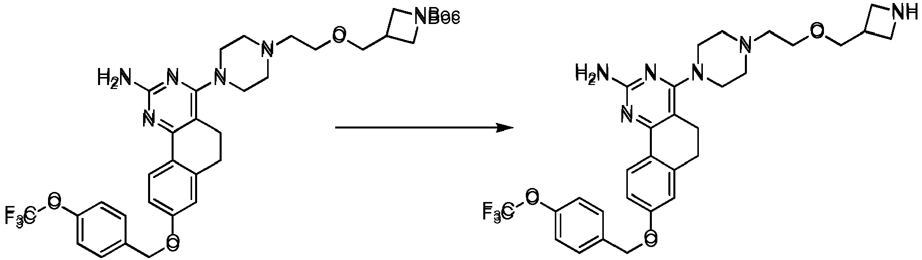

The title compound was obtained in the same method as in Reference Example 4(c) from the compound obtained in Example 38(a).
¹H-NMR (400 MHz, CDCl₃) δ: 2.56-2.70 (8H, m), 2.74-2.84 (2H, m) 2.88-3.04 (1H, m), 3.27-3.36 (4H, m), 3.42 (2H, dd, J=6.1, 8.0 Hz), 3.56-3.65 (4H, m), 3.71 (2H, t, J=8.1 Hz), 4.64-4.75 (2H, m), 5.10 (2H, s), 6.80 (1H, d, J=2.4 Hz), 6.90 (1H, dd, J=2.6, 8.6 Hz), 7.24 (2H, d, J=7.9 Hz), 7.47 (2H, d, J=8.7 Hz), 8.04 (1H, d, J=8.6 Hz).
MS (ESI) m/z: 585 [M+H]⁺.

### (Example 39)

### 3-((2-(4-(2-amino-8-((4-(trifluoromethoxy)benzyl)oxy)-5,6-dihydrobenzo[h]quinazolin-4-yl)piperazin-1-yl)etho xy)methyl)azetidine-1-carboxamide

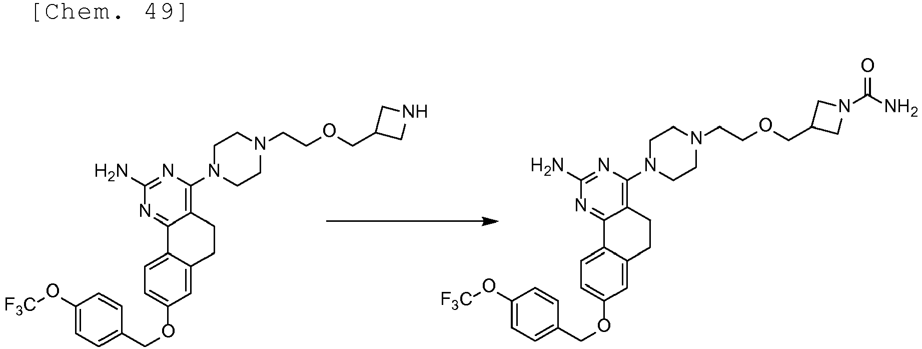

4-(4-(2-(azetidin-3-ylmethoxy)ethyl)piperazin-1-yl)-8-((4-(trifluoromethoxy)benzyl)oxy)-5,6-dihydroben zo[h]quinazolin-2-amine (117 mg, 0.2 mmol, 1.0 equivalent) obtained in Example 38 was dissolved in methylene chloride, and N,N-diisopropylethylamine (70 µL, 0.4 mmol, 2.0 equivalents) was added, followed by stirring at 0 °C. Thereafter, a methylene chloride solution (0.5 mL) of trimethylsilyl isocyanate (40 µL, 0.3 mmol, 1.5 equivalents) was added, followed by stirring at room temperature for 1.5 hours. A crude product obtained by distilling off the solvent under reduced pressure was purified through a silica gel column chromatography (eluent, methanol : chloroform) to obtain the title compound (115 mg).
¹H-NMR (400 MHz, CDCl₃) δ: 2.59-2.69 (8H, m), 2.75-2.81 (2H, m), 2.84 (1H, t, J=6.7 Hz), 3.33 (4H, brs), 3.60 (2H, d, J=6.6 Hz), 3.64 (2H, t, J=5.7 Hz), 3.73 (2H, dd, J=5.2, 8.0 Hz), 4.04 (2H, t, J=8.2 Hz), 4.20 (2H, brs), 4.71 (1H, brs), 5.10 (2H, s), 6.80 (1H, d, J=2.5 Hz), 6.90 (1H, dd, J=2.5, 8.6 Hz), 7.24 (2H, d, J=7.9 Hz), 7.47 (2H, d, J=8.7 Hz), 8.04 (1H, d, J=8.5 Hz).
MS (ESI) m/z: 628 [M+H]⁺.

### (Example 190)

### 3-(3-((2-(4-(2-amino-8-((4-(trifluoromethoxy)benzyl)ox y)-5,6-dihydrobenzo[h]quinazolin-4-yl)piperazin-1-yl)e thoxy)methyl)azetidin-1-yl)-4-hydroxy-1,2,5-thiadiazol e 1,1-dioxide

### (a) 3-(3-((2-(4-(2-amino-8-((4-(trifluoromethoxy)benzyl)ox y)-5,6-dihydrobenzo[h]quinazolin-4-yl)piperazin-1-yl)e thoxy)methyl)azetidin-1-yl)-4-ethoxy-1,2,5-thiadiazole 1,1-dioxide

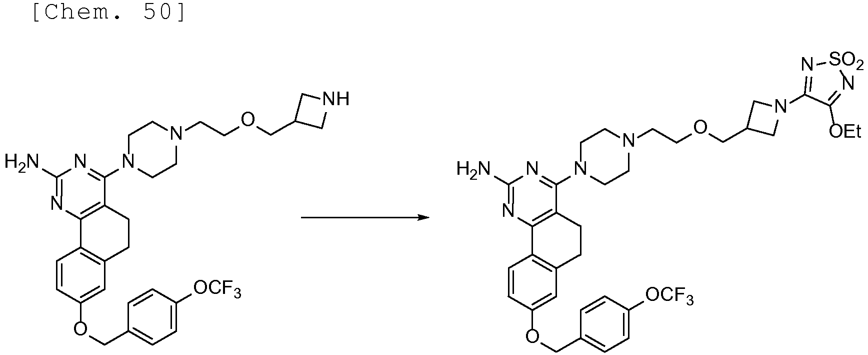

First, 4-(4-(2-(azetidin-3-ylmethoxy)ethyl)piperazin-1-yl)-8-((4-(trifluoromethoxy)benzyl)oxy)-5,6-dihydrobenzo[h]q uinazolin-2-amine (18.5 mg, 0.032 mmol) obtained in Example 38 was dissolved in THF (0.3 mL), which was added to a THF (0.3 mL)-solution of 3,4-diethoxy-1,2,5-thiadiazole 1,1-dioxide(7.8 mg, 0.038 mmol), followed by stirring at room temperature for 1 hour. The solvent was distilled off under reduced pressure and subjected to a thin-layer silica gel column chromatography (eluent, chloroform:methanol) to obtain the title compound (10 mg).

### (b) 3-(3-((2-(4-(2-amino-8-((4-(trifluoromethoxy)benzyl)ox y)-5,6-dihydrobenzo[h]quinazolin-4-yl)piperazin-1-yl)e thoxy)methyl)azetidin-1-yl)-4-hydroxy-1,2,5-thiadiazol e 1,1-dioxide

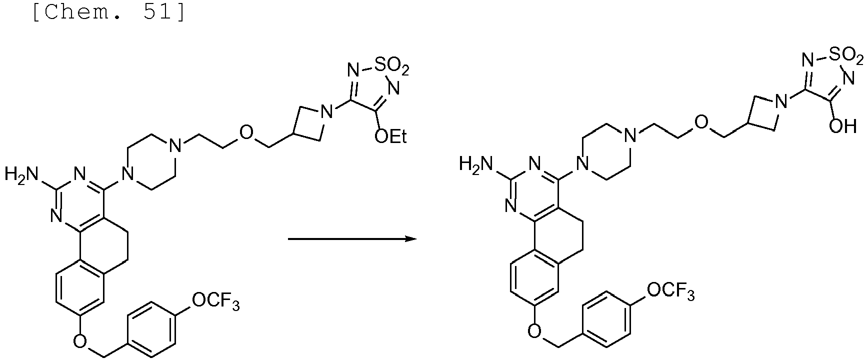

A composition (10 mg) of 3-(3-((2-(4-(2-amino-8-((4-(trifluoromethoxy)benzyl)ox y)-5,6-dihydrobenzo[h]quinazolin-4-yl)piperazin-1-yl)e thoxy)methyl)azetidin-1-yl)-4-ethoxy-1,2,5-thiadiazole 1,1-dioxide obtained in Example 190(a) was dissolved in acetonitrile/THF/water (2.0 mL, 2/1/1, v/v/v), followed by stirring at room temperature for 2.5 hours and further stirring at 50° for 20 hours. After the reaction solution was filtered, the solvent was distilled off under reduced pressure to obtain the title compound (5.7 mg).
¹H-NMR (400 MHz, DMSO-d₆) δ: 2.63 (2H, d, J=7.5 Hz), 2.71-2.80 (2H, m), 2.90-2.99 (1H, m), 3.00-3.18 (5H, m), 3.32 (superimposed by H₂O, m), 3.62 (2H, d, J=6.1 Hz), 3.72 (2H, brs), 3.80 (1H, dd, J=5.2, 10.2 Hz), 4.10 (1H, t, J=9.2 Hz), 4.21 (1H, dd, J=5.4, 10.4 Hz), 4.49 (1H, t, J=9.2 Hz), 5.19 (2H, s), 6.21 (2H, brs), 6.90-7.02 (2H, m), 7.40 (2H, d, J=8.1 Hz), 7.60 (2H, d, J=8.6 Hz), 7.93 (1H, d, J=9.2 Hz), 8.21 (1H, brs).
MS (ESI) m/z: 717 [M+H]⁺.

### (Example 40)

### N-(2-amino-4-(4-methylpiperazin-1-yl)-5,6-dihydrobenzo [h]quinazolin-8-yl)-3-phenoxypropane-1-sulfonamide

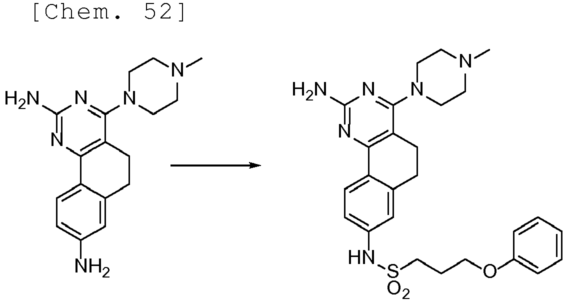

The compound (77 mg) obtained in Reference Example 3(c) was dissolved in chloroform (1.3 mL), and pyridine (31 µl, 1.5 equivalents) and 3-phenoxypropane-1-sulfonyl chloride (73 mg, 1.2 equivalents) were added. After stirring at room temperature for 19 hours, the product was purified through a silica gel column chromatography (eluent, ethyl acetate : hexane) to obtain 54 mg of the title compound.
¹H-NMR (400 MHz, CDCl₃) δ: 2.24-2.33 (2H, m), 2.33-2.38 (3H, m), 2.53 (4H, t, J=4.5 Hz), 2.59-2.69 (2H, m), 2.69-2.77 (2H, m), 3.27-3.40 (6H, m), 4.02 (2H, t, J=5.8 Hz), 4.78 (2H, brs), 6.77-6.83 (2H, m), 6.90-6.96 (1H, m), 7.03-7.08 (2H, m), 7.15-7.29 (2H, m), 8.00-8.05 (1H, m). MS (ESI) m/z: 509 [M+H]⁺.

### (Examples 41 to 56, 191 to 195)

Compounds described in Tables 21 to 23 were obtained in the same method as in Example 190 (b). Tables 21 to 23 show the structural formula, the compound name, the nuclear magnetic resonance spectrum (¹H-NMR) and its solvent, and the result of the electrospray ionization mass spectrum (ESI MS) of each compound.

**[Table 21]**

| Ex. No. | Structural formula | Compound name | ¹H-NMR (400 MHz) *δ* (ppm) from TMS | Solvent | ESI MS m/z |
|---|---|---|---|---|---|
| 41 | 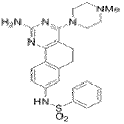 | N-(2-amino-4-(4-methylpiperazin-1-yl)-5,6-dihydrobenzo[h]quinazolin-8-yl)benzenesulfonamide | 2.34(3H, s), 2.54(4H, t, J=4.3 Hz), 2.57-2.64(2H, m), 2.64-2.74(2H, m), 3.33(4H, t, J=4.3 Hz), 4.81(2H, brs), 6.79-6.87(1H, m), 6.92-7.01(1H, m), 7.39(2H,m), 7.46-7.55(1H, m), 7.74-7.80(2H, m), 7.82(1H, m). | CDCl₃ | 451 [M+H]⁺ |
| 42 | 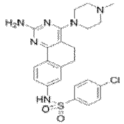 | N-(2-amino-4-(4-methylpiperazin-1-yl)-5,6-dihydrobenzo[h]quinazolin-8-yl)-4-chlorobenzenesulfonamide | 2.34(3H, s), 2.53(4H, d, J=4.4 Hz), 2.61-2.67(2H, m), 2.71-2.78(2H, m), 3.33(4H, t, J=4.4 Hz), 4.64-4.72(1H, m), 6.91-6.97(1H, m), 6.97-7.01(1H, m), 7.38-7.41(1H, m), 7.41-7.44(1H, m), 7.68-7.70(1 H, m), 7.70-7.74(1 H, m), 7.96(1 H, d, J=8.3 Hz). | CDCl₃ | 485 [M+H]⁺ |
| 43 | 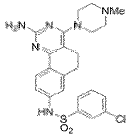 | N-(2-amino-4-(4-methylpiperazin-1-yl)-5,6-dihydrobenzo[h]quinazolin-8-yl)-3-chlorobenzenesulfonamide | 2.36(3H, s), 2.54(4H, t, J=4.3 Hz), 2.63(2H, t, J=7.1 Hz), 2.68-2.76(2H, m), 3.34(4H, t, J=4.3 Hz), 4.77(2H, brs), 6.85-6.94(1H, m), 6.95-7.00(1H, m), 7.31-7.39(1H, m), 7.46-7.53(1H, m), 7.59-7.65(1H, m), 7.81(1H, t, J=1.9 Hz), 7.88-7.96(1H, m). | CDCl₃ | 485 [M+H]⁺ |
| 44 | 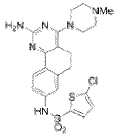 | N-(2-amino-4-(4-methylpiperazin-1-yl)-5,6-dihydrobenzo[h]quinazolin-8-yl)-5-chlorothiophene-2-sulfonamide | 2.34(3H, s), 2.52(4H, t, J=4.5 Hz), 2.60-2.68(2H, m), 2.72-2.79(2H, m), 3.33(4H, t, J=4.5 Hz), 4.72(2H, brs), 6.82(1 H, d, J=4.0 Hz), 6.99(1 H, dd, J=2.1, 8.2 Hz), 7.03(1H, d, J=2.1 Hz), 7.29(1H, d, J=4.0 Hz), 7.98(1 H, d, J=8.2 Hz). | CDCl₃ | 491 [M+H]⁺ |
| 45 | 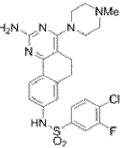 | N-(2-amino-4-(4-methylpiperazin-1-yl)-5,6-dihydrobenzo[h]quinazolin-8-yl)-4-chloro-3-fluorobenzenesulfonamide | 2.34(3H, s), 2.52(4H, t, J=4.5 Hz), 2.61(2H, t, J=6.7 Hz), 2.71(2H, t, J=6.7 Hz), 3.33(4H, t, J=4.5 Hz), 4.77(2H, brs), 6.88-6.93(1H, m), 6.94-6.97(1H, m), 7.41-7.48(1H, m), 7.49-7.53(1H, m), 7.59(1H, dd, J=2.0, 8.2 Hz), 7.88-7.94(1H, m). | CDCl₃ | 503 [M+H]⁺ |
| 46 | 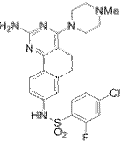 | N-(2-amino-4-(4-methylpiperazin-1-yl)-5,6-dihydrobenzo[h]quinazolin-8-yl)-4-chloro-2-fluorobenzenesulfonamide | 2.34(3H, s), 2.51(4H, t, J=4.5 Hz), 2.57-2.65(2H, m), 2.66-2.74(2H, m), 3.31(4H, t, J=4.5 Hz), 4.70(2H, brs), 6.93-6.99(2H, m), 7.15-7.18(1 H, m), 7.18-7.21(1H, m), 7.74-7.82(1H, m), 7.88-7.93(1H, m). | CDCl₃ | 503 [M+H]⁺ |
| 47 | 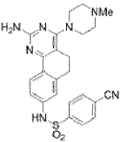 | N-(2-amino-4-(4-methylpiperazin-1-yl)-5,6-dihydrobenzo[h]quinazolin-8-yl)-4-cyanobenzenesulfonamide | 2.34(3H, s), 2.52(4H, t, J=4.5 Hz), 2.59-2.65(2H, m), 2.68-2.74(2H, m), 3.33(4H, t, J=4.5 Hz), 4.76(2H, brs), 6.89-6.93(1H, m), 6.93-6.96(1H, m), 7.69-7.72(1H, m), 7.72-7.74(1H, m), 7.86-7.88(1H, m), 7.88-7.90(1H, m), 7.91-7.95(1H, m). | CDCl₃ | 476 [M+H]⁺ |

**[Table 22]**

| Ex. No. | Structural formula | Compound name | ¹H-NMR (400 MHz) *δ* (ppm) from TMS | Solvent | ESI MS m/z |
|---|---|---|---|---|---|
| 48 | 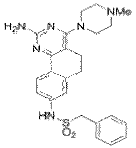 | N-(2-amino-4-(4-methylpiperazin-1-yl)-5,6-dihydrobenzo[h]quinazolin-8-yl)-1-phenylmethanesulfonamide | 2.34(3H, s), 2.53(4H, t, J=4.4 Hz), 2.67(2H, t, J=6.9 Hz), 2.79(2H, t, J=6.9 Hz), 3.34(4H, t, J=4.4 Hz), 4.36(2H, s), 4.75(2H, brs), 6.92-7.00(1 H, m), 7.04-7.07(1 H, m), 7.24-7.28(2H, m), 7.30-7.40(3H, m), 8.02-8.07(1 H, m). | CDCl₃ | 465 [M+H]⁺ |
| 49 | 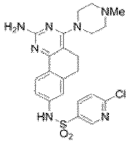 | N-(2-amino-4-(4-methylpiperazin-1-yl)-5,6-dihydrobenzo[h]quinazolin-8-yl)-6-chloropyridine-3-sulfonamide | 2.34(3H, s), 2.52(4H, t, J=4.8 Hz), 2.61-2.68(2H, m), 2.73-2.79(2H, m), 3.33(4H, t, J=4.8 Hz), 4.70(2H, brs), 6.94-6.98(1H, m), 7.00-7.02(1H, m), 7.37-7.40(1H, dd, J=0.7, 8.3 Hz), 7.93(1H, dd, J=2.6, 8.3 Hz), 7.97-8.01(1H, m), 8.78(1H, dd, J=0.7, 2.6 Hz). | CDCl₃ | 486 [M+H]⁺ |
| 50 | 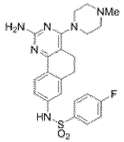 | N-(2-amino-4-(4-methylpiperazin-1-yl)-5,6-dihydrobenzo[h]quinazolin-8-yl)-4-fluorobenzenesulfonamide | 2.34(3H, s), 2.52(4H, t, J=4.6 Hz), 2.59-2.66(2H, m), 2.69-2.77(2H, m), 3.32(4H, t, J=4.6 Hz), 4.72(2H, brs), 6.89-6.94(1H, m), 6.95-6.98(1H, m), 7.06-7.13(2H, m), 7.76-7.83(2H, m), 7.91-7.95(1H, m). | CDCl₃ | 469 [M+H]⁺ |
| 51 | 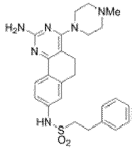 | N-(2-amino-4-(4-methylpiperazin-1-yl)-5,6-dihydrobenzo[h]quinazolin-8-yl)-2-phenylethane-1-sulfonamide | 2.34(3H, s), 2.53(4H, t, J=4.6 Hz), 2.60-2.68(2H, m), 2.70-2.78(2H, m), 3.08-3.18(2H, m), 3.29-3.40(6H, m), 4.79(2H, brs), 6.86-6.92(2H, m), 7.10-7.31(5H, m), 7.97-8.02(1H, m). | CDCl₃ | 479 [M+H]⁺ |
| 52 | 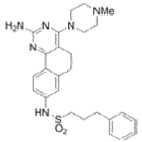 | N-(2-amino-4-(4-methylpiperazin-1-yl)-5,6-dihydrobenzo[h]quinazolin-8-yl)-3-phenylpropane-1-sulfonamide | 2.08-2.16(2H, m), 2.34(3H, s), 2.53(4H, t, J=4.5 Hz), 2.59-2.66(2H, m), 2.69(2H, t, J=7.5 Hz), 2.71-2.77(2H, m), 3.05-3.13(2H, m), 3.33(4H, t, J=4.5 Hz), 4.81(2H, brs), 6.96(1 H, dd, J=2.3, 8.3 Hz), 7.02(1H, d, J=2.3 Hz), 7.06-7.28(5H, m), 8.00(1H, m). | CDCl₃ | 493 [M+H]⁺ |
| 53 | 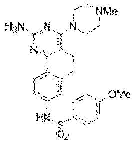 | N-(2-amino-4-(4-methylpiperazin-1-yl)-5,6-dihydrobenzo[h]quinazolin-8-yl)-4-methoxybenzenesulfonami de | 2.33(3H, s), 2.52(4H, t, J=4.3 Hz), 2.54-2.61(2H, m), 2.61-2.68(2H, m), 3.31(4H, t, J=4.3 Hz), 3.76(3H, s), 4.96(2H, brs), 6.76-6.84(3H, m), 6.90-6.93(1 H, m), 7.66-7.73(2H, m), 7.75-7.80(1H, m). | CDCl₃ | 481 [M+H]⁺ |
| 54 | 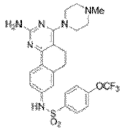 | N-(2-amino-4-(4-methylpiperazin-1-yl)-5,6-dihydrobenzo[h]quinazolin-8-yl)-4-(trifluoromethoxy)benzene sulfonamide | 2.34(3H, s), 2.52(4H, t, J=4.5 Hz), 2.56-2.63(2H, m), 2.64-2.71(2H, m), 3.33(4H, t, J=4.5 Hz), 4.86(2H, brs), 6.86(1 H, dd, J=2.3, 8.3 Hz), 692(1H, d, J=2.1 Hz), 7.20-7.26(2H, m), 7.80-7.83(1 H, m), 7.83-7.85(1 H, m), 7.87(1 H, m). | CDCl₃ | 535 [M+H]⁺ |

**[Table 23]**

| Ex. No. | Structural formula | Compound name | ¹H-NMR (400 MHz) *δ* (ppm) from TMS | Solvent | ESI MS m/z |
|---|---|---|---|---|---|
| 55 | 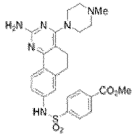 | methyl 4-(N-(2-amino-4-(4-methylpiperazin-1-yl)-5,6-dihydrobenzo[h]quinazolin-8-yl)sulfamoyl)benzoate | 2.33(3H, s), 2.52(4H, t, J=4.3 Hz), 2.55-2.62(2H, m), 2.62-2.69(2H, m), 3.32(4H, t, J=4.3 Hz), 3.90(3H, s), 4.90(2H, brs), 6.79(1H, dd, J=2.2, 8.3 Hz), 6.91(1H, d, J=2.2 Hz), 7.79-7.85(3H, m), 8.01-8.03(1H, m), 8.03-8.06(1H, m). | CDCl₃ | 509 [M+H]⁺ |
| 56 | 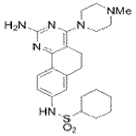 | N-(2-amino-4-(4-methylpiperazin-1-yl)-5,6-dihydrobenzo[h]quinazolin-8-yl)cyclohexanesulfonamide | 1.12-1.34(3H, m), 1.52-1.70(3H, m), 1.81-1.92(2H, m), 2.15(2H, m), 2.35(3H, s), 2.53(4H, t, J=4.6 Hz), 2.63-2.70(2H, m), 2.75-2.83(2H, m), 3.03(1H, tt, J=3.5, 12.1 Hz), 3.34(4H, t, J=4.6 Hz), 4.74(2H, brs), 705-711(2H, m), 8.00-8.05(1 H, m). | CDCl₃ | 457 [M+H]⁺ |
| 191 | 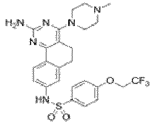 | N-(2-amino-4-(4-methylpiperazin-1-yl)-5,6-dihydrobenzo[h]quinazolin-8-yl)-4-(2,2,2-trifluoroethoxy)benzenesul fonamide | 2.34(3H, s), 2.48-2.56(4H, m), 2.58-2.65(2H, m), 2.68-2.75(2H, m), 3.29-3.36(4H, m), 4.36(2H, q, J=7.6 Hz), 4.73-4.78(2H, m), 6.88-6.97(4H, m), 7.73-7.78(2H, m), 791(1H, d, J=8.2 Hz). | CDCl₃ | 549 [M+H]⁺ |
| 192 | 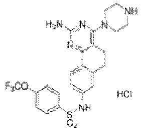 | N-(2-amino-4-(piperazin-1-yl)-5,6-dihydrobenzo[h]quinazolin-8-y\|)-4-(trifluoromethoxy)benzene sulfonamide hydrochloride | 2.57-2.80(4H, m), 3.21(4H, brs), 3.76(4H, brs), 7.16(2H, d, J=8.7 Hz), 7.59(2H, d, J=8.4 Hz), 7.86(1H, d, J=8.2 Hz), 8.00(2H, d, J=7.9 Hz), 9.24(2H, brs). | DMSO-d₆ | 521 [M+H]⁺ |
| 193 | 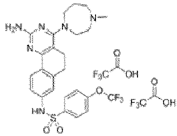 | N-(2-amino-4-(4-methyl-1,4-diazepan-1-yl)-5,6-dihydrobenzo[h]quinazolin-8-yl)-4-(trifluoromethoxy)benzene sulfonamide bis(trifluoroacetate) | 1.35-1.45(2H, m), 2.33(3H, s), 2.53-2.74(8H, m), 4.20-4.35(4H, m), 7.18(1 H, d, J=2.2 Hz), 7.22(1 H, dd, J=2.2, 8.6 Hz), 7.42-7.47(2H, m), 761(1H, d), 7.97-8.01(2H, m). | CD₃OD | 549 [M+H]⁺ |
| 194 | 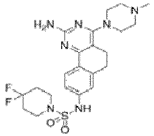 | N-(2-amino-4-(4-methylpiperazin-1-yl)-5,6-dihydrobenzo[h]quinazolin-8-yl)-4,4-difluoropiperidine-1-sulfonamide | 1.93-2.04(4H, m), 2.37(3H, s), 2.54(4H, t, J=4.6 Hz), 2.59-2.70(2H, m), 2.71-2.84(2H, m), 3.28-3.39(4H, m), 3.39-3.48(4H, m), 4.79(2H, s), 6.98(1 H, d, J=2.3 Hz), 7.05(1 H, dd, J=2.3, 8.3 Hz), 8.02(1H, d, J=8.3 Hz). | CDCl₃ | 494 [M+H]⁺ |
| 195 | 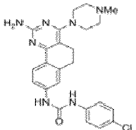 | 1-(2-amino-4-(4-methylpiperazin-1-yl)-5,6-dihydrobenzo[h]quinazolin-8-yl)-3-(4-chlorophenyl)urea | 2.34(3H, s), 2.51(4H, t, J=4.6 Hz), 2.56-2.64(3H, m), 2.71-2.77(2H, m), 3.32(4H, t, J=4.6 Hz), 4.77(2H, brs), 7.01-7.10(3H, m), 7.24-7.38(4H, m), 7.99(1H, d, J=8.4 Hz). | CDCl₃ | 464 [M+H]⁺ |

### (Examples 196 to 204)

Compounds described in Table 24 were each obtained using a corresponding heterocyclic derivative in place of 6-hydroxy-3,4-dihydronaphthalen-1(2H)-one and/or a corresponding halide in place of 1-(bromomethyl)-4-chlorobenzene in the same method as or a method similar to that in Example 2. Table 24 shows the structural formula, the compound name, the nuclear magnetic resonance spectrum (¹H-NMR) and its solvent, and the result of the electrospray ionization mass spectrum (ESI MS) of each compound.

**[Table 24]**

| Ex. No. | Structural formula | Compound name | ¹H-NMR (400 MHz) *δ* (ppm) from TMS | Solvent | ESI MS m/z |
|---|---|---|---|---|---|
| 196 | 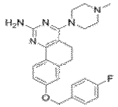 | 8-((4-fluorobenzyl)oxy)-4-(4-methylpiperazin-1-yl)-5,6-dihydrobenzo[h]quinazolin-2-amine | 2.36(3H, s), 2.55(4H, t, J=4.5 Hz), 2.62-2.69(2H, m), 2.73-2.82(2H, m), 3.35(4H, t, J=4.5 Hz), 4.72-4.90(2H, m), 5.06(2H, s), 6.81 (1H, d, J=2.5 Hz), 691(1H, dd, J=2.5, 8.6 Hz), 7.04-7.12,(2H, m), 7.37-7.45(2H, m), 8.05(1 H, d, 8.6 Hz). | CDCl₃ | 420 [M+H]⁺ |
| 197 | 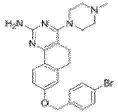 | 8-((4-bromobenzyl)oxy)-4-(4-methylpiperazin-1-yl)-5,6-dihydrobenzo[h]quinazolin-2-amine | 2.43(3H, brs), 2.57-2.83(8H, m), 3.40-3.54(4H, m), 4.74(2H, brs), 5.06(2H, s), 6.80(1H. d, J=2.3 Hz), 6.93(1H, dd, J=2.3, 8.6 Hz), 7.31(2H, d, J=8.3 Hz), 7.52(2H, d, J=8.3 Hz), 8.06(1 H, d, J=8.6 Hz). | CDCl₃ | 480 [M+H]⁺ |
| 198 | 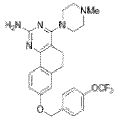 | 4-(4-methylpiperazin-1-yl)-8-((4-(trifluoromethoxy)benzyl)o xy)-5,6-dihydrobenzo[h]quinazolin-2-amine | 2.35(3H, s), 2.53(4H, t, J=4.6 Hz), 2.62-2.71(2H, m), 2.75-2.82(2H, m), 3.33(4H, t, J=4.6 Hz), 4.65-475(2H, brs), 5.10(2H, s), 6.79-6.82(1H, m), 6.88-6.94(1H, m), 7.20-7.25(2H, m), 7.44-7.50(2H, m), 8.02-8.07(1H, m). | CDCl₃ | 486 [M+H]⁺ |
| 199 | 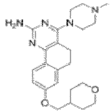 | 4-(4-methylpiperazin-1-yl)-8-((tetrahydro-2H-pyran-4-yl)methoxy)-5,6-dihydrobenzo[h]quinazolin-2-amine | 1.38-1.54(2H, m), 1.65-1.82(2H, m), 2.15-2.19(1H, m), 2.34(3H, s), 2.46-2.82(8H, m), 3.24-3.51(6H, m), 3.85(2H, d, J=6.4 Hz), 3.97-4.06(2H, m), 4.71(2H, s), 6.72(1 H, d, J=2.4 Hz), 6.83(1 H, dd, J=2.4, 8.6Hz), 8.03(1 H, d, J=8.6 Hz). | CDCl₃ | 410 [M+H]⁺ |
| 200 | 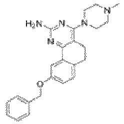 | 9-(benzyloxy)-4-(4-methylpiperazin-1-yl)-5,6-dihydrobenzo[h]quinazolin-2-amine | 2.35(3H, s), 2.54(4H, t, J=4.0 Hz), 2.63-2.67(2H, m), 2.73-2.77(2H, m), 3.34(4H, t, J=4.0 Hz), 4.71(2H, s), 5.13(2H, s), 695(1H, dd, J=2.0, 8.4 Hz), 7.11(1H, d, J=8.4 Hz), 7.30-7.40(3H m), 7.45-7.47(2H, m), 7.78(1 H, d, J=2.0 Hz). | CDCl₃ | 402 [M+H]⁺ |
| 201 | 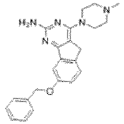 | 8-(benzyloxy)-4-(4-methylpiperazin-1-yl)-5H-indeno[1,2-d]pyrimidin-2-amine | 2.35(3H, s), 2.50(4H, t, J=8.0 Hz), 3.75(2H, s), 3.82(4H, t, J=8.0 Hz), 4.75(2H, s), 5.15(2H, s), 708(1H, dd, J=2.4, 8.0 Hz), 7.31-7.48(6H, m), 7.55(1H, d, J=2.4 Hz). | CDCl₃ | 388 [M+H]⁺ |
| 202 | 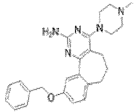 | 10-(benzyloxy)-4-(4-methylpiperazin-1-yl)-6,7-dihydro-5H-benzo[6,7]cyclohepta[1,2-d]pyrimidin-2-amine | 2.09-2.25(9H, m), 2.42(4H, s), 3.26(4H, s), 5.08(2H, s), 6.10(2H, s), 6.99(1H, d, J=8.0 Hz), 718(1H, d, J=8.0 Hz), 7.26-7.46(6H, m). | DMSO-d₆ | 416 [M+H]⁺ |
| 203 | 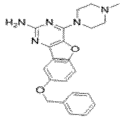 | 8-(benzyloxy)-4-(4-methylpiperazin-1-yl)benzofuro[3,2-d]pyrimidin-2-amine | 2.36(3H, s), 2.55(4H, t, J=5.0 Hz), 4.07(4H, t, J=5.0 Hz), 4.73(2H, s), 5.13(3H, s), 7.20(1H, dd, J=2.7, 9.0 Hz), 7.31-7.49(6H, m), 754(1H, d, J=2.7 Hz). | CDCl₃ | 390 [M+H]⁺ |
| 204 | 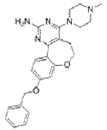 | 9-(benzyloxy)-4-(4-methylpiperazin-1-yl)-5,6-dihydrobenzo[2,3]oxepino[4,5-d]pyrimidin-2-amine | 2.35(3H, s), 2.53(4H, t, J=4.4 Hz), 2.74(2H, t, J=5.6 Hz), 3.36(4H, t, J=4.4 Hz), 4.51(2H, t, J=5.6 Hz), 4.74(2H, s), 5.09(2H, s), 6.68(1H, d, J=2.8 Hz), 682(1H, dd, J=2.8, 8.8 Hz), 7.31-7.44(5H, m), 7.90(1H, d, J=8.8 Hz). | CDCl₃ | 418 [M+H]⁺ |

### (Examples 205 to 215)

Compounds of Tables 25 to 26 were each obtained by the same methods as those in Examples 13, 14 except that a corresponding halide was used in place of 1-(bromomethyl)-4-chlorobenzene in Reference Example 1(a). Tables 25 to 26 show the structural formula, the compound name, the nuclear magnetic resonance spectrum (¹H-NMR) and its solvent, and the result of the electrospray ionization mass spectrum (ESI MS) of each compound.

**[Table 25]**

| Ex. No. | Structural formula | Compound name | ¹H-NMR (400 MHz) *δ* (ppm) from TMS | Solvent | ESI MS m/z |
|---|---|---|---|---|---|
| 205 | 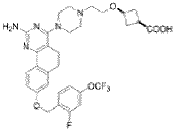 | cis-3-(2-(4-(2-amino-8-((2-fluoro-4-(trifluoromethoxy)benzyl)o xy)-5,6-dihydrobenzo[h]quinazolin-4-yl)piperazin-1-yl)ethoxy)cyclobutane-1-carboxylic acid | 201(2H, m), 2.45(8H, m), 2.62(3H, m), 2.75(2H, m), 3.21(4H, m), 3.42-3.52(2H, m), 3.85-3.92(1H, m), 5.22(2H, s), 6.18(2H, brs), 6.92-7.04(2H, m), 732(1H, m), 7.48(1H, m), 774(1H, t, J=8.5 Hz), 7.95(1H, d, J=7.7 Hz). | DMSO-d₆ | 632 [M+H]⁺ |
| 206 | 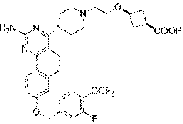 | cis-3-(2-(4-(2-amino-8-((3-fluoro-4-(trifluoromethoxy)benzyl)oxy)-5,6-dihydrobenzo[h]quinazolin-4-yl)piperazin-1-yl)ethoxy)cyclobutane-1-carboxylic acid | 201(2H, m), 2.45(8H, m), 2.62(3H, m), 2.75(2H, m), 3.22(4H, m), 3.42-3.52(2H, m), 3.89(1H, m), 5.21(2H, s), 6.18(2H, brs), 6.93-7.04(2H, m), 7.33-7.47(1H, m), 7.59-7.66(2H, m), 7.95(1H, d, J=7.2 Hz). | DMSO-d₆ | 632 [M+H]⁺ |
| 207 | 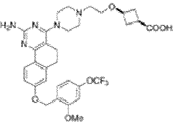 | cis-3-(2-(4-(2-amino-8-((2-methoxy-4-(trifluoromethoxy)benzyl)o xy)-5,6-dihydrobenzo[h]quinazolin-4-yl)piperazin-1-yl)ethoxy)cyclobutane-1-carboxvlic acid | 2.01(2H, m), 2.39-2.48(2H, m), 2.56-2.67(4H, m), 2.71-2.82(3H, m), 3.33(m, overlapped with DHO), 3.52-3.71(3H, m), 3.88-3.92(m, 4H), 5.11(2H, s), 6.22(2H, brs), 6.91-7.03(3H, m), 7.08(1H, d, J=1.9 Hz), 7.54(1H, d, J=8.3 Hz), 7.95(1H, d, J=9.2 Hz). | DMSO-d₆ | 644 [M+H]⁺ |
| 208 | 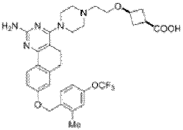 | cis-3-(2-(4-(2-amino-8-((2-methyl-4-(trifluoromethoxy)benzyl)o xy)-5,6-dihydrobenzo[h]quinazolin-4-yl)piperazin-1-yl)ethoxy)cyclobutane-1-carboxylic acid | 1.95-2.08(2H, m), 2.39(3H, s), 2.39-2.48(2H, m), 2.56-2.68(3H, m), 2.75-2.84(2H, m), 3.10-4.20(12H, brm), 3.92(1H, quin, J=7.1 Hz), 5.19(2H, s), 7.06(2H, brs), 7.22(1 H, d, J=.8.0 Hz), 7.27(1 H, s), 7.56(1 H, d, J=8.0 Hz), 8.03(1 H, d, J=9.1 Hz). | DMSO-d₆ | 628 [M+H]⁺ |
| 209 | 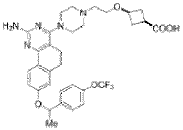 | cis-3-(2-(4-(2-amino-8-(1-(4-(trifluoromethoxy)phenyl)e thoxy)-5,6-dihydrobenzo[h]quinazolin-4-yl)piperazin-1-yl)ethoxy)cyclobutane-1-carboxylic acid | 1.56(3H, d, J=6.4 Hz), 1.88-2.00(2H, m), 2.37-2.46(2H, m), 2.47-2.61 (m, overlapped with DMSO), 2.62-2.73(2H, m), 3.15(4H, brs), 3.33(m, overlapped with DHO), 3.42(2H, t, J=5.9 Hz), 3.79-3.91(1 H, m), 5.65(1 H, q, J=6.3 Hz), 5.99(2H, brs), 6.84(2H, m), 7.36(2H, m), 7.52-7.60(2H, m), 7.85(1H, d, J=8.6 Hz). | DMSO-d₆ | 628 [M+H]⁺ |
| 210 | 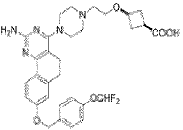 | cis-3-(2-(4-(2-amino-8-((4-(difluoromethoxy)benzyl)oxy)-5,6-dihydrobenzo[h]quinazolin-4-yl)piperazin-1-yl)ethoxy)cyclobutane-1-carboxylic acid | 1.96-2.10(2H, m), 2.39-2.49(2H, m), 2.55-2.69(3H, m), 2.71-2.83(2H, m), 3.00-4.15(13H, m), 5.16(2H, s), 6.19(1H, brs), 6.93-7.03(2H, m), 7.07-7.44(1 H, t, J=7.4 Hz), 7.22(2H, d, J=8.5 Hz), 7.49-7.57(2H, m), 7.95(1 H, d, J=8.5 Hz). | DMSO-d₆ | 596 [M+H]⁺ |
| 211 | 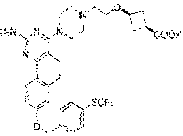 | cis-3-(2-(4-(2-amino-8-((4-((trifluoromethyl)thio)benzyl)oxy)-5,6-dihydrobenzo[h]quinazolin-4-yl)piperazin-1-yl)ethoxy)cyclobutane-1-carboxylic acid | 2.10-2.23(2H, m), 2.48-2.61(3H, m), 2.62-2.75(3H, m), 2.77-2.90(3H, m), 296-316(2H, m), 3.64(3H, brs), 3.83-3.96(6H, m), 5.13(2H, s), 6.83(1H, m), 6.94-7.06(1H, m), 7.47(2H, d, J=8.2 Hz), 7.67(2H, d, J=8.2 Hz), 8.31(1H, d, J=8.7 Hz). | CDCl₃ | 630 [M+H]⁺ |

**[Table 26]**

| Ex. No. | Structural formula | Compound name | ¹H-NMR (400 MHz) *δ* (ppm) from TMS | Solvent | ESI MS m/z |
|---|---|---|---|---|---|
| 212 | 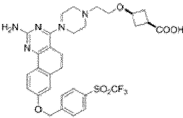 | cis-3-(2-(4-(2-amino-8-((4-((trifluoromethyl)sulfonyl)b enzyl)oxy)-5,6-dihydrobenzo[h]quinazolin-4-yl)piperazin-1-yl)ethoxy)cyclobutane-1-carboxylic acid | 2.15-2.29(2H, m), 2.47-2.58(2H, m), 2.60-2.87(11 H, m), 3.57(2H, t, J=4.9 Hz), 3.62-3.70(4H, brm), 3.88(1H, quin, J=7.2 Hz), 5.26(2H, s), 6.85(1 H, d, J=2.4 Hz), 6.98(1 H, dd, J=2.4, 8.5 Hz), 7.74(2H, d, J=8.6 Hz), 8.07(2H, d, J=8.6 Hz), 8.24(1H, d, J=8.5 Hz). | CDCl₃ | 662 [M+H]⁺ |
| 213 | 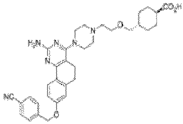 | trans-4-((2-(4-(2-amino-8-((4-cyanobenzyl)oxy)-5,6-dihydrobenzo[h]quinazolin-4-yl)piperazin-1-yl)ethoxy)methyl)cyclohexane-1-carboxylic acid | 0.93(2H, qd, J=3.3, 12.7 Hz), 1.22-1.35(2H, m), 1.46-1.50 (1H, m), 1.72-1.79(2H, m), 1.89(2H, dd, J=3.0, 13.5 Hz), 2.07-2.15(1H, m), 2.48-2.61(7H, m), 2.66-2.79(2H, m), 3.09-3.26(6H, m), 3.49(3H, t, J=5.8 Hz), 5.27(2H, s), 6.00(2H, s), 6.91-6.97(2H, m), 7.65(2H, d, J=7.8 Hz), 7.83(2H, d, J=8.3 Hz). 7.93(1H. d. J=8.3 Hz). | DMSO-d₆ | 597 [M+H]⁺ |
| 214 | 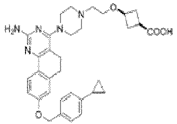 | cis-3-(2-(4-(2-amino-8-((4-cyclopropylbenzyl)oxy)-5,6-dihydrobenzo[h]quinazolin-4-yl)piperazin-1-yl)ethoxy)cyclobutane-1-carboxylic acid | 0.59-0.73(2H, m), 0.88-1.01(2H, m), 1.78-1.96(1H, m), 2.18(2H, m), 2.51(2H, brs), 2.58-2.69(3H, m), 2.75(2H, brs), 2.91-3.16(6H, m), 3.65(2H, brs), 3.78-3.94(5H, m), 4.99(2H, s), 677(1H, s), 693(1H, d, J=10.5 Hz), 7.05(2H, d, J=8.2 Hz), 7.25 (overlapped with CHCl₃), 8.15(1 H, d, J=8.5 Hz). | CDCl₃ | 570 [M+H]⁺ |
| 215 | 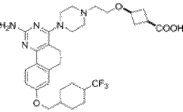 | cis-3-(2-(4-(2-amino-8-((4-(trifluoromethyl)cyclohexyl)methoxy)-5,6-dihydrobenzo[h]quinazolin-4-yl)piperazin-1-yl)ethoxy)cyclobutane-1-carboxylic acid | 1.05-1.88(6H, m), 1.94-2.27(5H, m), 2.47-2.57(2H, m), 2.60-2.94(11H, m), 3.54-3.78(6H, m), 3.82(1H, d, J=7.1 Hz), 3.88(1H, quin, J=7.1 Hz), 395(1H, d, J=7.1 Hz), 672-678(1H, m), 6.86-6.98(1H, m), 8.22(1H, d, J=8.6 Hz). | CDCl₃ | 604 [M+H]⁺ |

### (Test Example 1: Evaluation of Human H1 Receptor Affinity)

Membrane preparation from recombinant CHO-K1 cell expressing the human histamine H1 receptor (PerkinElmer Inc.), radiolabeled ligand 1.2 nM [³H]pyrilamine (PerkinElmer Inc.), and test compounds dissolved in DMSO were reacted at 25°C for 3 hours in a buffering solution containing 50 mM tris-hydrochloric acid, pH 7.4, and 5 mM magnesium chloride on a 96 well plate, followed by filtration using a GF/B filter (PerkinElmer Inc.) pretreated with 0.3% polyethylenimine. The residue was washed four times with a buffering solution of 50 mM tris-hydrochloric acid, pH 7.4. Thereafter, a scintillation cocktail (MicroScint 20: PerkinElmer Inc.) was added to the GF/B filter, and the radioactivity of each well was measured using a liquid scintillation counter for 96-well (TopCount: PerkinElmer Inc.). The non-specific binding was evaluated in the presence of 1.0 µM pyrilamine. The inhibition constant Kᵢ was calculated from the 50% inhibitory concentration (IC₅₀ value) obtained in each test and the K_{d} value (1.1 nM) unique to this assay system, according to the Cheng-Prusoff equation. The test compounds whose values of the inhibition constant Kᵢ for the histamine H1 receptor, measured by the above-described method, were less than 100 nM (<100 nM) are rated as A, and the test compounds whose values of the inhibition constant Kᵢ were 100 to 500 nM are rated as B. The results of evaluation using the compounds obtained in the respective Examples as the test substance are shown in Tables 27 to 33.

### (Test Example 2: Evaluation of Human H4 Receptor Affinity)

Membrane preparation from recombinant CHO-K1 cell expressing the human histamine H4 receptor (PerkinElmer Inc.), radiolabeled ligand 8.2 nM [³H]histamine (PerkinElmer Inc.), and compounds dissolved in DMSO were reacted at 25°C for 90 minutes in a buffering solution containing 50 mM tris-hydrochloric acid, pH 7.4, and 1.25 mM EDTA on a 96 well plate, followed by filtration using a GF/B filter (PerkinElmer Inc.) pretreated with 0.3% polyethylenimine. The residue was washed four times with a buffering solution of 50 mM tris-hydrochloric acid, pH 7.4. Thereafter, a scintillation cocktail (MicroScint 20 : PerkinElmer Inc.) was added to the GF/B filter, and the radioactivity of each well was measured using a liquid scintillation counter for 96-well (TopCount:PerkinElmer Inc.). The non-specific binding was evaluated in the presence of 1.0 µM histamine. The inhibition constant Kᵢ was calculated from the 50% inhibitory concentration (IC₅₀ value) obtained in each test and the K_{d} value (5.7 nM) unique to this assay system, according to the Cheng-Prusoff equation. The test compounds whose values of the inhibition constant Kᵢ for the histamine H4 receptor, measured by the above-described method, were less than 100 nM (<100 nM) are rated as A, and the test compounds whose values of the inhibition constant Kᵢ were 100 to 500 nM are rated as B. The results of evaluation using the compounds obtained in the respective Examples as the test substances are shown in Tables 27 to 33.

**[Table 27]**

| Ex. No. | Evaluation of H1 Receptor Affinity | Evaluation of H4 Receptor Affinity |
|---|---|---|
| 1 | A | A |
| 2 | A | A |
| 3 | A | A |
| 4 | A | A |
| 5 | A | A |
| 6 | A | A |
| 7 | A | B |
| 8 | A | A |
| 9 | A | A |
| 10 | A | A |
| 101 | B | A |
| 102 | B | A |
| 103 | B | A |
| 104 | B | A |
| 105 | A | B |
| 106 | B | B |
| 107 | A | B |
| 108 | A | A |
| 109 | A | B |
| 110 | A | A |
| 111 | A | A |
| 112 | A | B |
| 113 | A | A |
| 114 | A | B |
| 115 | A | B |
| 116 | A | B |
| 117 | B | A |

**[Table 28]**

| Ex. No. | Evaluation of H1 Receptor Affinity | Evaluation of H4 Receptor Affinity |
|---|---|---|
| 118 | A | A |
| 11(a) | A | A |
| 11(b) | A | A |
| 12 | A | A |
| 13 | A | A |
| 14 | A | A |
| 15(a) | A | B |
| 15(b) | A | A |
| 16(a) | A | B |
| 16(b) | A | A |
| 17(a) | A | A |
| 17(b) | A | A |
| 18(a) | A | A |
| 18(b) | A | A |
| 19(a) | B | B |
| 19(b) | B | A |
| 20(a) | A | B |
| 20(b) | A | A |
| 119 | A | B |
| 120 | A | A |
| 121 | A | B |
| 122 | A | B |
| 123 | A | B |
| 124 | A | A |
| 125 | A | B |
| 126 | A | A |

**[Table 29]**

| Ex. No. | Evaluation of H1 Receptor Affinity | Evaluation of H4 Receptor Affinity |
|---|---|---|
| 127(a) | A | A |
| 127(b) | A | B |
| 128(a) | A | B |
| 128(b) | A | A |
| 129(a) | A | B |
| 129(b) | A | B |
| 130 | A | B |
| 131(a) | A | B |
| 131(b) | A | B |
| 132 | A | B |
| 133 | A | A |
| 134 | A | B |
| 135(a) | A | A |
| 135(b) | A | A |
| 136 | A | B |
| 137 | A | A |
| 138 | A | B |
| 139 | A | B |
| 140 | A | B |
| 141 | A | B |
| 142 | A | A |
| 143 | A | A |

**[Table 30]**

| Ex. No. | Evaluation of H1 Receptor Affinity | Evaluation of H4 Receptor Affinity |
|---|---|---|
| 21 | A | A |
| 22 | A | A |
| 144 | A | A |
| 145 | A | A |
| 23(b) | A | A |
| 24 | A | B |
| 25(a) | A | B |
| 25(b) | A | A |
| 26(a) | A | B |
| 26(b) | A | B |
| 27 | A | A |
| 28 | A | A |
| 29 | A | A |
| 30 | A | A |
| 31 | A | B |
| 32 | A | A |
| 35 | A | A |
| 146 | A | B |
| 147 | A | A |
| 148 | A | B |
| 36 | A | A |
| 37 | A | A |
| 149 | A | B |
| 150 | A | B |
| 151 | A | A |
| 152 | A | A |
| 153(a) | A | A |
| 153(b) | A | A |
| 154 | A | B |
| 155 | A | A |
| 156 | A | A |
| 157 | A | A |
| 158 | A | B |
| 159(a) | A | B |
| 159(b) | A | A |
| 160(a) | A | B |
| 160(b) | A | A |

**[Table 31]**

| Ex. No. | Evaluation of H1 Receptor Affinity | Evaluation of H4 Receptor Affinity |
|---|---|---|
| 161 | A | A |
| 162 | B | B |
| 163 | A | A |
| 164 | A | A |
| 165 | A | A |
| 166(a) | A | B |
| 166(b) | A | B |
| 167 | A | B |
| 168 | A | B |
| 169 | A | B |
| 170 | A | A |
| 171 | A | B |
| 172 | A | A |
| 173 | A | A |
| 174 | A | A |
| 175 | A | A |
| 176 | A | B |
| 177 | A | B |
| 178 | A | A |
| 179 | A | A |
| 180 | A | A |
| 181 | A | B |
| 182 | A | B |
| 183 | A | A |
| 184 | A | A |
| 185 | A | A |
| 186 | A | A |

**[Table 32]**

| Ex. No. | Evaluation of H1 Receptor Affinity | Evaluation of H4 Receptor Affinity |
|---|---|---|
| 187 | A | B |
| 188 | A | A |
| 33 | A | A |
| 34 | A | A |
| 189 | A | A |
| 38(b) | A | A |
| 39 | A | A |
| 190(b) | A | A |
| 40 | A | A |
| 41 | B | B |
| 42 | A | A |
| 43 | B | B |
| 44 | B | B |
| 45 | A | B |
| 46 | A | B |
| 47 | B | B |
| 48 | B | B |
| 49 | B | B |
| 50 | A | A |
| 51 | A | A |
| 52 | A | A |
| 53 | A | B |
| 54 | A | A |
| 55 | B | B |
| 56 | B | B |
| 191 | A | A |
| 192 | A | A |
| 193 | A | A |
| 194 | B | B |
| 195 | B | A |

**[Table 33]**

| Ex. No. | Evaluation of H1 Receptor Affinity | Evaluation of H4 Receptor Affinity |
|---|---|---|
| 196 | A | A |
| 197 | A | A |
| 198 | A | A |
| 199 | B | B |
| 200 | A | A |
| 201 | A | A |
| 202 | B | A |
| 203 | A | A |
| 204 | A | A |
| 205 | A | A |
| 206 | A | A |
| 207 | A | A |
| 208 | A | A |
| 209 | A | A |
| 210 | A | A |
| 211 | A | A |
| 212 | A | A |
| 213 | A | B |
| 214 | A | A |
| 215 | A | B |

### (Test Example 3: Evaluation of Human H1 Receptor Function)

The antagonistic effect was evaluated by the method described below. GeneBLAzer(R) H1-NFAT-bla HEK 293T-cells (Invitrogen Corporation, recombinant cells expressing the human histamine H1 receptor) were incubated at 37 °C for 30 minutes in the presence of the test compounds dissolved in DMSO in a high glucose Dulbecco's Modified Eagle Medium (GlutaMAX, containing 10% fetal bovine serum, 100 µM nonessential amino acids, 25 mM HEPES, 100 U/mL penicillin, and 100 µg/mL streptomycin), and histamine was added such that the final concentration became 30 nM, followed by further incubation for 4.5 hours. Then, a mixture of LiveBLAzer™ FRET-B/G Loading Kit (Invitrogen Corporation) and Solution D (Invitrogen Corporation) was added, followed by leaving to stand at room temperature for 2 hours under shaded conditions. Thereafter, the fluorescence intensity was measured under two conditions of 400 nm/460 nm and 400 nm/535 nm (excitation light/fluorescence) by using a multilabel counter (EnVision: PerkinElmer Inc.). The 50% inhibitory concentration (IC₅₀ value) was calculated in accordance with (measured value -blank measured value at 400 nm/460 nm) / (measured value -blank measured value at 400 nm/535 nm) of each test sample. Further, to check if the compound exhibits an agonistic effect, a similar operation to the aforementioned was carried out but without adding histamine. As a result of the measurement by the above-described method using the compounds obtained in the respective Examples as test substances, none of the compounds shown in following Table 34 exhibited an agonistic effect. In addition, regarding the 50% inhibitory concentration (IC₅₀ value) of each test compound, the test compounds whose 50% inhibitory concentration were less than 200 nM (<200 nM) are rated as A, and the test compounds whose 50% inhibitory concentration were 200 to 2000 nM are rated as B. Results of the evaluation using the compounds obtained in the respective Examples as the test substances are shown in Table 34.

### (Test Example 4: Evaluation of H4 Receptor Function)

The antagonistic effect was evaluated by the method described below. Membrane preparation from recombinant CHO-K1 cell expressing the human histamine H4 receptor (PerkinElmer Inc.), 10 µM GDP, 0.1 µM histamine, and the compounds dissolved in DMSO were reacted at 30°C for 20 minutes in a buffering solution containing 20 mM HEPES, pH 7.4, 100 mM NaCl, 10 mM MgCl₂, 1 mM DTT, and 1 mM EDTA on an assay plate. Thereafter, SPA beads (Amersham plc) were added, followed by incubation for 60 minutes, and further 0.3 nM [³⁵S]GTPγS (PerkinElmer Inc.) was added, followed by incubation for 30 minutes. Then, the radioactivity of each well was measured using a scintillation counter (Microbeta 1450: PerkinElmer Inc.). The 50% inhibitory concentration (IC₅₀ value) of the [³⁵S]GTPγS binding affinity with addition of histamine was calculated for each test sample. In addition, to check if the compound exhibits an agonistic effect, a similar operation to the aforementioned was carried out but without adding of histamine. As a result of the measurement by the above-described method using the compounds obtained in the respective Examples as test substances, none of the tested compounds shown in following Table 34 exhibited an agonistic effect. In addition, regarding the 50% inhibitory concentration (IC₅₀ value) of each test compound, the test compounds whose 50% inhibitory concentration (IC₅₀ value) were less than 200 nM (<200 nM) are rated as A, and the test compounds whose 50% inhibitory concentration (IC₅₀ value) were 200 to 2000 nM are rated as B. Results of the evaluation using the compounds obtained in the respective Examples as the test substances are shown in Table 34.

**[Table 34]**

| Ex. No. | Evaluation of H1 Receptor Function | Evaluation of H4 Receptor Function |
|---|---|---|
| 1 | A | A |
| 2 | A | A |
| 3 | A | A |
| 102 | B | A |
| 111 | A | A |
| 11(b) | A | B |
| 12 | A | B |
| 13 | A | B |
| 14 | A | B |
| 15(b) | A | B |
| 16(b) | A | B |
| 17(b) | A | B |
| 18(b) | A | B |
| 19(b) | B | A |
| 20(b) | A | B |
| 122 | A | B |
| 127(b) | A | B |
| 129(b) | A | B |
| 143 | A | A |
| 21 | A | B |
| 22 | A | B |
| 23(b) | A | A |
| 29 | A | A |
| 32 | A | A |
| 36 | A | A |
| 152 | A | B |
| 178 | A | A |
| 184 | A | A |
| 37 | A | A |
| 39 | A | A |
| 42 | A | B |
| 54 | A | B |
| 196 | A | A |
| 201 | A | A |

### [Industrial Applicability]

As described above, the present invention can provide a compound with excellent dual modulatory function against the histamine H1 receptor and the histamine H4 receptor, which are useful in treating and/or preventing various allergies and inflammatory diseases involving the histamine H1 and/or H4 receptor, for example, and a pharmacologically acceptable salt thereof as well as pharmaceutical compositions containing the same.

## Claims

1. A compound represented by the following general formula (I) or a pharmacologically acceptable salt thereof: 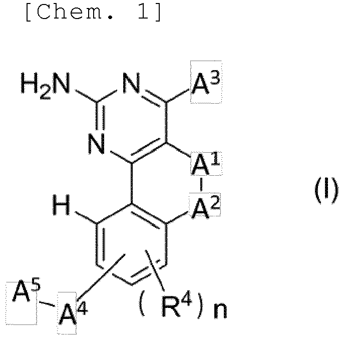 [in the formula (I),
A¹ represents a single bond, a methylene group, an ethylene group, or a sulfur atom,
A² represents a methylene group or an oxygen atom,
A³ represents an azetidinyl group, a pyrrolidinyl group, a piperidinyl group, a piperazinyl group, or a homopiperazinyl group which is optionally substituted with at least one substituent selected from the group consisting of -N(R¹)R², -N(R¹)R³, and -R³,
R¹ and R² may be the same or different and each represents a hydrogen atom, a C₁₋₆ alkyl group, or an optionally substituted C₆₋₁₀ monocyclic or polycyclic arylalkyl group,
R³ represents a group represented by the following formula: -D¹-D²-D³ or a group represented by the following formula: -D¹-ON=D⁴ (except where -N(R¹)R³ becomes -N(R¹)R²),
D¹ represents an optionally substituted C₁₋₆ alkylene group,
D² represents a single bond, -O-, -OC(O)-, -S-, -S(O)₂-, -N(R¹)S(O)₂-, -C(O)-, or -C(O)N(R¹)-,
D³ represents a hydrogen atom, an optionally substituted C₁₋₆ alkyl group, an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₁₋₆ alkyloxyalkyl group, a 3- to 10-membered monocyclic or bicyclic heterocyclyloxyalkyl group, an optionally substituted C₁₋₆ alkylthioalkyl group, or an optionally substituted C₁₋₆ alkylsulfonylalkyl group,
D⁴ represents an optionally substituted C₃₋₁₀ cycloalkylene group or an optionally substituted 3-to 10-membered monocyclic or bicyclic divalent heterocyclyl group,
A⁴ represents -O-, -S-, -S(O)-, -S(O)₂-, or -N(R¹)-,
A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, an optionally substituted C₆₋₁₀ monocyclic or polycyclic arylalkylsulfonyl group, an optionally substituted C₆₋₁₀ monocyclic or polycyclic aryloxyalkylsulfonyl group, or an optionally substituted C₆₋₁₀ monocyclic or polycyclic arylaminocarbonyl group,
R⁴ represents a halogen atom, a hydroxyl group, a C₁₋₆ alkyloxy group, or a cyano group, and
n is an integer of 0 or 1,
except where
A¹ is a methylene group, an ethylene group, or a sulfur atom,
A² is a methylene group or an oxygen atom,
A³ is an azetidinyl group, a pyrrolidinyl group, or a piperidinyl group which is substituted with at least one substituent selected from the group consisting of -N(R¹)R², -N(R¹)R³, and -R³, a piperazinyl group in which one carbon atom on the ring is substituted with a hydroxyalkyl group or a C₁₋₆ alkyloxyalkyl group, or a homopiperazinyl group in which a nitrogen atom on the ring is optionally substituted with a C₁₋₆ alkyl group, a hydroxyalkyl group, a C₁₋₆ alkyloxyalkyl group, or a C₃₋₁₀ cycloalkyloxyalkyl group,
A⁴ is -N(R¹)-, and
A⁵ is a C₃₋₁₀ cycloalkylsulfonyl group, a C₆-₁₀ monocyclic or polycyclic arylsulfonyl group, or a 3- to 10-membered monocyclic or bicyclic heterocyclylsulfonyl group].

2. The compound or a pharmacologically acceptable salt thereof according to claim 1, wherein
in the formula (I),
A³ represents an azetidinyl group, a pyrrolidinyl group, a piperidinyl group, a piperazinyl group, or a homopiperazinyl group which is optionally substituted with at least one substituent selected from the group consisting of -N(R¹)R³ and -R³,
R³ represents a group represented by the following formula: -D¹-D²-D³ or a group represented by the following formula: -D¹-ON=D⁴ (except where -N(R¹)R³ becomes -N(R¹)R²),
D¹ represents a C₁₋₆ alkylene group,
D² represents a single bond, -O-, -OC(O)-, -S(O)₂-, -N(R¹)S(O)₂-, -C(O)-, or -C(O)N(R¹)-,
A⁴ represents -O- or -N(R¹)-,
A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
n represents 0.

3. The compound or a pharmacologically acceptable salt thereof according to claim 1, wherein
in the formula (I),
both A¹ and A² represent methylene groups,
A³ represents an azetidinyl group, a pyrrolidinyl group, a piperidinyl group, a piperazinyl group, or a homopiperazinyl group which is optionally substituted with at least one substituent selected from the group consisting of -N(R¹)R³ and -R³,
R³ represents a group represented by the following formula: -D¹-D²-D³ or a group represented by the following formula: -D¹-ON=D⁴ (except where -N(R¹)R³ becomes -N(R¹)R²),
D¹ represents a C₁₋₆ alkylene group,
D² represents a single bond, -O-, -OC(O)-, -S(O)₂-, -N(R¹)S(O)₂-, -C(O)-, or -C(O)N(R¹)-,
A⁴ represents -O- or -N(R¹)-,
A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, an optionally substituted C₆₋₁₀ monocyclic or polycyclic arylaminocarbonyl group, and
n represents 0.

4. The compound or a pharmacologically acceptable salt thereof according to claim 1, wherein
in the formula (I),
both A¹ and A² represent methylene groups,
A³ represents an azetidinyl group, a pyrrolidinyl group, a piperidinyl group, a piperazinyl group, or a homopiperazinyl group which is optionally substituted at least one -N(R¹)R²,
R¹ and R² may be the same or different and each represents a hydrogen atom or a C₁₋₆ alkyl group,
A⁴ represents -O- or -N(R¹)-,
A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
n represents 0.

5. The compound or a pharmacologically acceptable salt thereof according to claim 1, wherein
in the formula (I),
both A¹ and A² represent methylene groups,
A³ represents a piperazinyl group optionally substituted with at least one -R³,
R³ represents a group represented by the following formula: -D¹-D²-D³ or a group represented by the following formula: -D¹-ON=D⁴,
D¹ represents a C₁₋₆ alkylene group,
D² represents a single bond, -O-, -OC(O)-, -S(O)₂-, -N(R¹)S(O)₂-, -C(O)-, or -C(O)N(R¹)-,
A⁴ represents -O- or -N(R¹)-,
A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
n represents 0.

6. The compound or a pharmacologically acceptable salt thereof according to claim 1, wherein
in the formula (I),
A¹ represents a single bond, a methylene group, or an ethylene group,
A³ represents a pyrrolidinyl group, a piperidinyl group, piperazinyl group, or a homopiperazinyl group which is optionally substituted with at least one substituent selected from the group consisting of -N(R¹)R² and -R³,
R¹ and R² may be the same or different and each represents a hydrogen atom or a C₁₋₆ alkyl group,
R³ represents a group represented by the following formula: -D¹-D²-D³,
D¹ represents a C₁₋₆ alkylene group,
D² represents a single bond or -O-,
D³ represents a hydrogen atom, an optionally substituted C₁₋₆ alkyl group, an optionally substituted C₃₋₁₀ cycloalkyl group, an optionally substituted C₆₋₁₀ monocyclic or polycyclic aryl group, an optionally substituted 3- to 10-membered monocyclic or bicyclic heterocyclyl group, an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, an optionally substituted C₁₋₆ alkyloxyalkyl group, a 3- to 10-membered monocyclic or bicyclic heterocyclyloxyalkyl group, or an optionally substituted C₁₋₆ alkylthioalkyl group,
A⁴ represents -O- or -N(R¹)-,
A⁵ represents an optionally substituted C₃₋₁₀ cycloalkylalkyl group, an optionally substituted C₆₋₁₀ monocyclic or polycyclic arylalkyl group, an optionally substituted 3- to 10-membered monocyclic or bicyclic heterocyclylalkyl group, a C₃₋₁₀ cycloalkylsulfonyl group, an optionally substituted C₆₋₁₀ monocyclic or polycyclic arylsulfonyl group, an optionally substituted 3- to 10-membered monocyclic or bicyclic heterocyclylsulfonyl group, a C₆₋₁₀ monocyclic or polycyclic arylalkylsulfonyl group, a C₆₋₁₀ monocyclic or polycyclic aryloxyalkylsulfonyl group, or an optionally substituted C₆₋₁₀ monocyclic or polycyclic arylaminocarbonyl group, and
n represents 0.

7. The compound or a pharmacologically acceptable salt thereof according to claim 1, wherein
the compound represented by the formula (I) is N-(2-amino-4-(4-methylpiperazin-1-yl)-5,6-dihydrobenzo [h]quinazolin-8-yl)benzenesulfonamide, 9-(benzyloxy)-4-(4-methylpiperazin-1-yl)-6,7-dihydro-5 H-benzo[6,7]cyclohepta[1,2-d]pyrimidin-2-amine, 8-(benzyloxy)-4-(4-methylpiperazin-1-yl)-6H-isothiochr omeno[4,3-d]pyrimidin-2-amine, 2-(4-(2-amino-8-(benzyloxy)-5,6-dihydrobenzo[h]quinazo lin-4-yl)piperazin-1-yl)ethan-1-ol, 2-(4-(2-amino-8-((4-chlorobenzyl)oxy)-5,6-dihydrobenzo [h]quinazolin-4-yl)piperazin-1-yl)ethyl 3-(2-methoxy-2-oxoethyl)azetidine-1-carboxylate, 1-(2-(4-(2-amino-8-((4-chlorobenzyl)oxy)-5,6-dihydrobe nzo[h]quinazolin-4-yl)piperazin-1-yl)ethyl)3-methyl azetidine-1,3-dicarboxylate,
methyl 1-(4-(4-(2-amino-8-((4-chlorobenzyl)oxy)-5,6-dihydrobe nzo[h]quinazolin-4-yl)piperazin-1-yl)butanoyl)azetidin e-3-carboxylate, N-(2-amino-4-(4-methylpiperazin-1-yl)-5,6-dihydrobenzo [h]quinazolin-8-yl)-4-chlorobenzenesulfonamide, N-(2-amino-4-(4-methylpiperazin-1-yl)-5,6-dihydrobenzo [h]quinazolin-8-yl)-3-chlorobenzenesulfonamide, N-(2-amino-4-(4-methylpiperazin-1-yl)-5,6-dihydrobenzo [h]quinazolin-8-yl)-5-chlorothiophene-2-sulfonamide, N-(2-amino-4-(4-methylpiperazin-1-yl)-5,6-dihydrobenzo [h]quinazolin-8-yl)-4-chloro-3-fluorobenzenesulfonamid e, N-(2-amino-4-(4-methylpiperazin-1-yl)-5,6-dihydrobenzo [h]quinazolin-8-yl)-4-chloro-2-fluorobenzenesulfonamid e, N-(2-amino-4-(4-methylpiperazin-1-yl)-5,6-dihydrobenzo [h]quinazolin-8-yl)-4-cyanobenzenesulfonamide, N-(2-amino-4-(4-methylpiperazin-1-yl)-5,6-dihydrobenzo [h]quinazolin-8-yl)-2-phenoxyethan-1-sulfonamide, N-(2-amino-4-(4-methylpiperazin-1-yl)-5,6-dihydrobenzo [h]quinazolin-8-yl)-1-phenylmethanesulfonamide, N-(2-amino-4-(4-methylpiperazin-1-yl)-5,6-dihydrobenzo [h]quinazolin-8-yl)-6-chloropyridine-3-sulfonamide, N-(2-amino-4-(4-methylpiperazin-1-yl)-5,6-dihydrobenzo [h]quinazolin-8-yl)-4-fluorobenzenesulfonamide, N-(2-amino-4-(4-methylpiperazin-1-yl)-5,6-dihydrobenzo [h]quinazolin-8-yl)-4-(trifluoromethyl)benzenesulfonam ide, N-(2-amino-4-(4-methylpiperazin-1-yl)-5,6-dihydrobenzo [h]quinazolin-8-yl)-3-phenoxypropane-1-sulfonamide, N-(2-amino-4-(4-methylpiperazin-1-yl)-5,6-dihydrobenzo [h]quinazolin-8-yl)-2-phenylethane-1-sulfonamide, N-(2-amino-4-(4-methylpiperazin-1-yl)-5,6-dihydrobenzo [h]quinazolin-8-yl)-3-phenylpropane-1-sulfonamide, N-(2-amino-4-(4-methylpiperazin-1-yl)-5,6-dihydrobenzo [h]quinazolin-8-yl)-4-methoxybenzenesulfonamide, N-(2-amino-4-(4-methylpiperazin-1-yl)-5,6-dihydrobenzo [h]quinazolin-8-yl)-4-(trifluoromethoxy)benzenesulfona mide,
methyl 4-(N-(2-amino-4-(4-methylpiperazin-1-yl)-5,6-dihydrobe nzo[h]quinazolin-8-yl)sulfamoyl)benzoate, N-(2-amino-4-(4-methylpiperazin-1-yl)-5,6-dihydrobenzo [h]quinazolin-8-yl)cyclohexanesulfonamide, 1-(2-amino-4-(4-methylpiperazin-1-yl)-5,6-dihydrobenzo [h]quinazolin-8-yl)-3-(4-chlorophenyl)urea, N-(2-amino-4-(4-methylpiperazin-1-yl)-5,6-dihydrobenzo [h]quinazolin-8-yl)-N-(4-chlorobenzyl)-4-nitrobenzenes ulfonamide, N⁸-(4-chlorobenzyl)-4-(4-methylpiperazin-1-yl)-5,6-dih ydrobenzo[h]quinazolin-2,8-diamine, 4-(((2-amino-4-(4-methylpiperazin-1-yl)-5,6-dihydroben zo[h]quinazolin-8-yl)oxy)methyl)-N-methylbenzenesulfon amide, 2-(4-(2-(4-(2-amino-8-((4-chlorobenzyl)oxy)-5,6-dihydr obenzo[h]quinazolin-4-yl)piperazin-1-yl)ethoxy)phenyl) -2-methylpropanoic acid, 3-(3-((2-(4-(2-amino-8-((4-chlorobenzyl)oxy)-5,6-dihyd robenzo[h]quinazolin-4-yl)piperazin-1-yl)ethoxy)methyl )oxetan-3-yl)propanoic acid, 3-((2-(2-(4-(2-amino-8-((4-chlorobenzyl)oxy)-5,6-dihyd robenzo[h]quinazolin-4-yl)piperazin-1-yl)ethoxy)ethoxy )methyl)oxetane-3-carboxylic acid, 3-(2-(2-(4-(2-amino-8-((4-chlorobenzyl)oxy)-5,6-dihydr obenzo[h]quinazolin-4-yl)piperazin-1-yl)ethoxy)ethoxy) oxetane-3-carboxylic acid, 5-((2-(4-(2-amino-8-((4-chlorobenzyl)oxy)-5,6-dihydrob enzo[h]quinazolin-4-yl)piperazin-1-yl)ethoxy)methyl)te trahydrofuran-2-carboxylic acid, 2-(3-(2-(4-(2-amino-8-((4-chlorobenzyl)oxy)-5,6-dihydr obenzo[h]quinazolin-4-yl)piperazin-1-yl)ethoxy)cyclobu tyl)acetic acid, 2-(3-(2-(4-(2-amino-8-((4-chlorobenzyl)oxy)-5,6-dihydr obenzo[h]quinazolin-4-yl)piperazin-1-yl)ethoxy)cyclobu tyl)-2-methylpropanoic acid, 3-(2-(4-(2-amino-8-((4-(trifluoromethoxy)benzyl)oxy)-5 ,6-dihydrobenzo[h]quinazolin-4-yl)piperazin-1-yl)ethox y)bicyclo[3.1.0]hexane-6-carboxylic acid, 2-(3-(2-(4-(2-amino-8-((4-(trifluoromethoxy)benzyl)oxy )-5,6-dihydrobenzo[h]quinazolin-4-yl)piperazin-1-yl)et hoxy)cyclobutyl)-2-methylpropanoic acid, 2-(3-(2-(4-(2-amino-8-((4-(trifluoromethoxy)benzyl)oxy )-5,6-dihydrobenzo[h]quinazolin-4-yl)piperazin-1-yl)et hoxy)cyclobutyl)acetic acid, 4-(2-(4-(2-amino-8-((4-(trifluoromethoxy)benzyl)oxy)-5 ,6-dihydrobenzo[h]quinazolin-4-yl)piperazin-1-yl)ethox y)tetrahydrofuran-2-carboxylic acid, 4-((2-(4-(2-amino-8-((4-(trifluoromethoxy)benzyl)oxy)-5,6-dihydrobenzo[h]quinazolin-4-yl)piperazin-1-yl)etho xy)methyl)oxetane-2-carboxylic acid, N-(2-amino-4-(piperazin-1-yl)-5,6-dihydrobenzo[h]quina zolin-8-yl)-4-(trifluoromethoxy)benzenesulfonamide,
ethyl 2-((2-(4-(2-amino-8-((4-chlorobenzyl)oxy)-5,6-dihydrob enzo[h]quinazolin-4-yl)piperazin-1-yl)ethoxy)methyl)th iazole-4-carboxylate,
methyl 4-((2-(4-(2-amino-8-((4-chlorobenzyl)oxy)-5,6-dihydrob enzo[h]quinazolin-4-yl)piperazin-1-yl)ethoxy)methyl)-3 -fluorobenzoate, 4-((2-(4-(2-amino-8-((4-chlorobenzyl)oxy)-5,6-dihydrob enzo[h]quinazolin-4-yl)piperazin-1-yl)ethoxy)methyl)-3 -fluorobenzene carboxylic acid, 2-((2-(4-(2-amino-8-((4-chlorobenzyl)oxy)-5,6-dihydrob enzo[h]quinazolin-4-yl)piperazin-1-yl)ethoxy)methyl)th iazole-4-carboxylic acid, 4-((2-(4-(2-amino-8-((4-chlorobenzyl)oxy)-5,6-dihydrob enzo[h]quinazolin-4-yl)piperazin-1-yl)ethoxy)methyl)pe ntacyclo[4.2.0.0^{2,5}.0^{3,8}.0^{4,7}]octane-1-carboxylic acid, 6-((2-(4-(2-amino-8-((4-chlorobenzyl)oxy)-5,6-dihydrob enzo[h]quinazolin-4-yl)piperazin-1-yl)ethoxy)methyl)sp iro[3.3]heptane-2-carboxylic acid, 4-((2-(4-(2-amino-8-((4-chlorobenzyl)oxy)-5,6-dihydrob enzo[h]quinazolin-4-yl)piperazin-1-yl)ethoxy)methyl)-1 -methyl-1H-pyrrole-2-carboxylic acid, 4-(2-(4-(2-amino-8-((4-chlorobenzyl)oxy)-5,6-dihydrobe nzo[h]quinazolin-4-yl)piperazin-1-yl)ethoxy)bicyclo[2. 2.2]octane-1-carboxylic acid, 3-(2-(4-(2-amino-8-((4-chlorobenzyl)oxy)-5,6-dihydrobe nzo[h]quinazolin-4-yl)piperazin-1-yl)ethoxy)-4,7,7-tri methylbicyclo[2.2.1]heptane-1-carboxylic acid, 4-(4-(2-((2-(tetrazol-5-yl)propan-2-yl)oxy)ethyl)piper azin-1-yl)-8-((4-chlorobenzyl)oxy)-5,6-dihydrobenzo[h] quinazolin-2-amine, 3-((2-(4-(2-amino-8-((4-chlorobenzyl)oxy)-5,6-dihydrob enzo[h]quinazolin-4-yl)piperazin-1-yl)ethoxy)imino)cyc lobutane-1-carboxylic acid, 3-((2-(4-(2-amino-8-((4-(trifluoromethoxy)benzyl)oxy)-5,6-dihydrobenzo[h]quinazolin-4-yl)piperazin-1-yl)etho xy)methyl)cyclohexane-1-carboxylic acid, 3-((2-(4-(2-amino-8-((4-(trifluoromethoxy)benzyl)oxy)-5,6-dihydrobenzo[h]quinazolin-4-yl)piperazin-1-yl)etho xy)methyl)-N-cyanoazetidine-1-carboximideamide, 4-(4-(2-(azetidin-3-ylmethoxy)ethyl)piperazin-1-yl)-8-((4-(trifluoromethoxy)benzyl)oxy)-5,6-dihydrobenzo[h]q uinazolin-2-amine, 4-(piperazin-1-yl)-8-((4-(trifluoromethoxy)benzyl)oxy) -5,6-dihydrobenzo[h]quinazolin-2-amine, 3-((2-(4-(2-amino-8-((4-(trifluoromethoxy)benzyl)oxy)-5,6-dihydrobenzo[h]quinazolin-4-yl)piperazin-1-yl)etho xy)methyl)azetidine-1-carboxamide, 3-(3-((2-(4-(2-amino-8-((4-(trifluoromethoxy)benzyl)ox y)-5,6-dihydrobenzo[h]quinazolin-4-yl)piperazin-1-yl)e thoxy)methyl)azetidin-1-yl)-4-hydroxy-1,2,5-thiadiazol 1,1-dioxide, 8-(benzyloxy)-4-(piperazin-1-yl)-5,6-dihydrobenzo[h]qu inazolin-2-amine, 2-(4-(2-amino-8-(benzyloxy)-5,6-dihydrobenzo[h]quinazo lin-4-yl)piperazin-1-yl)ethan-1-ol, 2-(2-(2-(4-(2-amino-8-(benzyloxy)-5,6-dihydrobenzo[h]q uinazolin-4-yl)piperazin-1-yl)ethoxy)ethoxy)acetic acid, 4-(4-methylpiperazin-1-yl)-8-(thiophen-3-ylmethoxy)-5, 6-dihydrobenzo[h]quinazolin-2-amine, 8-((4-chlorobenzyl)oxy)-4-(4-methylpiperazin-1-yl)-5,6 -dihydrobenzo[h]quinazolin-2-amine, 4-(4-methylpiperazin-1-yl)-8-((tetrahydro-2H-pyran-4-y l)methoxy)-5,6-dihydrobenzo[h]quinazolin-2-amine, 8-((4-chlorobenzyl)oxy)-4-(piperazin-1-yl)-5,6-dihydro benzo[h]quinazolin-2-amine, 8-(benzyloxy)-4-(3-(dimethylamino)pyrrolidin-1-yl)-5,6 -dihydrobenzo[h]quinazolin-2-amine, 8-((4-fluorobenzyl)oxy)-4-(4-methylpiperazin-1-yl)-5,6 -dihydrobenzo[h]quinazolin-2-amine, 8-(benzyloxy)-4-(4-(dimethylamino)piperidin-1-yl)-5,6-dihydrobenzo[h]quinazolin-2-amine, 8-(benzyloxy)-4-(4-methylpiperazin-1-yl)benzofuro[3,2-d]pyrimidin-2-amine, 2-(2-(2-(4-(2-amino-8-((4-chlorobenzyl)oxy)-5,6-dihydr obenzo[h]quinazolin-4-yl)piperazin-1-yl)ethoxy)ethoxy) acetic acid, N-(4-(4-(2-amino-8-(benzyloxy)-5,6-dihydrobenzo[h]quin azolin-4-yl)piperazin-1-yl)butyl)ethanesulfonamide, (4-(2-amino-8-(benzyloxy)-5,6-dihydrobenzo[h]quinazoli n-4-yl)-1-methylpiperazin-2-yl)methanol, 8-(benzyloxy)-4-(4-(3-(ethylsulfonyl)propyl)piperazin-1-yl)-5,6-dihydrobenzo[h]quinazolin-2-amine, 2-(2-(2-(4-(2-amino-8-(benzyloxy)-5,6-dihydrobenzo[h]q uinazolin-4-yl)piperazin-1-yl)ethoxy)ethoxy)-N-methyla cetamide, 2-((5-(4-(2-amino-8-(benzyloxy)-5,6-dihydrobenzo[h]qui nazolin-4-yl)piperazin-1-yl)pentyl)oxy)acetic acid, 2-(2-(2-(4-(2-amino-8-(benzyloxy)-5,6-dihydrobenzo[h]q uinazolin-4-yl)piperazin-1-yl)ethoxy)ethoxy)acetamide, 2-(2-(2-(4-(2-amino-8-((4-chlorobenzyl)oxy)-5,6-dihydr obenzo[h]quinazolin-4-yl)piperazin-1-yl)ethoxy)ethoxy) propanoic acid, N-(2-(2-(2-(4-(2-amino-8-(benzyloxy)-5,6-dihydrobenzo[ h]quinazolin-4-yl)piperazin-1-yl)ethoxy)ethoxy)ethyl)m ethanesulfonamide, 8-(benzyloxy)-4-(4-methylpiperazin-1-yl)-5H-chromeno[4 ,3-d]pyrimidin-2-amine, 2-(2-(2-(4-(2-amino-8-((4-chlorobenzyl)oxy)-5,6-dihydr obenzo[h]quinazolin-4-yl)piperazin-1-yl)ethoxy)ethoxy) -2-methylpropanoic acid, 2-(4-(4-(2-amino-8-((4-chlorobenzyl)oxy)-5,6-dihydrobe nzo[h]quinazolin-4-yl)piperazin-1-yl)butoxy)propanoic acid, 2-(2-(2-(4-(2-amino-8-((4-fluorobenzyl)oxy)-5,6-dihydr obenzo[h]quinazolin-4-yl)piperazin-1-yl)ethoxy)ethoxy) acetic acid, 4-(2-(4-(2-amino-8-((4-chlorobenzyl)oxy)-5,6-dihydrobe nzo[h]quinazolin-4-yl)piperazin-1-yl)acetamide)butanoi c acid, 2-(2-(2-(4-(2-amino-8-((4-chlorobenzyl)oxy)-5,6-dihydr obenzo[h]quinazolin-4-yl)piperazin-1-yl)ethoxy)ethoxy) butanoic acid, 2-(2-(2-(4-(2-amino-8-((4-chlorobenzyl)oxy)-5,6-dihydr obenzo[h]quinazolin-4-yl)piperazin-1-yl)ethoxy)ethoxy) -N-methylacetamide, N-(2-(2-(2-(4-(2-amino-8-((4-chlorobenzyl)oxy)-5,6-dih ydrobenzo[h]quinazolin-4-yl)piperazin-1-yl)ethoxy)etho xy)ethyl)methanesulfonamide,
methyl 2-(2-(2-(4-(2-amino-8-((4-chlorobenzyl)oxy)-5,6-dihydr obenzo[h]quinazolin-4-yl)piperazin-1-yl)ethoxy)ethoxy) acetate, 2-(2-(2-(4-(2-amino-8-((4-chlorobenzyl)oxy)-5,6-dihydr obenzo[h]quinazolin-4-yl)piperazin-1-yl)ethoxy)ethoxy) ethan-1-ol, 2-(2-(2-(4-(2-amino-8-((4-chlorobenzyl)oxy)-5,6-dihydr obenzo[h]quinazolin-4-yl)piperazin-1-yl)ethoxy)ethoxy) ethyl sulfamate, 2-(2-(2-(4-(2-amino-8-((4-chlorobenzyl)oxy)-5,6-dihydr obenzo[h]quinazolin-4-yl)piperazin-1-yl)ethoxy)ethoxy) -N-(methylsulfonyl)acetamide, 2-(2-(2-(2-(4-(2-amino-8-((4-chlorobenzyl)oxy)-5,6-dih ydrobenzo[h]quinazolin-4-yl)piperazin-1-yl)ethoxy)etho xy)ethyl)isoindoline-1,3-dione, 8-((4-bromobenzyl)oxy)-4-(4-methylpiperazin-1-yl)-5,6-dihydrobenzo[h]quinazolin-2-amine, 4-(4-(2-(2-(2-aminoethoxy)ethoxy)ethyl)piperazin-1-yl) -8-((4-chlorobenzyl)oxy)-5,6-dihydrobenzo[h]quinazolin -2-amine,
methyl 3-((2-(4-(2-amino-8-((4-chlorobenzyl)oxy)-5,6-dihydrob enzo[h]quinazolin-4-yl)piperazin-1-yl)ethoxy)methyl)be nzoate,
methyl 4-((2-(4-(2-amino-8-((4-chlorobenzyl)oxy)-5,6-dihydrob enzo[h]quinazolin-4-yl)piperazin-1-yl)ethoxy)methyl)be nzoate,
methyl 5-((2-(4-(2-amino-8-((4-chlorobenzyl)oxy)-5,6-dihydrob enzo[h]quinazolin-4-yl)piperazin-1-yl)ethoxy)methyl)fu ran-2-carboxylate, 5-((2-(4-(2-amino-8-((4-chlorobenzyl)oxy)-5,6-dihydrob enzo[h]quinazolin-4-yl)piperazin-1-yl)ethoxy)methyl)fu ran-2-carboxylic acid, N-(2-amino-4-(4-methylpiperazin-1-yl)-5,6-dihydrobenzo [h]quinazolin-8-yl)-4-(2,2,2-trifluoroethoxy)benzenesu lfonamide,
methyl 5-((2-(4-(2-amino-8-((4-(trifluoromethoxy)benzyl)oxy)-5,6-dihydrobenzo[h]quinazolin-4-yl)piperazin-1-yl)etho xy)methyl)furan-2-carboxylate, 3-((2-(4-(2-amino-8-((4-chlorobenzyl)oxy)-5,6-dihydrob enzo[h]quinazolin-4-yl)piperazin-1-yl)ethoxy)methyl)be nzoic acid, 4-((2-(4-(2-amino-8-((4-chlorobenzyl)oxy)-5,6-dihydrob enzo[h]quinazolin-4-yl)piperazin-1-yl)ethoxy)methyl)be nzoic acid, 5-((2-(4-(2-amino-8-((4-(trifluoromethoxy)benzyl)oxy)-5,6-dihydrobenzo[h]quinazolin-4-yl)piperazin-1-yl)etho xy)methyl)furan-2-carboxylic acid,
methyl 2-(2-(4-(2-amino-8-((4-chlorobenzyl)oxy)-5,6-dihydrobe nzo[h]quinazolin-4-yl)piperazin-1-yl)ethoxy) acetate, 8-((4-chlorobenzyl)oxy)-4-(4-(3-(ethylsulfonyl)propyl) piperazin-1-yl)-5,6-dihydrobenzo[h]quinazolin-2-amine, methyl 3-((2-(4-(2-amino-8-((4-(trifluoromethoxy)benzyl)oxy)-5,6-dihydrobenzo[h]quinazolin-4-yl)piperazin-1-yl)etho xy)methyl)benzoate,
methyl 6-((2-(4-(2-amino-8-((4-chlorobenzyl)oxy)-5,6-dihydrob enzo[h]quinazolin-4-yl)piperazin-1-yl)ethoxy)methyl)pi colinate, 3-((2-(4-(2-amino-8-((4-(trifluoromethoxy)benzyl)oxy)-5,6-dihydrobenzo[h]quinazolin-4-yl)piperazin-1-yl)etho xy)methyl)benzoic acid, 6-((2-(4-(2-amino-8-((4-chlorobenzyl)oxy)-5,6-dihydrob enzo[h]quinazolin-4-yl)piperazin-1-yl)ethoxy)methyl)pi colinic acid, N-(2-amino-4-(4-methyl-1,4-diazepan-1-yl)-5,6-dihydrob enzo[h]quinazolin-8-yl)-4-(trifluoromethoxy)benzenesul fonamide, 2-(2-(2-(4-(2-amino-8-((4-chlorobenzyl)oxy)-5,6-dihydr obenzo[h]quinazolin-4-yl)piperazin-1-yl)ethoxy)ethoxy) acetonitrile,
methyl 3-((2-(4-(2-amino-8-((4-chlorobenzyl)oxy)-5,6-dihydrob enzo[h]quinazolin-4-yl)piperazin-1-yl)ethoxy)methyl)-4 -fluorobenzoate,
methyl 5-((2-(4-(2-amino-8-((4-chlorobenzyl)oxy)-5,6-dihydrob enzo[h]quinazolin-4-yl)piperazin-1-yl)ethoxy)methyl)-2 -fluorobenzoate, 3-((2-(4-(2-amino-8-((4-chlorobenzyl)oxy)-5,6-dihydrob enzo[h]quinazolin-4-yl)piperazin-1-yl)ethoxy)methyl)-4 -fluorobenzoic acid, 5-((2-(4-(2-amino-8-((4-chlorobenzyl)oxy)-5,6-dihydrob enzo[h]quinazolin-4-yl)piperazin-1-yl)ethoxy)methyl)-2 -fluorobenzoic acid, 4-(4-(2-((tetrazol-5-yl)methoxy)ethyl)piperazin-1-yl)-8-((4-chlorobenzyl)oxy)-5,6-dihydrobenzo[h]quinazolin-2-amine, 4-(4-(2-(2-((1H-tetrazol-5-yl)methoxy)ethoxy)ethyl)pip erazin-1-yl)-8-((4-chlorobenzyl)oxy)-5,6-dihydrobenzo[ h]quinazolin-2-amine,
methyl 4-(2-(4-(2-amino-8-((4-chlorobenzyl)oxy)-5,6-dihydrobe nzo[h]quinazolin-4-yl)piperazin-1-yl)ethoxy)benzoate,
methyl 3-(3-(4-(2-amino-8-((4-chlorobenzyl)oxy)-5,6-dihydrobe nzo[h]quinazolin-4-yl)piperazin-1-yl)propoxy)benzoate, 2-(3-(2-(4-(2-amino-8-((4-chlorobenzyl)oxy)-5,6-dihydr obenzo[h]quinazolin-4-yl)piperazin-1-yl)ethoxy)phenyl) acetic acid, 4-(2-(4-(2-amino-8-((4-chlorobenzyl)oxy)-5,6-dihydrobe nzo[h]quinazolin-4-yl)piperazin-1-yl)ethoxy)benzoic acid, 3-(3-(4-(2-amino-8-((4-chlorobenzyl)oxy)-5,6-dihydrobe nzo[h]quinazolin-4-yl)piperazin-1-yl)propoxy)benzoic acid, 1-(4-(4-(2-amino-8-((4-chlorobenzyl)oxy)-5,6-dihydrobe nzo[h]quinazolin-4-yl)piperazin-1-yl)butyl)-1H-pyrazol e-4-carboxylic acid, 1-(2-(2-(4-(2-amino-8-((4-chlorobenzyl)oxy)-5,6-dihydr obenzo[h]quinazolin-4-yl)piperazin-1-yl)ethoxy)ethyl)-1H-pyrazole-4-carboxylic acid, 4-(4-(4-(2-amino-8-((4-chlorobenzyl)oxy)-5,6-dihydrobe nzo[h]quinazolin-4-yl)piperazin-1-yl)butoxy)benzoic acid, 4-(4-(3-((tetrazol-5-yl)methoxy)propyl)piperazin-1-yl) -8-((4-chlorobenzyl)oxy)-5,6-dihydrobenzo[h]quinazolin -2-amine, 4-(4-(4-((tetrazol-5-yl)methoxy)butyl)piperazin-1-yl)-8-((4-chlorobenzyl)oxy)-5,6-dihydrobenzo[h]quinazolin-2-amine, 4-(4-(2-(2-((tetrazol-5-yl)oxy)ethoxy)ethyl)piperazin-1-yl)-8-((4-chlorobenzyl)oxy)-5,6-dihydrobenzo[h]quina zolin-2-amine, 2-((2-(4-(2-amino-8-((4-(trifluoromethoxy)benzyl)oxy)-5,6-dihydrobenzo[h]quinazolin-4-yl)piperazin-1-yl)etho xy)methyl)thiazole-4-carboxylic acid, 2-(2-(2-(4-(2-amino-8-((4-chlorobenzyl)oxy)-5,6-dihydr obenzo[h]quinazolin-4-yl)piperazin-1-yl)ethoxy)phenyl) acetic acid, 2-(4-(2-(4-(2-amino-8-((4-chlorobenzyl)oxy)-5,6-dihydr obenzo[h]quinazolin-4-yl)piperazin-1-yl)ethoxy)phenyl) acetic acid, 1-(2-(2-(4-(2-amino-8-((4-chlorobenzyl)oxy)-5,6-dihydr obenzo[h]quinazolin-4-yl)piperazin-1-yl)ethoxy)ethyl)-1H-pyrazole-3-carboxylic acid, 2-(3-(2-(4-(2-amino-8-((4-chlorobenzyl)oxy)-5,6-dihydr obenzo[h]quinazolin-4-yl)piperazin-1-yl)ethyl)phenoxy) acetic acid, 2-(4-(2-(4-(2-amino-8-((4-chlorobenzyl)oxy)-5,6-dihydr obenzo[h]quinazolin-4-yl)piperazin-1-yl)ethyl)phenoxy) acetic acid, 4-((2-(4-(2-amino-8-((4-chlorobenzyl)oxy)-5,6-dihydrob enzo[h]quinazolin-4-yl)piperazin-1-yl)ethoxy)methyl)cy clohexane-1-carboxylic acid, 7-(4-methylpiperazin-1-yl)-2-(4-phenylbutyl)-2,4,5,3-( 4-(4-(2-amino-8-((4-chlorobenzyl)oxy)-5,6-dihydrobenzo [h]quinazolin-4-yl)piperazin-1-yl)butoxy)cyclobutane-1 -carboxylic acid, 3-(2-(4-(2-amino-8-((4-chlorobenzyl)oxy)-5,6-dihydrobe nzo[h]quinazolin-4-yl)piperazin-1-yl)ethoxy)cyclobutan e-1-carboxylic acid, N-(2-amino-4-(4-methylpiperazin-1-yl)-5,6-dihydrobenzo [h]quinazolin-8-yl)-4,4-difluoropiperidine-1-sulfonami de, 4-(2-(4-(2-amino-8-((4-chlorobenzyl)oxy)-5,6-dihydrobe nzo[h]quinazolin-4-yl)piperazin-1-yl)ethoxy)cyclohexan e-1-carboxylic acid, 4-((2-(4-(2-amino-8-((4-(trifluoromethoxy)benzyl)oxy)-5,6-dihydrobenzo[h]quinazolin-4-yl)piperazin-1-yl)etho xy)methyl)cyclohexane-1-carboxylic acid, 3-(2-(4-(2-amino-8-((4-(trifluoromethoxy)benzyl)oxy)-5 ,6-dihydrobenzo[h]quinazolin-4-yl)piperazin-1-yl)ethox y)cyclobutane-1-carboxylic acid, 4-((2-(4-(2-amino-8-((4-chlorobenzyl)amino)-5,6-dihydr obenzo[h]quinazolin-4-yl)piperazin-1-yl)ethoxy)methyl) cyclohexane-1-carboxylic acid, 4-((2-(4-(2-amino-8-(benzylamino)-5,6-dihydrobenzo[h]q uinazolin-4-yl)piperazin-1-yl)ethoxy)methyl)cyclohexan e-1-carboxylic acid, 4-((2-(4-(2-amino-8-((4-(trifluoromethoxy)benzyl)amino )-5,6-dihydrobenzo[h]quinazolin-4-yl)piperazin-1-yl)et hoxy)methyl)cyclohexane-1-carboxylic acid, 4-((2-(4-(2-amino-8-((4-cyanobenzyl)oxy)-5,6-dihydrobe nzo[h]quinazolin-4-yl)piperazin-1-yl)ethoxy)methyl)cyc lohexane-1-carboxylic acid, 4-((2-(4-(2-amino-8-((4-chlorobenzyl)(methyl)amino)-5, 6-dihydrobenzo[h]quinazolin-4-yl)piperazin-1-yl)ethoxy )methyl)cyclohexane-1-carboxylic acid, 3-(2-(4-(2-amino-8-((2-fluoro-4-(trifluoromethoxy)benz yl)oxy)-5,6-dihydrobenzo[h]quinazolin-4-yl)piperazin-1 -yl)ethoxy)cyclobutane-1-carboxylic acid, 3-(2-(4-(2-amino-8-((3-fluoro-4-(trifluoromethoxy)benz yl)oxy)-5,6-dihydrobenzo[h]quinazolin-4-yl)piperazin-1 -yl)ethoxy)cyclobutane-1-carboxylic acid, 3-(2-(4-(2-amino-8-((2-methoxy-4-(trifluoromethoxy)ben zyl)oxy)-5,6-dihydrobenzo[h]quinazolin-4-yl)piperazin-1-yl)ethoxy)cyclobutane-1-carboxylic acid, 3-(2-(4-(2-amino-8-((2-methyl-4-(trifluoromethoxy)benz yl)oxy)-5,6-dihydrobenzo[h]quinazolin-4-yl)piperazin-1 -yl)ethoxy)cyclobutane-1-carboxylic acid, 3-(2-(4-(2-amino-8-(1-(4-(trifluoromethoxy)phenyl)etho xy)-5,6-dihydrobenzo[h]quinazolin-4-yl)piperazin-1-yl) ethoxy)cyclobutane-1-carboxylic acid, 3-(2-(4-(2-amino-8-((4-cyclopropylbenzyl)oxy)-5,6-dihy drobenzo[h]quinazolin-4-yl)piperazin-1-yl)ethoxy)cyclo butane-1-carboxylic acid, 3-(2-(4-(2-amino-8-((4-(trifluoromethyl)cyclohexyl)met hoxy)-5,6-dihydrobenzo[h]quinazolin-4-yl)piperazin-1-y l)ethoxy)cyclobutane-1-carboxylic acid, 3-(2-(4-(2-amino-8-((4-((trifluoromethyl)thio)benzyl)o xy)-5,6-dihydrobenzo[h]quinazolin-4-yl)piperazin-1-yl) ethoxy)cyclobutane-1-carboxylic acid, 3-(2-(4-(2-amino-8-((4-(difluoromethoxy)benzyl)oxy)-5, 6-dihydrobenzo[h]quinazolin-4-yl)piperazin-1-yl)ethoxy )cyclobutane-1-carboxylic acid, 3-(2-(4-(2-amino-8-((4-((trifluoromethyl)sulfonyl)benz yl)oxy)-5,6-dihydrobenzo[h]quinazolin-4-yl)piperazin-1 -yl)ethoxy)cyclobutane-1-carboxylic acid, N-(2-amino-4-(4-methylpiperazin-1-yl)-5,6-dihydrobenzo [h]quinazolin-8-yl)-4,4-difluoropiperidine-1-sulfonami de, 4-(2-(4-(2-amino-8-((4-chlorobenzyl)oxy)-5,6-dihydrobe nzo[h]quinazolin-4-yl)piperazin-1-yl)ethoxy)cyclohexan e-1-carboxylic acid,
ethyl 3-(2-(4-(2-amino-8-((4-chlorobenzyl)oxy)-5,6-dihydrobe nzo[h]quinazolin-4-yl)piperazin-1-yl)ethoxy)-1-methylc yclobutane-1-carboxylate, 3-(2-(4-(2-amino-8-((4-chlorobenzyl)oxy)-5,6-dihydrobe nzo[h]quinazolin-4-yl)piperazin-1-yl)ethoxy)-1-methylc yclobutane-1-carboxylic acid,
ethyl 4-((2-(4-(2-amino-8-((4-chlorobenzyl)oxy)-5,6-dihydrob enzo[h]quinazolin-4-yl)piperazin-1-yl)ethoxy)methyl)-1 -cyanocyclohexane-1-carboxylate, 4-((2-(4-(2-amino-8-((4-chlorobenzyl)oxy)-5,6-dihydrob enzo[h]quinazolin-4-yl)piperazin-1-yl)ethoxy)methyl)-1 -cyanocyclohexane-1-carboxylic acid,
ethyl 4-(2-(4-(2-amino-8-((4-chlorobenzyl)oxy)-5,6-dihydrobe nzo[h]quinazolin-4-yl)piperazin-1-yl)ethoxy)-1-methylc yclohexane-1-carboxylate, 4-(2-(4-(2-amino-8-((4-chlorobenzyl)oxy)-5,6-dihydrobe nzo[h]quinazolin-4-yl)piperazin-1-yl)ethoxy)-1-methylc yclohexane-1-carboxylic acid, 4-((2-(4-(2-amino-8-((4-chlorobenzyl)oxy)-5,6-dihydrob enzo[h]quinazolin-4-yl)piperazin-1-yl)ethoxy)methyl)-1 -methylcyclohexane-1-carboxylic acid,
ethyl 4-((2-(4-(2-amino-8-((4-chlorobenzyl)oxy)-5,6-dihydrob enzo[h]quinazolin-4-yl)piperazin-1-yl)ethoxy)methyl)-4 -hydroxycyclohexane-1-carboxylate, 4-((2-(4-(2-amino-8-((4-chlorobenzyl)oxy)-5,6-dihydrob enzo[h]quinazolin-4-yl)piperazin-1-yl)ethoxy)methyl)-4 -hydroxycyclohexane-1-carboxylic acid, 4-(2-(4-(2-amino-8-((4-chlorobenzyl)oxy)-5,6-dihydrobe nzo[h]quinazolin-4-yl)piperazin-1-yl)ethoxy)-4-(hydrox ymethyl)cyclohexane-1-carboxylic acid, 4-(2-(4-(2-amino-8-((4-chlorobenzyl)oxy)-5,6-dihydrobe nzo[h]quinazolin-4-yl)piperazin-1-yl)ethoxy)-1-methylc yclohexane-1-carboxamide, 3-((2-(4-(2-amino-8-((4-chlorobenzyl)oxy)-5,6-dihydrob enzo[h]quinazolin-4-yl)piperazin-1-yl)ethoxy)methyl)cy clobutane-1-carboxylic acid, 3-(2-(4-(2-amino-8-((4-(trifluoromethoxy)benzyl)oxy)-5 ,6-dihydrobenzo[h]quinazolin-4-yl)piperazin-1-yl)ethox y)-1-methylcyclobutane-1-carboxylic acid, 3-(2-(4-(2-amino-8-((4-(trifluoromethoxy)benzyl)oxy)-5 ,6-dihydrobenzo[h]quinazolin-4-yl)piperazin-1-yl)ethox y)cyclobutane-1-carboxamide, 4-((2-(4-(2-amino-8-((4-(trifluoromethoxy)benzyl)oxy)-5,6-dihydrobenzo[h]quinazolin-4-yl)piperazin-1-yl)etho xy)methyl)-1-cyanocyclohexane-1-carboxylic acid, 4-((2-(4-(2-amino-8-((4-(trifluoromethoxy)benzyl)oxy)-5,6-dihydrobenzo[h]quinazolin-4-yl)piperazin-1-yl)etho xy)methyl)-1-cyanocyclohexane-1-carboxamide, 3-(2-(4-(2-amino-8-((4-chlorobenzyl)oxy)-5,6-dihydrobe nzo[h]quinazolin-4-yl)piperazin-1-yl)ethoxy)isoxazole-5-carboxylic acid,
methyl 3-((2-(4-(2-amino-8-((4-chlorobenzyl)oxy)-5,6-dihydrob enzo[h]quinazolin-4-yl)piperazin-1-yl)ethoxy)methyl)cy clopentane-1-carboxylate, 3-((2-(4-(2-amino-8-((4-chlorobenzyl)oxy)-5,6-dihydrob enzo[h]quinazolin-4-yl)piperazin-1-yl)ethoxy)methyl)cy clopentane-1-carboxylic acid, 2-(2-(4-(2-amino-8-((4-chlorobenzyl)oxy)-5,6-dihydrobe nzo[h]quinazolin-4-yl)piperazin-1-yl)ethoxy)cyclopropa ne-1-carboxylic acid, 3-(2-(4-(2-amino-8-((4-chlorobenzyl)oxy)-5,6-dihydrobe nzo[h]quinazolin-4-yl)piperazin-1-yl)ethoxy)cyclopenta ne-1-carboxylic acid,
methyl 3-((2-(4-(2-amino-8-((4-(trifluoromethoxy)benzyl)oxy)-5,6-dihydrobenzo[h]quinazolin-4-yl)piperazin-1-yl)etho xy)methyl)cyclopentane-1-carboxylate, 3-((2-(4-(2-amino-8-((4-(trifluoromethoxy)benzyl)oxy)-5,6-dihydrobenzo[h]quinazolin-4-yl)piperazin-1-yl)etho xy)methyl)cyclopentane-1-carboxylic acid,
ethyl 2-((2-(4-(2-amino-8-((4-chlorobenzyl)oxy)-5,6-dihydrob enzo[h]quinazolin-4-yl)piperazin-1-yl)ethoxy)methyl)cy clopropane-1-carboxylate,
ethyl 3-(2-(4-(2-amino-8-((4-chlorobenzyl)oxy)-5,6-dihydrobe nzo[h]quinazolin-4-yl)piperazin-1-yl)ethoxy)cyclopenta ne-1-carboxylate, 2-((2-(4-(2-amino-8-((4-chlorobenzyl)oxy)-5,6-dihydrob enzo[h]quinazolin-4-yl)piperazin-1-yl)ethoxy)methyl)cy clopropane-1-carboxylic acid,
ethyl 2-((2-(4-(2-amino-8-((4-(trifluoromethoxy)benzyl)oxy)-5,6-dihydrobenzo[h]quinazolin-4-yl)piperazin-1-yl)etho xy)methyl)cyclopropane-1-carboxylate, 2-((2-(4-(2-amino-8-((4-(trifluoromethoxy)benzyl)oxy)-5,6-dihydrobenzo[h]quinazolin-4-yl)piperazin-1-yl)etho xy)methyl)cyclopropane-1-carboxylic acid, 4-((2-(4-(2-amino-8-((4-chlorobenzyl)oxy)-5,6-dihydrob enzo[h]quinazolin-4-yl)piperazin-1-yl)ethoxy)methyl)bi cyclo[2.2.2]octane-1-carboxylic acid, 4-(4-(2-((3-(benzyloxy)isoxazol-5-yl)methoxy)ethyl)pip erazin-1-yl)-8-((4-chlorobenzyl)oxy)-5,6-dihydrobenzo[ h]quinazolin-2-amine, 3-(2-(4-(2-amino-8-((4-(trifluoromethoxy)benzyl)oxy)-5 ,6-dihydrobenzo[h]quinazolin-4-yl)piperazin-1-yl)ethox y)-N-hydroxycyclobutane-1-carboxamide, 6-(benzyloxy)-4-(3-(methylamino)pyrrolidin-1-yl)-5H-in deno[1,2-d]pyrimidin-2-amine, 7-(benzyloxy)-4-(4-methylpiperazin-1-yl)-5H-indeno[1,2 -d]pyrimidin-2-amine, 7-(benzyloxy)-4-(3-(methylamino)pyrrolidin-1-yl)-5H-in deno[1,2-d]pyrimidin-2-amine, 8-(benzyloxy)-4-(4-methylpiperazin-1-yl)-5H-indeno[1,2 -d]pyrimidin-2-amine, 8-(benzyloxy)-4-(3-(methylamino)pyrrolidin-1-yl)-5H-in deno[1,2-d]pyrimidin-2-amine, 8-(benzyloxy)-4-(4-methylpiperazin-1-yl)-5,6-dihydrobe nzo[h]quinazolin-2-amine, 8-(benzyloxy)-4-(3-(methylamino)pyrrolidin-1-yl)-5,6-d ihydrobenzo[h]quinazolin-2-amine, 9-(benzyloxy)-4-(4-methylpiperazin-1-yl)-5,6-dihydrobe nzo[h]quinazolin-2-amine, 9-(benzyloxy)-4-(3-(methylamino)pyrrolidin-1-yl)-5,6-d ihydrobenzo[h]quinazolin-2-amine, 10-(benzyloxy)-4-(4-methylpiperazin-1-yl)-6,7-dihydro-5H-benzo[6,7]cyclohepta[1,2-d]pyrimidin-2-amine, 10-(benzyloxy)-4-(3-(methylamino)pyrrolidin-1-yl)-6,7-dihydro-5H-benzo[6,7]cyclohepta[1,2-d]pyrimidin-2-amin e, 10-(benzyloxy)-4-(4-methylpiperazin-1-yl)-5,6-dihydrob enzo[2,3]oxepino[4,5-d]pyrimidin-2-amine, 10-(benzyloxy)-4-(3-(methylamino)pyrrolidin-1-yl)-5,6-dihydrobenzo[2,3]oxepino[4,5-d]pyrimidin-2-amine, 10-(benzyloxy)-4-(piperazin-1-yl)-5,6-dihydrobenzo[2,3 ]oxepino[4,5-d]pyrimidin-2-amine, 9-(benzyloxy)-4-(4-methylpiperazin-1-yl)-5,6-dihydrobe nzo[2,3]oxepino[4,5-d]pyrimidin-2-amine, 9-(benzyloxy)-4-(3-(methylamino)pyrrolidin-1-yl)-5,6-d ihydrobenzo[2,3]oxepino[4,5-d]pyrimidin-2-amine, 9-(benzyloxy)-4-(piperazin-1-yl)-5,6-dihydrobenzo[2,3] oxepino[4,5-d]pyrimidin-2-amine, 4-(4-methylpiperazin-1-yl)-8-((4-(trifluoromethoxy)ben zyl)oxy)-5,6-dihydrobenzo[h]quinazolin-2-amine, 2-(2-(4-(2-amino-8-((4-chlorobenzyl)oxy)-5,6-dihydrobe nzo[h]quinazolin-4-yl)piperazin-1-yl)ethoxy)ethan-1-ol , 2-(2-(2-(4-(2-amino-8-((4-(trifluoromethoxy)benzyl)oxy )-5,6-dihydrobenzo[h]quinazolin-4-yl)piperazin-1-yl)et hoxy)ethoxy)acetic acid, 2-((2-(2-(4-(2-amino-8-((4-(trifluoromethoxy)benzyl)ox y)-5,6-dihydrobenzo[h]quinazolin-4-yl)piperazin-1-yl)e thoxy)ethyl)thio)acetic acid, 5-(2-(4-(2-amino-8-((4-(trifluoromethoxy)benzyl)oxy)-5 ,6-dihydrobenzo[h]quinazolin-4-yl)piperazin-1-yl)ethox y)tetrahydro-2H-pyran-2-carboxylic acid, 3-(2-(4-(2-amino-8-((4-(trifluoromethoxy)benzyl)oxy)-5 ,6-dihydrobenzo[h]quinazolin-4-yl)piperazin-1-yl)ethox y)-6-fluorobicyclo[3.1.0]hexane-6-carboxylic acid, 2-(2-(4-(2-amino-8-((4-(trifluoromethoxy)benzyl)oxy)-5 ,6-dihydrobenzo[h]quinazolin-4-yl)piperazin-1-yl)ethox y)bicyclo[3.1.0]hexane-6-carboxylic acid, 5-((2-(4-(2-amino-8-((4-(trifluoromethoxy)benzyl)oxy)-5,6-dihydrobenzo[h]quinazolin-4-yl)piperazin-1-yl)etho xy)methyl)thiophene-2-carboxylic acid, or 4-(2-(4-(2-amino-8-((4-(trifluoromethoxy)benzyl)oxy)-5 ,6-dihydrobenzo[h]quinazolin-4-yl)piperazin-1-yl)ethox y)thiophene-2-carboxylic acid.

8. A pharmaceutical composition comprising at least one selected from the group consisting of the compound and a pharmacologically acceptable salt thereof according to any one of claims 1 to 7.

9. The pharmaceutical composition according to claim 8, for dually modulating a histamine H1 receptor and a histamine H4 receptor.

10. The pharmaceutical composition according to claim 8, for treating a disease attributable to a histamine H1 receptor and/or a histamine H4 receptor.

11. A method for dually modulating a histamine H1 receptor and a histamine H4 receptor by using at least one selected from the group consisting of the compound and a pharmacologically acceptable salt thereof according to any one of claims 1 to 7.

12. A method for treating a disease attributable to a histamine H1 receptor and/or a histamine H4 receptor, the method comprising:
administering at least one selected from the group consisting of the compound and a pharmacologically acceptable salt thereof according to any one of claims 1 to 7 and the pharmaceutical composition according to any one of claims 8 to 10 to a patient.

13. A use of the compound or a pharmacologically acceptable salt thereof according to any one of claims 1 to 7, for dually modulating a histamine H1 receptor and a histamine H4 receptor.

14. A use of the compound or a pharmacologically acceptable salt thereof according to any one of claims 1 to 7, for treating a disease attributable to a histamine H1 receptor and/or a histamine H4 receptor.

15. A use of the compound or a pharmacologically acceptable salt thereof according to any one of claims 1 to 7, for producing a dual modulator for a histamine H1 receptor and a histamine H4 receptor.

16. A use of the compound or a pharmacologically acceptable salt thereof according to any one of claims 1 to 7, for producing a therapeutic agent for a disease attributable to a histamine H1 receptor and/or a histamine H4 receptor.
